# EUROPEAN PATENT APPLICATION

(11) **EP 4 488 378 A1**
(43) Date of publication of application: **08.01.2025**
(21) Application number: 23763495.1
(22) Date of filing: 01.03.2023
(51) Int. Cl.: C12P 21/08, C07K 1/18, C07K 16/28, C12N 9/24

(54) **METHOD FOR PRODUCING FC-CONTAINING MOLECULE**

(30) Priority: 02.03.2022 JP 2022032199
(71) Applicant: Daiichi Sankyo Company, Limited, Tokyo 103-8426 (JP)
(72) Inventor: MUTO, Hiroshi, Tokyo 103-8426 (JP); IWAMOTO, Mitsuhiro, Tokyo 103-8426 (JP); ARAKAWA, Shiori, Tokyo 103-8426 (JP); SAKAI, Yuta, Tokyo 103-8426 (JP); NARA, Kaori, Tokyo 103-8426 (JP); TANAKA, Yoshifumi, Tokyo 103-8426 (JP); NAKATANI, Yuki, Tokyo 103-8426 (JP)
(74) Representative: Marks & Clerk LLP
(86) International application number: PCT/JP2023/007583
(87) International publication number: WO 2023/167238

(57) **Abstract**

[Problem] To establish a method for producing an Fc-containing molecule having a homogeneous glycan structure with high purity with the smallest possible amount of a glycan donor molecule used, the method allowing free selection of an enzyme to be used from a wide range of options regardless of residual hydrolysis activity or the identity of the enzyme, and the like. [Solution] A novel method for producing an Fc-containing molecule having a desired N297-linked glycan, characterized by combining Steps 1 and 2 that are described in detail in the present specification, and the like are provided.

## Description

### Technical Field

The present invention relates to a method for producing an Fc-containing molecule having an N297-linked glycan including a glycan derived from a glycan donor molecule, a novel glycan donor molecule suitably used in the method, a method for isolating an Fc-containing molecule having an N297-linked glycan, and the like.

### Background Art

An antibody is a glycoprotein molecule having an N-linked glycan linked to the side chain of Asn at position 297 (N297-linked glycan) located in the Fc region of a heavy chain molecule thereof. An antibody is an important molecule in basic research and the medical field, and research and development, particularly of an antibody drug, have been actively pursued, and various influences of a glycan have been clarified (Non Patent Literature 1). A medical antibody mainly used at present is an IgG class molecule, and such an antibody is generally produced by using cultured animal cells such as CHO cells and NS0 cells. The N297-linked glycan of an antibody produced in these animal cells is a biantennary complex type glycan, and is heterogeneous at core fucose, a terminal sialyl group and galactosyl group, and a bisecting GlcNAc (Non Patent Literature 2). It has been revealed that the N297-linked glycan of an antibody greatly affects effector activities including ADCC activity (Antibody-Dependent Cell-Mediated Cytotoxicity) and CDC activity (Complement-Dependent Cytotoxicity) (Non Patent Literature 3, Non Patent Literature 4), and the possibility of also affecting the blood half-life has been pointed out (Non Patent Literature 5). In addition, it has been revealed that an antibody in which a non-reducing end of the N297-linked glycan is 2,6-sialylated is a major active component of IVIG (intravenous immunoglobulin) (Non Patent Literature 6). Furthermore, in recent years, an antibody-drug conjugate and a peptide-Fc conjugate using a chemically-modified glycan as a linker have been developed (Patent Literatures 1 to 4, Non Patent Literatures 7 to 9).

An enzymatic transglycosylation reaction is known as a method for achieving a homogenous glycan added to an antibody for medical use or a glycoprotein molecule including an antibody Fc region (Non Patent Literatures 10 to 12). In the transglycosylation reaction, a method for transferring a glycan having an oxazoline at a reducing end to a GlcNAc (N-acetylglucosamine) acceptor by using a single ENGase (Patent Literatures 5 and 6, Non Patent Literatures 10 and 11), and a one-pot method involving directly transfering a glycan to a GlcNAc acceptor by using two ENGases (Non Patent Literature 12, Patent Literature 1) are known.

Until now, a transglycosylation reaction using an oxazoline form (a glycan donor molecule having an activated reducing end) according to the former has been widely used. However, it is known that when an oxazoline form, which is unstable in an aqueous solution, is used as a glycan donor molecule, the instability thereof causes glycation at antibody Lys, which is a non-enzyme-mediated chemical reaction, to proceed (Non Patent Literatures 7 and 9). As one method for solving this problem, the one-pot method, which is a method using a glycan donor molecule having an unactivated reducing end, has been proposed. However, in order to obtain an Fc-containing molecule having a homogeneous glycan structure with high purity, a large amount of a glycan donor molecule used, 100 equivalents to 300 equivalents, is required (Patent Literature 1, Non Patent Literature 12). In addition, as the ENGases used in this one-pot method, a combination of two enzymes, an Endo-S mutant enzyme and an Endo-M mutant enzyme or an Endo-CC mutant, is known, and it has also been confirmed that when the amount of a glycan donor molecule used is reduced, the transglycosylation rate decreases.

When an Fc-containing molecule having a homogenous glycan structure is used for a medical use, it is desirable to reduce the amount of a glycan donor molecule, which is an expensive raw material, used, in order to suppress the production cost thereof. In order to improve the efficiency of the transglycosylation reaction and obtain an Fc-containing molecule having a homogenous glycan structure with high purity with a smaller amount of a glycan donor molecule used in a method for achieving a homogenous glycan of an Fc-containing molecule by using an endo-β-N-acetylglucosaminidase, the approach of searching for a transglycosylation enzyme having suppressed hydrolysis activity is possible. On the other hand, in the one-pot method, when a mutant enzyme having suppressed hydrolysis activity has already been selected, it is very difficult to reduce the amount of a glycan donor molecule used through the use of the same approach. In addition, endo-β-N-acetylglucosaminidases have different substrate specificities depending on the biological species from which these are derived, and need to be used properly according to the purpose, and thus in the case of this approach, the combinations of enzymes that can be used are limited depending on the degree of suppression of hydrolysis activity. Because of this, desired is a method for obtaining an Fc-containing molecule having a homogeneous glycan structure with high purity with a smaller amount of a glycan donor molecule used while an endo-β-N-acetylglucosaminidase can be freely selected from a wide range of options regardless of residual hydrolysis activity or the identity of the enzyme.

### Citation List

### Patent Literature

Patent Literature 1: International Publication No. WO 2018/003983
Patent Literature 2: International Publication No. WO 2018/090045
Patent Literature 3: International Publication No. WO 2019/065964
Patent Literature 4: International Publication No. WO 2020/050406
Patent Literature 5: International Publication No. WO 2017/124084
Patent Literature 6: International Publication No. WO 2018/039373

### Non Patent Literature

Non Patent Literature 1: Arnold JN, et al., Annu Rev Immunol. 2007, 25, 21-50
Non Patent Literature 2: Jefferis R, Biotechnol Prog. 2005, 21, 11-16
Non Patent Literature 3: Nimmejahn F, et al., Nat Rev Immunol. 2008, 8, 34-47
Non Patent Literature 4: Jefferis R, Nat Rev Drug Discov. 2009, 8, 226-234
Non Patent Literature 5: Bumbaca D, et al., AAPS J. 2012, 14, 554-558
Non Patent Literature 6: Anthony RM, et al., Science. 2008, 320, 373-376
Non Patent Literature 7: Parsons TB, , et al., Angew. Chem. Int. Ed. Engl. 2016, 55, 2361-2367
Non Patent Literature 8: Iwamoto M, et al., Bioconjugate Chem. 2018, 29, 2829-2837
Non Patent Literature 9: Manabe S, et al., Bioconjugate Chem. 2019, 30, 1343-1355
Non Patent Literature 10: Wang LX, Trends Glycosci Glycotechnol. 2011, 23, 33-52
Non Patent Literature 11: Huang W, et al., J Am Chem Soc. 2012, 134, 12308-12318
Non Patent Literature 12: Iwamoto M, et al., PLoS ONE 2018, 13, e0193534

### Summary of Invention

### Technical Problem

Objects of the present invention are, for example, to establish a method for producing an Fc-containing molecule having a homogeneous glycan structure with high purity with the smallest possible amount of a glycan donor molecule used, the method allowing free selection of an enzyme to be used from a wide range of options regardless of residual hydrolysis activity or the enzyme type, to provide a novel glycan donor molecule that can be suitably used in the method, and to establish a method for selectively isolating such an Fc-containing molecule.

### Solution to Problem

The present inventors have carried out intensive studies in order to solve the above problems, and as a result, found that it is possible to set a reaction condition under which an Fc-containing molecule having a moderate or higher transglycosylation rate can be obtained even with a small amount of a glycan donor molecule used, regardless of which endo-β-N-acetylglucosaminidase (in the one-pot method, a combination of two or more endo-β-N-acetyl glucosaminidases) is used in the transglycosylation reaction, and that by contacting a transglycosylation reaction mixture with a cation exchange chromatography medium or a multimode chromatography medium under an acidic condition, an Fc-containing molecule to which a glycan is added, which is a target product, and an unreacted Fc-containing molecule (acceptor molecule) can be isolated, and as a result, an Fc-containing molecule having a homogenous glycan structure with high purity can be collected at a high yield. Taking advantage of these findings, the present inventors have found a production method for obtaining an Fc-containing molecule having a homogeneous glycan structure with high purity with the smallest possible amount of a glycan donor molecule used, and also found that if this production method is used, in a one-pot method, a combination of endo-β-N-acetylglucosaminidases can be selected from a wide range of options, and also found a novel glycan donor molecule suitable for a transglycosylation reaction using a specific combination of enzymes among these, and further found that the isolation step using a cation exchange chromatography medium or a multimode chromatography medium under an acidic condition as described above can be applied not only to a method for producing an Fc-containing molecule, but also simply to the isolation of a desired Fc-containing molecule, leading to the completion of the present invention.

The present invention provides the following inventions.
[1] A method for producing an Fc-containing molecule having an N297-linked glycan comprising a glycan derived from a glycan donor molecule, comprising the following Steps 1 and 2:
   [Step 1] reacting an acceptor molecule, which is an Fc-containing molecule having, as an N297-linked glycan, a core GlcNAc to which fucose is optionally added, with a glycan donor molecule comprising a glycan in the presence of an endo-β-N-acetylglucosaminidase (Enzyme-A), which prefers, as a substrate, an N297-linked glycan of an Fc-containing molecule, so as to obtain a reaction mixture; and
   [Step 2] contacting the reaction mixture with a cation exchange chromatography medium or a multimode chromatography medium under an acidic condition and collecting the Fc-containing molecule having an N297-linked glycan comprising the glycan derived from the glycan donor molecule.
[2] The method according to [1], wherein the glycan donor molecule comprises a GlcNAc having an unactivated reducing end.
[3] The method according to [2], wherein in Step 1, the acceptor molecule is reacted with the glycan donor molecule in the presence of Enzyme-A and an endo-β-N-acetylglucosaminidase (Enzyme-B), which prefers, as a substrate, a glycan of a glycan donor molecule, so as to obtain a reaction mixture.
[4] The method according to [3], wherein the endo-β-N-acetylglucosaminidase (Enzyme-B), which prefers, as a substrate, a glycan of a glycan donor molecule, is an enzyme having a property of acting on the glycan donor molecule in the presence of Enzyme-A to promote a transglycosylation reaction of the glycan derived from the glycan donor molecule to the acceptor molecule by Enzyme-A.
[5] The method according to [3] or [4], wherein Enzyme-B is an enzyme having an activity of transglycosylation from SGP to a GlcNAc derivative.
[6] The method according to any one of [3] to [5], wherein Enzyme-B is a mutant enzyme of Endo-M, Endo-Om, Endo-CC, or Endo-Rp, and the mutant enzyme has lower hydrolysis activity than a corresponding wild type enzyme thereof.
[7] The method according to any one of [3] to [6], wherein Enzyme-B is selected from the group consisting of Endo-Rp N172Q, Endo-Rp N172H, Endo-Rp N172A, Endo-Rp N172C, Endo-Rp N172D, Endo-Rp N172E, Endo-Rp N172G, Endo-Rp N172I, Endo-Rp N172L, Endo-Rp N172M, Endo-Rp N172P, Endo-Rp N172S, Endo-Rp N172T, Endo-Rp N172V, Endo-Rp W278F/S216V, Endo-Rp W278F/N246D, Endo-Rp W278F/D276N, Endo-Rp W278F/A310D, Endo-Rp W278F/N172D/F307Y, Endo-Rp W278F/N172D/F307H, Endo-Rp W278F/N172D/A310D, Endo-Rp W214F/F307Y/L306I, Endo-M N175Q, Endo-M N175Q/Y217F, Endo-CC N180H, and Endo-Om N194Q.
[8] The method according to any one of [3] to [7], wherein Enzyme-A and Enzyme-B are fused.
[9] The method according to any one of [1] to [8], wherein in Step 1, 2 equivalents to 40 equivalents of the glycan donor molecule are used per equivalent of the acceptor molecule.
[10] The method according to any one of [1] to [9], wherein in step 1, a transglycosylation rate exceeds 80%.
[11] The method according to any one of [1] to [10], wherein the glycan included in the glycan donor molecule is an N-linked glycan or an O-linked glycan having an optionally chemically-modified non-reducing end.
[12] The method according to [11], wherein the glycan donor molecule is SGP, AG(9)-P, AG(7)-P, SG-Asn, AG(9)-Asn, AG(7)-Asn, SG-OR, AG(9)-OR, AG(7)-OR, (MSG1)-Asn, (MSG2)-Asn, a mixture of (MSG1)-Asn and (MSG2)-Asn, MSG1-OR, MSG2-OR, or a mixture of MSG1-OR and MSG2-OR, having an optionally chemically-modified non-reducing end, wherein R is any substituent linked to the oxygen atom via at least one carbon atom.
[13] The method according to [12], wherein R represents a C1-C6 alkyl group or an optionally substituted aryl group.
[14] The method according to [13], wherein R is selected from the group consisting of a methyl group, an ethyl group, a n-propyl group, an i-propyl group, a n-butyl group, an i-butyl group, a s-butyl group, a t-butyl, a n-pentyl group, and a n-hexyl group, or is selected from the group consisting of a phenyl group, a benzyl group, an indenyl group, a naphthyl group, a fluorenyl group, an anthranyl group, and a phenanthrenyl group, optionally substituted with a C1-C6 alkoxy group, a C1-C6 alkyl group, or a halogen group.
[15] The method according to any one of [12] to [14], wherein R is selected from the group consisting of PMP, Me, A-Mor, iPr, nPr, PrOMe, and A-PEG-N₃ wherein A represents an acetyl group in a glycolic acid unit, that is, a moiety present between an anomeric hydroxyl group and an amino group in Mor or PEG.
[16] The method according to [11] or [12], wherein the glycan donor molecule is ([N₃-PEG(3)]₂-SG)-P-PEG(3)-N₃, ([N₃-PEG(3)]₂-SG)-Asn-PEG(3)-N₃, ([N₃-PEG(3)]-MSG1)-Asn-PEG(3)-N₃, ([N₃-PEG(3)]-MSG2)-Asn-PEG(3)-N₃, or a mixture of ([N₃-PEG(3)]-MSG1)-Asn-PEG(3)-N₃ and ([N₃-PEG(3)]-MSG2)-Asn-PEG(3)-N₃.
[17] The method according to any one of [12] to [15], wherein the glycan donor molecule is ([N₃-PEG(3)]₂-SG)-OR, ([N₃-PEG(3)]-MSG1)-OR, ([N₃-PEG(3)]-MSG2)-OR, or a mixture of ([N₃-PEG(3)]-MSG1)-OR and ([N₃-PEG(3)]-MSG2)-OR.
[18] The method according to any one of [11] to [17], wherein the glycan donor molecule is a compound represented by a formula selected from the group consisting of the following formulas. 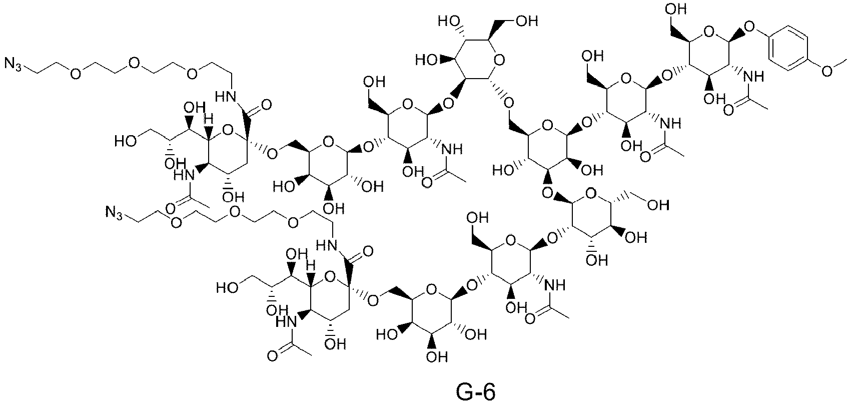 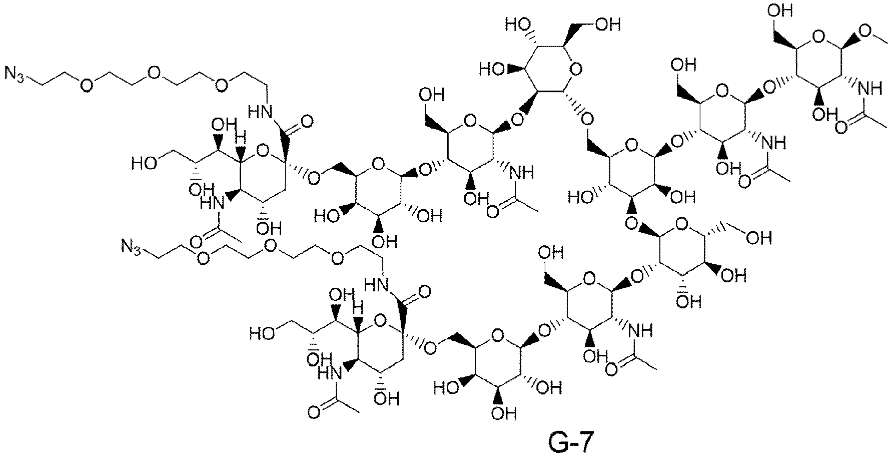 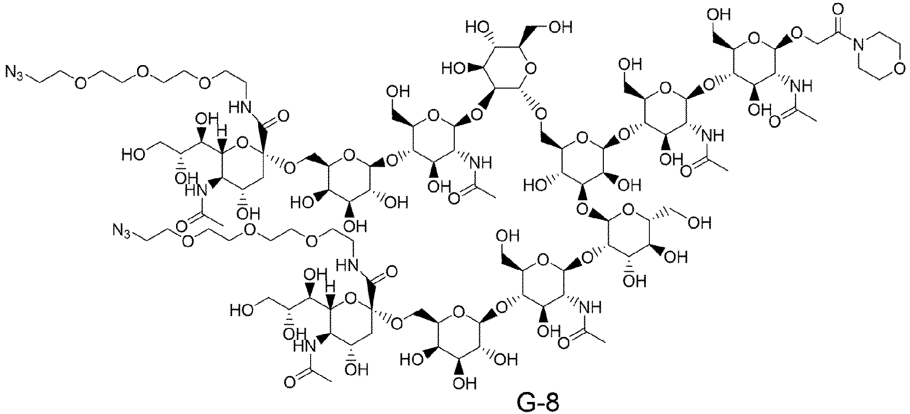 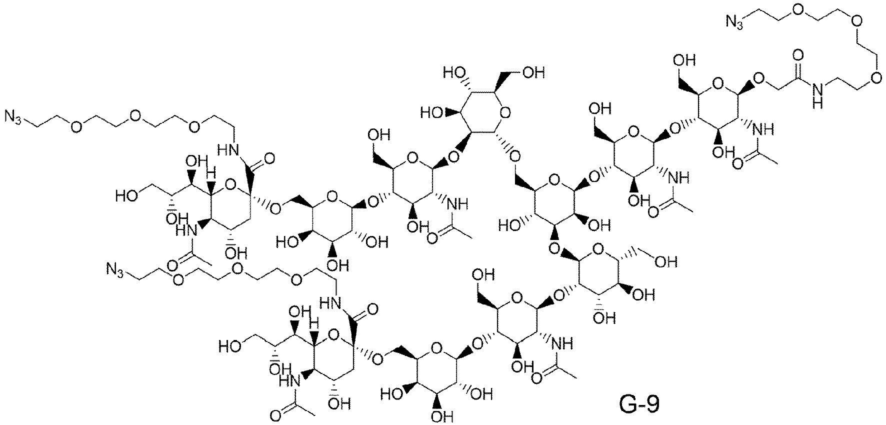 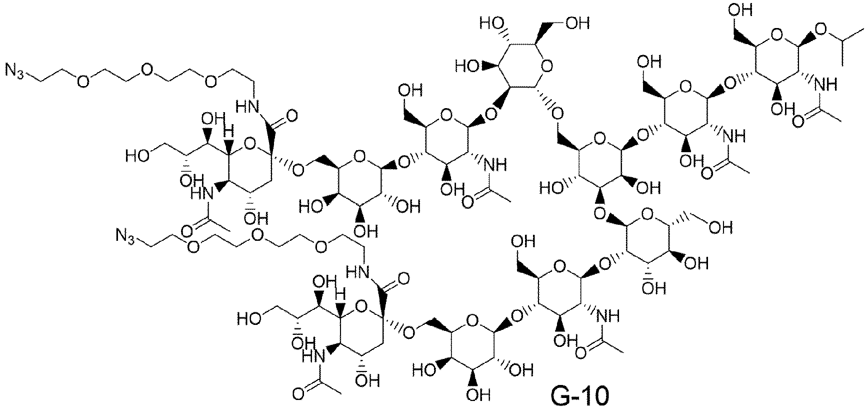 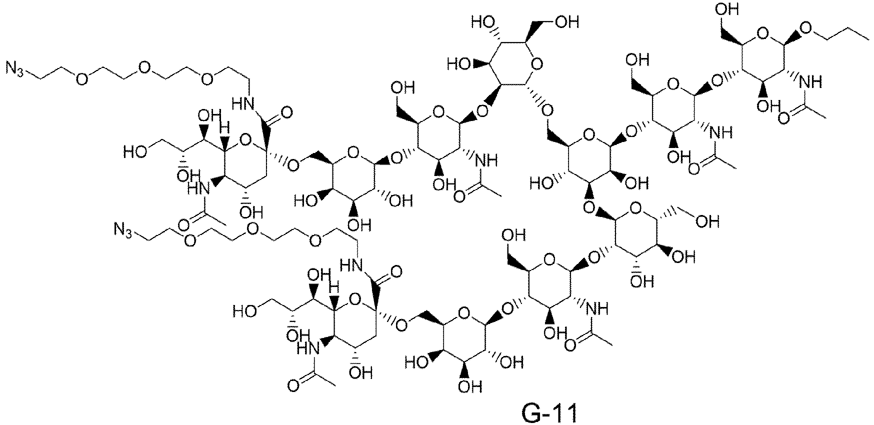 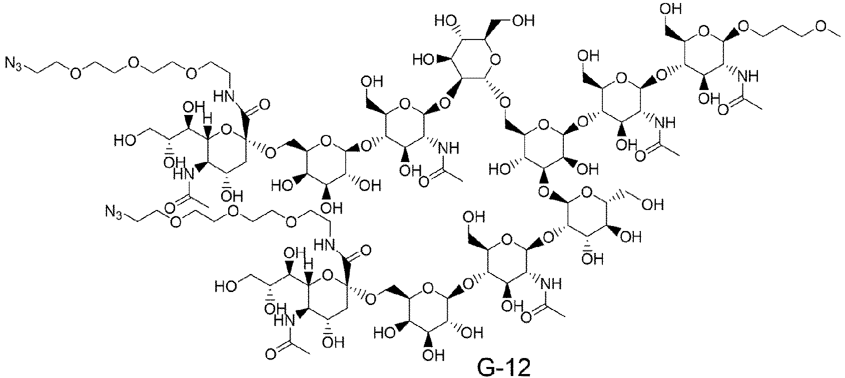 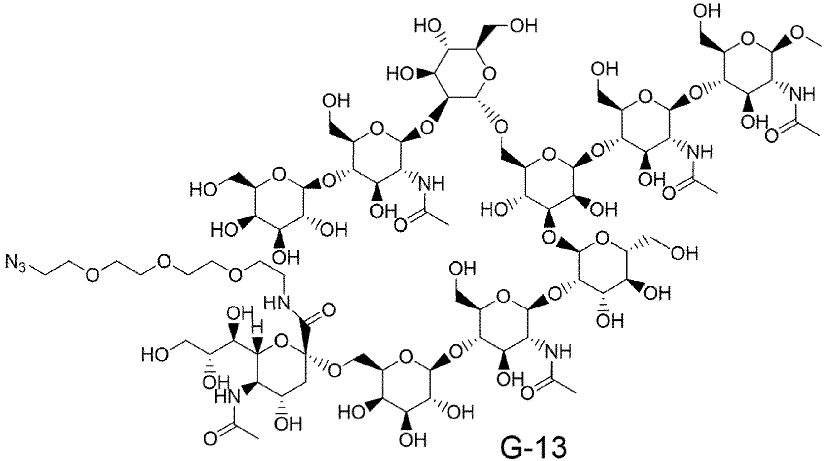 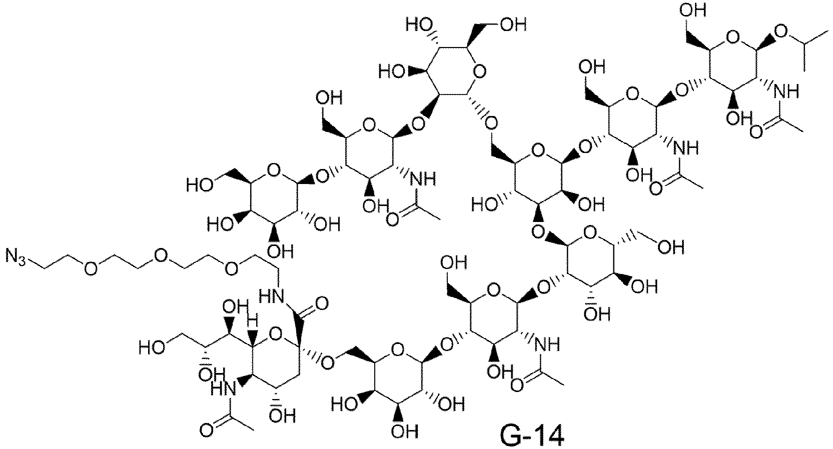 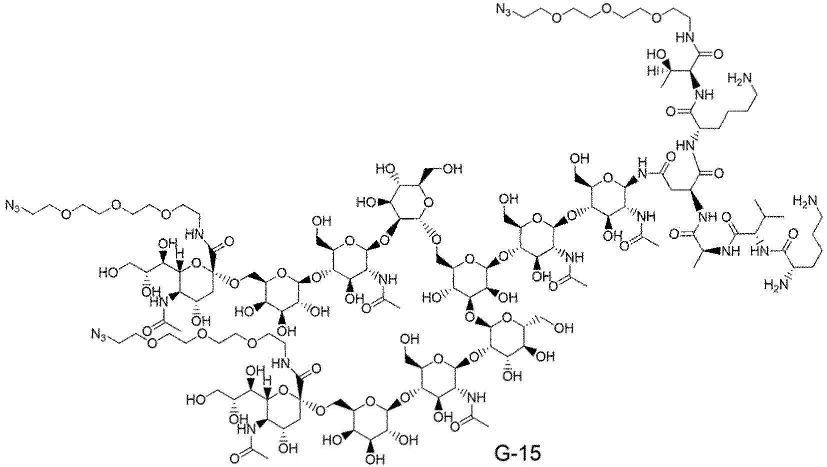 and 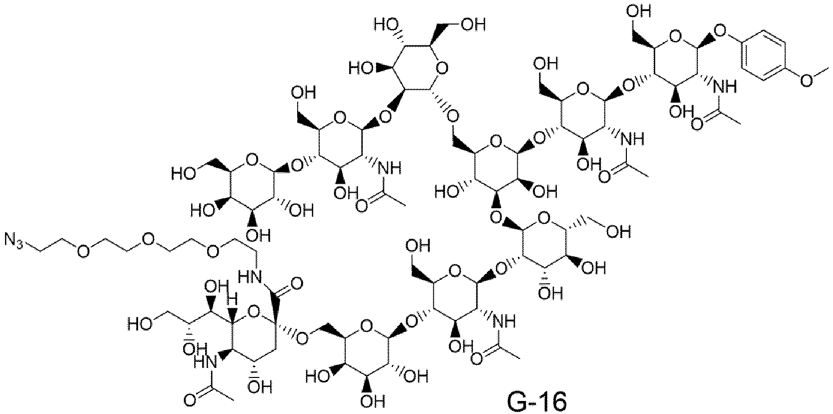
[19] The method according to any one of [1] to [18], wherein the Fc-containing molecule is an immunoglobulin, CLCH, or an Fc-containing fragment.
[20] The method according to any one of [1] to [19], wherein the endo-β-N acetylglucosaminidase (Enzyme-A), which prefers, as a substrate, an N297-linked glycan, is an enzyme that can act on the glycan of the Fc-containing molecule and optionally has residual hydrolysis activity.
[21] The method according to any one of [1] to [20], wherein Enzyme-A is a mutant enzyme of Endo-S, Endo-S2, Endo-Si, Endo-Se, Endo-Sd, or Endo-Sz, and the mutant enzyme has lower hydrolysis activity than a corresponding wild type enzyme thereof.
[22] The method according to [21], wherein Enzyme-A is Endo-S D233Q, Endo-S D233Q/Q303L, Endo-S D233Q/E350A, Endo-S D233Q/E350Q, Endo-S D233Q/E350D, Endo-S D233Q/E350N, Endo-S D233Q/D405A, Endo-Si D241Q, Endo-Si D241Q/Q311L, Endo-Si D241Q/E360Q, Endo-Si D241M, Endo-Si D241M/Q311L, Endo-Si D241M/E360Q, Endo-Si T190Q, Endo-Si T190/D241Q, Endo-Si T190Q/D241M, Endo-S2 D184M, Endo-S2 T138Q, Endo-S2 D184Q/Q250L, Endo-S2 D184M/Q250L, Endo-Se D233M, Endo-Sz D234M, Endo-Sz D234M/Q304L, Endo-Si D241X₁ wherein X₁ represents any amino acid residue other than K, R, and D, Endo-Si T190X₂ wherein X₂ represents an amino acid residue of F, H, K, M, Q, R, W, or Y, Endo-Si Q311X₃ wherein X₃ represents an amino acid residue of F, N, or Y, or Endo-Si E360K.
[23] The method according to any one of [1] to [22], wherein pH in a reaction system in Step 1 is in the range of 6.5 to 8.0.
[24] The method according to any one of [1] to [23], wherein a final acceptor molecule concentration in a reaction system in Step 1 is 20 mg/mL or more and 100 mg/mL or less.
[25] The method according to any one of [1] to [24], wherein the Fc-containing molecule having an N297-linked glycan comprising the glycan derived from the glycan donor molecule is collected in a flow-through fraction from the cation exchange chromatography medium or the multimode chromatography medium.
[26] The method according to any one of [1] to [25], wherein the acidic condition in Step 2 means a pH range of 3.5 to 4.5.
[27] The method according to any one of [1] to [26], wherein the chromatography medium is particulate, membranous, or monolithic.
[28] The method according to any one of [1] to [27], wherein the chromatography medium is a cation exchange chromatography medium.
[29] The method according to any one of [1] to [28], wherein the glycan has a non-reducing end chemically-modified with a substituent having an azido group (N₃-), and the method further comprises reacting a molecule having an alkyne structure with the azido group (N₃-) of the glycan.
[30] The method according to [29], wherein the molecule having an alkyne structure is selected from a chemotherapeutic agent, a molecular target drug, an immunostimulant, a toxin, a photosensitizer, an antimicrobial agent, an antiviral agent, a diagnostic agent, a protein, a peptide, an amino acid, a nucleic acid molecule, a nucleic acid, an antigen, a vitamin, and a hormone.
[31] The method according to [30], wherein the chemotherapeutic agent is selected from camptothecin, pyrrolobenzodiazepine, doxorubicin, auristatin, taxane, and derivatives thereof.
[32] The method according to [30], wherein the immunostimulant is selected from a STING agonist, a TLR agonist, and an A2AR antagonist.
[33] The method according to any one of [29] to [32], wherein the molecule having an alkyne structure is selected from the group consisting of (A) to (E):
   (A) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide,
   (B) N-[4-(11,12-didehydrodibenzo[b,f]azocin-5(6H)-yl)-4-oxobutanoyl]glycylglycyl-L-valyl-N-[4-({[(11'S,11'aS)-11'-hydroxy-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl)phenyl]-L-alaninamide,
   (C) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',11a'-dihydro-1'H-spiro[cyclopropane-1,2'-pyrrolo[2,1-c][1,4]benzodiazepin]-8'-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl }oxy)methyl]phenyl }-L-alaninamide,
   (D) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',10',11',11a'-tetrahydro-1'H-spiro[cyclopropane-1,2'-pyrrolo[2,1-c][1,4]benzodiazepin]-8'-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide, and
   (E) (bis(N,N-diethylethanaminium)N-[4-(11,12-didehydrodibenzo[b,f]azocin-5(6H)-yl)-4-oxobutanoyl]glycylglycyl-L-phenylalanyl-N-[(2-{9-[(5R,7R,8R,12aR,14R,15R,15aR,16R)-15-fluoro-16-hydroxy-2,10-dioxo-2,10-disulfide-14-(6,7,8,9-tetrahydro-2H-2,3,5,6-tetraazabenzo[cd]azulen-2-yl)octahydro-2H,10H,12H-5,8-methano-2λ⁵,10λ⁵ -furo[3,2-l][1,3,6,9,11,2,10]pentaoxadiphosphacyclotetradecyn-7-yl]-6-oxo-6,9-dihydro-1H-purin-1-yl} ethoxy)methyl]glycinamide.
[34] An Fc-containing molecule produced by the method according to any one of [1] to [33].
[35] A method for isolating an Fc-containing molecule having an N297-linked glycan, comprising contacting a solution comprising the Fc-containing molecule and one or more impurities with a cation exchange chromatography medium or a multimode chromatography medium under an acidic condition to selectively trap the impurities in the cation exchange chromatography medium or the multimode chromatography medium, or to selectively trap the Fc-containing molecule in the cation exchange chromatography medium or the multimode chromatography medium.
[36] The method according to [35], wherein the N297-linked glycan comprises a complex type glycan.
[37] The method according to [35] or [36], wherein the N297-linked glycan is not a core GlcNAc to which fucose is optionally added, and the one or more impurities comprise a molecule that is the same as the Fc-containing molecule except for having, as an N297-linked glycan, a core GlcNAc to which fucose is optionally added.
[38] The method according to any one of [35] to [37], wherein the chromatography medium is a cation exchange chromatography medium.
[39] A method for isolating an Fc-containing molecule having an N297-linked glycan comprising a glycan derived from a glycan donor molecule, comprising the following Steps 1 and 2:
   [Step 1] reacting an acceptor molecule, which is an Fc-containing molecule having, as an N297-linked glycan, a core GlcNAc to which fucose is optionally added, with a glycan donor molecule comprising a glycan in the presence of an endo-β-N-acetylglucosaminidase (Enzyme-A), which prefers, as a substrate, an N297-linked glycan of an Fc-containing molecule, so as to obtain a reaction mixture; and
   [Step 2] contacting the reaction mixture with a cation exchange chromatography medium or a multimode chromatography medium under an acidic condition and isolating the Fc-containing molecule having an N297-linked glycan comprising the glycan derived from the glycan donor molecule.
[40] The method according to [39], wherein the chromatography medium is a cation exchange chromatography medium.
[41] The method or the Fc-containing molecule according to any one of [1] to [40], wherein the Fc-containing molecule having an N297-linked glycan is derived from an antibody selected from the group consisting of an anti-HER2 antibody, an anti-HER3 antibody, an anti-DLL3 antibody, an anti-FAP antibody, an anti-CDH11 antibody, an anti-CDH6 antibody, an anti-A33 antibody, an anti-CanAg antibody, an anti-CD19 antibody, an anti-CD20 antibody, an anti-CD22 antibody, an anti-CD30 antibody, an anti-CD33 antibody, an anti-CD56 antibody, an anti-CD70 antibody, an anti-CD98 antibody, an anti-TROP2 antibody, an anti-CEA antibody, an anti-Cripto antibody, an anti-EphA2 antibody, an anti-G250 antibody, an anti-MUC1 antibody, an anti-GPNMB antibody, an anti-Integrin antibody, an anti-PSMA antibody, an anti-Tenascin-C antibody, an anti-SLC44A4 antibody, an anti-Mesothelin antibody, an anti-ENPP3 antibody, an anti-CD47 antibody, an anti-EGFR antibody, an anti-GPR20 antibody, and an anti-DR5 antibody.
[42] A compound represented by a formula selected from the group consisting of the following formulas. 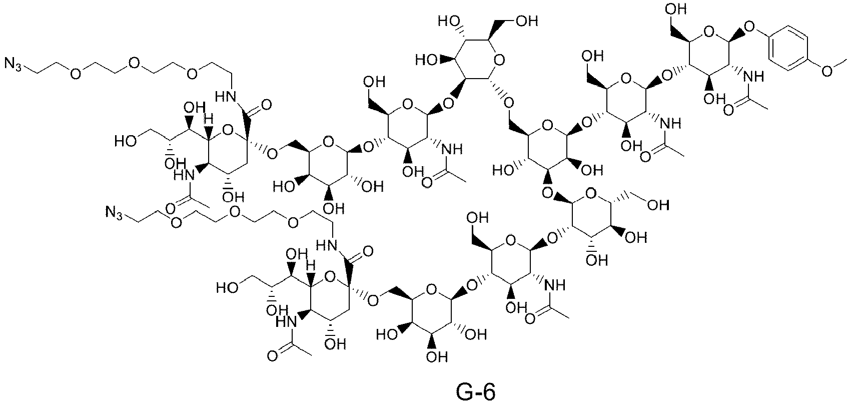 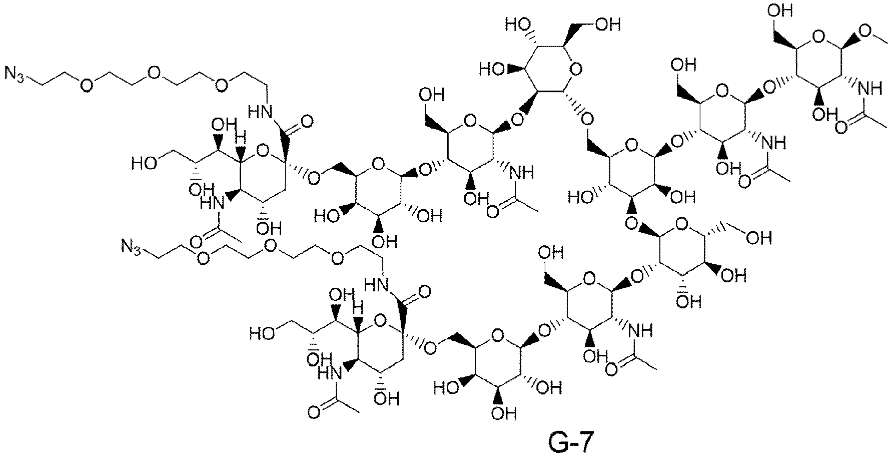 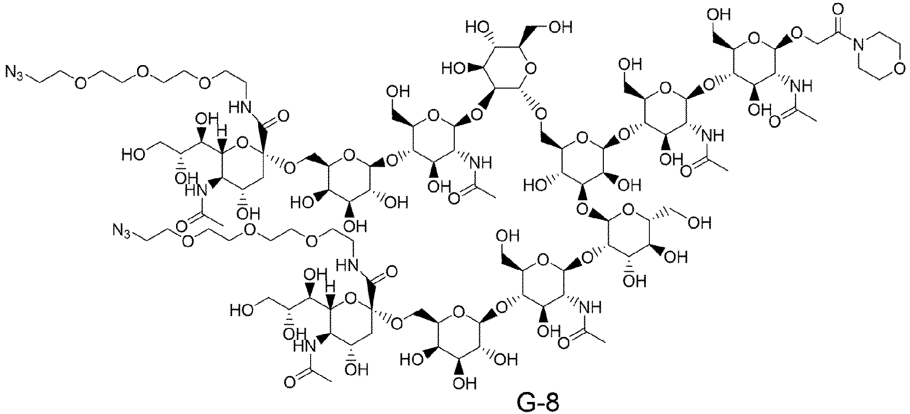 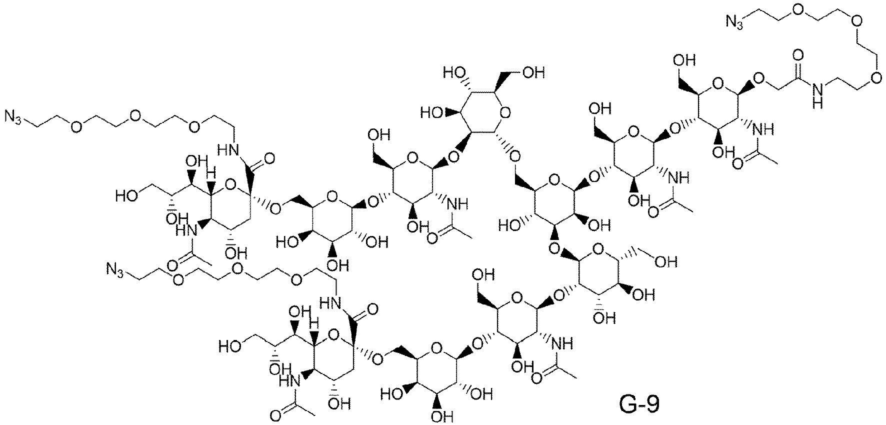 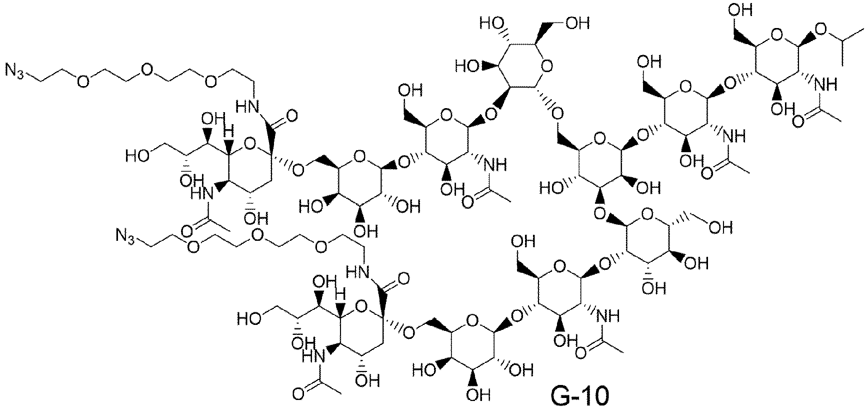 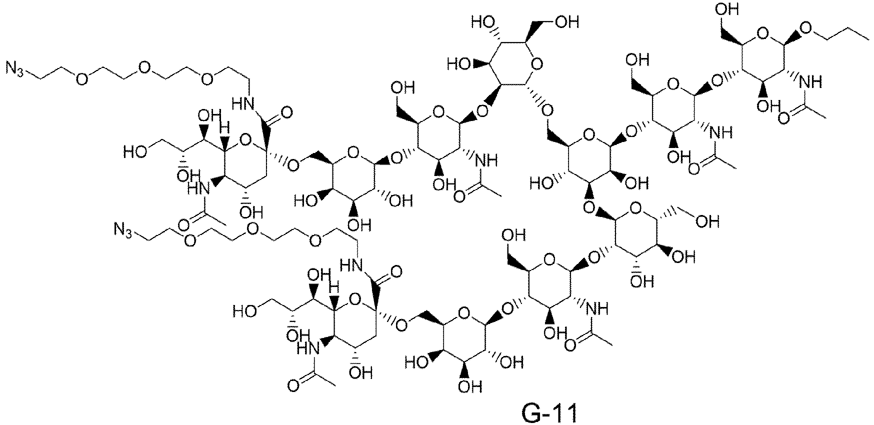 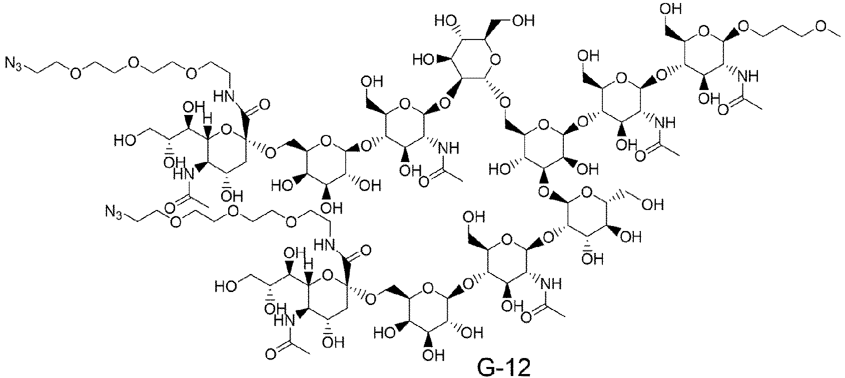 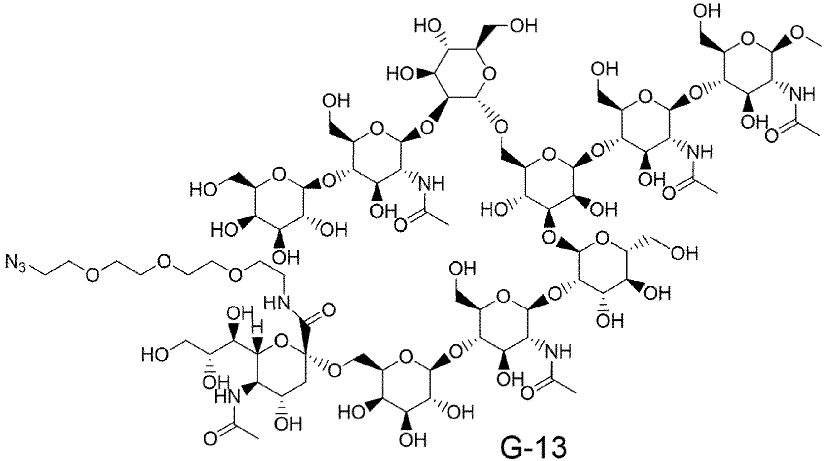 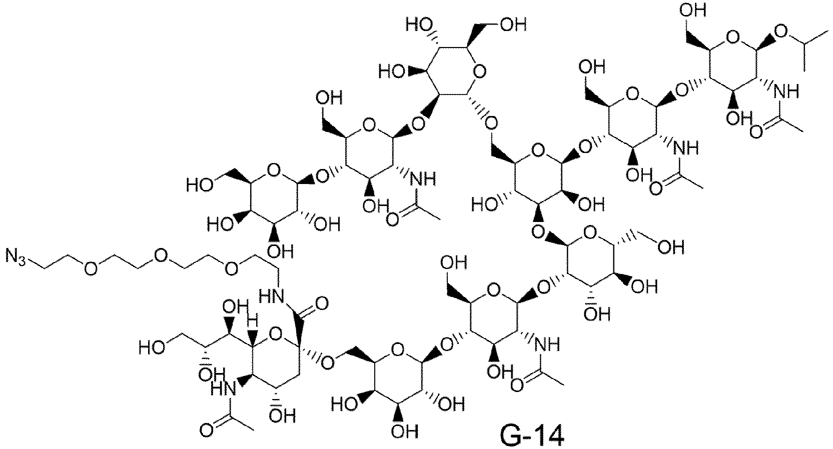 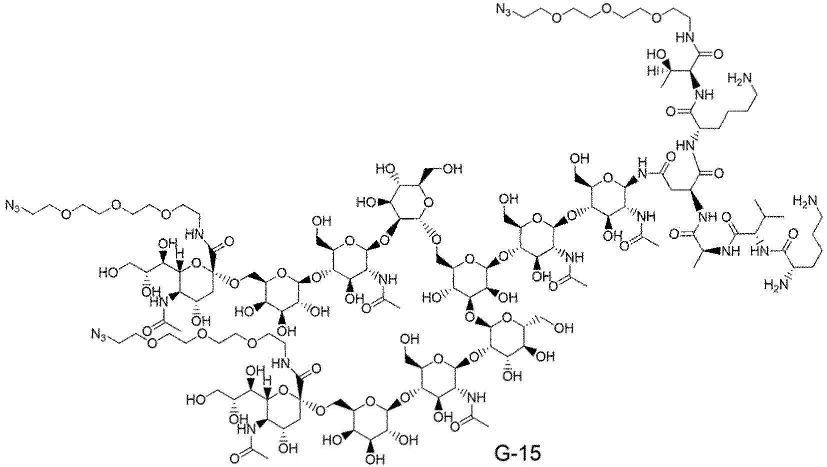 and 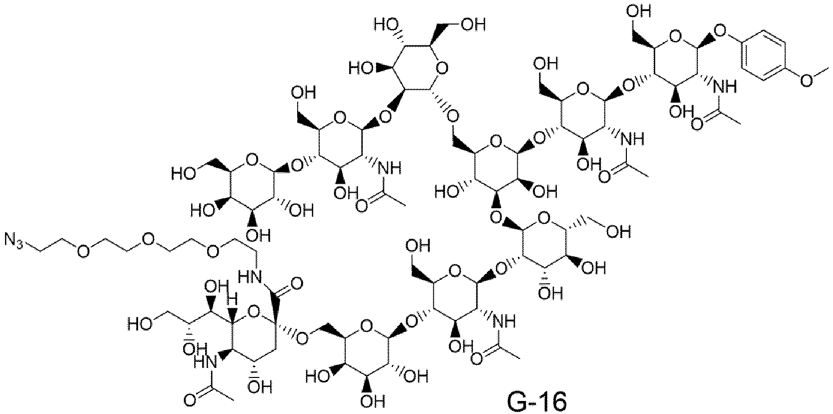
[43] A fusion protein of Enzyme-A and Enzyme-B, wherein
   Enzyme-A is an endo-β-N-acetylglucosaminidase, which prefers, as a substrate, an N297-linked glycan of an Fc-containing molecule, and
   Enzyme-B is an endo-β-N-acetylglucosaminidase, which prefers, as a substrate, a glycan of a glycan-containing molecule having a GlcNAc having an unactivated glycan reducing end, but does not prefer, as a substrate, the N297-linked glycan, or has low reactivity with the N297-linked glycan.

### Advantageous Effects of Invention

In the production method of the present invention, by cation exchange chromatography under an acidic condition, it is possible to separate an Fc-containing molecule to which a glycan is added, which is a target product, and an unreacted Fc-containing molecule (acceptor molecule), and thus it is possible to produce an Fc-containing molecule having a homogeneous glycan structure with high purity with the smallest possible amount of a glycan donor molecule used. In addition, in particular, when the production method of the present invention is applied to the one-pot method, the purity of the desired Fc-containing molecule can be increased in the isolation step, and thus the need to extremely increase the efficiency of the transglycosylation reaction itself is eliminated, and as a result, it is possible to freely select Enzyme-A, which exhibits transglycosylation activity on the N297-linked glycan of the Fc-containing molecule, and Enzyme-B, which exhibits the property of activating the glycan in the glycan donor molecule, from a wide range of options. That is, from the viewpoint of enzyme productivity and physical properties, it is possible to freely choose an enzyme suitable for a mass production process.

In addition, when the production method of the present invention is applied to the one-pot method, it is possible to freely select a glycan donor molecule from an N-linked glycan or an O-linked glycan having a chemically unactivated reducing end. That is, unlike when an oxazoline form having a chemically activated reducing end is chosen as a glycan donor molecule, it is possible to choose a glycan donor molecule having a chemically stable structure that does not spontaneously degrade in an aqueous solution near neutrality and does not directly form a chemical bond with Lys in the Fc-containing molecule.

From the above, it is possible to reduce the amount of a glycan donor molecule required for efficiently obtaining an Fc-containing molecule having a homogeneous glycan structure, leading to reduction in the production cost of a glycan-remodeled Fc-containing molecule.

In addition, one aspect of the present invention provides a novel glycan donor molecule suitable for a transglycosylation reaction in the one-pot method, particularly for a transglycosylation reaction using a combination of specific endo-β-N-acetylglucosaminidases, and furthermore, another aspect of the present invention provides a novel isolation method including isolating a desired Fc-containing molecule from an impurity.

### Brief Description of Drawings

[Figure 1] Figure 1 shows a schematic diagram of a transglycosylation reaction in a one-pot reaction in which an acceptor molecule ((Fucα1,6)GlcNAc-mAb), a glycan donor molecule (X-SG-Z), Enzyme-A, and Enzyme-B are mixed.
[Figure 2] Figure 2 shows a list of structural formulas of representative examples of a glycan donor molecule
[Figure 3] Figure 3 shows a schematic diagram of a transglycosylation reaction when using a glycan donor molecule and any of G-1, G-3, or G-4.
[Figure 4] Figure 4 shows a schematic diagram of a transglycosylation reaction when using a glycan donor molecule and any of G-1 to G-17.
[Figure 5] Figure 5 shows a schematic diagram of a hydrolysis reaction that is a reaction competitive with a one-pot reaction.
[Figure 6] Figure 6 shows a schematic diagram of a hydrolysis activity reaction of mAb2 using Enzyme-A.
[Figure 7] Figure 7 shows the amino acid sequence of wild type Endo-S (SEQ ID NO: 69). An amino acid residue corresponding to a catalytic activity center site of the enzyme is indicated in bold and underlined, and an amino acid residue substituted in the mutant enzymes used in the Examples or an amino acid residue that can be expected to control enzyme activity by adding a mutation is indicated in bold.
[Figure 8] Figure 8 shows the amino acid sequence of wild type Endo-S2 (SEQ ID NO: 70). An amino acid residue corresponding to a catalytic activity center site of the enzyme is indicated in bold and underlined, and an amino acid residue substituted in the mutant enzymes used in the Examples or an amino acid residue that can be expected to control enzyme activity by adding a mutation is indicated in bold.
[Figure 9] Figure 9 shows the amino acid sequence of wild type Endo-Si (SEQ ID NO: 71). An amino acid residue corresponding to a catalytic activity center site of the enzyme is indicated in bold and underlined, and an amino acid residue substituted in the mutant enzymes used in the Examples or an amino acid residue that can be expected to control enzyme activity by adding a mutation is indicated in bold.
[Figure 10] Figure 10 shows the amino acid sequence of wild type Endo-M (SEQ ID NO: 72). An amino acid residue corresponding to a catalytic activity center site of the enzyme is indicated in bold and underlined, and an amino acid residue substituted in the mutant enzymes used in the Examples or an amino acid residue that can be expected to control enzyme activity by adding a mutation is indicated in bold.
[Figure 11] Figure 11 shows the amino acid sequence of wild type Endo-Rp (SEQ ID NO: 73). An amino acid residue corresponding to a catalytic activity center site of the enzyme is indicated in bold and underlined, and an amino acid residue substituted in the mutant enzymes used in the Examples or an amino acid residue that can be expected to control enzyme activity by adding a mutation is indicated in bold.
[Figure 12] Figure 12 shows the amino acid sequence of wild type Endo-CC (SEQ ID NO: 74). An amino acid residue corresponding to a catalytic activity center site of the enzyme is indicated in bold and underlined, and an amino acid residue that can be expected to control enzyme activity by adding a mutation is indicated in bold.
[Figure 13] Figure 13 shows the amino acid sequence of wild type Endo-Om (SEQ ID NO: 75). An amino acid residue corresponding to a catalytic activity center site of the enzyme is indicated in bold and underlined, and an amino acid residue that can be expected to control enzyme activity by adding a mutation is indicated in bold.
[Figure 14] Figure 14 shows the amino acid sequence of the anti-CD70 antibody 1 light chain (SEQ ID NO: 1) and the amino acid sequence of the anti-CD70 antibody 1 heavy chain (SEQ ID NO: 2).
[Figure 15] Figure 15 shows the amino acid sequence of the anti-CD70 antibody 2 light chain (SEQ ID NO: 3) and the amino acid sequence of the anti-CD70 antibody 2 heavy chain (SEQ ID NO: 4).
[Figure 16] Figure 16 shows the amino acid sequence of the anti-TROP2 antibody 1 light chain (SEQ ID NO: 5) and the amino acid sequence of the anti-TROP2 antibody 1 heavy chain (SEQ ID NO: 6).
[Figure 17] Figure 17 shows the amino acid sequence of the anti-TROP2 antibody 2 light chain (SEQ ID NO: 7) and the amino acid sequence of the anti-TROP2 antibody 2 heavy chain (SEQ ID NO: 8).
[Figure 18] Figure 18 shows the amino acid sequence of the anti-EGFR antibody 1 light chain (SEQ ID NO: 9) and the amino acid sequence of the anti-EGFR antibody 1 heavy chain (SEQ ID NO: 10).
[Figure 19] Figure 19 shows the amino acid sequence of the anti-EGFR antibody 2 light chain (SEQ ID NO: 11) and the amino acid sequence of the anti-EGFR antibody 2 heavy chain (SEQ ID NO: 12).
[Figure 20] Figure 20 shows the CDRL1 amino acid sequence of anti-CD70 antibody 1 (SEQ ID NO: 13), the CDRL2 amino acid sequence of anti-CD70 antibody 1 (SEQ ID NO: 14), the CDRL3 amino acid sequence of anti-CD70 antibody 1 (SEQ ID NO: 15), the CDRH1 amino acid sequence of anti-CD70 antibody 1 (SEQ ID NO: 16), the CDRH2 amino acid sequence of anti-CD70 antibody 1 (SEQ ID NO: 17), and the CDRH3 amino acid sequence of anti-CD70 antibody 1 (SEQ ID NO: 18). The CDR sequences were determined according to the Kabat definition.
[Figure 21] Figure 21 shows the CDRL1 amino acid sequence of anti-CD70 antibody 2 (SEQ ID NO: 19), the CDRL2 amino acid sequence of anti-CD70 antibody 2 (SEQ ID NO: 20), the CDRL3 amino acid sequence of anti-CD70 antibody 2 (SEQ ID NO: 21), the CDRH1 amino acid sequence of anti-CD70 antibody 2 (SEQ ID NO: 22), the CDRH2 amino acid sequence of anti-CD70 antibody 2 (SEQ ID NO: 23), and the CDRH3 amino acid sequence of anti-CD70 antibody 2 (SEQ ID NO: 24). The CDR sequences were determined according to the Kabat definition.
[Figure 22] Figure 22 shows the CDRL1 amino acid sequence of anti-TROP2 antibody 1 (SEQ ID NO: 25), the CDRL2 amino acid sequence of anti-TROP2 antibody 1 (SEQ ID NO: 26), the CDRL3 amino acid sequence of anti-TROP2 antibody 1 (SEQ ID NO: 27), the CDRH1 amino acid sequence of anti-TROP2 antibody 1 (SEQ ID NO: 28), the CDRH2 amino acid sequence of anti-TROP2 antibody 1 (SEQ ID NO: 29), and the CDRH3 amino acid sequence of anti-TROP2 antibody 1 (SEQ ID NO: 30). The CDR sequences were determined according to the Kabat definition.
[Figure 23] Figure 23 shows the CDRL1 amino acid sequence of anti-TROP2 antibody 2 (SEQ ID NO: 31), the CDRL2 amino acid sequence of anti-TROP2 antibody 2 (SEQ ID NO: 32), the CDRL3 amino acid sequence of anti-TROP2 antibody 2 (SEQ ID NO: 33), the CDRH1 amino acid sequence of anti-TROP2 antibody 2 (SEQ ID NO: 34), the CDRH2 amino acid sequence of anti-TROP2 antibody 2 (SEQ ID NO: 35), and the CDRH3 amino acid sequence of anti-TROP2 antibody 2 (SEQ ID NO: 36). The CDR sequences were determined according to the Kabat definition.
[Figure 24] Figure 24 shows the CDRL1 amino acid sequence of anti-EGFR antibody 1 (SEQ ID NO: 37), the CDRL2 amino acid sequence of anti-EGFR antibody 1 (SEQ ID NO: 38), the CDRL3 amino acid sequence of anti-EGFR antibody 1 (SEQ ID NO: 39), the CDRH1 amino acid sequence of anti-EGFR antibody 1 (SEQ ID NO: 40), the CDRH2 amino acid sequence of anti-EGFR antibody 1 (SEQ ID NO: 41), and the CDRH3 amino acid sequence of anti-EGFR antibody 1 (SEQ ID NO: 42). The CDR sequences were determined according to the Kabat definition.
[Figure 25] Figure 25 shows the CDRL1 amino acid sequence of anti-EGFR antibody 2 (SEQ ID NO: 43), the CDRL2 amino acid sequence of anti-EGFR antibody 2 (SEQ ID NO: 44), the CDRL3 amino acid sequence of anti-EGFR antibody 2 (SEQ ID NO: 45), the CDRH1 amino acid sequence of anti-EGFR antibody 2 (SEQ ID NO: 46), the CDRH2 amino acid sequence of anti-EGFR antibody 2 (SEQ ID NO: 47), and the CDRH3 amino acid sequence of anti-EGFR antibody 2 (SEQ ID NO: 48). The CDR sequences were determined according to the Kabat definition.
[Figure 26] Figure 26 shows the amino acid sequence of the trastuzumab light chain (SEQ ID NO: 49) and the amino acid sequence of the trastuzumab heavy chain (SEQ ID NO: 50).
[Figure 27] Figure 27 shows the amino acid sequence of the engineered HER2 antibody light chain (SEQ ID NO: 49) and the amino acid sequence of the engineered HER2 antibody heavy chain (SEQ ID NO: 51).
[Figure 28] Figure 28 shows the amino acid sequence of the pertuzumab light chain (SEQ ID NO: 52) and the amino acid sequence of the pertuzumab heavy chain (SEQ ID NO: 53).
[Figure 29] Figure 29 shows the amino acid sequence of the engineered HER2 antibody 2 light chain (SEQ ID NO: 52) and the amino acid sequence of the engineered HER2 antibody 2 heavy chain (SEQ ID NO: 54).
[Figure 30] Figure 30 shows the amino acid sequence of the anti-CDH6 antibody light chain (SEQ ID NO: 55) and the amino acid sequence of the anti-CD33 antibody heavy chain (SEQ ID NO: 56).
[Figure 31] Figure 31 shows the CDRL1 amino acid sequence of pertuzumab (SEQ ID NO: 57), the CDRL2 amino acid sequence of pertuzumab (SEQ ID NO: 58), the CDRL3 amino acid sequence of pertuzumab (SEQ ID NO: 59), the CDRH1 amino acid sequence of pertuzumab (SEQ ID NO: 60), the CDRH2 amino acid sequence of pertuzumab ( SEQ ID NO: 61), and the CDRH3 amino acid sequence of pertuzumab (SEQ ID NO: 62). The CDR sequences were determined according to the IMGT definition.
[Figure 32] Figure 32 shows the CDRL1 amino acid sequence of an anti-CDH6 antibody (SEQ ID NO: 63), the CDRL2 amino acid sequence of the anti-CDH6 antibody (SEQ ID NO: 64), the CDRL3 amino acid sequence of the anti-CDH6 antibody (SEQ ID NO: 65), the CDRH1 amino acid sequence of the anti-CDH6 antibody (SEQ ID NO: 66), the CDRH2 amino acid sequence of the anti-CDH6 antibody (SEQ ID NO: 67), and the CDRH3 amino acid sequence of the anti-CDH6 antibody (SEQ ID NO: 68). The CDR sequences were determined according to the Kabat definition.

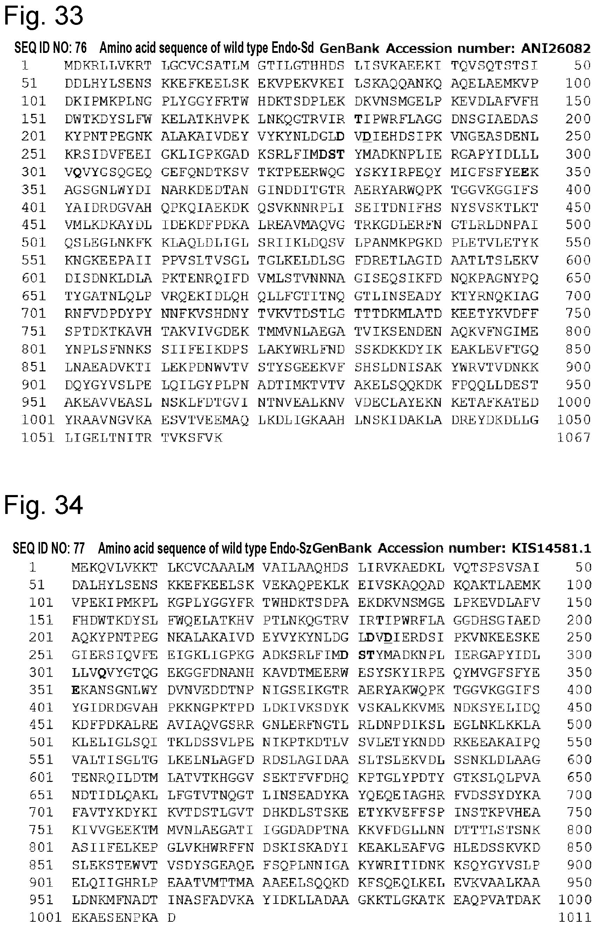

[Figure 33] Figure 33 shows the amino acid sequence of wild type Endo-Sd (SEQ ID NO: 76). An amino acid residue corresponding to a catalytic activity center site of the enzyme is indicated in bold and underlined, and an amino acid residue substituted in the mutant enzymes used in the Examples or an amino acid residue that can be expected to control enzyme activity by adding a mutation is indicated in bold.
[Figure 34] Figure 34 shows the amino acid sequence of wild type Endo-Sz (SEQ ID NO: 77). An amino acid residue corresponding to a catalytic activity center site of the enzyme is indicated in bold and underlined, and an amino acid residue substituted in the mutant enzymes used in the Examples or an amino acid residue that can be expected to control enzyme activity by adding a mutation is indicated in bold.

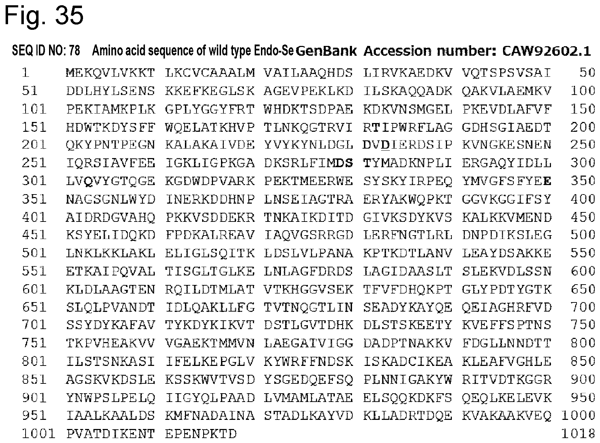

[Figure 35] Figure 35 shows the amino acid sequence of wild type Endo-Se (SEQ ID NO: 78). An amino acid residue corresponding to a catalytic activity center site of the enzyme is indicated in bold and underlined, and an amino acid residue substituted in the mutant enzymes used in the Examples or an amino acid residue that can be expected to control enzyme activity by adding a mutation is indicated in bold.

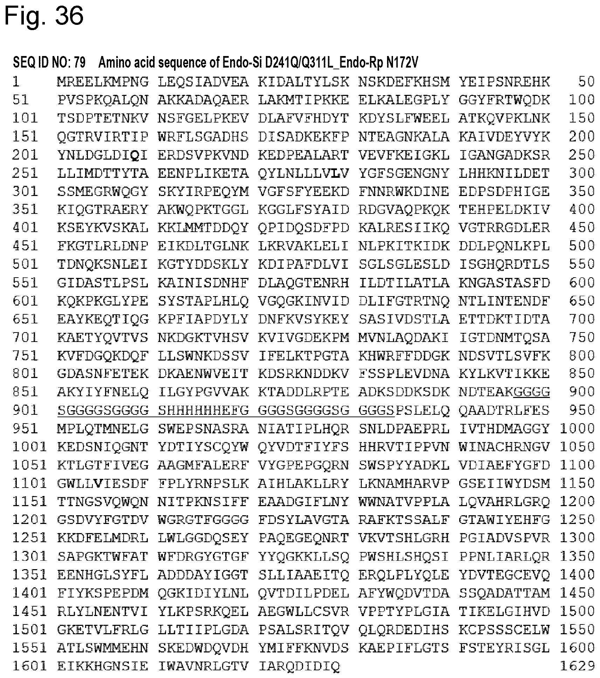

[Figure 36] Figure 36 shows the sequence of SEQ ID NO: 79 ([Endo-Si D241Q/Q311L]-[Endo-Rp N172V] fusion protein). An amino acid residue substituted in the fusion protein described in the Examples is indicated in bold, and an amino acid linker sequence is underlined.

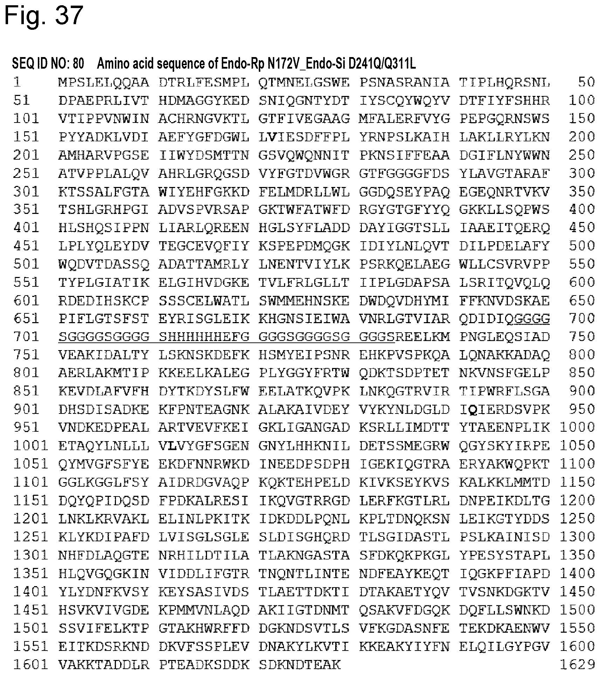

[Figure 37] Figure 37 shows the sequence of SEQ ID NO: 80 ([Endo-Rp N172V]-[Endo-Si D241Q/Q311L] fusion protein). An amino acid residue substituted in the fusion protein used in the Examples is indicated in bold, and an amino acid linker sequence is underlined.

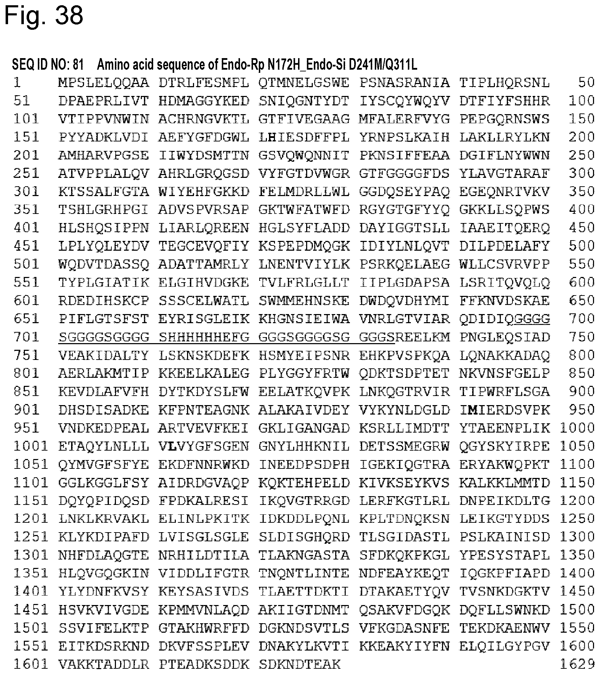

[Figure 38] Figure 38 shows the sequence of SEQ ID NO: 81 ([Endo-Rp N172H]-[Endo-Si D241M/Q311L] fusion protein). An amino acid residue substituted in the fusion protein used in the Examples is indicated in bold, and an amino acid linker sequence is underlined.

### Description of Embodiments

Hereinafter, the present invention will be described in detail.

Herein, the notation of an amino acid included in a molecule is according to a convention in the present field, and when a mutation site is indicated, it is represented by the one-letter code for the wild type amino acid followed by the number thereof (for example, "D241" for Asp at position 241). In addition, a mutation is represented by the one-letter code for the wild type amino acid followed by the number thereof followed by the one-letter code for the amino acid after mutation (for example, "D241Q" for a mutation in which Asp at position 241 is substituted with Gln). In addition, a specific mutant having a mutation is represented by a molecule name and the mutation (for example, "Endo-Si D241Q" for a mutant in which Asp at position 241 of Endo-Si is substituted with Gln), and a mutant having a plurality of mutations is represented in such a manner that the mutations are separated by "/" (for example, "Endo-Si D241Q/Q311L" for a mutant having an additional mutation in which Gln at position 311 is substituted with Leu in Endo-Si D241Q).

In one aspect of the present invention, provided is a method for producing an Fc-containing molecule having an N297-linked glycan including a glycan derived from a glycan donor molecule, including the following Steps 1 and 2.
[Step 1] reacting an acceptor molecule, which is an Fc-containing molecule having, as an N297-linked glycan, a core GlcNAc to which fucose is optionally added, with a glycan donor molecule including a glycan in the presence of an endo-β-N-acetylglucosaminidase (Enzyme-A), which prefers, as a substrate, an N297-linked glycan of an Fc-containing molecule, so as to obtain a reaction mixture; and
[Step 2] contacting the reaction mixture with a cation exchange chromatography medium or a multimode chromatography medium under an acidic condition and collecting the Fc-containing molecule having an N297-linked glycan including the glycan derived from the glycan donor molecule.

### <N297-linked glycan>

As used herein, the "N297-linked glycan" means an N-linked glycan that is linked to the side chain of Asn at position 297 of an IgG heavy chain, and even if the addition position of the N-linked glycan of an IgG heavy chain is changed by any method, the N-linked glycan is included in the N297-linked glycan as long as the N-linked glycan receives the action of Enzyme-A. For example, in one aspect of the present invention, when an IgG heavy chain is fragmented, even a glycan linked to the corresponding Asn in a peptide fragment including the Asn is included in the N297-linked glycan. Usually, the N297-linked glycan in IgG produced in an animal or the like has a basic structure consisting of the structure of the following formula (I) or (II), and the non-reducing end thereof is optionally further chemically-modified, and for example, Gal or sialic acid is optionally added.

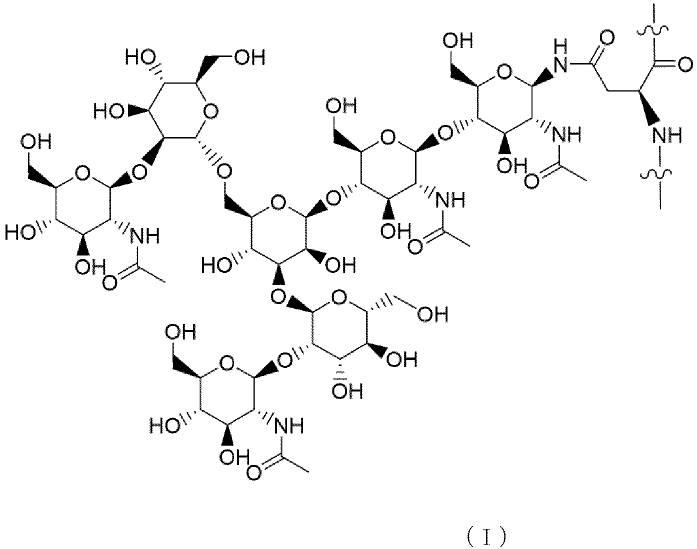

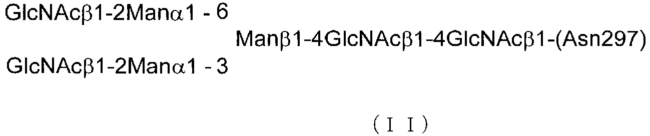

Many of the N297-linked glycans of IgG produced by cells have a diversity of glycan structures, including one in which a glycan is further linked to a GlcNAc (core GlcNAc) at a reducing end, a non-reducing end, a branched sugar, or the like in this basic structure. The N297-linked glycan may have a structure having core fucose in which fucose (Fuc) is α1,6-linked at position 6 of a core GlcNAc ((Fucα1,6)GlcNAc). In some cases, Man, which is a branched sugar, forms a triantennary glycan in which a glycan including a GlcNAc is further linked at position 5 thereof. In some cases, a glycan including galactose (Gal) or sialic acid (Sia) is further linked to the GlcNAc at a non-reducing end.

### <Glycan donor molecule>

As used herein, the "glycan donor molecule" (also referred to as a "glycan donor") means a molecule that donates a glycan to an acceptor molecule.

When a glycan donor molecule is used for glycan remodeling for the purpose of drug discovery, it is preferable to adopt a glycan donor molecule having a human type glycan or a human-compatible type glycan that causes few problems when applied to a human. Such a glycan is a glycan that is known not to exhibit antigenicity in the human body, and known examples of such a glycan that is an N-linked glycan include a high mannose type, a hybrid type, and a complex type. These three have a common basic structure. The high mannose type is a glycan having a mannose-rich structure in which a plurality of mannose molecules are continuously present in two branched chains (1-3 chain and 1-6 chain) branched from mannose at a position close to the reducing end (β-mannose). The hybrid type has a structure in which one of two branched chains (1-3 chain and 1-6 chain) branched from mannose at a position close to the reducing end (β-mannose) has a GlcNAc. The complex type has a structure having a GlcNAc in two branched chains (1-3 chain and 1-6 chain) branched from mannose at a position close to the reducing end (β-mannose), wherein the structure is diversified depending on the presence or absence of galactose, the presence or absence of sialic acid, and the linkage isomerism or position isomerism thereof. As the complex type glycan, a biantennary type, a triantennary type, and a tetraantennary type are known.

Hereinafter, examples of the high mannose type, hybrid type, and complex type structures will be shown. The common basic structure thereof is indicated by a dotted square (inside the dotted square, there is a mixture of a structure including a GlcNAc introduced at position 4 of β-mannose (bisecting GlcNAc) and a structure not including the same, and herein, all refer to the common basic structure).

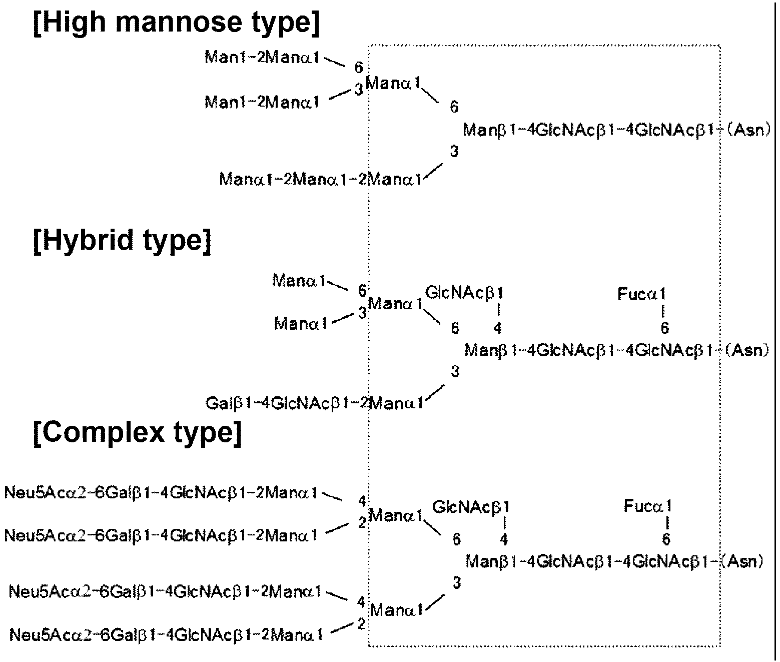

In the present invention, the glycan moiety in the glycan donor molecule may be any of a high mannose type glycan, a hybrid type glycan, and a complex type glycan, and is preferably a high mannose type glycan or a complex type glycan, and particularly preferably a complex type glycan. Therefore, in one aspect of the present invention, the glycan donor molecule includes a complex type glycan. In addition, in the present invention, the complex type glycan may be any of a biantennary type, a triantennary type, or a tetraantennary type, and the glycan donor molecule of the present invention preferably includes a biantennary complex type glycan.

In one aspect of the present invention, the glycan donor molecule in the present invention is a glycan-containing molecule having a GlcNAc having an unactivated glycan reducing end, or a glycan-containing molecule having a GlcNAc having an activated glycan reducing end (for example, an oxazolinated GlcNAc). Herein, the activation of the reducing end of a glycan donor molecule synthesized by a chemical method refers to a state in which the reactivity of the anomeric position of the reducing end of the glycan donor molecule is chemically enhanced, and typically, refers to a state in which the anomeric position is oxazolinated or halogenated. That is, the activation of the reducing end refers to a state in which isolation and purification with an activated glycan donor molecule alone are possible.

On the other hand, activation in which enzyme-B acts on a glycan donor molecule including a glycan including a GlcNAc having an unactivated reducing end to bring the glycan donor molecule into a state in which Enzyme-A can be used for the desired transglycosylation reaction refers to a state in which the reactivity of the anomeric position at the reducing end of the glycan donor molecule is enzymologically enhanced. That is, the activation means that an activated intermediate is generated, and refers to a state in which isolation and purification with an activated glycan donor molecule alone are impossible.

In one aspect of the present invention, the glycan donor molecule is a glycan-containing molecule having a GlcNAc having an unactivated glycan reducing end, and preferable examples thereof include SGP, (SG-)Asn, (MSG1-)Asn, (MSG2-)Asn, or a mixture of (MSG1-)Asn and (MSG2-)Asn. These glycan moieties are representative of an N-linked glycan, and include a sialyl glycan (hereinafter referred to as "SG") consisting of a structure represented by the following structural formula and sequence formula. The linkage mode of the glycan on the reducing end side may be changed from the N-linked type to the O-linked type. Herein, unless otherwise specified, a partial structure when an amino acid is linked at a side chain thereof to a glycan is denoted in such a manner that the side chain moiety is indicated in parentheses, for example, as "(SG-)Asn" as described above. In addition, herein, a glycan structure having sialic acid deleted at the non-reducing end of only one of the branched chains of β-Man of SG is referred to as MSG; and one having sialic acid only in the 1-3 glycan of the branched chains is denoted as MSG1, and one having sialic acid only in the 1-6 glycan of the branched chains is denoted as MSG2. 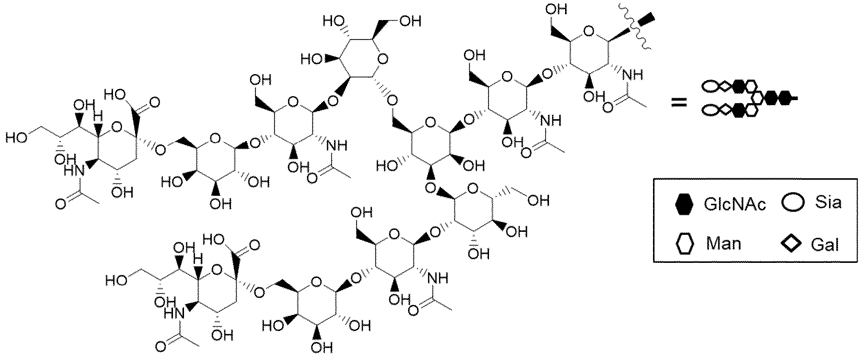 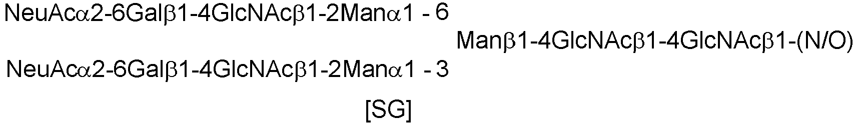 wherein "-(N/O)" represents a glycoside linkage with an N atom or an O atom.

Specific examples of the glycan moiety in the glycan donor molecule that can be used in the present invention include AG(9) obtained by treating SG with neuraminidase and deleting NeuAc at two non-reducing ends) (AG(9) of the following structural formula and sequence formula), and AG(7) obtained by treating AG(9) with galactosidase and deleting Gal at two non-reducing ends (AG(7) of the following structural formula and sequence formula). 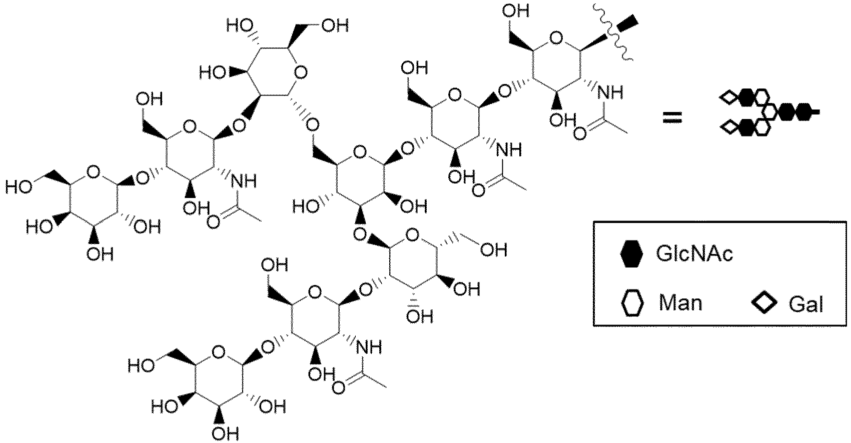 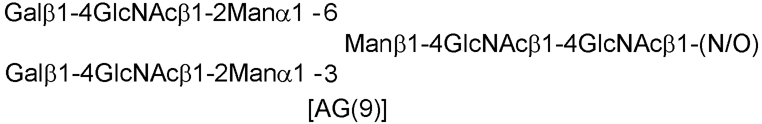 wherein "-(N/O)" represents a glycoside linkage with an N atom or an O atom. 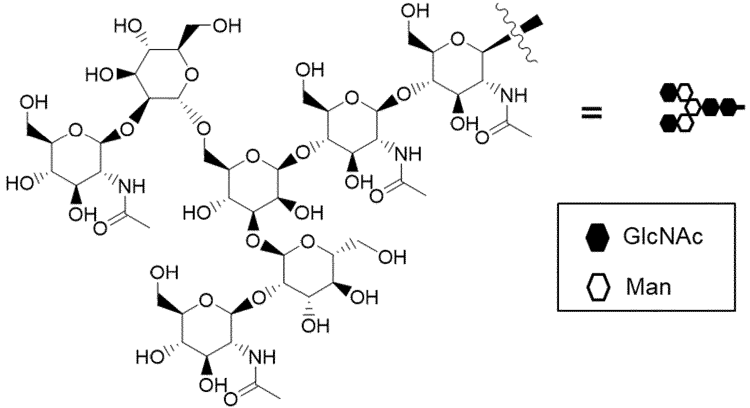 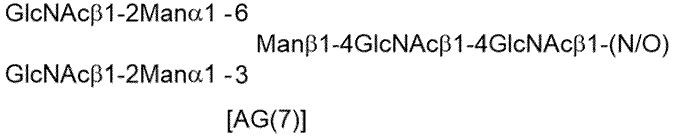 wherein "-(N/O)" represents a glycoside linkage with an N atom or an O atom.

In the present invention, the glycan donor molecule is optionally chemically-modified, and for example, the non-reducing end is optionally chemically-modified.

Examples of a glycan moiety in a glycan donor molecule having an optionally chemically-modified non-reducing end that can be used in the present invention include a derivative (SG type) in which a site on wild type sialic acid is chemically-modified with any substituent. 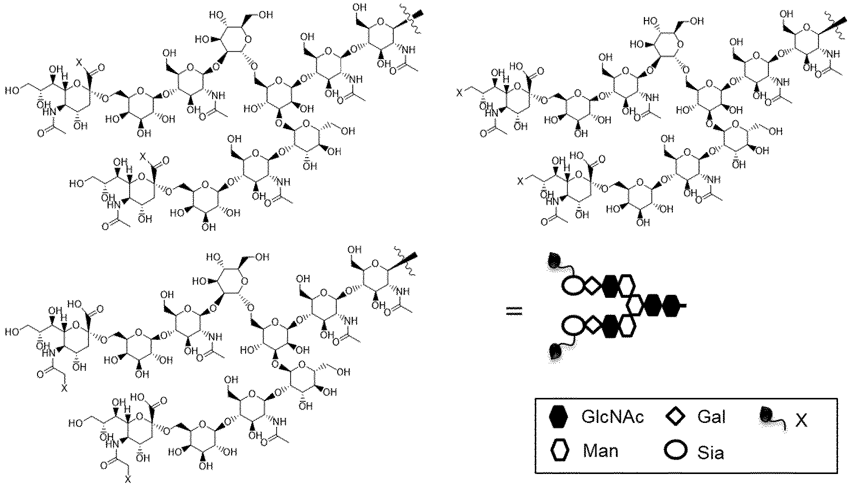 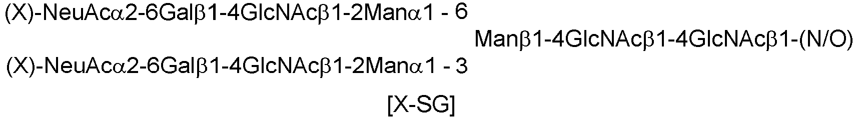 wherein X represents a substituent introduced on sialic acid at the non-reducing ends on both the 1-3 chain side and the 1-6 chain side of the branched chains of β-Man, and is a substituent that can be synthesized by applying various methods known in the field of organic synthetic chemistry, and with reference to substituents used in binding methods applied to synthesis of an antibody-drug conjugate (Bioconjugate Chem. 2015, 26, 2198-2215), examples thereof include, but are not limited to, an acetylene group, an azido group (N₃-), a dibenzylcyclooctene (DBCO) group, a bicyclo[6.1.0]non-4-ene (BCN) group, a structure including a 1,2,4,5-tetrazine ring, an aldehyde group, a SH group, and a methylsulfonyltriazole group, preferable examples thereof include, but are not limited to, an acetylene group and an azido group (N₃-), and more preferable examples thereof include, but are not limited to, a substituent having an azido group (N₃-). In addition, the "substituent having an azido group (N₃-)" is, for example, a polyethylene glycol linker having N₃, and that is, the upper limit of the number of ethylene glycol units (-CH₂-CH₂-O-) included in the linker structure is about 20 or less, preferably about 11 or less, and more preferably about 7 or less, and examples of the "substituent having an azido group (N₃-)" include, but are not limited to, a polyethylene glycol linker having N₃ (for example, a linear substituent such as a 35-azido-3,6,9,12,15,18,21,24,27,30,33-undecaoxapentatriacontan-1-amino group, a 32-azido-3,6,9,12,15,18,21,24,27,30-decaoxadotriacontan-1-amino group, a 29-azido-3,6,9,12,15,18,21,24,27-nonaoxanonacosan-1-amino group, a 26-azido-3,6,9,12,15,18,21,24-octaoxahexacosan-1-amino group, a 23-azido-3,6,9,12,15,18,21-heptaoxatricosan-1-amino group, a 20-azido-3,6,9,12,15,18-hexaoxaeicosan-1-amino group, a 17-azido-3,6,9,12,15-pentaoxaheptadecan-1-amino group, a 14-azido-3,6,9,12-tetraoxatetradecan-1-amino group, a 11-azido-3,6,9-trioxaundecan-1-amino group, a 2-[2-(2-azidoethoxy)ethoxy]ethylamino group, a 2-(azidoethoxy)ethylamino group, a 2-azidoethylamino group, or a 2-[2-[2-(2-azidoethoxy)ethoxy]-N-[2-[2-[2-(2-azidoethoxy)ethoxy]ethoxy]ethyl]ethanamine group, or a branched substituent such as a 1,3-bis[2-[2-(2-azidoethoxy)ethoxy]ethoxy]propan-2-amino group or a 3-[2-[2-(2-azidoethoxy)ethoxy]ethoxy]-2-[2-[2-(2-azidoethoxy)ethoxy]ethoxymethyl]propan-1-amino group); and wherein "-(N/O)" represents a glycoside linkage with an N atom or an O atom.

Of the X-SG type structures shown here, the X-MSG1 type, which is a structure lacking chemically-modified sialic acid on the 1-6 chain side of the branched chains of β-Man, and the X-MSG2 type, which is a structure lacking chemically-modified sialic acid on the 1-3 chain side are also examples of a glycan moiety in a glycan donor molecule having an optionally chemically-modified non-reducing end.

Furthermore, an optionally chemically-modified site other than sialic acid is also possible, and examples thereof include an AG(9) type derivative and an AG(7) type derivative shown below. 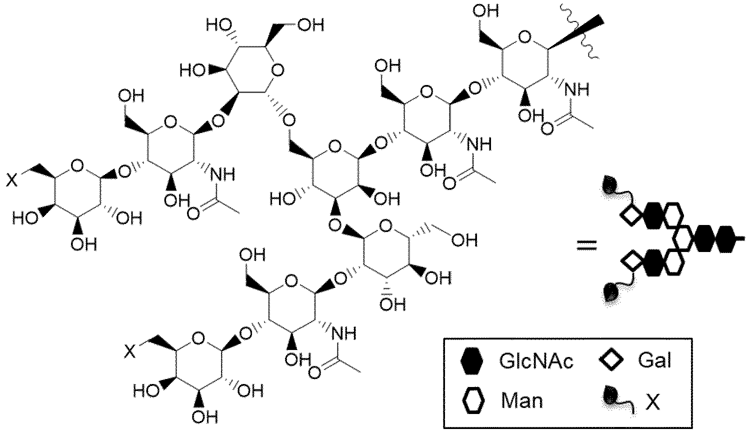 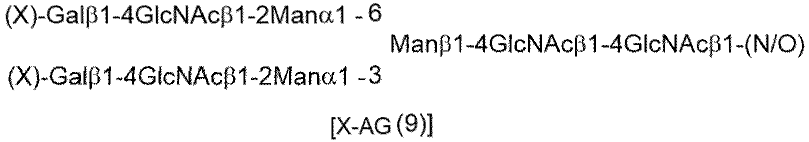 wherein X represents a substituent introduced on galactose at the non-reducing ends of both the 1-3 chain side and the 1-6 chain side of the branched chains of β-Man, and specifically, represents the same meaning as defined above; and wherein "-(N/O)" represents a glycoside linkage with an N atom or an O atom. 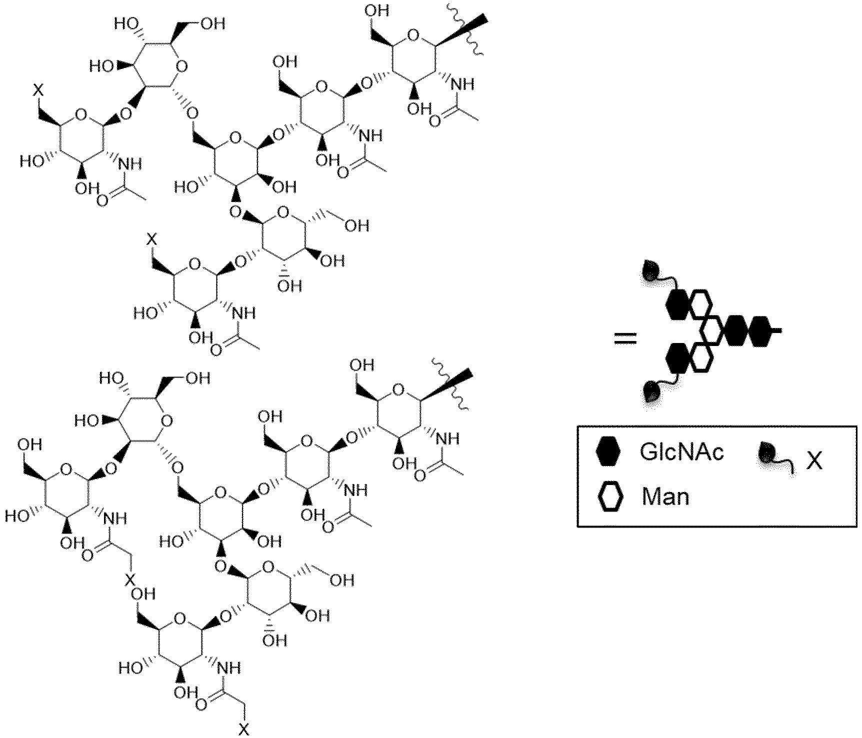 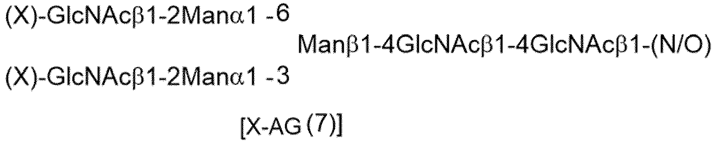 wherein X represents a substituent introduced on the GlcNAc at the non-reducing ends of both the 1-3 chain side and the 1-6 chain side of the branched chains of β-Man, and specifically, represents the same meaning as defined above; and wherein "-(N/O)" represents a glycoside linkage with a N atom or an O atom.

Of the X-AG(9) type structures shown here, a structure lacking chemically-modified galactose on the 1-6 chain side of the branched chains of β-Man and a structure lacking chemically-modified galactose on the 1-3 chain side, or among the X-AG(7) type structures, a structure lacking the chemically-modified GlcNAc on the 1-6 chain side of the branched chains of β-Man and a structure lacking the chemically-modified GlcNAc on the 1-3 chain side are also examples of a glycan moiety in the donor molecule.

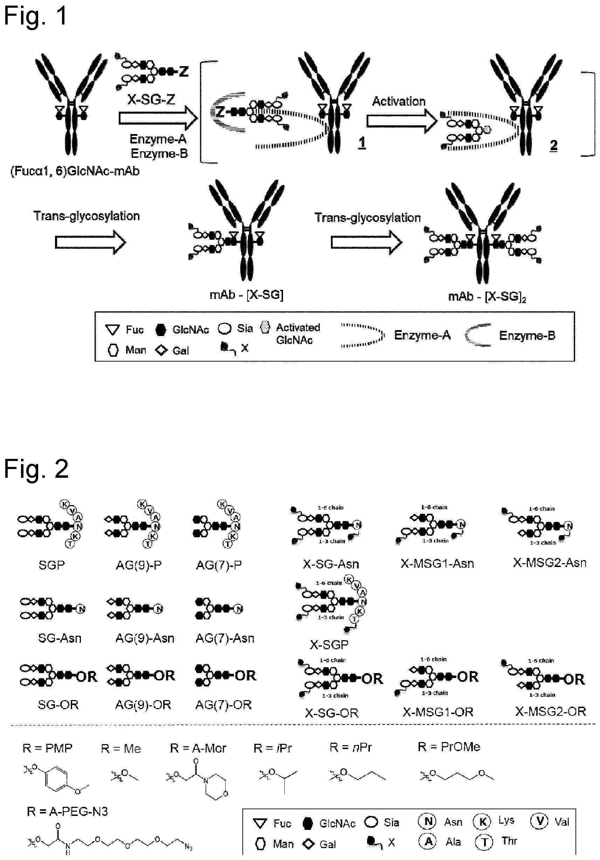

Preferably, the glycan donor molecule of the present invention is SGP, AG(9)-P, AG(7)-P, SG-Asn, AG(9)-Asn, AG(7)-Asn, SG-OR, AG(9)-OR, AG(7)-OR, (MSG1)-Asn, (MSG2)-Asn, a mixture of (MSG1)-Asn and (MSG2)-Asn, MSG1-OR, MSG2-OR, or a mixture of MSG1-OR and MSG2-OR, having an optionally chemically-modified non-reducing end, or is a glycan donor molecule having the structure shown in Figure 2. In the above structural formulas, "P" represents a peptide moiety derived from SGP, and "X" in Figure 2 represents the same meaning as defined above. The glycan donor molecule of the present invention is further preferably, as one including an N-linked glycan, ([N₃-PEG(3)]₂-SG)-P-PEG(3)-N₃, ([N₃-PEG(3)]₂-SG)-Asn-PEG(3)-N₃, ([N₃-PEG(3)]-MSG1)-Asn-PEG(3)-N₃, ([N₃-PEG(3)]-MSG2)-Asn-PEG(3)-N₃, or a mixture of ([N₃-PEG(3)]-MSG1)-Asn-PEG(3)-N₃ and ([N₃-PEG(3)]-MSG2)-Asn-PEG(3)-N₃, and, as one including an O-linked glycan, ([N₃-PEG(3)]₂-SG)-OR, ([N₃-PEG(3)]-MSG1)-OR, ([N₃-PEG(3)]-MSG2)-OR, or a mixture of ([N₃-PEG(3)]-MSG1)-OR and ([N₃-PEG(3)]-MSG2)-OR. Furthermore, in one aspect of the present invention, the glycan donor molecule is preferably a novel glycan donor molecule specifically disclosed in the chapter <Novel glycan donor molecule>. The glycan donor molecule of the present invention is preferably appropriately selected according to various conditions such as a combination of Enzyme-A and Enzyme-B to be used.

In the above chemical formulas, "R" represents any substituent linked to an oxygen atom via at least one carbon atom, and represents preferably a C1-C6 alkyl group or an optionally substituted aryl group. The "C1-C6 alkyl group" means a linear or branched alkyl group having 1 to 6 carbon atoms, and examples thereof include, but are not limited to, a methyl group, an ethyl group, a n-propyl group, an i-propyl group, a n-butyl group, an i-butyl group, a s-butyl group, a t-butyl, a n-pentyl group, and n-hexyl. In addition, examples of the "aryl group" include, but are not limited to, a phenyl group, a benzyl group, an indenyl group, a naphthyl group, a fluorenyl group, an anthranyl group, and a phenanthrenyl group. Examples of a substituent for the aryl group include, but are not limited to, a C1-C6 alkoxy group (for example, a methoxy group, an ethoxy group, or an isopropoxy group), a C1-C6 alkyl group (for example, a methyl group or an ethyl group), and a halogen group. Examples of a substituted aryl group include, but are not limited to, a para-methoxyphenyl group and a para-methylphenyl group. In one aspect of the present invention, "R" is selected from the group consisting of PMP, Me, A-Mor, iPr, nPr, PrOMe, and A-PEG-N₃ wherein A represents an acetyl group in a glycolic acid unit, that is, a moiety present between an anomeric hydroxyl group and an amino group in Mor or PEG.

In one aspect of the present invention, the glycan donor molecule includes a high mannose type glycan. Examples of such a glycan donor molecule include, but are not limited to, Man9-GlcNAc₂-Asn ("M9-Asn") having the following structure, and Man8-GlcNAc₂-Asn ("M8-Asn"), Man7-GlcNAc₂-Asn ("M7-Asn"), and Man6-GlcNAc₂-Asn ("M6-Asn"), having a structure lacking one, two, or three mannose molecules, respectively, from M9-Asn.

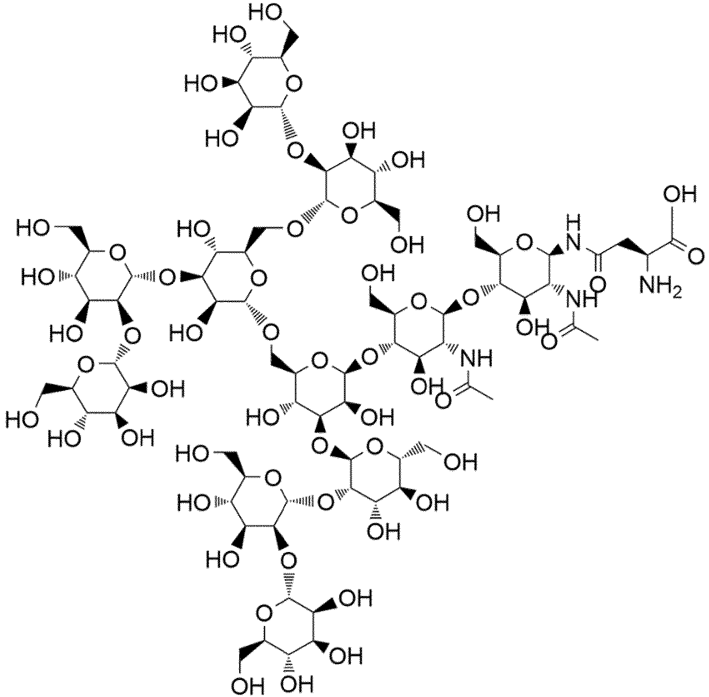

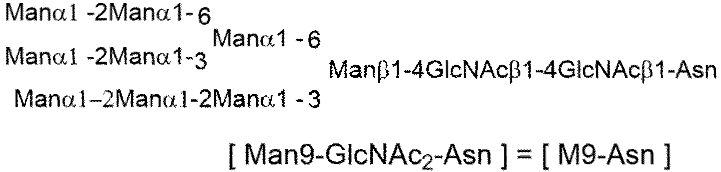

In one aspect of the present invention, the glycan donor molecule is a glycan-containing molecule having a GlcNAc having an activated glycan reducing end, preferably an oxazolinated GlcNAc. As such a glycan donor molecule, molecules having various glycan structures can be used. Examples of such a glycan donor molecule include, but are not limited to, SG-Ox wherein "Ox" represents oxazoline, MSG1-Ox, MSG2-Ox, or a mixture of MSG1-Ox and MSG2-Ox.

A glycan-containing molecule having a GlcNAc having an activated glycan reducing end is also optionally chemically-modified as is a glycan-containing molecule having a GlcNAc having an unactivated glycan reducing end, and for example, the non-reducing end is optionally chemically-modified. Examples of the chemical modification of a non-reducing end include the same modifications as described for the glycan-containing molecule having a GlcNAc having an unactivated glycan reducing end, and examples thereof include, but are not limited to, [N₃-PEG(3)]₂-SG)-Ox, [N₃-PEG(3)]-MSG1-Ox, [N₃-PEG(3)]-MSG2-Ox, or a mixture of [N₃-PEG(3)]-MSG1-Ox and [N₃-PEG(3)]-MSG2-Ox.

The glycan donor molecule can be appropriately purchased as a commercial product or prepared by using or applying a known method, and for example, can be prepared by using or applying the method shown in Example 7 or Reference Example 23-1.

### <Fc-containing molecule>

As used herein, the "Fc-containing molecule" (sometimes referred to as an "Fc region-containing molecule") refers to a molecule containing an Fc region, and examples thereof include an antibody (immunoglobulin), an Fc-fusion protein, and an Fc fragment (or an Fc-containing fragment). The antibody (immunoglobulin) in the present invention may be any of IgG, IgA, IgM, IgE, and IgD, and may be either a monoclonal antibody or a polyclonal antibody, and furthermore may be naturally derived or synthesized, and is preferably IgG, and particularly an IgG type monoclonal antibody. Examples of an Fc-containing fragment other than the antibody include, but are not limited to, CLCH, such as CLCH consisting only of an Fc fragment and constant regions of IgG (particularly an IgG type monoclonal antibody) (Patent Literature 1), and a bispecific antibody including an Fc fragment (Expert Opin Ther Pat. 2018 28, 251-276).

### <What is an acceptor molecule?>

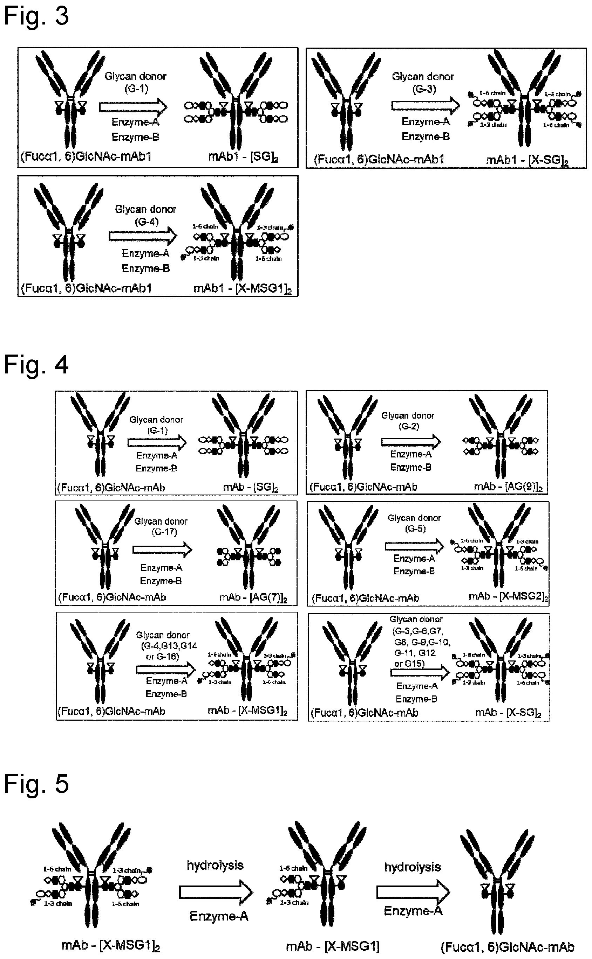

As used herein, the "acceptor molecule" means a molecule that accepts a glycan from a glycan donor molecule. A typical acceptor molecule is an IgG, or an Fc fragment thereof, that is derived from a monoclonal antibody and has an N297-linked glycan consisting only of a core GlcNAc to which core Fuc is optionally linked. Herein, the "core Fuc" (or "core fucose") means fucose linked to the core GlnNAc. Core Fuc may or may not be linked to the core GlcNAc, depending on the antibody from which the acceptor molecule is derived or the method for producing the same. Therefore, in one aspect of the present invention, the acceptor molecule is an Fc-containing molecule (for example, an antibody or an Fc-containing fragment thereof) having, as an N297-linked glycan, a core GlcNAc to which fucose is optionally added, and preferably an Fc-containing molecule (for example, an antibody or an Fc-containing fragment thereof) having an N297-linked glycan consisting of a GlcNAc or a (Fucα1,6)GlcNAc. As what the acceptor molecule is derived from, various monoclonal antibodies, glycan-containing molecules, or Fc region-containing molecules (for example, Fc, or CLCH formed by combining CH consisting only of a constant region obtained by deleting a variable region from a heavy chain and CL consisting only of a constant region of a light chain) can be used. Preferable examples of the acceptor molecule of the present invention include (Fucα1,6)-GlcNAc-IgG (for example, (Fucα1,6)GlcNAc-mAb1 in Figure 4), (Fucα1,6)-GlcNAc-Fc, (Fucα1,6)-GlcNAc-CLCH, and GlcNAc-mAb4 (Patent Literature 1).

In one aspect of the present invention, the Fc-containing molecule having an N297-linked glycan can be derived from an antibody, and examples of the antibody include, but are not limited to, an anti-HER2 antibody, an anti-HER3 antibody, an anti-DLL3 antibody, an anti-FAP antibody, an anti-CDH11 antibody, an anti-CDH6 antibody, an anti-A33 antibody, an anti-CanAg antibody, an anti-CD19 antibody, an anti-CD20 antibody, an anti-CD22 antibody, an anti-CD30 antibody, an anti-CD33 antibody, an anti-CD56 antibody, an anti-CD70 antibody, an anti-CD98 antibody, an anti-TROP2 antibody, an anti-CEA antibody, an anti-Cripto antibody, an anti-EphA2 antibody, an anti-G250 antibody, an anti-MLTC1 antibody, an anti-GPNMB antibody, an anti-Integrin antibody, an anti-PSMA antibody, an anti-Tenascin-C antibody, an anti-SLC44A4 antibody, an anti-Mesothelin antibody, an anti-ENPP3 antibody, an anti-CD47 antibody, an anti-EGFR antibody, an anti-GPR20 antibody, or an anti-DR5 antibody. The antibody of the present invention is preferably an anti-HER2 antibody (for example, trastuzumab or pertuzumab), an anti-CDH6 antibody, an anti-CD33 antibody, an anti-EphA2 antibody, an anti-CD70 antibody, an anti-TROP2 antibody, or an anti-EGFR antibody, and more preferably an anti-HER2 antibody, an anti-CDH6 antibody, an anti-CD70 antibody, an anti-TROP2 antibody, or an anti-EGFR antibody.

Such an antibody can be obtained by immunizing an animal with a polypeptide that serves as an antigen, and collecting and purifying an antibody produced in vivo, by using a method commonly practiced in this field. The origin of an antigen is not limited to a human, and an animal can also be immunized with an antigen derived from a non-human animal such as a mouse or a rat. In this case, an antibody applicable to a human disease can be screened for by testing the cross-reactivity between an antibody that binds to the obtained heterologous antigen and a human antigen.

In addition, according to a known method (for example, Kohler and Milstein, Nature (1975) 256, p.495-497, Kennett, R. ed., Monoclonal Antibodies, p.365-367, Plenum Press, N.Y. (1980)), a monoclonal antibody can also be obtained by fusing an antibody-producing cell that produces an antibody against an antigen with a myeloma cell to establish a hybridoma.

The antigen can be obtained by genetically engineering a host cell to produce a gene encoding an antigen protein. In the present invention, the "host cell" can be appropriately selected from a cell commonly used for protein production such as an animal cell, a plant cell, Escherichia coli, or a yeast.

The above antibody may be a humanized antibody, and can be obtained according to a known method (for example, Proc. Natl. Acad. Sci. USA, 81, 6851-6855, (1984), Nature (1986) 321, p.522-525, WO90/07861).

For example, an anti-HER2 antibody (US5821337, WO2004/008099, WO2020/050406, or the like), an anti-CD33 antibody (WO2014/057687, WO2020/050406, or the like), an anti-EphA2 antibody (WO2009/028639, WO2020/050406, or the like), an anti CDH6 antibody (WO2018/212136, WO2020/050406, or the like), an anti-CD70 antibody (WO2004/073656, WO2007/038637, WO2021/177438, or the like), an anti-TROP2 antibody (WO2015/098099, WO2021/177438, or the like), and an anti-EGFR antibody (WO1998/050433, WO2002/092771, WO2021/177438, or the like) can be obtained by known means.

Hereinafter, the antibody will be described in detail, it is known that the constant region of the antibody has a plurality of allotypes, and examples thereof for an IgG1 heavy chain include G1m17, G1m3, G1m1, and G1m2. The constant region of the antibody used in the present invention is not particularly limited, and it is preferable to use G1m17 or G1m3. In addition, when IgG1 is used as the isotype of the antibody of the present invention, it is possible to adjust the effector function by substituting a part of the amino acid residues of the constant region; as one aspect, it is possible to enhance or attenuate antibody-dependent cellular cytotoxicity activity, enhance or attenuate antibody-dependent cell-mediated phagocytosis, or enhance or attenuate complement-dependent cytotoxicity activity, and one aspect of a mutant is one in which leucine at position 234 and leucine at position 235 specified by EU index numbering (Proc. Natl. Acad. Sci. USA, Vol. 63, No. 1 (May 15, 1969), pp.78-85) in the amino acid sequence of the constant region of wild type human IgG1 are substituted with alanine and alanine, respectively, or one in which leucine at position 234, leucine at position 235, and proline at position 329 specified by EU index numbering are substituted with alanine, alanine, and alanine, respectively. In addition, the antibody used in the present invention can have one or two amino acids deleted at the carboxyl end of one or both of the heavy chains.

The above anti-HER2 antibody is not particularly limited, and desirably has the following properties, for example.
(1) An anti-HER2 antibody characterized by having the following properties;
   (a) specifically binding to HER2;
   (b) having the activity of internalizing into an HER2-expressing cell by binding to HER2.
(2) The antibody according to (1) above, wherein the antibody binds to an extracellular domain of HER2.
(3) The antibody according to (1) or (2) above, wherein the antibody is a monoclonal antibody.
(4) The antibody according to any one of (1) to (3) above, wherein the antibody has antibody-dependent cellular cytotoxicity (ADCC) activity and/or complement-dependent cytotoxicity (CDC) activity.
(5) The antibody according to any one of (1) to (4) above, wherein the antibody is a mouse monoclonal antibody, a chimeric monoclonal antibody, or a humanized monoclonal antibody.
(6) The antibody according to (1) to (3) above, wherein a heavy chain constant region is a heavy chain constant region of human IgG1 and comprises a mutation that causes reduced ADCC and ADCP activities.
(7) The antibody according to any one of (1) to (4) above, wherein the antibody is a humanized monoclonal antibody comprising a heavy chain consisting of the amino acid sequence set forth in SEQ ID NO: 50 and a light chain consisting of the amino acid sequence set forth in SEQ ID NO: 49.
(8) The antibody according to (6) above, wherein the heavy chain constant region is a heavy chain constant region of human IgG1, and leucine at position 234 and leucine at position 235 specified by EU index numbering are each substituted with alanine.
(9) The antibody according to (8) above, wherein the antibody is a humanized monoclonal antibody comprising a heavy chain consisting of the amino acid sequence set forth in SEQ ID NO: 51 and a light chain consisting of the amino acid sequence set forth in SEQ ID NO: 49.
(10) The antibody according to any one of (1) to (4) above, wherein the antibody is a humanized monoclonal antibody comprising a heavy chain consisting of the amino acid sequence set forth in SEQ ID NO: 53 and a light chain consisting of the amino acid sequence set forth in SEQ ID NO: 52.
(11) The antibody according to (8) above, wherein the antibody is a humanized monoclonal antibody comprising a heavy chain consisting of the amino acid sequence set forth in SEQ ID NO: 54 and a light chain consisting of the amino acid sequence set forth in SEQ ID NO: 52.
(12) The antibody according to any one of (1) to (3), (6) or (8), wherein the antibody is a humanized monoclonal antibody comprising a light chain comprising CDRL1 consisting of the amino acid sequence set forth in SEQ ID NO: 57, CDRL2 consisting of the amino acid sequence set forth in SEQ ID NO: 58, and CDRL3 consisting of the amino acid sequence set forth in SEQ ID NO: 59, and a heavy chain comprising CDRH1 consisting of the amino acid sequence set forth in SEQ ID NO: 60, CDRH2 consisting of the amino acid sequence set forth in SEQ ID NO: 61, and CDRH3 consisting of the amino acid sequence set forth in SEQ ID NO: 62.
(13) The antibody according to any one of (1) to (12) above, wherein the antibody has one or two amino acids deleted at a heavy chain carboxyl end.
(14) An antibody obtained by a method for producing an antibody comprising: a step of culturing a host cell transformed with an expression vector containing a polynucleotide encoding the antibody according to any one of (1) to (13) above; and a step of collecting a target antibody from a culture obtained in the above step.

The anti-CD70 antibody used in the production of an antibody-immunostimulant conjugate of the present invention is not particularly limited, and desirably has the following properties, for example.
(1) An anti-CD70 antibody specifically that binds to CD70.
(2) The antibody according to (1) above, wherein the antibody binds to an extracellular domain of CD70.
(3) The antibody according to (1) or (2) above, wherein the antibody is a monoclonal antibody.
(4) The antibody according to any one of (1) to (3) above, wherein the antibody has antibody-dependent cellular cytotoxicity (ADCC) activity and/or complement-dependent cytotoxicity (CDC) activity.
(5) The antibody according to any one of (1) to (4) above, wherein the antibody is a mouse monoclonal antibody, a chimeric monoclonal antibody, or a humanized monoclonal antibody.
(6) The antibody according to (1) to (3) above, wherein a heavy chain constant region is a heavy chain constant region of human IgG1 and comprises a mutation that causes reduced ADCC and ADCP activities.
(7) The antibody according to (6) above, wherein the heavy chain constant region is a heavy chain constant region of human IgG1, and leucine at position 234 and leucine at position 235 specified by EU index numbering are each substituted with alanine.
(8) The antibody according to (7) above, wherein the antibody is a humanized monoclonal antibody comprising a heavy chain consisting of the amino acid sequence set forth in SEQ ID NO: 2 and a light chain consisting of the amino acid sequence set forth in SEQ ID NO: 1.
(9) The antibody according to (7) above, wherein the antibody is a humanized monoclonal antibody comprising a heavy chain consisting of the amino acid sequence set forth in SEQ ID NO: 4 and a light chain consisting of the amino acid sequence set forth in SEQ ID NO: 3.
(10) The antibody according to any one of (1) to (3), (6) or (7), wherein the antibody is a humanized monoclonal antibody comprising a light chain comprising CDRL1 consisting of the amino acid sequence set forth in SEQ ID NO: 13, CDRL2 consisting of the amino acid sequence set forth in SEQ ID NO: 14, and CDRL3 consisting of the amino acid sequence set forth in SEQ ID NO: 15, and a heavy chain comprising CDRH1 consisting of the amino acid sequence set forth in SEQ ID NO: 16, CDRH2 consisting of the amino acid sequence set forth in SEQ ID NO: 17, and CDRH3 consisting of the amino acid sequence set forth in SEQ ID NO: 18.
(11) The antibody according to any one of (1) to (3), (6) or (7), wherein the antibody is a humanized monoclonal antibody comprising a light chain comprising CDRL1 consisting of the amino acid sequence set forth in SEQ ID NO: 19, CDRL2 consisting of the amino acid sequence set forth in SEQ ID NO: 20, and CDRL3 consisting of the amino acid sequence set forth in SEQ ID NO: 21, and a heavy chain comprising CDRH1 consisting of the amino acid sequence set forth in SEQ ID NO: 22, CDRH2 consisting of the amino acid sequence set forth in SEQ ID NO: 23, and CDRH3 consisting of the amino acid sequence set forth in SEQ ID NO: 24.
(12) The antibody according to any one of (1) to (11) above, wherein the antibody has one or two amino acids deleted at a heavy chain carboxyl end.
(13) An antibody obtained by a method for producing an antibody comprising: a step of culturing a host cell transformed with an expression vector containing a polynucleotide encoding the antibody according to any one of (1) to (12) above; and a step of collecting a target antibody from a culture obtained in the above step.

The anti-TROP2 antibody used in the production of an antibody-immunostimulant conjugate of the present invention is not particularly limited, and desirably has the following properties, for example.
(1) An anti-TROP2 antibody that specifically binds to TROP2.
(2) The antibody according to (1) above, wherein the antibody binds to an extracellular domain of TROP2.
(3) The antibody according to (1) or (2) above, wherein the antibody is a monoclonal antibody.
(4) The antibody according to any one of (1) to (3) above, wherein the antibody has antibody-dependent cellular cytotoxicity (ADCC) activity and/or complement-dependent cytotoxicity (CDC) activity.
(5) The antibody according to any one of (1) to (4) above, wherein the antibody is a mouse monoclonal antibody, a chimeric monoclonal antibody, or a humanized monoclonal antibody.
(6) The antibody according to (1) to (3) above, wherein a heavy chain constant region is a heavy chain constant region of human IgG1 and comprises a mutation that causes reduced ADCC and ADCP activities.
(7) The antibody according to any one of (1) to (4) above, wherein the antibody is a humanized monoclonal antibody comprising a heavy chain consisting of the amino acid sequence set forth in SEQ ID NO: 6 and a light chain consisting of the amino acid sequence set forth in SEQ ID NO: 5.
(8) The antibody according to (6) above, wherein the heavy chain constant region is a human IgG1 heavy chain constant region, and leucine at position 234 and leucine at position 235 specified by EU index numbering are each substituted with alanine.
(9) The antibody according to (8) above, wherein the antibody is a humanized monoclonal antibody comprising a heavy chain consisting of the amino acid sequence set forth in SEQ ID NO: 8 and a light chain consisting of the amino acid sequence set forth in SEQ ID NO: 7.
(10) The antibody according to any one of (1) to (3), (6), or (8), wherein the antibody is a humanized monoclonal antibody comprising a light chain comprising CDRL1 consisting of the amino acid sequence set forth in SEQ ID NO: 25, CDRL2 consisting of the amino acid sequence set forth in SEQ ID NO: 26, and CDRL3 consisting of the amino acid sequence set forth in SEQ ID NO: 27, and a heavy chain comprising CDRH1 consisting of the amino acid sequence set forth in SEQ ID NO: 28, CDRH2 consisting of the amino acid sequence set forth in SEQ ID NO: 29, and CDRH3 consisting of the amino acid sequence set forth in SEQ ID NO: 30.
(11) The antibody according to any one of (1) to (3), (6) or (8), wherein the antibody is a humanized monoclonal antibody comprising a light chain comprising CDRL1 consisting of the amino acid sequence set forth in SEQ ID NO: 31, CDRL2 consisting of the amino acid sequence set forth in SEQ ID NO: 32, and CDRL3 consisting of the amino acid sequence set forth in SEQ ID NO: 33, and a heavy chain comprising CDRH1 consisting of the amino acid sequence set forth in SEQ ID NO: 34, CDRH2 consisting of the amino acid sequence set forth in SEQ ID NO: 35, and CDRH3 consisting of the amino acid sequence set forth in SEQ ID NO: 36.
(12) The antibody according to any one of (1) to (11) above, wherein the antibody has one or two amino acids deleted at a heavy chain carboxyl end.
(13) An antibody obtained by a method for producing an antibody comprising: a step of culturing a host cell transformed with an expression vector containing a polynucleotide encoding the antibody according to any one of (1) to (12) above; and a step of collecting a target antibody from a culture obtained in the above step.

The anti-EGFR antibody used in the production of an antibody-immunostimulant conjugate of the present invention is not particularly limited, and desirably has the following properties, for example.
(1) An anti-EGFR antibody that specifically binds to EGFR.
(2) The antibody according to (1) above, wherein the antibody binds to an extracellular domain of EGFR.
(3) The antibody according to (1) or (2) above, wherein the antibody is a monoclonal antibody.
(4) The antibody according to any one of (1) to (3) above, wherein the antibody has antibody-dependent cellular cytotoxicity (ADCC) activity and/or complement-dependent cytotoxicity (CDC) activity.
(5) The antibody according to any one of (1) to (4) above, wherein the antibody is a mouse monoclonal antibody, a chimeric monoclonal antibody, or a humanized monoclonal antibody.
(6) The antibody according to (1) to (3) above, wherein a heavy chain constant region is a heavy chain constant region of human IgG1 and comprises a mutation that causes reduced ADCC and ADCP activities.
(7) The antibody according to (6) above, wherein the heavy chain constant region is a chain constant region of human IgG1 heavy, and leucine at position 234 and leucine at position 235 specified by EU index numbering are each substituted with alanine.
(8) The antibody according to (7) above, wherein the antibody is a humanized monoclonal antibody comprising a heavy chain consisting of the amino acid sequence set forth in SEQ ID NO: 10 and a light chain consisting of the amino acid sequence set forth in SEQ ID NO: 9.
(9) The antibody according to (7) above, wherein the antibody is a humanized monoclonal antibody comprising a heavy chain consisting of the amino acid sequence set forth in SEQ ID NO: 12 and a light chain consisting of the amino acid sequence set forth in SEQ ID NO: 11.
(10) The antibody according to any one of (1) to (3), (6) or (7), wherein the antibody is a humanized monoclonal antibody comprising a light chain comprising CDRL1 consisting of the amino acid sequence set forth in SEQ ID NO: 37, CDRL2 consisting of the amino acid sequence set forth in SEQ ID NO: 38, and CDRL3 consisting of the amino acid sequence set forth in SEQ ID NO: 39, and a heavy chain comprising CDRH1 consisting of the amino acid sequence set forth in SEQ ID NO: 40, CDRH2 consisting of the amino acid sequence set forth in SEQ ID NO: 41, and CDRH3 consisting of the amino acid sequence set forth in SEQ ID NO: 42.
(11) The antibody according to any one of (1) to (3), (6) or (7), wherein the antibody is a humanized monoclonal antibody comprising a light chain comprising CDRL1 consisting of the amino acid sequence set forth in SEQ ID NO: 43, CDRL2 consisting of the amino acid sequence set forth in SEQ ID NO: 44, and CDRL3 consisting of the amino acid sequence set forth in SEQ ID NO: 45, and a heavy chain comprising CDRH1 consisting of the amino acid sequence set forth in SEQ ID NO: 46, CDRH2 consisting of the amino acid sequence set forth in SEQ ID NO: 47, and CDRH3 consisting of the amino acid sequence set forth in SEQ ID NO: 48.
(12) The antibody according to any one of (1) to (11) above, wherein the antibody has one or two amino acids deleted at a heavy chain carboxyl end.
(13) An antibody obtained by a method for producing an antibody comprising: a step of culturing a host cell transformed with an expression vector containing a polynucleotide encoding the antibody according to any one of (1) to (12) above; and a step of collecting a target antibody from a culture obtained in the above step.

The anti-CDH6 antibody used in the production of an antibody-immunostimulant conjugate of the present invention is not particularly limited, and desirably has the following properties, for example.
(1) An anti-CDH6 antibody that specifically binds to CDH6.
(2) The antibody according to (1) above, wherein the antibody binds to an extracellular domain of CDH6.
(3) The antibody according to (1) or (2) above, wherein the antibody is a monoclonal antibody.
(4) The antibody according to any one of (1) to (3) above, wherein the antibody has antibody-dependent cellular cytotoxicity (ADCC) activity and/or complement-dependent cytotoxicity (CDC) activity.
(5) The antibody according to any one of (1) to (4) above, wherein the antibody is a mouse monoclonal antibody, a chimeric monoclonal antibody, or a humanized monoclonal antibody.
(6) The antibody according to (1) to (3) above, wherein a heavy chain constant region is a heavy chain constant region of human IgG1 and comprises a mutation that causes reduced ADCC and ADCP activities.
(7) The antibody according to (6) above, wherein the heavy chain constant region is a heavy chain constant region of human IgG1, and leucine at position 234 and leucine at position 235 specified by EU index numbering are each substituted with alanine.
(8) The antibody according to (7) above, wherein the antibody is a humanized monoclonal antibody comprising a heavy chain consisting of the amino acid sequence set forth in SEQ ID NO: 56 and a light chain consisting of the amino acid sequence set forth in SEQ ID NO: 55.
(9) The antibody according to any one of (1) to (3), (6) or (7), wherein the antibody is a humanized monoclonal antibody comprising a light chain comprising CDRL1 consisting of the amino acid sequence set forth in SEQ ID NO: 63, CDRL2 consisting of the amino acid sequence set forth in SEQ ID NO: 64, and CDRL3 consisting of the amino acid sequence set forth in SEQ ID NO: 65, and a heavy chain comprising CDRH1 consisting of the amino acid sequence set forth in SEQ ID NO: 66, CDRH2 consisting of the amino acid sequence set forth in SEQ ID NO: 67, and CDRH3 consisting of the amino acid sequence set forth in SEQ ID NO: 68.
(10) The antibody according to any one of (1) to (9) above, wherein the antibody has one or two amino acids deleted at a heavy chain carboxyl end.
(11) An antibody obtained by a method for producing an antibody comprising: a step of culturing a host cell transformed with an expression vector containing a polynucleotide encoding the antibody according to any one of (1) to (10) above; and a step of collecting a target antibody from a culture obtained in the above step.

The acceptor molecule can be appropriately purchased as a commercial product or prepared by using or applying a known method, and for example, can be prepared by using or applying the method shown in Example 1. The acceptor molecule can typically be prepared by treating an N297-linked glycan of an Fc-containing molecule (typically, an Fc-containing molecule such as an IgG type monoclonal antibody or an Fc fragment or CLCH consisting only of constant regions of the antibody) to be subjected to glycan engineering (also referred to as glycan remodeling) with an ENGase that retains the activity of specifically hydrolyzing the 1,4-glycoside linkage between GlcNAc molecules (GlcNAcβ1-4GlcNAc) in the core chitobiose structure of the N297-linked glycan. In this case, as the ENGase, various ones such as Endo-Si WT, Endo-A, Endo-D, Endo-E, Endo-F3, Endo-H, Endo-S, Endo-S2, or Endo-Si can be used. Therefore, in one aspect of the present invention, the acceptor molecule can be provided in a step independent of the transglycosylation reaction in Step 1, by a method such as reacting the same or a different enzyme from Enzyme-A (enzyme having the activity of transferring a glycan in a glycan donor molecule to an acceptor molecule) used in Step 1 with an Fc-containing molecule (an Fc-containing molecule to be subjected to glycan engineering, typically an Fc-containing molecule having a host cell-derived N297-linked glycan, a heterogeneous N297-linked glycan, or a host cell-derived heterogeneous N297-linked glycan) as a raw material. In addition, a characteristic of Enzyme-A that can be used in Step 1 is "optionally having residual hydrolysis activity." Because of this, the acceptor molecule may be prepared in situ by allowing Enzyme-A to act on an Fc-containing molecule having a host cell-derived heterogeneous N297-linked glycan, instead of prior treatment with such an ENGase. Therefore, in one aspect of the present invention, the acceptor molecule is prepared in situ and/or in one pot by allowing Enzyme-A to act on an Fc-containing molecule (an Fc-containing molecule to be subjected to glycan engineering, typically an Fc-containing molecule having a host cell-derived N297-linked glycan, a heterogeneous N297-linked glycan, or a host cell-derived heterogeneous N297-linked glycan) as a raw material. In addition, IgG or the Fc-containing molecule used for glycan engineering (glycan remodeling) may be preferably one that is derived from an IgG heavy chain consisting of the same amino acid sequence and is produced in such a way as to have an N297-linked glycan. The method for producing the same is not limited, and IgG produced by a commonly known method for producing a monoclonal antibody, CLCH of the IgG, an Fc fragment obtained by enzymatic treatment thereof, or the like can be used. In addition, as such IgG or an Fc fragment, a mixture of samples obtained by different production methods or from different lots may be adopted.

### <Endo-β-N-acetylglucosaminidase>

The "endo-β-N-acetylglucosaminidase" is a glycohydrolase that is also known as ENGase, and is responsible for cleaving a chitobiose structure in a glycan structure and forming a linkage. In addition, the endo-β-N-acetylglucosaminidase is called differently depending on the origin thereof. Herein, an endo-β-N-acetylglucosaminidase, which prefers, as a substrate, an N297-linked glycan of an Fc-containing molecule, is referred to as "Enzyme-A," and an endo-β-N-acetylglucosaminidase, which prefers, as a substrate, a glycan of a glycan donor molecule, such as a glycan of a glycan raw material free of fucose (for example, SGP) is referred to as "Enzyme-B." Hereinafter, Enzyme-A and Enzyme-B in the present invention will be described.

### <Enzyme-A>

"Enzyme-A" means an endo-β-N-acetylglucosaminidase, which prefers, as a substrate, an N297-linked glycan of an Fc-containing molecule (for example, an antibody). Enzyme-A generally includes a domain having high affinity for an Fc-containing molecule, a domain having high affinity for a glycan, and a glycosylation reaction catalytic domain, and typically prefers, as a substrate, an N297-linked glycan of an Fc-containing molecule (for example, an antibody) and has both hydrolysis activity and transglycosylation activity, and the hydrolysis activity may be attenuated or eliminated, because the activity of transferring a glycan (particularly a glycan activated by a chemical method or an enzymological method) in a glycan donor molecule to an acceptor molecule is important in the present invention. Rather, an enzyme that retains strong hydrolysis activity can hydrolyze, as a substrate, even the glycan transferred to the core GlcNAc of the acceptor molecule by the transglycosylation activity, and thus the hydrolysis activity of Enzyme-A in the present invention is preferably relatively reduced. As described above, when Enzyme-A has residual hydrolysis activity, this can benefit from the advantage that an acceptor molecule can be prepared in situ in the same reaction tank in Step 1 without separately preparing the acceptor molecule in Step 1. Therefore, in one aspect of the present invention, Enzyme-A has activity that can act on a glycan of an Fc-containing molecule (particularly transglycosylation activity that prefers, as a substrate, an N297-linked glycan of an Fc-containing molecule), and in another aspect of the present invention, Enzyme-A has activity that can act on a glycan of an Fc-containing molecule, and has residual hydrolysis activity or has the hydrolysis activity eliminated.

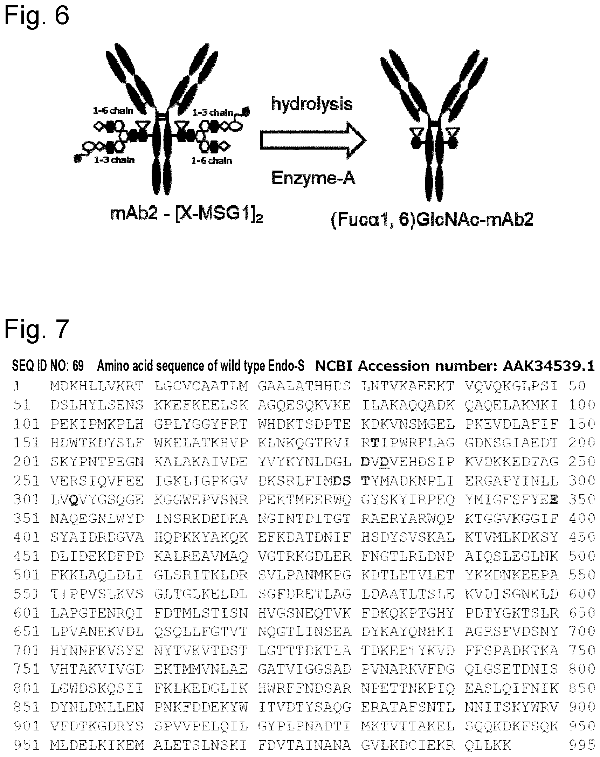

The hydrolysis activity of Enzyme-A means the activity of specifically hydrolyzing a β1,4-glycoside linkage included in core chitobiose in the common basic structure of a glycan, and particularly the activity of specifically hydrolyzing a β1,4-glycoside linkage included in core chitobiose in the common basic structure of an N297-linked glycan of an Fc-containing molecule. A reaction schematic diagrams of the hydrolysis activity of Enzyme-A are shown in Figure 5 and Figure 6.

The transglycosylation activity of Enzyme-A means the activity of glycosidically linking a reducing end of the glycan donor molecule (a glycan-containing molecule having a GlcNAc having an activated reducing end or a GlcNAc having an unactivated reducing end) to an acceptor molecule including an Fc site having only a core GlcNAc (to which core fucose may or may not be added) at N297. A reaction schematic diagram of the glycosylation activity of Enzyme-A when the glycan donor molecule includes a GlcNAc having an unactivated reducing end is shown in Figure 1.

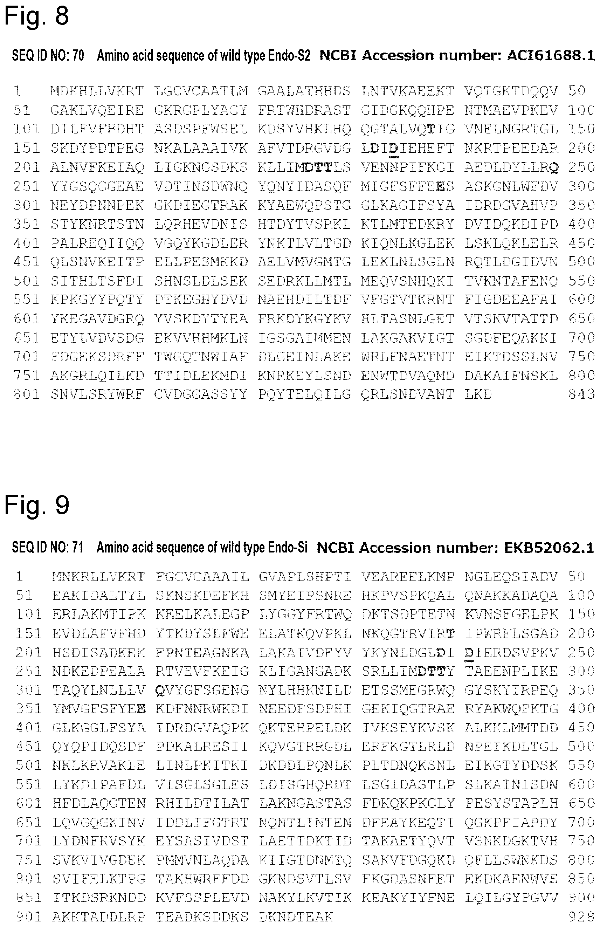

Examples of Enzyme-A in the present invention include Endo-S (*Streptococcus pyogenes*-derived enzyme) (see Collin M and Olsen A., EMBO J. 2001, 20, 3046-3055, and Goodfellow JJ, et al., J Am Chem Soc. 2012, 134, 8030-8033), EndoS2 or EndoS49 (see Sjogren J, et al., Biochem J. 2013, 455, 107-118, and Shivatare SS, et al., Chem Commun (Camb). 2018, 54, 6161-6164), Endo-F3 (Flavobacterium meningosepticum-derived enzyme) (see Huang W, et al., Chembiochem. 2011, 12, 932-941, and Giddens JP, et al., J Biol Chem. 2016, 291, 9356-9370), or mutant enzymes thereof. In addition, examples of Enzyme-A in the present invention include an endo-β-N-acetylglucosaminidase derived from *Streptococcus iniae* (see Pier GB and Madin SH., Int J Syst Bacteriol. 1976 26, 545-553) (hereinafter referred to as "Endo-Si"; the amino acid sequence thereof (SEQ ID NO: 71) is shown in Figure 9),or a mutant enzyme thereof. Furthermore, examples of Enzyme-A in the present invention include an enzyme whose sequence is specified from the genome information of the genus *Storeptococcus* (see Vincent P. Richard, et al., Genome Biol. Evol. 2014, 6, 741-753), or a mutant enzyme thereof.

Enzyme-A of the present invention is not limited to the specific enzymes used in the Examples as long as it has the above properties, and may be an enzyme isolated from a naturally occurring source or may be an enzyme artificially prepared or engineered based on the sequence information of an enzyme of the present invention. In the case of isolation from a naturally occurring source, the biological species that is used as the isolation source is not particularly limited, and is preferably a bacterium.

The active domain and the carbohydrate-binding module (CBM) of Endo-Si are expected to be located in the regions of amino acid numbers 106 to 447 and amino acid numbers 762 to 897, respectively, of SEQ ID NO: 71 from a sequence comparison with EndoS whose crystal structure has been analyzed (B. Trastoy et al., PNAS (2014) vol 111, No. 18, pp6714-6719), and these two are considered to be important sites for the interaction between hydrolysis activity and/or transfer activity and an antibody. Therefore, examples of Enzyme-A of the present invention include a polypeptide that includes the amino acid sequences set forth in amino acid numbers 106 to 447 and/or amino acid numbers 762 to 897 of SEQ ID NO: 71, preferably the amino acid sequence set forth in amino acid numbers 106 to 897 of SEQ ID NO: 71, more preferably the amino acid sequence set forth in amino acid numbers 106 to 928 of SEQ ID NO: 71, and further preferably the amino acid sequence set forth in amino acid numbers 34 to 928 of SEQ ID NO: 71, and exhibits transglycosylation activity.

As described above, the endo-β-N-acetylglucosaminidase usually has both hydrolysis activity and transglycosylation activity, and in some cases, an enzyme retaining strong hydrolysis activity hydrolyzes, as a substrate, even the glycan transferred to a core GlcNAc of an acceptor molecule (an antibody having, as an N297-linked glycan, a core GlcNAc, or a molecule containing an Fc region thereof) through the glycosylation activity, making it impossible to appropriately obtain the desired transglycosylated form. Because of this, in synthesizing a glycan-remodeled antibody or a glycan-modified compound, a mutant enzyme having relatively reduced hydrolysis activity as compared with transglycosylation activity is useful. Therefore, in one aspect of the present invention, Enzyme-A is a mutant enzyme of Endo-S, EndoS-2, Endo-Si, Endo-Sd, Endo-Se, or Endo-Sz, the mutant enzyme having lower hydrolysis activity than that of the corresponding wild type enzyme thereof, and preferably a mutant enzyme in which the amino acid residues indicated in bold in Figure 7 to Figure 9 are appropriately substituted, and particularly preferable examples thereof include, but are not limited to, Endo-S D233Q, Endo-S D233Q/Q303L, Endo-S D233Q/E350A, Endo-S D233Q/E350Q, Endo-S D233Q/E350D, Endo-S D233Q/E350N, Endo-S D233Q/D405A, Endo-Si D241Q, Endo-Si D241Q/Q311L, Endo-Si D241Q/E360Q, Endo-Si D241M, Endo-Si D241M/Q311L, Endo-Si D241M/E360Q, Endo-Si T190Q, Endo-Si T190/D241Q, Endo-Si T190Q/D241M, Endo-Si D241X₁ wherein X₁ represents any amino acid residue other than K, R, and D, specifically A, N, C, Q, E, G, H, I, L, M, F, P, S, T, W, Y, or V, and preferably A, Q, E, H, I, L, M, F, P, S, T, W, Y, or V, Endo-Si T190X₂ wherein X₂ represents an amino acid residue of F, H, K, M, Q, R, W, or Y, Endo-Si Q311X₃ wherein X₃ represents an amino acid residue of F, N, or Y, Endo-Si E360K, Endo-S2 D184M, Endo-S2 T138Q, Endo-S2 D184Q, Endo-S2 D184Q/Q250L, Endo-S2 D184Q/E289Q, Endo-S2 D184M/Q250L, Endo-S2 D184M/E289Q, Endo-S2 D182Q, Endo-S2 D226Q, Endo-S2 T227Q, Endo-S2 T228Q, Endo-Sd D232Q, Endo-Sd D232M, Endo-Sd D232Q/Q302L, Endo-Sd D232M/Q302L, Endo-Se D233Q, Endo-Se D233M, Endo-Se D233Q/Q303L, Endo-Se D233M/Q303L, Endo-Sz D234Q, Endo-Sz D234M, Endo-Sz D234Q/Q304L, or Endo-Sz D234M/Q304L. In addition, in addition to the specific mutations described above, a further mutation may be added to Enzyme-A, and for example, with reference to Patent Literature (International Publication No. WO 2022/050300) or the like, one to several amino acid residues may be substituted, deleted, inserted, and/or added as long as the present enzyme activities are not affected. In addition, with reference to Patent Literature (International Publication No. WO 2022/050300) or the like, examples of the amino acid sequence of Enzyme-A include an amino acid sequence having at least 80% or more, preferably 85% or more, more preferably 90% or more, further preferably 95%, 96%, 97%, 98%, or 99% or more homology or identity to the amino acid residues other than a specific mutant amino acid residue.

As used herein, "several" means an integer of 30 or 20 or less, preferably an integer of 10 or less, further preferably an integer of 5 or less, and most preferably 4, 3, 2, or 1.

### <Enzyme-B>

"Enzyme-B" means an endo-β-N-acetylglucosaminidase, which prefers, as a substrate, a glycan of a glycan donor molecule, particularly an endo-β-N-acetylglucosaminidase, which prefers, as a substrate, a glycan of a glycan donor molecule, but does not prefer, as a substrate, an N297-linked glycan (in other words, has low reactivity, preferably extremely low reactivity, with an N297-linked glycan). The glycan moiety on the glycan donor molecule that is preferred, as a substrate, by Enzyme-B may be any of a high mannose type glycan, a hybrid type glycan, and a complex type glycan, and is preferably a high mannose type glycan or a complex type glycan, and particularly preferably a complex type glycan. Therefore, in one aspect of the present invention, the glycan moiety on the glycan donor molecule that is preferred, as a substrate, by Enzyme-B is a high mannose type glycan or a complex type glycan, and preferably a complex type glycan. In addition, in the present invention, the complex type glycan may be any of a biantennary type, a triantennary type, or a tetraantennary type, and the glycan moiety on the glycan donor molecule that is preferred, as a substrate, by Enzyme-B is preferably a biantennary complex type glycan. In one aspect of the present invention, Enzyme-B is an endo-β-N-acetylglucosaminidase, which prefers, as a substrate, a fucose-free glycan raw material (a glycopeptide or a glycoprotein such as SGP), and preferably an endo-β-N-acetylglucosaminidase, which prefers, as a substrate, a fucose-free glycan raw material including a biantennary complex type glycan.

Enzyme-B of the present invention is preferably an enzyme that has the property of acting on a glycan donor molecule including a glycan including a GlcNAc having an unactivated reducing end in the presence of Enzyme-A to generate an activated intermediate in which the reducing end can be used for a transglycosylation reaction, and as a result, promoting the transglycosylation reaction of a glycan derived from a glycan donor molecule by Enzyme-A to an acceptor molecule. Here, the transglycosylation activity of Enzyme-B itself is considered to be correlated with the ability thereof to activate a glycan donor molecule, and thus in one aspect of the present invention, Enzyme-B can be selected from an endo-β-N-acetylglucosaminidase, which prefers, as a substrate, a glycan of a glycan donor molecule (for example, SGP) including a GlcNAc having an unactivated reducing end, but does not prefer, as a substrate, an N297-linked glycan, by using transglycosylation activity to an acceptor having a GlcNAc (for example, a GlnNAc derivative such as 1-methylethyl-2-(acetylamino)-2-deoxy-β-D-glucopyranoside or 2-(acetylamino)-2-deoxy-β-D-glucopyranoside) as an indicator. Therefore, in one aspect of the present invention, Enzyme-B is an enzyme having an activity of transglycosylation from SGP to a GlcNAc derivative.

As shown in Figure 1, the glycan donor molecule activating action of Enzyme-B is to act on a glycan donor molecule including a GlcNAc having an unactivated reducing end to generate an activated intermediate in which the reducing end can be used for a transglycosylation reaction, and when the hydrolysis activity of Enzyme-B is high, the activated intermediate may not react with an acceptor molecule but react with a H₂O molecule present in the reaction system, via Enzyme-A, to generate many free glycans. Therefore, Enzyme-B in the present invention preferably has relatively reduced hydrolysis activity as compared with the glycan-activating action.

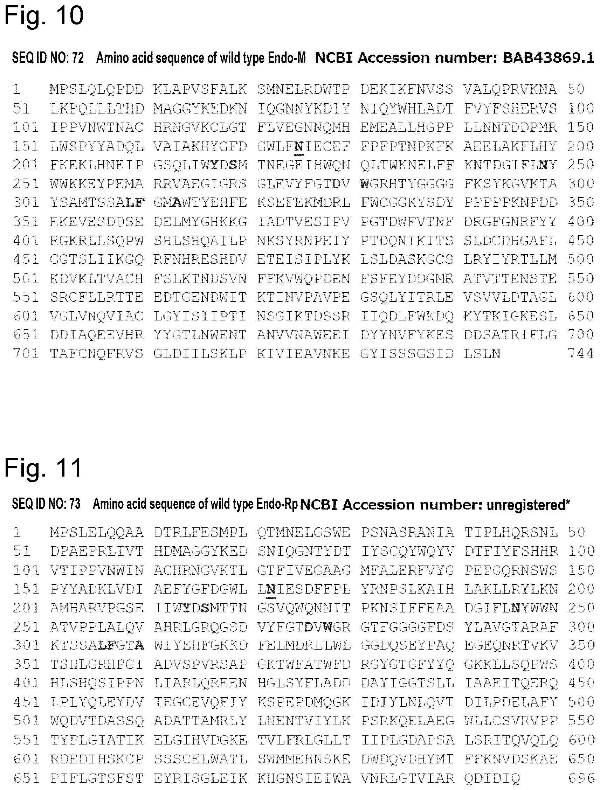

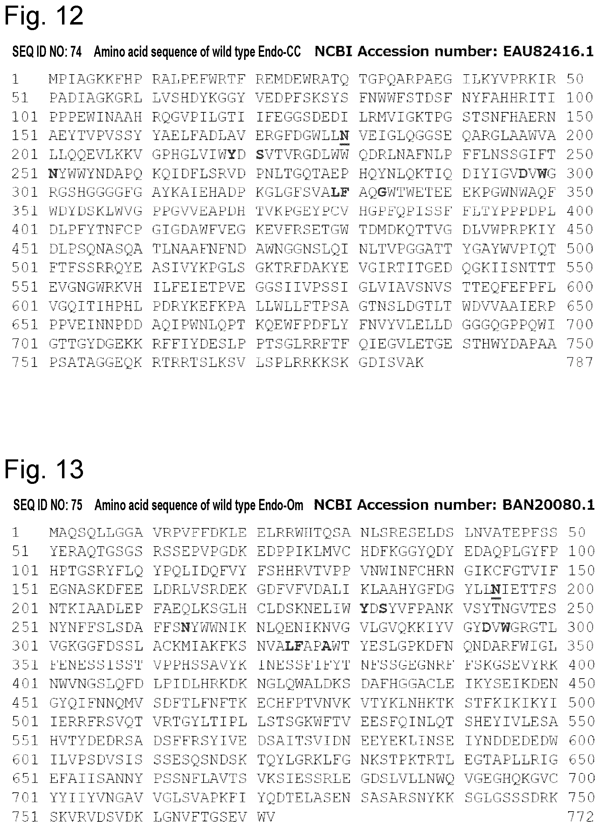

Examples of Enzyme-B of the present invention include Endo-M (Mucor hiemalis-derived enzyme) (see Yamamoto K, et al., Biochem Biophys Res Commun. 1994, 203, 244-252, and Umekawa M, et al., J. Biol Chem. 2021, 285, 511-521), Endo-CC (Coprinopsis cinerea-derived enzyme) (see Eshima Y, et al., PLoS One. 2015, 10, e0132859), Endo-Om (Ogataea minuta-derived enzyme) (see Murakami S, et al., Glycobiology. 2013, 23, 736-744), Endo-Rp (Rhizomucor pusillus-derived enzyme) (WO2018101454), and mutant enzymes thereof (preferably mutant enzymes having reduced hydrolysis activity as compared with the wild type). In one aspect of the present invention, Enzyme-B is a mutant enzyme of Endo-M, Endo-Om, Endo-CC, or Endo-Rp, the mutant enzyme having lower hydrolysis activity than that of the corresponding wild type enzyme thereof, and preferably a mutant enzyme in which the amino acid residues indicated in bold in Figure 10 to Figure 13 are appropriately substituted, and particularly preferable examples thereof include, but are not limited to, an enzyme selected from the group consisting of Endo-Rp N172Q, Endo-Rp N172H, Endo-Rp N172A, Endo-Rp N172C, Endo-Rp N172D, Endo-Rp N172E, Endo-Rp N172G, Endo-Rp N 172I, Endo-Rp N172L, Endo-Rp N172M, Endo-Rp N172P, Endo-Rp N172S, Endo-Rp N172T, Endo-Rp N172V, Endo-Rp W278F/S216V, Endo-Rp W278F/N246D, Endo-Rp W278F/D276N, Endo-Rp W278F/A310D, Endo-Rp W278F/N172D/F307Y, Endo-Rp W278F/N172D/F307H, Endo-Rp W278F/N172D/A310D, Endo-Rp W214F/F307Y/L306I, Endo-M N175Q, Endo-M N175Q/Y217F, Endo-CC N180H, and Endo-Om N194Q. In addition, in addition to the specific mutations described above, a further mutation may be added to Enzyme-B, and for example, with reference to Patent Literature (International Publication No. WO 2022/050300) or the like, one to several amino acid residues may be substituted, deleted, inserted, and/or added as long as the present enzyme activities are not affected. In addition, with reference to Patent Literature (International Publication No. WO 2022/050300) or the like, examples of the amino acid sequence of Enzyme-B include an amino acid sequence having at least 80% or more, preferably 85% or more, more preferably 90% or more, further preferably 95%, 96%, 97%, 98%, or 99% or more homology or identity to the amino acid residues other than a specific mutant amino acid residue.

In a typical embodiment of the production method according to the present invention, Enzyme-A and Enzyme-B are each added as a separate molecule to Step 1, and Enzyme-A and Enzyme-B may be added to the reaction system in a directly or indirectly bound state as long as Enzyme-A and Enzyme-B play their respective desired roles. Therefore, in one aspect of the present invention, Enzyme-A and Enzyme-B may be added to the reaction system in a directly or indirectly chemically bound state, and for example, both molecules may be added to the reaction system in the form of an immobilized enzyme immobilized on any solid phase carrier, or a fusion protein bound with any amino acid, a polymer such as PEG, an oligomer linker, or the like. In other words, in one aspect of the present invention, Enzyme-A and Enzyme-B are directly or indirectly chemically bound (= Enzyme-A and Enzyme-B are fused), and for example, can be added to the reaction system as an immobilized enzyme immobilized on any solid phase carrier, or a fusion protein bound with an arbitrary amino acid, a polymer such as PEG, an oligomer linker, or the like, preferably an amino acid linker. In the present invention, even if Enzyme-A and Enzyme-B are functional fragments rather than full-length molecules without a deletion, the fragments are also regarded as one aspect of Enzyme-A and Enzyme-B as long as the functional fragments have each the desired function. Therefore, Enzyme-A and Enzyme-B in the present invention also include, for example, a state in which "Enzyme-A" and "Enzyme-B," "an enzyme fragment having the function of Enzyme-A" and "an enzyme fragment having the function of Enzyme-B," "Enzyme-A" and "an enzyme fragment having the function of Enzyme-B," and "an enzyme fragment having the function of Enzyme-A" and "Enzyme-B" are directly or indirectly chemically bound. Examples of the "enzyme fragment having the function of Enzyme-A" include a fragment having an amino acid sequence that is a specific functional sequence fragmented from the full-length sequence of Enzyme-A, or Endo-BI1 (GenBank Accession number: ACJ53522.1), Endo-BI2 (GenBank Accession number: BAJ71450.1), Endo-BT-3987 (GenBank Accession number: AAO79092.1), Endo-E (GenBank Accession number: AAR20477.1), Endo-F (GenBank Accession number: AAA24922.1), Endo-F2 (GenBank Accession number: AAA24923.1), Endo-F3 (GenBank Accession number: AAA24924.1), Endo-CoM (GenBank Accession number: XP006673222.1), or Endo-SB (GenBank Accession number: BBB35949.1), and that may have a specific mutation added thereto. In addition, the "enzyme fragment having the function of Enzyme-B" include a fragment having an amino acid sequence that is a specific functional sequence fragmented from the full-length sequence of the catalytic activity domain of Enzyme-B described above, or Endo-A (GenBank Accession number: AAD10851.1), Endo-CE (GenBank Accession number: BAB84821.1), Endo-Tsp1006 (GenBank Accession number: CCY37287.1), Endo-Tsp1263 (GenBank Accession number: CDD88945.1), Endo-Tsp1457 (GenBank Accession number: CDD89351.1), Endo-BB (GenBank Accession number: AAN25135.1), Endo-BH (GenBank Accession number: BAB04504.1), Endo-BN (GenBank Accession number: WP_007484749.1), Endo-CC1 (GenBank Accession number: XP_001839402.1), Endo-CC2 (GenBank Accession number: XP_002911817.1), Endo-D (GenBank Accession number: AAK74656.1), or Endo-Pm (GenBank Accession number: ADK97032.1), and that may have a specific mutation added thereto. For example, in the functional sequence of each of Enzyme-A and Enzyme-B, with reference to patent literature (International Publication No. WO 2022/050300) or the like, one to several amino acid residues may be substituted, deleted, inserted, and/or added as long as the above function is not affected, and with reference to Patent Literature (International Publication No. WO 2022/050300) or the like, examples of each amino acid sequence include an amino acid sequence having at least 80% or more, preferably 85% or more, more preferably 90% or more, further preferably 95%, 96%, 97%, 98%, or 99% or more homology or identity to the amino acid residues other than a specific amino acid residue affecting the activity.

Any combination of Enzyme-A and Enzyme-B can be used as long as Enzyme-A and Enzyme-B play their respective roles. For example, any combination of Enzyme-A and Enzyme-B shown in the following table can be used.

| | **Enzyme-A** | | **Enzyme-B** |
|---|---|---|---|
| A-1 | Endo-Si D241M Q311L | B-1 | Endo-Rp N172H |
| A-2 | Endo-Si D241Q | B-2 | Endo-Rp N172Q |
| A-3 | Endo-Si D241Q E360Q | B-3 | Endo-Rp N172V |
| A-4 | Endo-Si D241M | B-4 | Endo-M N175Q |
| A-5 | Endo-Si D241Q Q311L | B-5 | Endo-M N175Q Y217F |
| A-6 | Endo-Si D241M E360Q | | |
| A-7 | Endo-Si WT | | |
| A-8 | Endo-S D233Q E350Q | | |
| A-9 | Endo-S WT | | |
| A-10 | Endo-S2 D184M | | |
| A-11 | Endo-S2 T138Q | | |
| A-12 | Endo-S2 WT | | |

| | **Enzyme-A** | | **Enzyme-A** |
|---|---|---|---|
| A-13 | Endo-Si T190F | A-26 | Endo-Si D241L |
| A-14 | Endo-Si T190H | A-27 | Endo-Si D241P |
| A-15 | Endo-Si T190K | A-28 | Endo-Si D241R |
| A-16 | Endo-Si 1190M | A-29 | Endo-Si D241S |
| A-17 | Endo-Si T190Q | A-30 | Endo-Si D241T |
| A-18 | Endo-Si T190R | A-31 | Endo-Si D241V |
| A-19 | Endo-Si T190W | A-32 | Endo-Si D241W |
| A-20 | Endo-Si T190Y | A-33 | Endo-Si D241Y |
| A-21 | Endo-Si D241A | A-34 | Endo-Si Q311F |
| A-22 | Endo-Si D241E | A-35 | Endo-Si Q311K |
| A-23 | Endo-Si D241F | A-36 | Endo-Si Q311N |
| A-24 | Endo-Si D241H | A-37 | Endo-Si Q311Y |
| A-25 | Endo-Si D241I | A-38 | Endo-Si E360K |

| | **Enzyme-A** |
|---|---|
| A-39 | Endo-S2 D184M/Q250L |
| A-40 | Endo-S2 D184Q/Q250L |
| A-41 | Endo-Sd WT |
| A-42 | Endo-Se WT |
| A-43 | Endo-Se D233M |
| A-44 | Endo-Sz WT |
| A-45 | Endo-Sz D234M |
| A-46 | Endo-Sz D234M/Q304L |

In addition, as described above, when Enzyme-A and Enzyme-B are fused, any combination of Enzyme-A and Enzyme-B can be used as long as Enzyme-A and Enzyme-B play their respective roles. More specifically, for example, Enzyme-A is an Endo-Si mutant and Enzyme-B is an Endo-Rp mutant, and for example, Enzyme-A is Endo-Si D241M/Q311L or Endo-Si D241Q/Q311L and Enzyme-B is Endo-Rp N172H or Endo-Rp N172V. In yet another specific aspect, the fusion enzyme is a fusion protein of Endo-Rp N172H and Endo-Si D241M/Q311L, a fusion protein of Endo-Rp N172V and Endo-Si D241Q/Q311L, or Endo-Si D241Q/Q311L and Endo-Rp N172V, and examples thereof include the [Enzyme-A]-[Enzyme-B] fusion protein enzyme and the [Enzyme-B]-[Enzyme-A] fusion protein enzyme shown in the following table.

| | **[Enzyme-A]-[Enzyme-B] fusion protein enzyme or [Enzyme-B]-[Enzyme-A] fusion protein enzyme** |
|---|---|
| BA-1 | [Endo-Rp N172H]-[Endo-Si D241M/Q311L] |
| BA-2 | [Endo-Rp N172V]-[Endo-Si D241Q/Q311L] |

A fusion protein of Enzyme-A and Enzyme-B can be produced according to a conventional method with reference to Non Patent Literature (Joaquim, et al., Processes. 2022, 10, 494.) or the like, and for example, can be produced by binding Enzyme-A and Enzyme-B to any solid phase carrier via a physical or chemical interaction. Alternatively, with reference to Non Patent Literature (F Grueninger-Leitch, et al., Protein Sci. 1996, 12, 2617-22, and Emily MK, et al., Protein Eng Des Sel. 2005, 10, 497-501), Patent Literature (WO2017137459), and the like, a host cell can be genetically engineered to produce the fusion protein. That is, after respective genes encoding Enzyme-A and Enzyme-B are bound, a host cell can be genetically engineered to produce a single enzyme having respective functions derived from Enzyme-A and Enzyme-B. When a host cell is genetically engineered to produce a fusion enzyme as described above, there is no need to separately provide Enzyme-A and Enzyme-B, and the number of enzymes to be provided can be reduced, and thus the enzyme production cost and effort can be reduced. When Enzyme-A and Enzyme-B are bound via a linker consisting of an amino acid, in one aspect, the linker is composed of one or more amino acids, and with reference to Non Patent Literature (Chen, et al., Adv Drug Deliv Rev. 2013 October 15; 65(10): 1357-1369.) or the like, one having a typical property known in the art can be used, a flexible or rigid linker, a linker cleavable or noncleavable in nature may be used, and a long or short linker can be used. In one aspect, the linker can include (SGGS)n, (GGGS)n, (GGGGS)n, (G)n, (EAAAK)n, (EF)n, or (GGS)n, or a combination thereof, as an amino acid sequence fragment wherein n is an integer of 1 to 30, preferably an integer of 1 to 20, further preferably an integer of 1 to 10, and more preferably an integer of 1 to 6. In addition, the linker can include an amino acid sequence commonly used as a purification tag, such as a polyhistidine tag, an HA tag, a FLAG tag, a CBD tag, an Fc tag, or a GST tag. In one aspect, the purification tag is a polyhistidine tag, an HA tag, a FLAG tag, an Fc tag, or a GST tag, preferably a polyhistidine tag, an HA tag, a FLAG tag, or a CBD tag, further preferably a polyhistidine tag or a FLAG tag, and more preferably a polyhistidine tag.

### <Step 1>

In the production method according to the present invention, Step 1 is reacting an acceptor molecule, which is an Fc-containing molecule having, as an N297-linked glycan, a core GlcNAc to which fucose is optionally added, with a glycan donor molecule including a glycan in the presence of an endo-β-N-acetylglucosaminidase (Enzyme-A), which prefers, as a substrate, an N297-linked glycan of an Fc-containing molecule, so as to obtain a reaction mixture. In one aspect of the present invention, in Step 1, the acceptor molecule is reacted with the glycan donor molecule in the presence of Enzyme-A and an endo-β-N-acetylglucosaminidase (Enzyme-B), which prefers, as a substrate, a glycan of a glycan donor molecule, so as to obtain a reaction mixture. The reaction conditions for Step 1 can be appropriately selected according to the reaction conditions for a known transglycosylation reaction using a GlcNAc having an activated reducing end (for example, an oxazolinated GlcNAc) or a GlcNAc having an unactivated reducing end as a glycan donor molecule, or by using or applying reaction conditions for a known transglycosylation reaction (for example, Patent Literature 3).

The reaction in step 1 is preferably carried out in a buffer and desirably carried out under a condition that does not promote the degradation of a glycan donor molecule including a GlcNAc having an activated reducing end or having an unactivated reducing end. From this viewpoint, in one aspect of the present invention, when the glycan donor molecule includes a GlcNAc having an unactivated reducing end, the pH in the reaction system in Step 1 can be appropriately selected between 5.8 and 9.5, and is in the range of preferably 6.2 to 8.5, more preferably 6.5 to 8.0, and further preferably 6.5 to 7.5. In addition, when the glycan donor molecule includes a GlcNAc having an activated reducing end, the pH in the reaction system in Step 1 can be appropriately selected between 5.5 and 8.0, and is in the range of preferably 6.0 to 7.5, more preferably 6.2 to 7.5, and further preferably 6.5 to 7.5. The buffer can be appropriately selected from phosphate buffer (pH 6.0 to 7.5), MOPS-NaOH buffer (pH 6.5 to 8.0), Tris-HCl buffer (pH 7.0 to 9.0), and the like, and is preferably Tris-HCl buffer (pH 7.0 to 9.0). An additive that does not inhibit the enzyme reaction may be added to the reaction solution for the purpose of stabilizing an enzyme.

The reaction temperature can be appropriately selected between 4°C and 50°C, and is preferably 15°C to 45°C, more preferably 20°C to 40°C, and further preferably 25°C to 40°C.

The reaction time can be appropriately selected between 10 minutes and 96 hours, and is preferably 0.5 hours to 80 hours, more preferably 2 hours to 70 hours, further preferably 4 hours to 60 hours, particularly preferably is 6 to 48 hours, and most preferably 8 to 30 hours. A small amount of the reaction solution may be collected over time to determine the completion of the reaction while confirming the degree of progress of the transglycosylation reaction. In general, the degree of progress of the transglycosylation reaction can be monitored by sodium dodecyl sulfate-polyacrylamide gel electrophoresis (SDS-PAGE), capillary electrophoresis, microchip electrophoresis, a fully automated electrophoresis system, liquid chromatography mass spectrometry (LC-MS), or the like, and for example, can be monitored by fragmenting a glycan-remodeled antibody into a heavy chain and a light chain and then using a fully automated electrophoresis system to confirm that the retention time changes only on the heavy chain side to which an N297-linked glycan is added.

When the glycan donor molecule includes a GlcNAc having an unactivated reducing end, Step 1 can be carried out as a one-pot method involving directly transferring a glycan of the glycan donor molecule to an antibody having, as an N297-linked glycan, a core GlcNAc to which fucose is optionally added or a molecule containing an Fc region thereof, which is an acceptor molecule. In this aspect, Step 1 requires Enzyme-A as well as Enzyme-B as described above.

As will be described in detail in Example 8, in glycan remodeling using a glycan donor molecule including a glycan having an oxazolinated reducing end, glycation, which is a non-Endo-enzyme-mediated chemical reaction, is likely to proceed on Lys in an acceptor molecule, and thus it is common practice to set the antibody concentration in the reaction solution low. Therefore, in the production method according to the present invention, when the glycan donor molecule includes a GlcNAc having an activated reducing end, it is preferable to adopt a relatively low acceptor molecule concentration in Step 1. The acceptor molecule concentration in such an embodiment can be appropriately set according to a known method, and for example, the final acceptor molecule concentration in the reaction system in Step 1 is 0.6 mg/mL to 18 mg/mL, preferably 1.2 mg/mL to 16 mg/mL, more preferably 3 mg/mL to 14 mg/mL, further preferably 4.2 mg/mL to 13.2 mg/mL, and particularly preferably 6 mg/mL to 12 mg/mL.

On the other hand, when a glycan donor molecule including a GlnNAc having an unactivated reducing end is used, there is no need to worry about glycation because a chemically synthesized oxazoline form solution, which causes glycation, is not added, and thus a relatively high acceptor molecule concentration can be adopted. Rather, it is effective to add a high concentration of an acceptor molecule from the viewpoint of increasing the frequency of intermolecular contact. Therefore, when the glycan donor molecule includes a GlcNAc having an unactivated reducing end, the acceptor molecule concentration in Step 1 can be appropriately set according to a known method, and for example, the upper limit value of the final acceptor molecule concentration in the reaction system in Step 1 is 200 mg/mL, preferably 160 mg/mL, more preferably 120 mg/mL, further preferably 100 mg/mL, and particularly preferably 80 mg/mL, and the lower limit value thereof is 10 mg/mL, preferably 14 mg/mL, more preferably 18 mg/mL, and further preferably 20 mg/mL, and the concentration range is a combination of any of the upper limit values and any of the lower limit values described above, or is 10 mg/mL to 200 mg/mL, preferably 14 mg/mL to 160 mg/mL, more preferably 18 mg/mL to 120 mg/mL, further preferably 20 mg/mL to 100 mg/mL, and particularly preferably 20 mg/mL to 80 mg/mL, but is not limited thereto.

The amount of the glycan donor molecule added in Step 1 can be appropriately set by using or applying a known method. In particular, in a glycan remodeling reaction using a glycan donor molecule including a GlnNAc having an unactivated reducing end, the amount of the glycan donor molecule added affects the transglycosylation rate, and thus it is conventional common practice to use a relatively large amount of the glycan donor molecule (100 to 300 equivalents, Patent Literature 1, Non Patent Literature 12). On the other hand, as described above, in the production method of the present invention, an Fc-containing molecule to which a glycan is added, which is a target product, and an unreacted Fc-containing molecule (acceptor molecule) can be separated by cation exchange chromatography under an acidic condition, and thus there is no need to extremely increase the efficiency of the transglycosylation reaction itself in Step 1, and therefore the amount of the glycan donor molecule added can be made relatively small. A glycan donor molecule is often expensive, and thus this advantage is particularly useful in a one-pot method, which requires a large amount of a glycan donor molecule used in order to obtain an Fc-containing molecule having a homogeneous glycan structure with high purity. Therefore, in one aspect of the present invention, examples of the lower limit value of the amount of the glycan donor molecule added (used) in Step 1 include, but are not limited to, 2 equivalents, 3 equivalents, 4 equivalents, 5 equivalents, 6 equivalents, 7 equivalents, 8 equivalents, 9 equivalents, 10 equivalents, 11 equivalents, 12 equivalents, 13 equivalents, 14 equivalents, 15 equivalents, 16 equivalents, 17 equivalents, 18 equivalents, 19 equivalents, and 20 equivalents, per equivalent of the acceptor molecule, and examples of the upper limit value of the amount of the glycan donor molecule include, but are not limited to, 300 equivalents, 200 equivalents, 150 equivalents, 100 equivalents, 90 equivalents, 80 equivalents, 75 equivalents, 70 equivalents, 65 equivalents, 60 equivalents, 55 equivalents, 45 equivalents, 40 equivalents, 35 equivalents, 30 equivalents, 25 equivalents, and 20 equivalents. In addition, the range of the amount of the glycan donor molecule added (used) in Step 1 is a combination of any of the upper limit values and any of the lower limit values described above, or is 5 to 80 equivalents, preferably 7 to 60 equivalents, more preferably 8 to 50 equivalents, and particularly preferably 10 to 40 equivalents per equivalent of the acceptor molecule, but is not limited thereto.

As described above, in the production method of the present invention, an Fc-containing molecule to which a glycan is added, which is a target product, and an unreacted Fc-containing molecule (acceptor molecule) can be separated by cation exchange chromatography under an acidic condition, and thus there is no need to extremely increase the efficiency of the transglycosylation reaction itself in Step 1. Therefore, the transglycosylation rate in Step 1 can be set to any numerical value, and for example, can be set to 50% or more, 55% or more, 60% or more, 65% or more, 70% or more, 75% or more, 80% or more, 85% or more, 90% or more, or 95% or more can be set, and on the other hand, from the viewpoint of no need to extremely increase the efficiency of the transglycosylation reaction itself, the transglycosylation rate can also be set to 95% or less, 90% or less, 85% or less, 80% or less, 75% or less, 70% or less, 65% or less, or 60% or less. In one aspect of the present invention, the transglycosylation rate in Step 1 is more than 80%, preferably more than 85%, and particularly preferably more than 90%. The above "transglycosylation rate" can be determined according to a conventional method, and for example, can be determined by using the fully automated electrophoresis system disclosed in Example 8. The timing of measuring the transglycosylation rate varies depending on the enzyme used, and is 1 minute or more and 100 hours or less, preferably 1 hour or more and 80 hours or less, and more preferably 2 hours or more and 72 hours or less.

The acceptor molecule used in Step 1 can be prepared by a separate preparation method independent of Step 1. Therefore, in one aspect of the present invention, the method of the present invention further includes, prior to Step 1, a step of separately preparing an acceptor molecule. In this aspect, the acceptor molecule can be provided, for example, in a step independent of the transglycosylation reaction in Step 1, by a method such as reacting the same or a different enzyme from Enzyme-A (an enzyme having the activity of transferring a glycan in a glycan donor molecule to an acceptor molecule) used in Step 1 with an Fc-containing molecule (an Fc-containing molecule to be subjected to glycan engineering, typically an Fc-containing molecule having a host cell-derived N297-linked glycan, a heterogeneous N297-linked glycan, or a host cell-derived heterogeneous N297-linked glycan) as a raw material. In one aspect of the present invention, the acceptor molecule can be prepared, for example, by treating an N297-linked glycan of an Fc-containing molecule to be subjected to glycan engineering with an ENGase that retains the activity of specifically hydrolyzing the 1,4-glycoside linkage between GlcNAc molecules (GlcNAcβ1-4GlcNAc) in the core chitobiose structure of the N297-linked glycan.

Alternatively, when Enzyme-A used in Step 1 has residual hydrolysis activity, the acceptor molecule in Step 1 can be prepared in situ in the same tank as in Step 1. Accordingly, Step 1 of the present invention includes an aspect in which the acceptor molecule is prepared in situ. In this aspect, the step of separately preparing an acceptor molecule prior to Step 1 can be omitted, and thus the production step is simplified. The simplification of the production step eliminates the need for a step of removing the ENGase used in the separate step, and further eliminates the need for a treatment such as separating and purifying the relatively unstable acceptor molecule generated in the separate step, and thus is very beneficial. In addition, the same enzyme-A is used as an enzyme that hydrolyzes an N297-linked glycan of an Fc-containing molecule as a raw material, and an enzyme that transfers a glycan in a glycan donor molecule, and thus this can also benefit from the advantage of the production step that the number and types of enzymes used can be reduced (this point becomes more remarkable when a fusion protein of Enzyme-A and Enzyme-B is used). In this aspect, in Step 1, rather than an acceptor molecule itself, which is a molecule that directly receives a glycan from a glycan donor molecule, an Fc-containing molecule (typically, an Fc-containing molecule such as an IgG type monoclonal antibody or an Fc fragment or CLCH consisting only of constant regions of the antibody) to be subjected to glycan engineering is added to the reaction system. The N297-linked glycan of the Fc-containing molecule to be added as such a raw material is not particularly limited as long as the N297-linked glycan is different from the glycan derived from the glycan donor molecule, which is a target product, and typical examples thereof include an N297-linked glycan derived from a host cell used when producing the Fc-containing molecule, a heterogeneous N297-linked glycan, or a host cell-derived heterogeneous N297-linked glycan. When such an Fc-containing molecule is introduced into the reaction system in Step 1, the 1,4-glycoside linkage between GlcNAc molecules (GlcNAcβ1-4GlcNAc) in the core chitobiose structure of the N297-linked glycan of the Fc-containing molecule is specifically hydrolyzed by the hydrolysis activity of Enzyme-A to generate an acceptor molecule in situ. Subsequently, by the glycosylation activity of Enzyme-A, the glycan of the glycan donor molecule is transferred to the acceptor molecule generated in situ, to produce an Fc-containing molecule having an N297-linked glycan including the glycan derived from the glycan donor molecule, which is a target product, in one pot. In this aspect, the acceptor molecule is generated in situ, but is not isolated or purified, and thus it appears as if the N297-linked glycan of the Fc-containing molecule added as a raw material were directly exchanged with the glycan derived from the glycan donor molecule in one step. Because of this, herein, such an aspect is appropriately referred to as "direct exchange reaction of the N297-linked glycan."

In the direct exchange reaction of the N297-linked glycan, all glycan donor molecules as described above can be used without particular limitation, and for example, any of a glycan donor molecule having an unactivated glycan reducing end and a glycan donor molecule having an activated glycan reducing end can be used. However, when a glycan having an activated reducing end (for example, a glycan having an oxazolinated reducing end) is used as a glycan donor molecule, the glycan is rapidly hydrolyzed in water and loses the reactivity with an antibody, and thus the constraint is that the reaction needs to be completed in a short time; and thus in the direct exchange reaction of the N297-linked glycan, it is preferable to use a glycan donor molecule having an unactivated glycan reducing end. Therefore, in one aspect of the present invention, the glycan donor molecule used in the direct exchange reaction of the N297-linked glycan is a glycan donor molecule having an unactivated glycan reducing end. In other words, in one aspect of the present invention, when Step 1 includes an aspect in which the acceptor molecule is prepared in situ, the glycan donor molecule used in Step 1 is a glycan donor molecule having an unactivated glycan reducing end. Such a glycan donor molecule is not limited, and examples thereof include a novel glycan donor molecule specifically disclosed in the chapter <Novel glycan donor molecule>. For example, when G-1 is selected as the glycan donor molecule, Fc-containing molecule-[SG]₂ is obtained, and G-4, G-13, G-14, or G-16 is selected as the glycan donor molecule, Fc-containing molecule-[X-MSG1]₂ is obtained, and furthermore, G-3, G-6, G-7, G-8, G-9, G-10, G-11, G-12, or G-15 is selected, Fc-containing molecule-[X-SG]₂ is obtained.

On the other hand, in the direct exchange reaction of the N297-linked glycan, when a glycan having an activated reducing end (for example, a glycan having an oxazolinated reducing end) is used as the glycan donor molecule, as described above, the glycan is rapidly hydrolyzed in water and loses the reactivity with an antibody, and thus the constraint is that the reaction needs to be completed in a short time. When an acceptor molecule is prepared in situ, it is conceivable to increase the concentration of an Fc-containing molecule to be added as a raw material in order to generate the acceptor molecule in a short time, but if this concentration is too high, the frequency of contact between the glycan having an activated reducing end and the Fc-containing molecule added as a raw material increases, and non-enzyme-mediated glycation can proceed predominantly. Therefore, in order to carry out the direct exchange reaction of the N297-linked glycan by using a glycan having an activated reducing end, it is preferable to set the concentration of the Fc-containing molecule to be added as a raw material low in order to suppress the glycation reaction. Alternatively, in order to generate an acceptor molecule in a short time and complete the reaction in a short time while avoiding hydrolysis of an activated glycan (for example, an oxazolinated glycan), it is preferable to use Enzyme-A that has stronger hydrolysis activity for an N297-linked glycan. Non Patent Literature (Xiao Zhang, et al., ACS Chem Biol. 2021, 11, 2502-2514) and Patent Literature (WO2022226420) have reported that by adding an oxazolinated disaccharide and wild type Endo-S2 to an antibody having a host cell-derived heterogeneous glycan, a glycan derived from a glycan donor molecule can be added to the antibody with a high transglycosylation rate. According to these reports, the glycan derived from the disaccharide oxazoline transferred to the antibody is resistant to the hydrolysis activity of Endo-S2, and thus the reaction can be completed in a short time by using wild type Endo-S2 having high hydrolysis activity. Therefore, in the direct exchange reaction of the N297-linked glycan in the present invention as well, such a known method can be used to appropriately select a glycan donor molecule and Enzyme-A used in the reaction.

Various conditions such as the reaction temperature in the direct exchange reaction of the N297-linked glycan are as described above, and the reaction time is preferably set to a longer time than in an aspect that includes a step of separately preparing an acceptor molecule.

### <Step 2>

In the production method according to the present invention, Step 2 is contacting the reaction mixture obtained in Step 1 with a cation exchange chromatography medium or a multimode chromatography medium under an acidic condition and collecting an Fc-containing molecule having an N297-linked glycan including the glycan derived from the glycan donor molecule, and preferably contacting the reaction mixture obtained in Step 1 with a cation exchange chromatography medium or a multimode chromatography medium under an acidic condition and isolating and collecting the Fc-containing molecule having an N297-linked glycan including the glycan derived from the glycan donor molecule included in the reaction mixture from a partially or completely unreacted Fc-containing molecule (acceptor molecule). In one aspect of the present invention, Step 2 is using the reaction mixture obtained in Step 1 as a loading solution as it is, or appropriately adding a buffer to the reaction mixture to prepare a loading solution, and contacting the loading solution with a cation exchange chromatography medium or a multimode chromatography medium under an acidic condition to selectively adsorb an unreacted acceptor molecule on the medium, and on the other hand collecting a flow-through solution including an Fc-containing molecule having an N297-linked glycan including the glycan derived from the glycan donor molecule, to selectively collect the Fc-containing molecule.

As described above, in the production method of the present invention, in Step 2, the Fc-containing molecule to which the glycan is added, which is a target product, and the unreacted Fc-containing molecule (acceptor molecule) can be separated, and thus even when only the glycan donor molecule is used in a small amount in Step 1, it is possible to produce an Fc-containing molecule having a homogeneous glycan structure with high purity. In the present idea, in order to produce an Fc-containing molecule having a glycan structure having a high glycosylation rate while suppressing the amount of the glycan donor molecule used in Step 1, Step 2 needs to have high selectivity and a high recovery. When the selectivity is low, the Fc-containing molecule to which the glycan is added, which is a target product, cannot be isolated from the unreacted Fc-containing molecule (acceptor molecule), and thus the glycosylation rate cannot be increased, and when the recovery is low, the amount of the acceptor molecule fed in Step 1 needs to be increased in order to increase the yield of the Fc-containing molecule, and as a result, the amount of the glycan donor molecule used increases, and the target effect cannot be obtained. In addition, when the present invention is applied as a method for producing an antibody for medical use on an industrial scale, it is necessary to be able to efficiently isolate the antibody, which is a target product, in Step 2 while keeping the desired biological activity.

As a conventional isolation technique, capillary gel electrophoresis (CE-SDS) is known as an analytical method for detecting an antibody to which no glycan is added (free of core GlcNAc) included in antibodies to which the N297-linked glycan is added (Richard R. Rustandi et al., Electrophoresis. 2008 Sep;29(17):3612-20). The present method can isolate a fragmented antibody heavy chain according to the hydrodynamic size of the molecule under a reducing condition, whereas the antibody denatures and loses the biological activity thereof in the process of pretreatment, and in addition, the throughput is limited to about several hundred µg because the antibody is isolated by using a medium packed in a fine capillary. In addition, a method using hydrophilic interaction chromatography (HILIC) is known as an analytical method for isolating an antibody having a core GlcNAc prepared by using an ENGase from an antibody having an N297-linked glycan prior to the action of the ENGase (Matthew A et al., Measuring the Glycan Occupancy of Intact mAbs using HILIC and Detection by Intrinsic Fluorescence). The present method does not require antibody fragmentation and can separate an antibody having an N297-linked glycan and an antibody having a core GlcNAc, whereas the antibody is denatured by heating and exposure to an organic solvent and loses the biological activity thereof, and in addition, the throughput is limited to the order of several µg because the isolation requires an analytical column having high number of theoretical plates and an ultra-high performance liquid chromatography apparatus (UHPLC). In addition, a method using cation exchange chromatography is known as an analytical method for isolating an antibody having a core GlcNAc prepared by using an ENGase from an antibody having an N297-linked glycan prior to the action of the ENGase (Masaki Kurogochi et al., PLOS ONE|DOI: 10.1371/journal.pone.0132848 July 22, 2015, and Shiyi Wang et al., Journal of Chromatography A, 1217 (2010) 6496-6502). The present method can separate an antibody having an N297-linked glycan and an antibody having a core GlcNAc without denaturing the antibodies, whereas in order to achieve high resolution separation, the method requires a gradient elution scheme that uses an analytical column having high number of theoretical plates and mixes two solutions by using a complicated program, has a throughput limited to about several hundred µg, and in addition, has the following drawback: a satisfactory yield cannot be obtained. Therefore, prior to the present invention, a method for isolating an antibody according to the presence or absence of glycan addition that can be applied to isolation and purification purposes on an industrial scale has not been reported at all.

Under these circumstances, the present inventors have found that by an extremely simple method involving contacting the reaction mixture with a cation exchange chromatographic medium or a multimode chromatographic medium under an acidic condition, an Fc-containing molecule to which a glycan is added, which is a target product, can be selectively isolated and purified at a high yield without denaturation.

Step 2 can be carried out according to a conventional method in cation exchange chromatography or multimode chromatography. For example, the reaction mixture obtained in Step 1 is mixed with an acidic buffer to obtain a loading solution, and the loading solution is passed through a column packed with a cation exchange chromatography medium or a multimode chromatography medium, making it possible to separate a molecule adsorbed on the column from a molecule that has flowed through the column. In Step 2, subsequently, an equilibration solution (or wash solution) is passed through to wash away a molecule non-specifically adsorbed on the column, and then an eluant (or a regeneration solution) is passed through, making it possible to elute a molecule specifically adsorbed on the column. In Step 2, after that, a cleaning solution can appropriately be passed through for the purpose of cleaning in place.

In step 2, cation exchange chromatography or multimode chromatography is carried out under an acidic condition. In one aspect of the present invention, the loading solution (or the loading solution and the equilibration solution) in Step 2 has an acidic pH. In Step 2, the lower the pH of the loading solution (or the loading solution and the equilibration solution), the higher the improving effect on the transglycosylation rate, and the improving effect on the transglycosylation rate tends to decrease as the pH increases. As the "acidic condition" in the present invention, any acidic pH (<7.0) can be used as long as the desired isolation can be achieved, and for example, as the lower limit value (the lower limit value of the pH of the loading solution and the equilibration solution), any pH selected from 2.5, 2.6, 2.7, 2.8, 2.9, 3.0, 3.1, 3.2, 3.3, 3.4, 3.5, 3.6, 3.7, 3.8, 3.9, and 4.0 can be adopted, and as the upper limit value (the upper limit value of the pH of the loading solution and the equilibration solution), any pH selected from 5.0, 4.9, 4.8, 4.7, 4.6, 4.5, 4.4, 4.3, 4.2, 4.1, 4.0, 3.9, 3.8, 3.7, 3.6, and 3.5 can be adopted. In addition, the pH range (the pH range of the loading solution and the equilibration solution) in the "acidic condition" is a combination of any of the upper limit vales and any of the lower limit values described above, or is 2.5 to 5.0, preferably 3.0 to 4.8, further preferably 3.3 to 4.7, particularly preferably 3.4 to 4.6, and most preferably 3.5 to 4.5, but is not limited thereto. Therefore, in one aspect of the present invention, the acidic condition in Step 2 means a range of pH 2.5 to 5.0, preferably pH 3.0 to 4.8, further preferably pH 3.3 to 4.7, particularly preferably pH 3.4 to 4.6, and most preferably pH 3.5 to 4.5.

In Step 2, the reaction mixture obtained in Step 1 can be used as a loading solution for passing through the column as it is, or a buffer can be appropriately added to the reaction mixture to prepare a loading solution. As the buffer for preparing a loading solution, any buffer can be used as long as the loading solution has an acidic pH, and examples thereof include acetate buffer, citrate buffer, phosphate buffer, glycine buffer, and phthalate buffer.

In addition, by appropriately adding a salt to the loading solution, the salt concentration can also be adjusted to the desired level. The salt for preparing a loading solution is not particularly limited as long as the desired isolation can be achieved, and examples thereof include sodium chloride, potassium chloride, and magnesium chloride. In addition, the salt concentration is, for example, 30 to 2000 mM, preferably 150 to 1000 mM, more preferably 200 to 750 mM, further preferably 280 to 600 mM, and particularly preferably 310 to 500 mM. The salt concentration in the loading solution affects the yield and purity of the final target product, and the optimum salt concentration varies depending on the target product and the type of the cation exchange chromatography medium or the multimode chromatography medium, and thus it is preferable to adopt the optimum salt concentration depending on the given chromatographic environment.

The concentration of the Fc-containing molecule in the loading solution is not particularly limited as long as the desired isolation can be achieved, and is, for example, 0.1 to 100 mg/mL, preferably 0.5 to 50 mg/mL, more preferably 1.5 to 30 mg/mL, further preferably 2 to 20 mg/mL, and particularly preferably 3 to 10 mg/mL. In addition, the volume of the loading solution itself to be passed through the medium can be appropriately determined depending on the volume of the medium or the like, according to a conventional method.

As the equilibration solution in Step 2, any equilibration solution can be used as long as it can equilibrate the pH and salt concentration in the medium before isolation, and wash away a molecule non-specifically adsorbed on the medium without substantially isolating a molecule adsorbed on the medium. For the buffer that can be used as the equilibration solution and the salt concentration in the equilibration solution, see the description of the loading solution. The volume of the equilibration solution passed can be appropriately determined according to the volume of the medium or the like, according to a conventional method, and is 1 to 100 column volume (CV), preferably 5 to 50 CV, more preferably 8 to 40 CV, further preferably 10 to 30 CV, and particularly preferably 12 to 20 CV, but is not limited thereto.

The eluant in Step 2 can elute a molecule adsorbed on the medium, and any eluant can be used, and any cleaning solution can be used as the cleaning solution as long as it enables cleaning in place (CIP) of the medium. Therefore, the eluant and the cleaning solution can be appropriately determined according to a known method or by applying a known method.

In the production method of the present invention, the transglycosylation rate after the completion of Step 2 can be set to any numerical value, and for example, can be set to 85% or more, 90% or more, or 95% or more. In one aspect of the present invention, the transglycosylation rate in Step 2 is more than 90%, preferably more than 95%, and particularly preferably more than 99%. In addition, the step yield of Step 2 can be set to any numerical value, and as described above, the amount of the glycan donor molecule used in Step 1 can be reduced as the step yield of step 2 is increased, and thus a higher step yield is desirable. However, the step yield is not particularly limited as long as the desired isolation can be achieved, and is, for example, 1 to 99%, preferably 5 to 95%, more preferably 20 to 95%, and particularly preferably 40 to 90%.

As used herein, the cation exchange chromatography medium means a medium having a cation exchange group. The cation exchange chromatography medium is classified into a strong acid type having a sulfonic acid group (R-SO₃H) bound to the surface thereof, and a weak acid type having a carboxyl group (R-COOH), a phenolic hydroxyl group (R-OH), a phosphonic acid group [R-P(=O)(OH)₂], or a phosphinic acid group [R-P(=O)(OH)-R] bound thereto. Therefore, in one aspect of the present invention, the cation exchange chromatography medium is a strong acid type or weak acid type medium, and/or a medium having a sulfonic acid group, a carboxyl group, a phenolic hydroxyl group, a phosphonic acid groups, or a phosphinic acid group as a cation exchange group. The specific form of the ion exchange chromatography media may be any of particulate, membranous, monolithic, plate-like, chip-like, a fibrous, and the like, and from the viewpoint of a characteristic of a molecule to be adsorbed on the medium, examples thereof include, but are not limited to, particulate, membranous, and monolithic.

Examples of the particulate cation exchange chromatography medium include, but are not limited to, SP sepharose FF (Cytiva), SOURCE 15S (Cytiva), Capto S Impact (Cytiva), Eshmuno CP-FT (Merck), Exhmuno CPX (Merck), POROS HS (Thermo), POROS XS (Thermo), Nuvia HR-S (BiO-RAD), and Cellufine Max GS (JNC).

Examples of the membranous cation exchange chromatography medium include, but are not limited to, Sartobind S (Sartorius) and Mustang S (Pall).

Examples of the monolithic cation exchange chromatography medium include, but are not limited to, CIM monolith SO3 (BIA Separation).

The cation exchange chromatography in Step 2 can be carried out by using a known apparatus, and examples of such an apparatus include, but are not limited to, AKTA avant 25 (Cytiva). In addition, the flow rate of the mobile phase in the cation exchange chromatography in Step 2 can be appropriately determined according to a conventional method.

In the present invention, the multimode chromatography medium includes a cation exchange group and at least one functional group capable of a different action from the cation exchange group, and in one embodiment, the functional group capable of a different action is a functional group capable of hydrophobic interaction. Examples of the cation exchange group include the same ones as described for the cation exchange chromatography medium. In addition, examples of the form of the multimode chromatography medium include the same ones as described for the cation exchange chromatography medium. Examples of the multimode chromatography medium include, but are not limited to, Capto(TM) MMC and TOYOPEARL(TM) MX-TRP-650M. The multimode chromatography in Step 2 can be carried out by using a known apparatus. In addition, the flow rate of the mobile phase in the multimode chromatography in Step 2 can be appropriately determined according to a conventional method.

### <Conjugate>

The Fc-containing molecule obtained via Step 1 and Step 2 of the present invention can be further chemically or biochemically-modified. By using a glycan donor molecule having a substituent having an azido group (N₃-), such as a polyethylene glycol linker (L(PEG)) having N₃ in moiety X in the structural formula of the glycan donor molecule described above, an Fc-containing molecule having an azido group at a glycan non-reducing end, such as an Fc-containing molecule having the structure of N297-(Fuc)SG-L(PEG)₂, N297-(Fuc)MSG1-L(PEG), or N297-(Fuc)MSG2-L(PEG), represented by the following formula can be prepared. 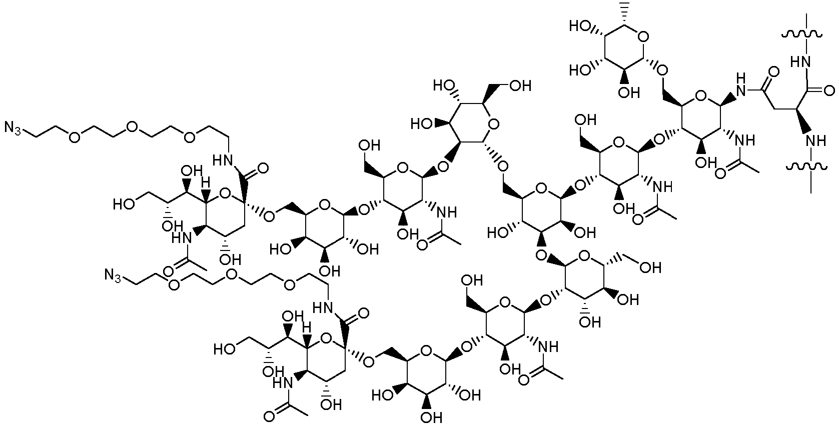 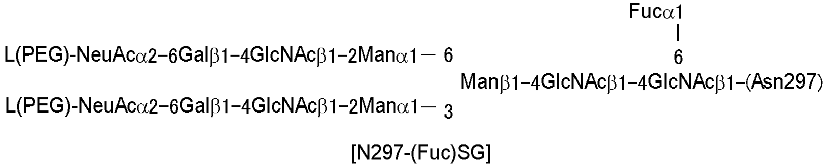 wherein L(PEG) represents binding to the carbonyl group bound to position 2 of sialic acid at the non-reducing ends on both the 1-3 chain side and the 1-6 chain side of the branched chains of β-Man; and L(PEG) is a linker structure that includes an ethylene glycol structure and optionally includes a further bound structure and/or modified structure. 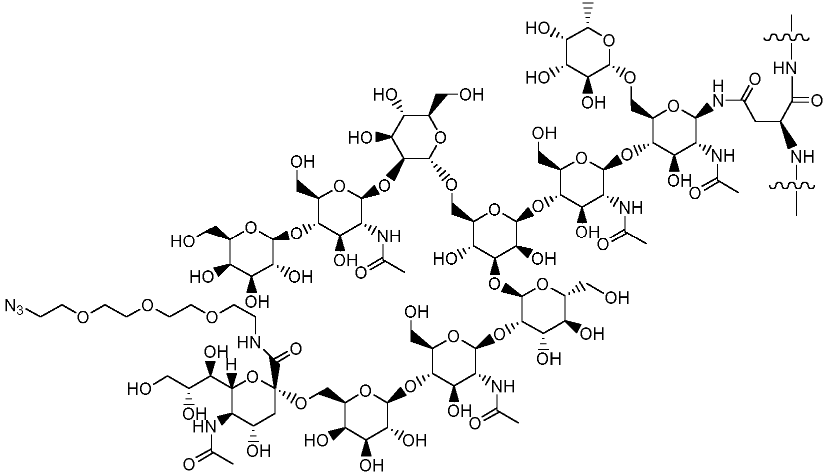 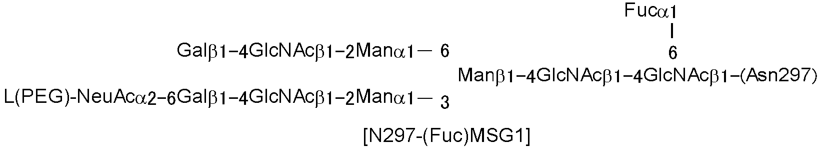 wherein L(PEG) represents binding to the carbonyl group bound to position 2 of sialic acid at the non-reducing end on the 1-3 chain side of the branched chains of β-Man; and L(PEG) is a linker structure that includes an ethylene glycol structure and optionally includes a further bound structure and/or modified structure. 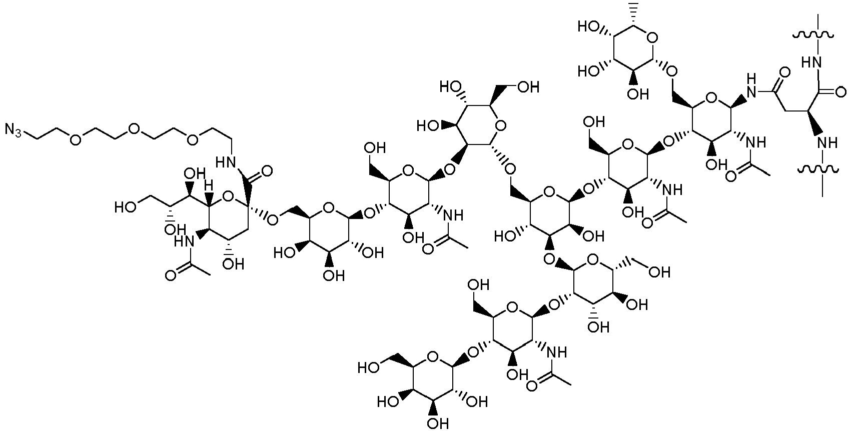 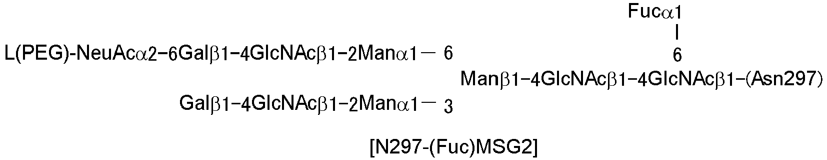 wherein L(PEG) represents binding to the carbonyl group bound to position 2 of sialic acid at the non-reducing end on the 1-6 chain side of the branched chains of β-Man; and L(PEG) is a linker structure that includes an ethylene glycol structure and optionally includes a further bound structure and/or modified structure.

The azido group (N₃-) forms a 1,2,3-triazole ring by reacting with an alkyne structure such as a (hetero)cycloalkynyl group (for example, DBCO (Dibenzocyclooctyne)) (SPAAC (strain-promoted alkyne azide cycloaddition: Agard NJ, et al., J Am Chem Soc. 2004, 126, 46, 15046-15047). Therefore, in one aspect of the present invention, when the glycan derived from a glycan donor molecule has a non-reducing end chemically-modified by a substituent having an azido group (N₃-), or the glycan itself derived from a glycan donor molecule does not have an azido group but after Step 1 or Step 2, a substituent having an azido group is introduced into a non-reducing end of the glycan, the production method according to the present invention can further include a step of reacting a molecule having an alkyne structure with the azido group (N₃-) of the glycan. Herein, the Fc-containing molecule bound to a molecule having an alkyne structure as described above is sometimes referred to as a "conjugate." The Fc-containing molecule obtained via Step 1 and Step 2 of the present invention that has been conjugated with another molecule by a reaction other than SPAAC is also sometimes referred to as a "conjugate."

Examples of the molecule having an alkyne structure include a molecule having a (hetero)cycloalkynyl group and having desired activity, and preferable examples thereof include, but are not limited to, a pharmacologically active compound (for example, a chemotherapeutic agent, a molecular target drug, an immunostimulant (for example, a STING agonist (WO2020/050406, WO2014/099824, WO2014/179335, WO2014/189805, WO2014/189806, WO2015/074145, WO2015/185565, WO2016/096714, WO2016/012305, WO2016/145102, WO2017/027646, WO2017/027645, WO2017/075477, WO2017/093933, WO2017/100305, WO2017/123669, WO2017/161349, WO2017/175147, WO2017/175156, WO2018/009466, WO2018/045204, WO2018/060323, WO2018/067423, WO2018/065360, WO2014/093936, WO2018/009648, WO2018/100558), a TLR agonist, an A2AR antagonist, an IDO inhibitor, antagonists of CTLA-4, LAG-3, and PD-1 pathways, a checkpoint inhibitor, a vascular endothelial growth factor (VEGF) receptor inhibitor, a smoothen inhibitor, an alkylating agent, an antimetabolite, a retinoid and an anticancer vaccine, an adjuvant, a lipid, a liposome, a toxin, an antimicrobial, an antiviral agent, a diagnostic agent, a protein, a peptide, an amino acid, a nucleic acid, an antigen, a vitamin, or a hormone). Examples of the chemotherapeutic agent or the toxin include, but are not limited to, camptothecin (for example, WO2014/057687), pyrrolobenzodiazepine (for example, WO2013/173496, WO2014/130879, WO2017/004330, WO2017/004025, WO2017/020972, WO2016/036804, WO2015/095124, WO2015/052322, WO2015/052534, WO2016/011519, WO2015/052321, WO2015/031693, WO2011/130613, or WO2019/065964), doxorubicin, auristatin, taxane, or derivatives thereof. In addition, examples of the immunostimulant include, but are not limited to, a STING agonist, a TLR agonist, and an A2AR antagonist. Preferable examples of the molecule having an alkyne structure include a molecule selected from the group consisting of (A) to (E) below:
(A) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide,
(B) N-[4-(11,12-didehydrodibenzo[b,f]azocin-5(6H)-yl)-4-oxobutanoyl]glycylglycyl-L-valyl-N-[4-({[(11'S,11'aS)-11'-hydroxy-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl)phenyl]-L-alaninamide,
(C) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',11a'-dihydro-1'H-spiro[cyclopropane-1,2'-pyrrolo[2,1-c][1,4]benzodiazepin]-8'-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide,
(D) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',10',11',11a'-tetrahydro-1'H-spiro[cyclopropane-1,2'-pyrrolo[2,1-c][1,4]benzodiazepin]-8'-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide, and
(E) (bis(N,N-diethylethanaminium)N-[4-(11,12-didehydrodibenzo[b,f]azocin-5(6H)-yl)-4-oxobutanoyl]glycylglycyl-L-phenylalanyl-N-[(2-{9-[(5R,7R,8R,12aR,14R,15R,15aR,16R)-15-fluoro-16-hydroxy-2,10-dioxo-2,10-disulfide-14-(6,7,8,9-tetrahydro-2H-2,3,5,6-tetraazabenzo[cd]azulen-2-yl)octahydro-2H,10H,12H-5,8-methano-2λ5,10λ5-furo[3,2-l][1,3,6,9,11,2,10]pentaoxadiphosphacyclotetradecyn-7-yl]-6-oxo-6,9-dihydro-1H-purin-1-yl}ethoxy)methyl]glycinamide.

An Fc-containing molecule produced by the production method described above is also within the scope of the present invention. Therefore, in one aspect thereof, the present invention encompasses an Fc-containing molecule produced by the production method described above.

### isolation method>

The finding that a glycoprotein having a desired glycan (particularly an Fc-containing molecule having an N297-linked glycan) discovered by the present inventors and a glycoprotein not having the glycan can be separated by cation exchange chromatography or multimode chromatography under an acidic condition can also be applied to a use in which a target glycoprotein is simply isolated independently of the production method described above. Therefore, in one aspect of the present invention, provided is a method for isolating a glycoprotein (particularly an Fc-containing a molecule having an N297-linked glycan), including contacting a solution including the glycoprotein (Fc-containing molecule) and one or more impurities with a cation exchange chromatographic medium or a multimode chromatographic medium under an acidic condition to selectively trap the impurities in the cation exchange chromatographic medium or the multimode chromatographic medium. In one aspect of the present invention, the above isolation method is an isolation method including contacting a solution including the glycoprotein (Fc-containing molecule) and one or more impurities with a cation exchange chromatography medium or a multimode chromatography medium under an acidic condition to selectively trap the impurities in the cation exchange chromatography medium or the multimode chromatography medium, and on the other hand, collecting a flow-through solution including a glycoprotein having a desired glycan (an Fc-containing molecule having an N297-linked glycan) that is not selectively trapped in the medium, to selectively collect the glycoprotein (Fc-containing molecule).

In one aspect of the isolation method of the present invention, the glycoprotein is an Fc-containing molecule, typically an Fc-containing molecule having an N297-linked glycan. The N297-linked glycan may be any of a high mannose type glycan, a hybrid type glycan, and a complex type glycan, and is preferably a complex type glycan. Therefore, in one aspect of the present invention, the N297-linked glycan is a complex type glycan. In addition, typical examples of the impurities to be separated from a glycoprotein having a desired glycan (for example, an Fc-containing molecule having an N297-linked glycan) include a molecule having the same structure as that of the glycoprotein (Fc-containing molecule) except that the structure of the glycan (for example, the N297-linked glycan) is different from that of the glycoprotein (Fc-containing molecule), which is a target product, or the glycan (for example, the N297-linked glycan) is deleted. For example, when the N297-linked glycan in the Fc-containing molecule, which is a target product, is not a core GlcNAc to which fucose is optionally added (for example, when the N297-linked glycan is any of a high mannose type glycan, a hybrid type glycan, and a complex type glycan), the one or more impurities include a molecule identical to the Fc-containing molecule except for having, as an N297-linked glycan, a core GlcNAc to which fucose is optionally added.

The isolation method according to the present invention is basically the same as Step 2 of the production method according to the present invention, except that the isolation method does not necessarily need to be incorporated as one step in the method for producing an Fc-containing molecule. Therefore, the description of Step 2 provided above also applies as it is when the isolation method according to the present invention is carried out.

In addition, the isolation method according to the present invention can also be combined with a desired transglycosylation reaction. Therefore, in one aspect of the present invention, provided is a method for isolating an Fc-containing molecule having an N297-linked glycan including a glycan derived from a glycan donor molecule, including the following Steps 1 and 2:
[Step 1] reacting an acceptor molecule, which is an Fc-containing molecule having, as an N297-linked glycan, a core GlcNAc to which fucose is optionally added, with a glycan donor molecule including a glycan in the presence of an endo-β-N-acetylglucosaminidase (Enzyme-A), which prefers, as a substrate, an N297-linked glycan of an Fc-containing molecule, so as to obtain a reaction mixture; and
[Step 2] contacting the reaction mixture with a cation exchange chromatography medium or a multimode chromatography medium under an acidic condition and isolating, collecting, or isolating and collecting the Fc-containing molecule having an N297-linked glycan including the glycan derived from the glycan donor molecule.

Steps 1 and 2 in the isolation method are the same as Step 1 and Step 2 in the production method according to the present invention described above. Therefore, the description of Step 1 and Step 2 provided above also applies as it is when the isolation method is carried out.

### <Novel glycan donor molecule>

In yet another aspect of the present invention, a novel glycan donor molecule represented by a formula selected from the group consisting of the following formulas: 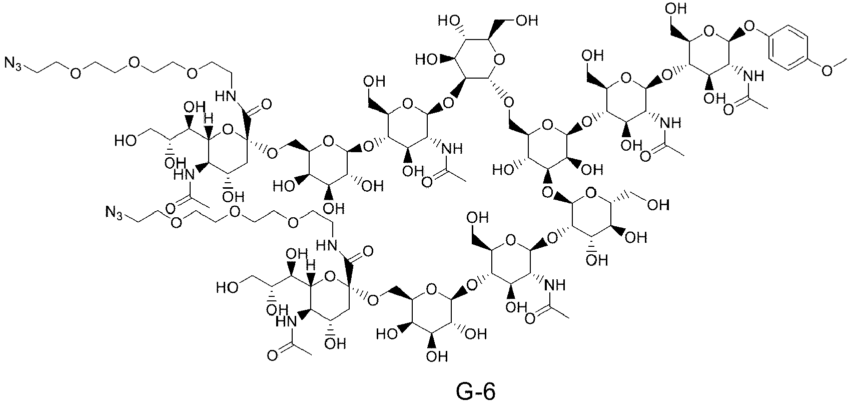 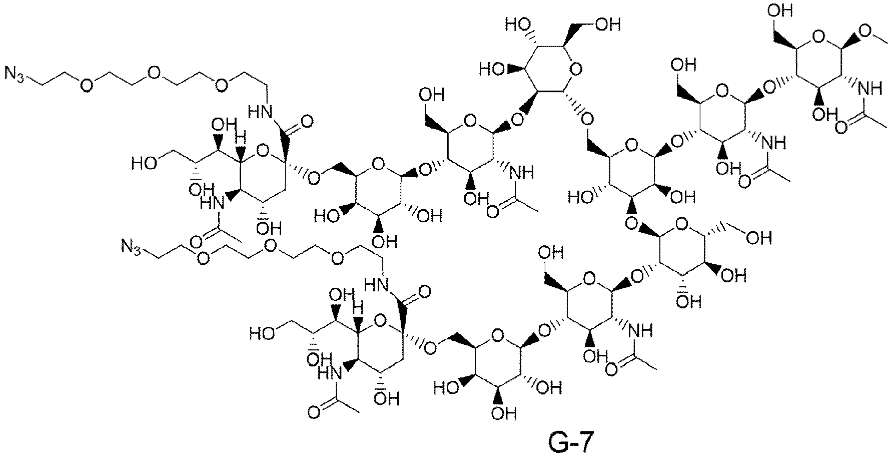 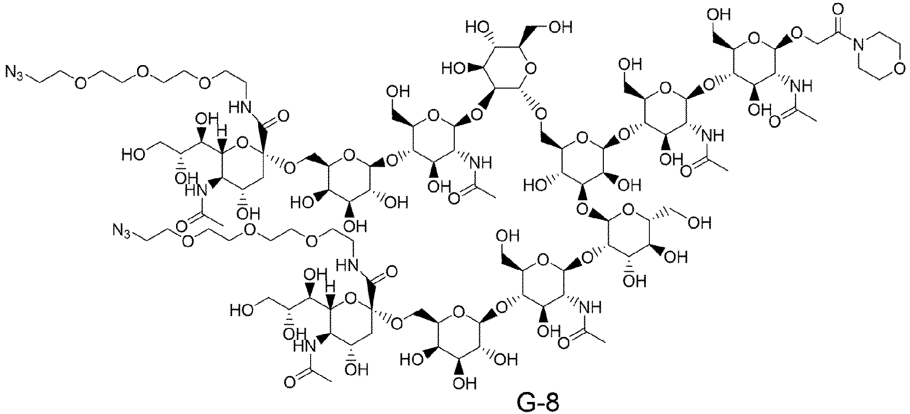 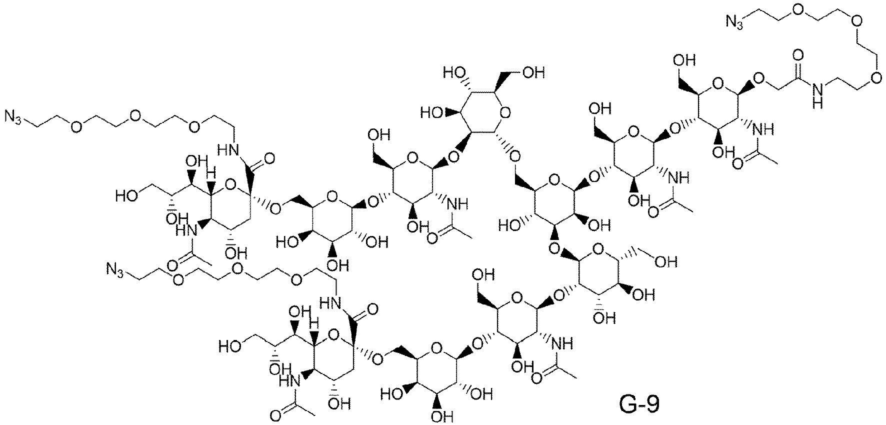 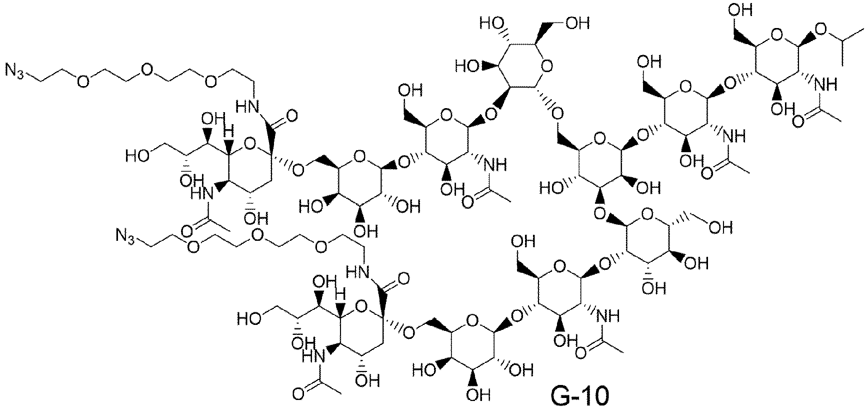 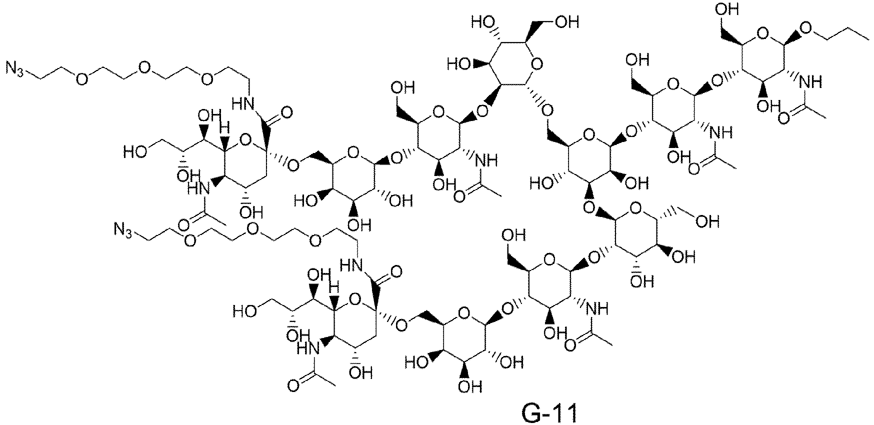 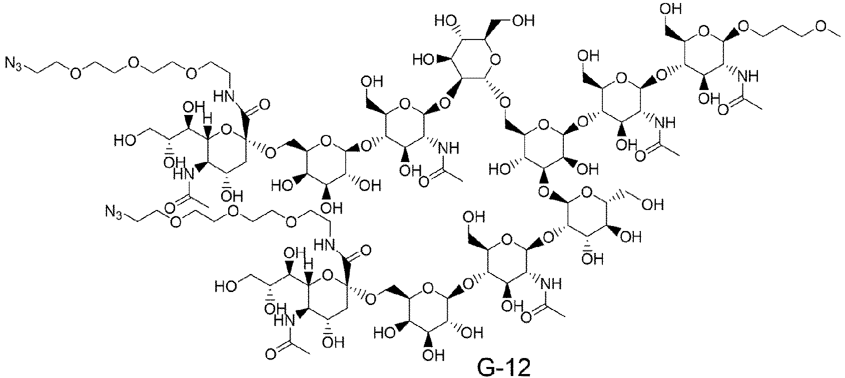 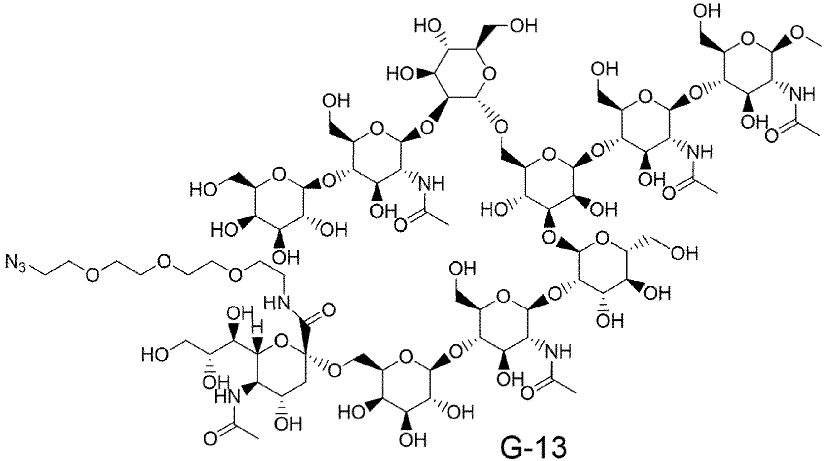 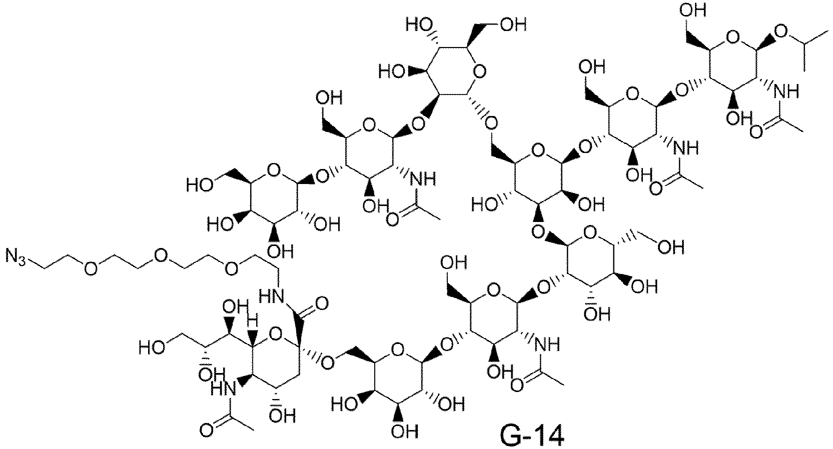 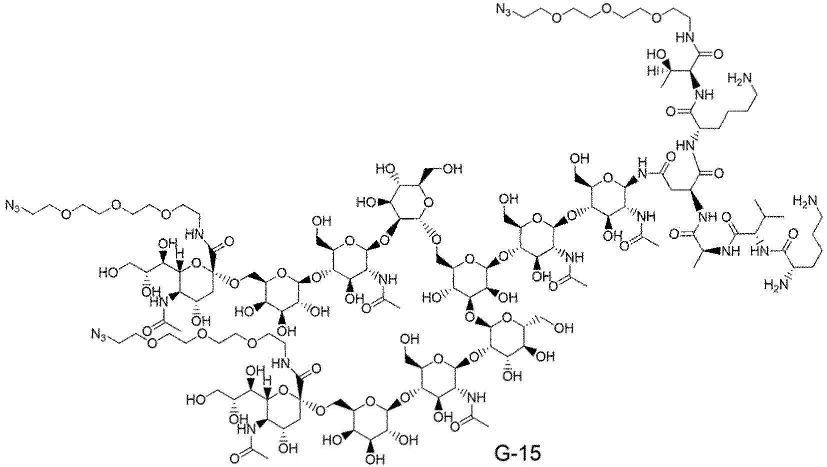 and 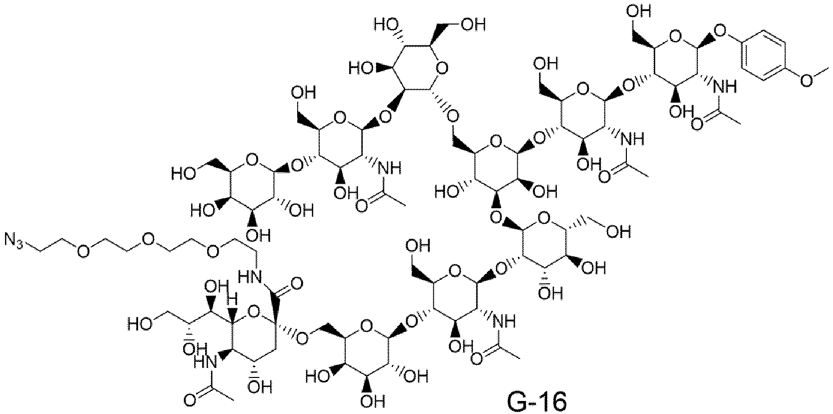 is provided. As will be specifically described in the Examples below, it has been demonstrated that these novel glycan donor molecules can be suitably used in the production method according to the present invention, and it can be expected that these can also be used for a glycan remodeling reaction (transglycosylation reaction) other than the same. When the novel glycan donor molecules are used in the production method according to the present invention, it is preferable to appropriately determine each glycan donor molecule in consideration of the compatibility with a combination of Enzyme-A and Enzyme-B to be used or the like, with reference to the test results in the Examples.

### <Method for producing antibody-immunostimulant conjugate (Rp,Rp-XIII)>

In one aspect of the present invention, provided is a method for producing an antibody-immunostimulant conjugate (Rp,Rp-XIII), including:
[Step 1] reacting an acceptor molecule, which is an Fc-containing molecule having, as an N297-linked glycan, a core GlcNAc to which fucose is optionally added, with a glycan donor molecule including a glycan in the presence of an endo-β-N-acetylglucosaminidase (Enzyme-A), which prefers, as a substrate, an N297-linked glycan of an Fc-containing molecule and an endo-β-N-acetylglucosaminidase (Enzyme-B), which prefers, as a substrate, a glycan of a glycan donor molecule, so as to obtain a reaction mixture, wherein the Fc-containing molecule is an antibody, and the glycan donor molecule is a compound represented by formula G-10;
[Step 2] contacting the reaction mixture with a cation exchange chromatography medium or a multimode chromatography medium under an acidic condition and collecting the Fc-containing molecule having an N297-linked glycan including the glycan derived from the glycan donor molecule; and
[Step 3] binding a cyclic dinucleotide (CDN) conjugate precursor represented by the following formula (Rp,Rp-XII): 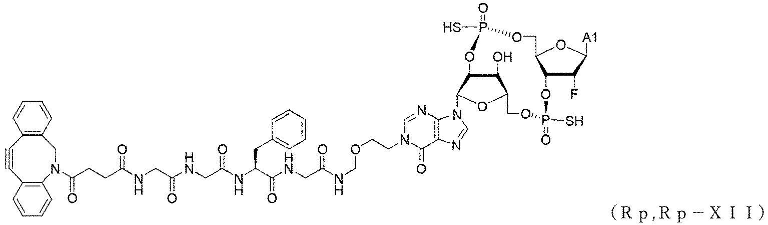
to an azido group (N₃-) of the glycan by a cycloaddition reaction. In the above production method, the descriptions in the above chapters <Step 1> and <Step 2> are applied to Step 1 and Step 2 as they are. Therefore, hereinafter, Step 3 and the antibody-immunostimulant conjugate produced by the method of the present invention will be described in detail.

Step 3 is shown by the following schematic diagram. 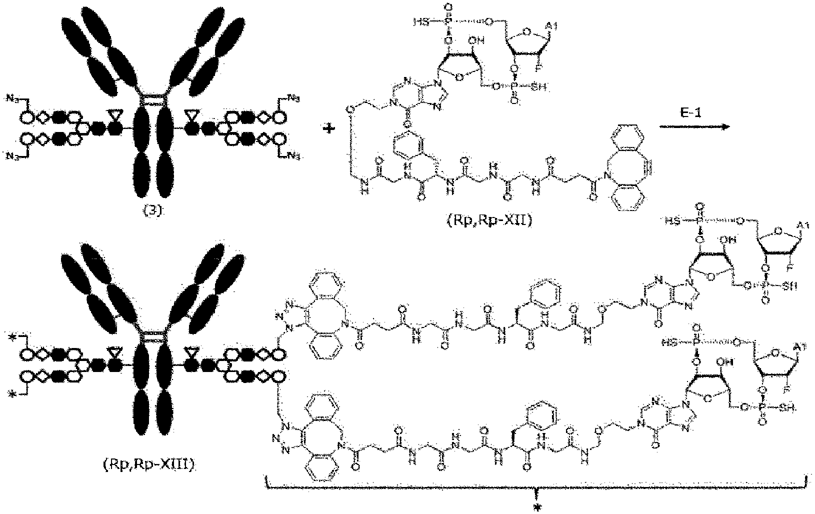 wherein
(3) schematically shows the Fc-containing molecule having an N297-linked glycan including the glycan derived from the glycan donor molecule, obtained in Step 2, the two asterisks (*) on the left side of the antibody-immunostimulant conjugate (Rp,Rp-XIII) represent the CDN-linker moiety indicated by the asterisk on the right side, and
A1 represents any of

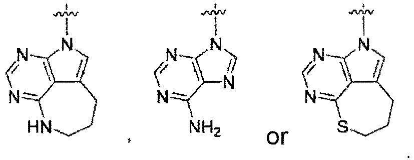

The antibody-immunostimulant conjugate (Rp,Rp-XIII) can be produced by binding the Fc-containing molecule (3) obtained in Step 2 to a CDN conjugate precursor (Rp,Rp-XII) by a cycloaddition reaction. The CDN conjugate precursor (Rp,Rp-XII) can be produced with reference to WO2020/050406, WO2021/177438, or WO2022/163846. Examples of the cycloaddition reaction include the Diels-Alder reaction and a 1,3-dipolar cycloaddition reaction, and a 1,3-dipolar cycloaddition reaction is preferably used for production. Examples of the 1,3-dipolar cycloaddition reaction include a cycloaddition reaction between azide and a terminal alkyne, and an SPAAC (strain-promoted azide-alkyne cycloaddition reaction: J. Am. Chem. Soc. 2004, 126, 15046-15047) reaction, and an SPAAC reaction is preferably used for production. Therefore, in one aspect of the present invention, Step 3 includes binding the Fc-containing molecule (3) obtained in Step 2 to a CDN conjugate precursor (Rp,Rp-XII) by an SPAAC reaction.

### (E-1 step)

Hereinafter, the SPAAC reaction in Step 3 will be described. The SPAAC reaction can be carried out by mixing a buffer solution (phosphate buffer, acetate buffer, borate buffer, or the like) of the Fc-containing molecule (3) obtained in Step 2 and a solution obtained by dissolving a CDN conjugate precursor (Rp,Rp-XII) in an appropriate solvent (dimethylsulfoxide, N,N-dimethylformamide, N,N-dimethylacetamide, N-methylpyrrolidone, propylene glycol, or a mixed solvent thereof). The CDN conjugate precursor (Rp,Rp-XII) is 2 mol to excess mol, and preferably 4 mol to 30 mol, per mol of the Fc-containing molecule (3), and the ratio of the organic solvent to the buffer solution of the Fc-containing molecule (3) is preferably 1% to 200% (v/v). The reaction temperature is 0°C to 37°C, and preferably 15°C to 25°C, and the reaction time is 1 hour to 150 hours, and preferably 6 hours to 72 hours. The pH of the reaction solution is preferably 5 to 9. Appropriately, the reaction solution can be isolated according to the method described in Common Operation D described later to obtain the antibody-immunostimulant conjugate (Rp,Rp-XIII).

The antibody-immunostimulant conjugate can be identified by buffer exchange, isolation, antibody concentration measurement, and measurement of the average number of immunostimulant molecules bound per antibody molecule by Common Operations D to G described later.

### Common Operation A: Concentration of antibody aqueous solution

An antibody or antibody-immunostimulant conjugate solution can be placed in an Amicon(registered trademark) Ultra centrifugal filter device (50,000 NMWL, Merck Millipore Ltd.) and subjected to a centrifugal operation (centrifugation at 2000 G to 4000 G for 5 to 20 minutes) using a centrifuge (Allegra X-15R, Beckman Coulter, Inc.) to concentrate the antibody and antibody-immunostimulant conjugate solution.

### Common Operation B: Measurement of antibody concentration

The antibody concentration can be measured by using a UV measuring instrument (Nanodrop 1000, Thermo Fisher Scientific, Inc.) according to the method specified by the manufacturer. At this time, a different extinction coefficient at 280 nm (1.3 mLmg⁻¹cm⁻¹ to 1.8 mLmg⁻¹cm⁻¹) is used for each antibody.

### Common Operation C: Antibody buffer exchange

A buffer (phosphate buffered saline (pH 6.0), phosphate buffer (pH 6.0), or the like) is added to an antibody aqueous solution, and the resulting antibody aqueous solution is concentrated according to the method described in Common Operation A. This operation is carried out several times, and then the antibody concentration is measured according to the method described in Common Operation B. To this antibody buffer solution, a buffer (phosphate buffered saline (pH 6.0), phosphate buffer (pH 6.0), or the like) can be appropriately added to prepare the antibody buffer solution having a target concentration (for example, about 10 mg/mL).

### Common Operation D: Isolation of antibody-immunostimulant conjugate (gel filtration chromatography)

An NAP column (NAP-5, NAP-10, or NAP-25 (manufactured by GE Healthcare)) is equilibrated with acetate buffer (10 mM Acetate Buffer, 5% Sorbitol, pH 5.5; herein referred to as ABS) or a suitable buffer other than the same. This NAP column is charged with an antibody-immunostimulant conjugate reaction solution, and the amount of the buffer specified by the manufacturer is caused to flow down naturally to collect an antibody fraction. The NAP column is again charged with this fraction, and the amount of the buffer specified by the manufacturer is caused to flow down naturally to collect an antibody fraction. By repeating this operation two or three times in total, an antibody-immunostimulant conjugate excluding an unbound immunostimulant linker, dimethylsulfoxide, and propylene glycol can be obtained. If necessary, the concentration of the antibody-immunostimulant conjugate solution can be regulated by Common Operations A and C.

Common Operation E: Measurement of antibody concentration and average number of immunostimulant molecules bound per antibody molecule in antibody-immunostimulant conjugate (UV method)

The bound immunostimulant concentration of the antibody-immunostimulant conjugate can be calculated by using an absorptiometer (UV/VIS Spectrometer Lambda 25, PerkinElmer, Inc.) according to the method disclosed in WO2020/050406 or WO2021/177438 to measure the absorbances of the antibody-immunostimulant conjugate aqueous solution at two wavelengths of 280 nm and 250 nm.

Common Operation F: Measurement of antibody concentration and average number of immunostimulant molecules bound per antibody molecule in antibody-immunostimulant conjugate (reversed-phase high-performance liquid chromatography method: RP-HPLC)

The antibody concentration and the average number of the immunostimulant molecules bound per antibody molecule in the antibody-immunostimulant conjugate can be determined by high-performance liquid chromatography analysis according to the method disclosed in WO2020/050406 or WO2021/177438, in addition to Common Operation E described above.

Common Operation G: Measurement of antibody concentration and average number of immunostimulant molecules bound per antibody molecule in antibody-immunostimulant conjugate (hydrophobic interaction-high-performance liquid chromatography method: HI-HPLC)

The antibody concentration and the average number of the immunostimulant molecules bound per antibody molecule in the antibody-immunostimulant conjugate can be determined by high-performance liquid chromatography analysis according to the method disclosed in WO2020/050406 or WO2021/177438, in addition to Common Operations E and F described above.

An antibody-immunostimulant conjugate produced by the production method described above is also within the scope of the present invention. Therefore, in one aspect thereof, the present invention encompasses an antibody-immunostimulant conjugate produced by the production method described above.

The antibody-immunostimulant conjugate produced by the method of the present invention may have a stereoisomer or an optical isomer derived from an asymmetric carbon atom, a geometric isomer, a tautomer, or an optical isomer such as a d-form, a l-form, or an atropisomer, and all of these isomers, these optical isomers, and mixtures thereof are included in the present invention.

In the antibody-immunostimulant conjugate produced by the method of the present invention, the number of immunostimulant molecules bound to one antibody molecule is an important factor affecting the efficacy and safety thereof. The production of an antibody-immunostimulant conjugate is carried out by specifying a reaction condition such as the amounts of a raw material and a reagent to be reacted such that the number of immunostimulant molecules bound is constant, and unlike a chemical reaction of a low molecular weight compound, the antibody-immunostimulant conjugate is usually obtained as a mixture in which different numbers of immunostimulant molecules are bound. However, in the method of the present invention, in Steps 1 and 2, an Fc-containing molecule having a homogeneous glycan structure with high purity can be produced, and thus as a result, an antibody-immunostimulant conjugate having a uniform number of immunostimulant molecules bound can be obtained with a high purity. The number of immunostimulant molecules bound to one antibody molecule can be specified by the average value, that is, the average number of immunostimulant molecules bound (DAR: Drug to Antibody Ratio). The number of cyclic dinucleotide derivative molecules bound to an antibody molecule can be controlled, and as the average number of immunostimulant molecules bound per antibody, a range of 1 to 4 cyclic dinucleotide derivative molecules can be bound, and the average number is preferably 2 to 4, more preferably 3 to 4, further preferably 3.2 to 4, and particularly preferably 3.5 to 4.

In yet another aspect of the present invention, provided is a method for producing an antibody-immunostimulant conjugate having a range of 1 to 3 cyclic dinucleotide derivative molecules bound as the average number of immunostimulant molecules bound per antibody in the same manner as the above method except that G-14 is used instead of G-10 as the glycan donor molecule in [Step 1] above. In this case, the Fc-containing molecule obtained in Step 2 has the structure represented by (3') below instead of (3). The number of cyclic dinucleotide derivative molecules bound to an antibody molecule can be controlled, and the average number of immunostimulant molecules bound per antibody is in the range of preferably 1.0 to 2.5, and more preferably 1.2 to 2.2.

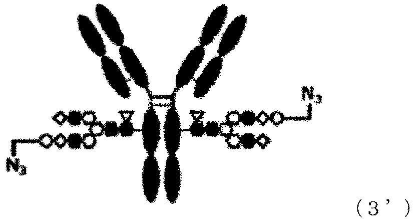

The antibody-immunostimulant conjugate produced by the method of the present invention may absorb water, have adsorbed water attached thereto, or become a hydrate when left in the atmosphere or recrystallized, and such a water-containing compound and salt are also encompassed by the present invention.

The antibody-immunostimulant conjugate produced by the method of the present invention has a basic group such as an amino group, and thus can be formed into a pharmaceutically acceptable salt if desired. Examples of such a salt include a hydrohalide such as a hydrochloride or a hydroiodide; an inorganic acid salt such as a nitrate, a perchlorate, a sulfate, or a phosphate; a lower alkanesulfonate such as a methanesulfonate, a trifluoromethanesulfonate, or an ethanesulfonate; an arylsulfonate such as a benzenesulfonate or a p-toluenesulfonate; an organic acid salt such as formic acid, acetic acid, malic acid, a fumarate, a succinate, a citrate, a tartrate, an oxalate, or a maleate; and an amino acid salt such as an ornithinate, a glutamate, or an aspartate.

The antibody-immunostimulant conjugate produced by the method of the present invention includes a phosphate group and/or a thiophosphate group in the structure thereof, and thus can generally form a base addition salt. Examples of the pharmaceutically acceptable salt include an alkali metal salt such as a sodium salt, a potassium salt, or a lithium salt; an alkaline earth metal salt such as a calcium salt or a magnesium salt; an inorganic salt such as an ammonium salt; and an organic amine salt such as a dibenzylamine salt, a morpholine salt, a phenylglycine alkyl ester salt, an ethylenediamine salt, a N-methylglucamine salt, a diethylamine salt, a triethylamine salt, a tert-butylamine salt, a cyclohexylamine salt, a dicyclohexylamine salt, a N,N'-dibenzylethylenediamine salt, a diethanolamine salt, a N-benzyl-N-(2-phenylethoxy)amine salt, a piperazine salt, a tetramethylammonium salt, or a tris(hydroxymethyl)aminomethane salt.

The antibody-immunostimulant conjugate produced by the method of the present invention may be present as a hydrate because of absorption of water in the air or the like. The solvate of the present invention is not particularly limited as long as the solvate is pharmaceutically acceptable, and specific preferable examples thereof include a hydrate, an ethanolate, and a 2-propanolate. In addition, a nitrogen atom is present in the antibody-immunostimulant conjugate of the present invention, and thus the antibody-immunostimulant conjugate may be a N-oxide form, and these solvates and the N-oxide form are also included in the scope of the present invention. In addition, a sulfur atom is present in the antibody-immunostimulant conjugate of the present invention, and thus the antibody-immunostimulant conjugate may be a sulfoxide form, and these solvates and the sulfoxide form are also included in the scope of the present invention.

In addition, compounds labeled with various radioactive or non-radioactive isotopes are also encompassed by the antibody-immunostimulant conjugate produced by the method of the present invention. One or more of the atoms constituting the antibody-immunostimulant conjugate produced by the methods of the present invention can also contain a non-natural proportion of an atomic isotope. Examples of the atomic isotope include deuterium (2H), tritium (3H), iodine-125 (125I), and carbon-14 (14C). In addition, the antibody-immunostimulant conjugate produced by the method of the present invention can be radiolabeled with a radioisotope such as tritium (3H), iodine-125 (125I), or carbon-14 (14C). A radiolabeled compound is useful as a therapeutic or preventive agent, a research reagent, such as an assay reagent, and a diagnostic agent, such as an in vivo diagnostic imaging agent. All isotopic variants of the antibody-immunostimulant conjugate of the present invention, whether radioactive or not, are encompassed by the scope of the present invention.

### <Fusion protein of Enzyme-A and Enzyme-B and method for producing Fc-containing molecule using the fusion protein>

In a further aspect of the present invention, a fusion protein of Enzyme-A and Enzyme-B is provided. Herein, the fusion protein of Enzyme-A and Enzyme-B means a protein in which Enzyme-A and Enzyme-B are bound directly or indirectly. In one aspect of the present invention, the fusion protein of Enzyme-A and Enzyme-B is an immobilized enzyme in which both Enzyme-A and Enzyme-B are immobilized on any solid phase carrier, and in another aspect of the present invention, the fusion protein of Enzyme-A and Enzyme-B is a fusion protein in which Enzyme-A and Enzyme-B are bound directly, or bound via any linker such as any amino acid, a polymer such as PEG, or an oligomeric linker, preferably an amino acid linker. As Enzyme-A and Enzyme-B used in the fusion protein of Enzyme-A and Enzyme-B, any combination of Enzyme-A and Enzyme-B can be used as long as Enzyme-A and Enzyme-B play their respective roles. Examples thereof include the [Enzyme-A]-[Enzyme-B] fusion protein enzyme and the [Enzyme-B]-[Enzyme-A] fusion protein enzyme shown in the following table.

| | **[Enzyme-A]-[Enzyme-B] fusion protein enzyme or [Enzyme-B]-[Enzyme-A] fusion protein enzyme** |
|---|---|
| BA-1 | [Endo-Rp N172H]-[Endo-Si D241M/Q311L] |
| BA-2 | [Endo-Rp N172V]-[Endo-Si D241Q/Q311L] |

The fusion protein of Enzyme-A and Enzyme-B, when used in the production of an Fc-containing molecule, can bring an advantage such as being able to reduce the number of enzymes to be prepared or provided in the production step, which is an advantage obtained independently of Step 2 of the present invention. Therefore, in one aspect of the present invention, provided is a method for producing an Fc-containing molecule having an N297-linked glycan including a glycan derived from a glycan donor molecule, including the following Step 1': reacting an acceptor molecule, which is an Fc-containing molecule having, as an N297-linked glycan, a core GlcNAc to which fucose is optionally added, with a glycan donor molecule including a glycan in the presence of a fusion protein of an endo-β-N-acetylglucosaminidase (Enzyme-A), which prefers, as a substrate, an N297-linked glycan of an Fc-containing molecule and an endo-β-N-acetylglucosaminidase (Enzyme-A), which prefers, as a substrate, a glycan of a glycan donor molecule, so as to obtain a reaction mixture. The descriptions for Step 1 above can be applied as they are to the acceptor molecule, the glycan donor molecule, the reaction conditions, and the like used in Step 1'. In addition, a higher transglycosylation rate can also be achieved by Step 1' followed by Step 2.

### Examples

Hereinafter, the present invention will be specifically described with reference to Examples. What is shown in the Examples is an example of an embodiment of the present invention, and the present invention is not limited thereto.

The protein concentrations described herein were quantified by using an ultratrace spectrophotometer NanoDrop 8000 (manufactured by Thermo Fisher Scientific) or an ultraviolet-visible spectrophotometer system SoloVPE (manufactured by C Technologies).

SGP (sialylglycopeptide) in the Examples can be prepared by the method disclosed in Example 1 of WO2017110984 or the method disclosed in Example 1 of WO2014208742. AG(9)-P in the Examples can be prepared by the method disclosed in Step 1-17A of Example 1-17 of WO2014115797. AG(7)-P in the Examples can be prepared by the method disclosed in Step 1-17B of Example 1-17 of WO2014115797.

([N₃-PEG(3)]₂-SG)-Asn-PEG(3)-N₃ in the Examples can be prepared by the method disclosed in Step 1-12A of Example 1-12 of WO2018003983. ([N₃-PEG(3)]-MSG1)-Asn-PEG(3)-N₃ and ([N₃-PEG(3)]-MSG2)-Asn-PEG(3)-N₃ in the Examples can be prepared by the method disclosed in Step 3 of Example 154 of WO2019065964.

mAb1 in the Examples represents Trastuzumab. (Fucα1,6)GlcNAc-mAb1 represents a hydrolyzed form of a glycan of Trastuzumab, which can be prepared by the method disclosed in Example 3 of WO2017010559. mAb2 in the Examples represents an antibody that can be prepared by the method disclosed in Example 136 of WO2019065964 and SEQ ID NOs: 36 and 52 in WO2019065964. (Fucα1,6)GlcNAc-mAb2 represents a hydrolyzed form of a glycan, (Fucα1,6)GlcNAc-anti-CLDN6 antibody (H1L1), which can be prepared by the method disclosed in Step 1 of Example 61 of WO2019065964. In the Examples, mAb2-[MSG1-N₃]₂ represents anti-CLDN6 antibody (H1L1)-[MSG1-N₃]₂, which can be prepared by the method disclosed in Step 2 of Example 61 of WO2019065964. mAb3 in the Examples represents engineered HER2 antibody 2, which can be prepared by the method disclosed in Reference Example 5 of WO2020050406 and SEQ ID NOs: 28 and 30 in WO2020050406. (Fucα1,6)GlcNAc-mAb3 represents a hydrolyzed form of a glycan, (Fucα1,6)GlcNAc-engineered anti-HER2 antibody 2, which can be prepared by the method disclosed in Step 1 of Example 85 of WO2020050406. In the Examples, mAb3-[SG-N₃]₂ represents engineered anti-HER2 antibody-[MSG1-N₃]₂, which can be prepared by the method disclosed in Step 2 of Example 85 of WO2020050406.

The progress of glycan hydrolysis and transglycosylation reaction was confirmed by using protein gel electrophoresis (WO2013/120066, Huang W, et al., J Am Chem Soc. 2012, 134, 12308-12318). LabChip GX II (manufactured by PerkinElmer) was used as an apparatus for a fully automated protein electrophoresis system and Protein Express Assay LabChip and Protein Express Reagent Kit (manufactured by PerkinElmer) were used as reagents therefor.

### <Example 1> Method for selecting Enzyme-A and Enzyme-B

When Enzyme-A and Enzyme-B work concertedly, direct transfer of a glycan unit of a chemically unactivated glycan donor molecule from the glycan donor molecule to a GlcNAc of an Fc-containing molecule (acceptor molecule) is possible. In the present Example, Enzyme-A is an enzyme having a domain having affinity for an Fc-containing molecule, a domain having affinity for a glycan, and a transglycosylation reaction catalytic domain, and Enzyme-B is an enzyme that activates a fucose-free biantennary glycan donor molecule to a state (activated intermediate) in which Enzyme-A can be used for a transglycosylation reaction to an Fc-containing molecule (see the following schematic diagram or Figure 1). In order to confirm general properties and effective reaction conditions of Enzyme-A and Enzyme-B that can be combined in Example 8 and beyond, the enzymes prepared in Example 2 to Example 6 were designed as shown in Table Y-1 below.

The identity of the enzyme is not limited as long as Enzyme-A and Enzyme-B play their respective roles, and a desired transglycosylated form can be obtained by combining these arbitrarily.

As representative examples of Enzyme-A, representative examples of an Endo-S mutant, representative examples of an Endo-S2 mutant, and representative examples of an Endo-Si mutant, which have residual hydrolysis activity but can be expected to have a suppressed hydrolysis activity as compared with the corresponding respective wild type enzymes, were selected. The transglycosylation activity of Enzyme-B itself is considered to be correlated with the ability thereof to activate a glycan donor, and thus as representative examples of Enzyme-B, representative examples of an Endo-M mutant and representative examples of an Endo-Rp mutant, which are known to have an activity of transglycosylation from SGP, which is a fucose-free biantennary glycan donor molecule, to a GlcNAc derivative, were selected.

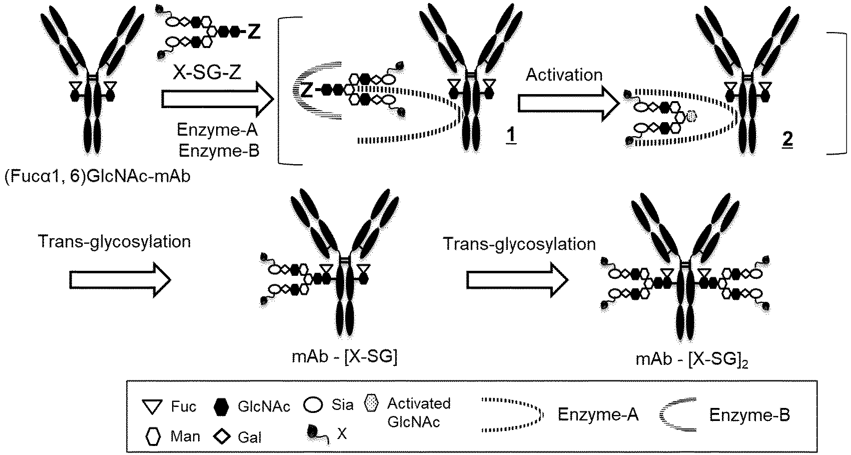

In the above schematic diagram and Figure 1, X on sialic acid represents any substituent including a hydrogen atom. For example, X is PEG-azide. In the figure, Z at the glycan reducing end is any substituent, and is preferably represented by NHR or OR bonded via a nitrogen atom or an oxygen atom. For example, R is a substituent selected from the group consisting of PMP, Me, A-Mor, iPr, nPr, PrOMe, and A-PEG-N₃ wherein A represents an acetyl group in a glycolic acid unit, that is, a moiety present between an anomeric hydroxyl group and an amino group in Mor or PEG.

**Table Y-1. Enzymes prepared in Example 2 to Example 6**

| | **Enzyme-A** | | **Enzyme-B** |
|---|---|---|---|
| A-1 | Endo-Si D241M Q311L | B-1 | Endo-Rp N172H |
| A-2 | Endo-Si D241Q | B-2 | Endo-Rp N172Q |
| A-3 | Endo-Si D241Q E360Q | B-3 | Endo-Rp N172V |
| A-4 | Endo-Si D241M | B-4 | Endo-M N175Q |
| A-5 | Endo-Si D241Q Q311L | B-5 | Endo-M N175Q Y217F |
| A-6 | Endo-Si D241M E360Q | | |
| A-7 | Endo-Si WT | | |
| A-8 | Endo-S D233Q E350Q | | |
| A-9 | Endo-S WT | | |
| A-10 | Endo-S2 D184M | | |
| A-11 | Endo-S2 T138Q | | |
| A-12 | Endo-S2 WT | | |

### <Example 2> Preparation of Endo-Si enzyme

### (Example 2-1) Expression of Endo-Si enzyme

An Endo-Si expression plasmid encoding a gene corresponding to SEQ ID NO: 3 was introduced into the methanol-assimilating yeast Ogataea minuta to obtain an Endo-Si producing strain. Subsequently, methanol-induced expression of Endo-Si protein was carried out under a shaking culture condition or under an aeration and agitation culture condition. In the case of shaking culture, a 500 ml baffled flask (CORNING, 431401) was used, methanol induction was carried out on day 1 after inoculation, the culture was terminated on day 3 after inoculation, and cells were collected by centrifugation. In the case of aeration and agitation culture, a 3 L culture tank (ABLE Corporation, BMS-03KP3 pressurized type) was used, methanol induction was carried out on day 1 after inoculation, culture was terminated on day 2 after inoculation, and cells were collected by centrifugation.

### (Example 2-2) Purification of Endo-Si enzyme

The cells collected in Example 2-1 were disrupted by using YeastBuster Protein Extraction Reagent (Merck, 71186) or a high pressure homogenizer, and centrifuged, and the resulting supernatant was purified by a combination of Capto Chelating, Capto DEAE, and Capto Phenyl ImpRes (Cytiva). Finally, the buffer was replaced with PBS(-) (FUJIFILM Wako Pure Chemical Corporation) by using Amicon Ultra-15, 30 kDa (Merck) to obtain an enzyme solution.

**Table Z1. Endo-Si enzyme solutions**

| **Enzyme-A solution** | **Mutant** | **Concentration (mg/mL)** | **Volume (mL)** |
|---|---|---|---|
| Si-001-1 | D241M Q311L | 3.98 | 5.3 |
| Si-001-2 | D241M Q311L | 4.07 | 225.5 |
| Si-002 | D241Q | 3.99 | 4.8 |
| Si-003 | D241Q E360Q | 3.95 | 13.1 |
| Si-004 | D241M | 3.99 | 6.1 |
| Si-005 | D241Q Q311L | 3.98 | 11.6 |
| Si-006 | D241M E360Q | 3.96 | 6.9 |
| Si-007 | Wild Type (WT) | 4.02 | 27.5 |

### <Example 3> Preparation of Endo-Rp enzyme

### (Example 3-1) Expression of Endo-Rp enzyme

An Endo-Rp expression plasmid encoding a gene corresponding to SEQ ID NO: 5 was introduced into the methanol-assimilating yeast Ogataea minuta to obtain an Endo-Rp producing strain. Subsequently, methanol-induced expression of Endo-Rp protein was carried out under a shaking culture condition or under an aeration and agitation culture condition. In the case of shaking culture, a 1000 ml baffled flask (CORNING, 431403) was used, methanol induction was carried out on day 1 after inoculation, the culture was terminated on day 2 or day 3 after inoculation, and cells were collected by centrifugation. In the case of aeration and agitation culture, a 3 L culture tank (ABLE Corporation, BMS-03KP3 pressurized type) was used, methanol induction was carried out on day 1 after inoculation, culture was terminated on day 4 after inoculation, and cells were collected by centrifugation.

### (Example 3-2) Purification of Endo-Rp enzyme

The cells collected in Example 3-1 were disrupted by using YeastBuster Protein Extraction Reagent (Merck, 71186) or a high pressure homogenizer, and centrifuged, and the resulting supernatant was purified by using any two or all of Capto Chelating, POROS 50 HQ (Thermo Fisher Scientific), and Capto MMC (Cytiva) to obtain an enzyme solution. A portion thereof was concentrated or buffer exchanged by using Amicon Ultra-15, 30 kDa (Merck) to obtain an enzyme solution.

**Table Z2. Endo-Rp enzyme solutions**

| **Enzyme-B solution** | **Mutant** | **Concentration (mg/mL)** | **Volume (mL)** |
|---|---|---|---|
| Rp-001-1 | N172H | 2.79 | 2.7 |
| Rp-001-2 | N172H | 2.80 | 15.3 |
| Rp-001-3 | N172H | 4.07 | 86.1 |
| Rp-002 | N172Q | 3.84 | 3.5 |
| Rp-003 | N172V | 2.89 | 4.24 |

### <Example 4> Preparation of Endo-S2 enzyme

### (Example 4-1) Expression of Endo-S2 enzyme

An Endo-S2 expression plasmid encoding a gene corresponding to SEQ ID NO: 2 was introduced into the methanol-assimilating yeast Ogataea minuta to obtain an Endo-S2 producing strain. Subsequently, methanol-induced expression of Endo-S2 protein was carried out under a shaking culture condition. In the case of shaking culture, a 1000 ml baffled flask (CORNING, 431403) was used, methanol induction was carried out on day 1 after inoculation, the culture was terminated on day 3 after inoculation, and cells were collected by centrifugation.

### (Example 4-2) Purification of Endo-S2 enzyme

The cells collected in Example 4-1 were disrupted by using YeastBuster Protein Extraction Reagent (Merck, 71186), and centrifuged, and the resulting supernatant was purified by using Capto Chelating. Finally, the buffer was replaced with PBS(-) (FUJIFILM Wako Pure Chemical Corporation) by using Amicon Ultra-15, 30 kDa (Merck) to obtain an enzyme solution.

**Table Z3. Endo-S2 enzyme solutions**

| **Enzyme-A solution** | **Mutant** | **Concentration (mg/mL)** | **Volume (mL)** |
|---|---|---|---|
| S2-001 | D184M | 4.00 | 1.54 |
| S2-002 | T138Q | 4.00 | 0.75 |
| S2-003 | Wild Type (WT) | 3.74 | 1.8 |

### <Example 5> Preparation of Endo-S enzyme

### (Example 5-1) Expression of Endo-S enzyme

An Endo-S expression plasmid encoding a gene corresponding to SEQ ID NO: 1 was introduced into the methanol-assimilating yeast Ogataea minuta to obtain an Endo-S producing strain. Subsequently, methanol-induced expression of Endo-S protein was carried out under an aeration and agitation culture condition. A 42 L culture tank (Infors HT) was used, methanol induction was carried out on one day 1 after inoculation, the culture was terminated on day 4 after inoculation, and cells were collected by centrifugation.

### (Example 5-2) Purification of Endo-S enzyme

The cells collected in Example 5-1 were disrupted by using a high pressure homogenizer and centrifuged, and the resulting supernatant was purified by a combination of purification using Capto Chelating, Capto MMC (Cytiva), and POROS 50 HQ (Thermo Fisher Scientific). Finally, the buffer was replaced with PBS(-) (Merck) by using Pellicon 3 cassette, 30 kDa (Merck) to obtain an enzyme solution.

**Table Z4. Endo-S enzyme solutions**

| **Enzyme-A solution** | **Mutant** | **Concentration (mg/mL)** | **Volume (mL)** |
|---|---|---|---|
| S-001 | D233Q E350Q | 3.9 | 863.6 |
| S-002 | Wild Type (WT) | 4.1 | 2021.2 |

### <Example 6> Preparation of Endo-M enzyme

### (Example 6-1) Expression of Endo-M enzyme

An Endo-M expression plasmid encoding a gene corresponding to SEQ ID NO: 4 was introduced into the methanol-assimilating yeast Ogataea minuta to obtain an Endo-M producing strain. Subsequently, methanol-induced expression of Endo-M protein was carried out under an aeration and agitation culture condition. In the case of aeration and agitation culture, a 3 L culture tank (ABLE Corporation, BMS-03KP3 pressurized type) was used, methanol induction was carried out on day 1 after inoculation, culture was terminated on day 2 after inoculation, and cells were collected by centrifugation.

### (Example 6-2) Purification of Endo-M enzyme

The cells collected in Example 6-1 were disrupted by using YeastBuster Protein Extraction Reagent (Merck, 71186), and centrifuged, and the resulting supernatant was purified by using Capto Chelating and Capto DEAE (Cytiva). Finally, the buffer composition was changed to 50 mM Tris, 150 mM NaCl, 30% Glycerol, pH 8.0 to obtain an enzyme solution.

**Table Z5. Endo-M enzyme solutions**

| **Enzyme-B solution** | **Mutant** | **Concentration (mg/mL)** | **Volume (mL)** |
|---|---|---|---|
| M-001 | N175Q | 4.14 | 7.0 |
| M-002 | N175Q Y217F | 4.06 | 14.8 |

### <Example 7> Synthesis of glycan donor

The glycan donor of the present invention can be produced according to the method described later (enzymatic conversion and chemical conversion of a glycan raw material or a glycan intermediate). After completion of each reaction, the target compound of each reaction is collected from the reaction mixture according to a conventional method. For example, the target compound can be obtained by appropriately neutralizing the reaction mixture, removing any insoluble matter present by filtration, and distilling off the solvent. The obtained compound can be separated and purified by a conventional method such as silica gel column chromatography or ion exchange chromatography, if necessary. Alternatively, the obtained compound can be purified by reprecipitation, recrystallization, and ultrafiltration.

The masses of the glycan derivatives described herein were confirmed by the following method. Q Exactive (manufactured by Thermo Fisher Scientific Inc), Ultimate 3000 (manufactured by Thermo Fisher Scientific Inc), and CORETECS UPLC C18 (manufactured by Waters Inc) (1.6 µm, 2.1 × 50 mm) were used as apparatuses, acetonitrile was used as mobile phase A, an aqueous solution containing 0.1% formic acid was used as mobile phase B, and a gradient changing from 2% to 95% of mobile phase A over 4 minutes was used to carry out analysis at a flow rate of 0.4 mL/min and 40°C.

Alternatively, Xevo G2-XS Family mass spectrometer (manufactured by Waters) and ACQUITY BEH C18 (manufactured by Waters) (1.7 µm, 2.1 × 50 mm) were used as apparatuses, acetonitrile was used as mobile phase A, a 10 mM ammonium acetate aqueous solution was used as mobile phase B, and a gradient changing from 1% to 30% of mobile phase A over 5 minutes was used to carry out analysis at a flow rate of 0.8 mL/min and 40°C.

### (Example 7-1) Synthesis of glycan donor raw materials (glycan raw material 3 and glycan raw material 3-1)

### (Synthesis scheme)

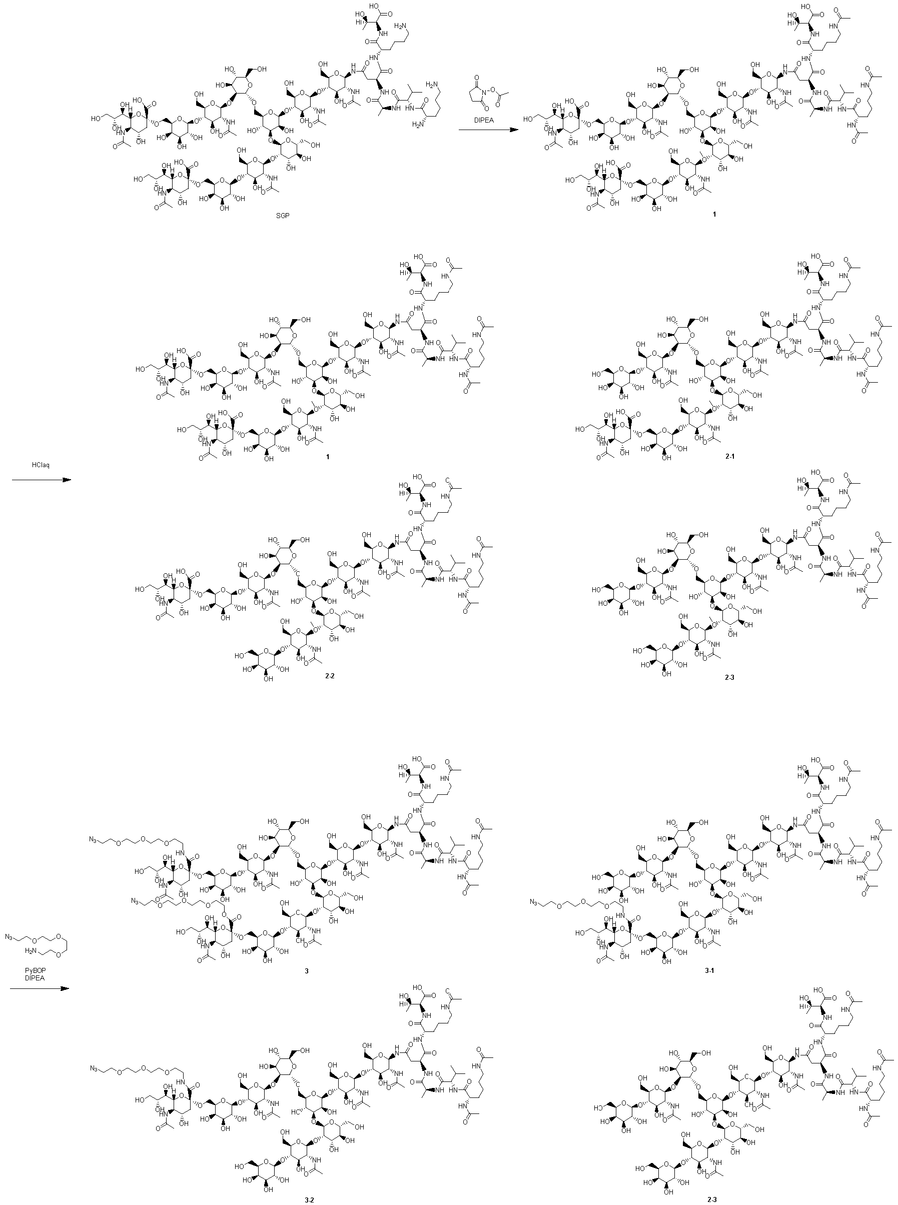

### (7-1a) [N-Acetylation and hydrolysis reaction]

SGP (G-1) (25.0 g) was dissolved in purified water (100 mL), N,N-diisopropylethylamine (6.77 g) dissolved in acetonitrile (50 mL) and N-(acetoxycarbonyloxy)succinimide (8.22 g) dissolved in acetonitrile (50 mL) was sequentially added, and the resulting mixture was stirred at room temperature for 2 hours. After confirming the completion of the reaction, a sodium hydroxide aqueous solution (5 N, 25 mL) was added, and the resulting mixture was stirred for 1 hour.

This solution was cooled, then hydrochloric acid (5 N, 36.6 g) was added while checking the pH with a pH meter to adjust the pH to 2.20, and then the resulting mixture was stirred for 3 and a half hours while keeping the temperature at 57 to 59°C. The mixture was cooled to room temperature, and then a sodium hydroxide aqueous solution (5 N, 25 mL) was added while checking the pH with a pH meter to adjust the pH to 5.98.

### [Purification]

A portion corresponding to 14 g of SGP of the solution adjusted to pH 5.98 was purified by using SEPHADEX G-25 Fine (GE HealthCare Japan) (purified water was used as the eluant). A fraction including the target product was collected, concentrated under reduced pressure and freeze-dried to obtain a solid (13.5 g), which was a mixture of glycan raw material 1, glycan raw material 2-1, glycan raw material 2-2, and glycan raw material 2-3.

### (7-1b) [Condensation reaction]

The solid (11.0 g) obtained in Step (7-1a) was dissolved in DMF (121.0 mL) and DMSO (33.0 mL), N,N-diisopropylethylamine (3.16 g, 24.4 mmol) and 11-azido-3,6,9-trioxaundecan-1-amine (5.33 g, 24.4 mmol) were sequentially added, and the resulting mixture was cooled to -7°C. A solution of 1H-benzotriazol-1-yloxytripyrrolidinophosphonium hexafluorophosphate (12.72 g, 24.4 mmol) in DMF (22.0 mL) solution was added to the mixture over 15 minutes, and then the resulting mixture was stirred near -5°C for 1 hour.

### [Crude purification]

After completion of the reaction, the reaction solution was warmed to room temperature and added dropwise to ethanol (550 mL). Furthermore, the reaction vessel was washed with ethanol (110 mL) and rinsed. The resulting slurry was collected by filtration, and the cake was washed with ethanol (110 mL) and then dried under reduced pressure at room temperature to obtain 15.7 g of a crude reaction product.

Purified water (198 g) was added to the crude reaction product to dissolve the same. A slight amount of the residue left was removed by filtration, and the obtained filtrate was concentrated under reduced pressure and then adjusted to 88 g with purified water. This aqueous solution was added dropwise to ethanol (550 mL), and furthermore, the aqueous solution was rinsed with purified water (22 mL). The resulting slurry was collected by filtration, and the cake was washed with a mixture of ethanol (275 mL) and purified water (55 mL) to obtain a wet solid (162.0 g). This was dissolved in purified water (220 mL), and then the resulting solution was concentrated to half the volume thereof by concentration under reduced pressure to remove ethanol. This was freeze-dried to obtain a crude product (8.81 g), which was a mixture of glycan raw material 3, glycan raw material 3-1, glycan raw material 3-2, and glycan raw material 2-3.

### (7-1c) [Preparative isolation of glycan raw material 3 and glycan raw material 3-1 using Triart C18]

A mixture of two lots on different scales obtained by the method described in Step (7-1b), that is, a crude product (10.05 g in total), which was a mixture of glycan raw material 3, glycan raw material 3-1, glycan raw material 3-2, and glycan raw material 2-3, was separated and purified by using acetonitrile and purified water as eluents, using K-Prep Lab 100G (manufactured by YMC) as an apparatus, and using Triart C18 (manufactured by YMC) as a column. The main peak detected by UV detection (210 nm) was collected and concentrated under reduced pressure. This concentrate was freeze-dried to obtain the target compound glycan raw material 3 (2.09 g) and the target compound glycan raw material 3-1 (2.02 g) as white powders.

### (Example 7-5) Synthesis of glycan donor (G-10)

### Synthesis method 1 of G-10

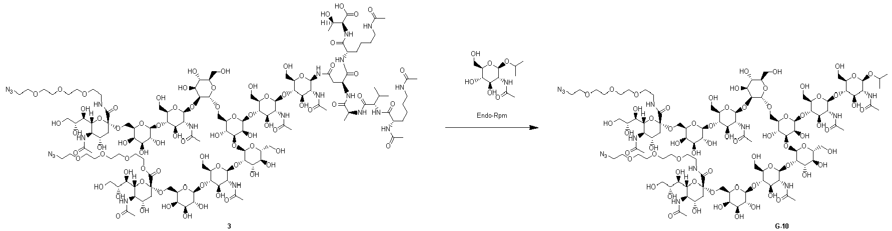

### (7-5-1) [Glycan exchange reaction]

Glycan raw material 3 (200 mg) obtained in Step (7-1c) and 1-Methylethyl 2-(acetylamino)-2-deoxy-β-D-glucopyranoside (733 mg, 50 equiv) were dissolved in phosphate buffer (0.5 mL, pH 6.7, 0.2 mol/L), and then the resulting mixed solution was heated to 50°C. Endo-Rp N172Q (1.3 mg) was added to this mixed solution, and the resulting mixture was shaken at that temperature for 90 hours.

### [Purification]

After completion of the reaction, 8 mL of purified water was added to the reaction solution at room temperature, and then insoluble matter was removed by filtration. This filtrate was separated and purified by using acetonitrile and water as eluents and using YMC-Actus Triart C18 (manufactured by YMC) as a column. The main peak detected by UV detection (210 nm) was collected, concentrated under reduced pressure, and freeze-dried to obtain the target product G-10 (82 mg). ESI-MS: Calcd for C103H176N14O66: [M+2H]²⁺ 1333.5496 (most abundant mass), Found 1333.5450

### Synthesis method 2 of G-10

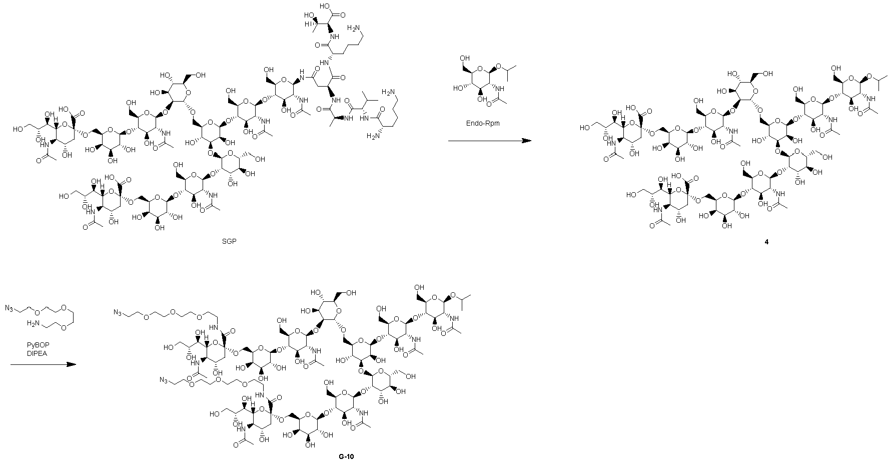

### (7-5-2a) [Glycan exchange reaction]

SGP (G-1) (20.0 g) and 1-Methylethyl 2-(acetylamino)-2-deoxy-β-D-glucopyranoside (36.8 g, 20 equiv) were dissolved in phosphate buffer (280 mL, pH 5.5, 0.2 mol/L), and then the resulting mixed solution was heated to 50°C. Endo-Rp N172H (9.1 mg) was added to this mixed solution, and the resulting mixture was stirred at that temperature for 72 hours.

### [Purification]

After completion of the reaction, the reaction solution was filtered at room temperature. A portion of this filtrate was purified by Sartocon Hydrosart-2 kDa (Sartorius) followed by Amberlite(registered trademark) IR120 H form (Merck) and then freeze-dried to obtain solid 4 (3.72 g from SGP 5.6 g).

### (7-5-2b) [Condensation reaction]

Solid 4 (3.0 g) obtained in Step (7-5-2a) was dissolved in DMF (59.1 mL) and purified water (0.9 mL), and then N,N-diisopropylethylamine (1.03 g, 7.94 mmol) and 11-azido-3,6,9-trioxaundecan-1-amine (867 mg, 3.97 mmol) were added. To this solution, 1H-benzotriazol-1-yloxytripyrrolidinophosphonium hexafluorophosphate (6.20 g, 11.9 mmol) was added in three portions at 20°C, and then the resulting mixture was stirred at that temperature for 23 hours.

### [Purification]

After the reaction, the reaction solution was diluted with purified water, and then purified and concentrated with Sartocon Hydrosart-2 kDa (Sartorius). This concentrate was separated and purified by using acetonitrile and water as eluents and using YMC-Actus Triart Prep C18-S (manufactured by YMC) as a column. The main peak detected by UV detection (210 nm) was collected and concentrated under reduced pressure to obtain the target product G-10 (2.77 g).

### (Example 7-9) Synthesis of glycan donor (G-14)

### Synthesis method 1 of G-14

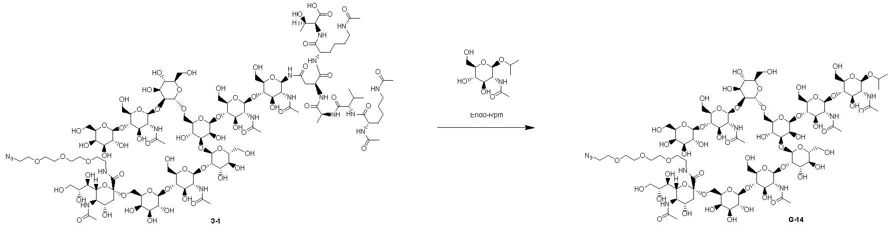

### (7-9-1) [Glycan exchange reaction]

Glycan raw material 3-1 (400 mg) obtained in Step (7-1c), 1-Methylethyl 2-(acetylamino)-2-deoxy-β-D-glucopyranoside (679 mg, 20 equiv), and Endo-Rp N172Q (4.2 mg) were heated to 50°C in phosphate buffer (13.0 mL, pH 6.8, 0.2 mol/L). This mixture was shaken at that temperature for 6 days.

### [Purification]

After completion of the reaction, the reaction solution was diluted with purified water at room temperature, and insoluble matter was removed by filtration. The filtrate was purified and concentrated with Vivaspin 15R, 2,000 MWCO Hydrosart (Sartorius). This concentrate was separated and purified by using acetonitrile and water as eluents and using Sfaer C18 (manufactured by Biotage) as a column. The main peak detected by UV detection (210 nm) was collected and concentrated under reduced pressure to obtain the target product G-14 (174 mg). ESI-MS: Calcd for C84H143N9O56: [M+2H]²⁺ 1087.9382 (most abundant mass), Found 1087.9357

### Synthesis method 2 of G-14

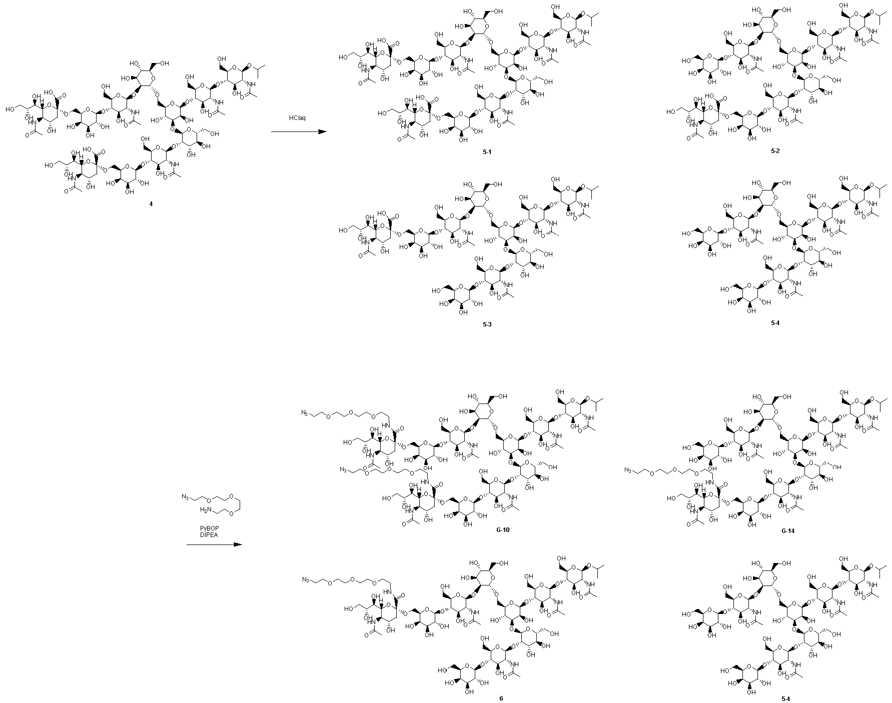

### (7-9-2a) [Hydrolysis reaction]

Water (20 mL) was added to solid 4 (1.0 g) obtained in Step (7-5-2a) to dissolve the same, and then the pH of the resulting solution was adjusted to 2.5 by using dilute hydrochloric acid (1.0 mol/L). This aqueous solution was heated to 60°C and stirred at that temperature for 90 minutes.

### [Purification]

After completion of the reaction, the aqueous solution was cooled to room temperature and then adjusted to pH 7.0 by using a NaOH aqueous solution (1 mol/L). The aqueous solution was purified by using Sartocon Hydrosart-2 kDa (Sartorius) and then freeze-dried to obtain a solid (1.06 g), which was a mixture of glycan raw material 5-1, glycan raw material 5-2, glycan raw material 5-3, and glycan raw material 5-4.

### (7-9-2b) [Condensation reaction]

DMF (8.0 mL) and N,N-diisopropylethylamine (0.13 g, 1.0 mmol) were added to the solid (0.8 g) obtained in Step (7-9-2a), which was a mixture of glycan raw material 5-1, glycan raw material 5-2, glycan raw material 5-3, and glycan raw material 5-4, and the resulting mixture was cooled to 0°C. To this slurry solution, 11-azido-3,6,9-trioxaundecan-1-amine (0.23 g, 1.0 mmol) and 1H-benzotriazol-1-yloxytripyrrolidinophosphonium hexafluorophosphate (0.55 g, 1.0 mmol) were sequentially added. The resulting mixture was stirred at 0°C for 30 minutes, and then stirred at room temperature for 1.5 hours. The slurry was not dissolved, and then the mixture was cooled again to 0°C, and N,N-diisopropylethylamine (0.13 g, 1.0 mmol), 11-azido-3,6,9-trioxaundecan-1-amine (0.23 g, 1.0 mmol), and 1H-benzotriazol-1-yloxytripyrrolidinophosphonium hexafluorophosphate (0.55 mg, 1.0 mmol) were sequentially added. The resulting mixture was stirred at room temperature for 13.5 hours and then cooled to 0°C, and further, 1H-benzotriazol-1-yloxytripyrrolidinophosphonium hexafluorophosphate (0.41 g, 0.78 mmol), 11-azido-3,6,9-trioxaundecan-1-amine (57 mg, 0.26 mmol), and N,N-diisopropylethylamine (33 mg, 0.26 mmol) were sequentially added. The resulting mixture was stirred at room temperature for 1 hour, and water (68 mL) was added to terminate the reaction to obtain a reaction solution including G-14, G-10, glycan raw material 6, and glycan raw material 5-4.

### [Purification]

The reaction solution was further diluted with purified water, and then purified and concentrated with Sartocon Hydrosart-2 kDa (Sartorius). This concentrate was separated and purified by using acetonitrile and water as eluents and using Triart C18 (manufactured by YMC) as a column. The main peak detected by UV detection (210 nm) was collected and concentrated under reduced pressure. This concentrate was freeze-dried to obtain the target compound G-14 (98 mg) as a white powder.

### (Example 7-2) Synthesis of glycan donor (G-7)

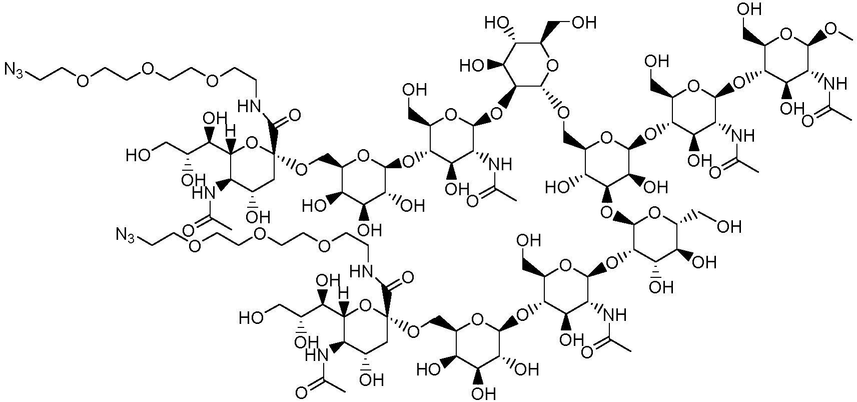

G-7 (64.9 mg) was obtained in the same manner as in Step (7-5-1) described in "Synthesis method 1 of G-10" by using glycan 3 (200 mg) synthesized in Step (7-1c) and Methyl 2-(acetylamino)-2-deoxy-β-D-glucopyranoside (655 mg, 50 equiv). ESI-MS: Calcd for C101H172N14O66: [M+2H]²⁺ 1319.5340 (most abundant mass), Found 1319.5292

### (Example 7-3) Synthesis of glycan donor (G-8)

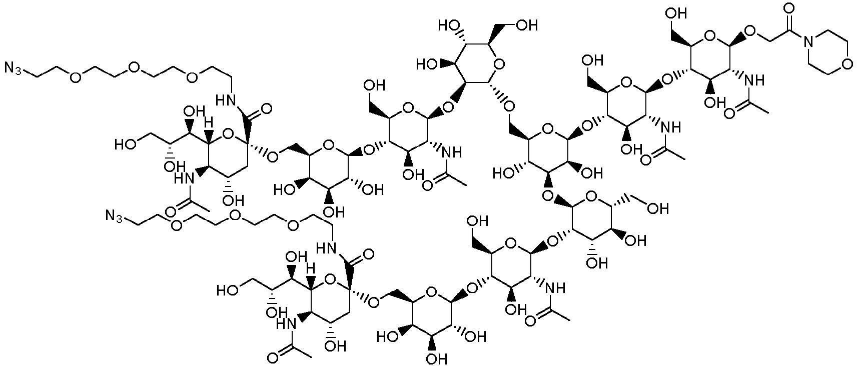

G-7 (32.0 mg) was obtained in the same manner as in Step (7-5-1) described in "Synthesis method 1 of G-10" by using glycan 3 (200 mg) synthesized in Step (7-1c) and 2-(Morpholin-4-yl)-2-oxoethyl 2-(acetylamino)-2-deoxy-β-D-glucopyranoside (194 mg, 20 equiv).
ESI-MS: Calcd for C106H179N15O68: [M+2H]²⁺ 1376.0578 (most abundant mass), Found 1376.0525

### (Example 7-4) Synthesis of glycan donor (G-9)

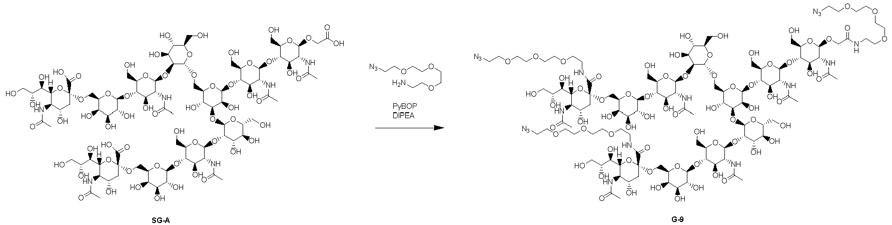

G-9 (23.8 mg) was obtained in the same manner as in Step (7-5-2b) described in "Synthesis method 2 of G-10" except that SG-A (30 mg) was used as a raw material.
ESI-MS: Calcd for C110H188N18070: [M+2H]²⁺ 1441.5925 (most abundant mass), Found 1733.78.

### (Example 7-6) Synthesis of glycan donor (G-11)

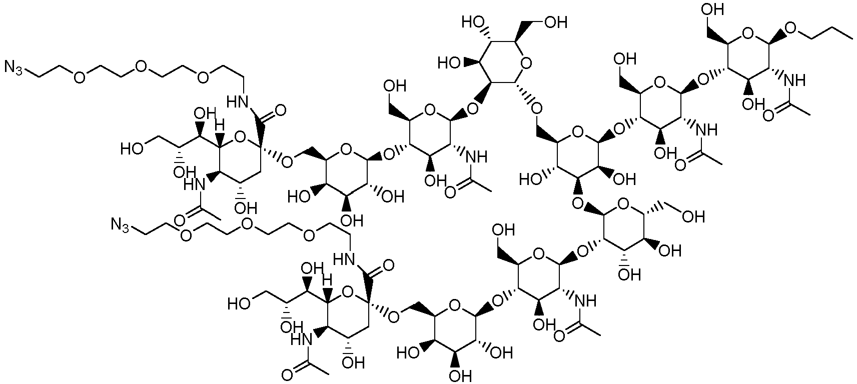

G-11 (80.9 mg) was obtained in the same manner as in Step (7-5-1) described in "Synthesis method 1 of G-10" by using glycan 3 (200 mg) synthesized in Step (7-1c) and Propyl 2-(acetylamino)-2-deoxy-β-D-glucopyranoside (733 mg, 50 equiv). ESI-MS: Calcd for C103H176N14O66: [M+2H]²⁺ 1333.5496 (most abundant mass), Found 1333.5471

### (Example 7-7) Synthesis of glycan donor (G-12)

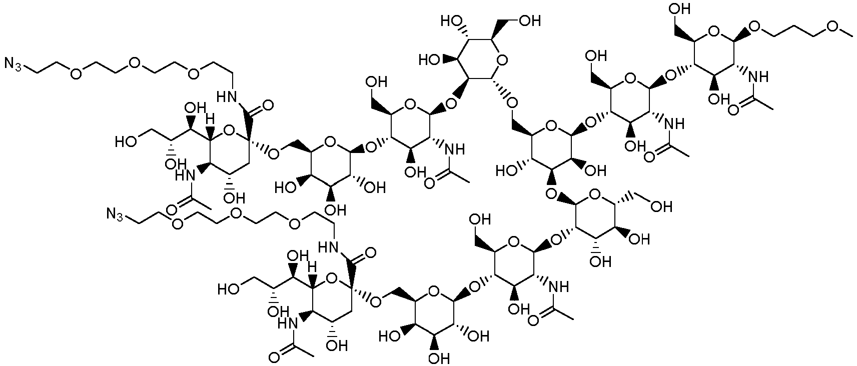

G-12 (70.0 mg) was obtained in the same manner as in Step (7-5-1) described in "Synthesis method 1 of G-10" by using glycan 3 (200 mg) synthesized in Step (7-1c) and 3-Methoxypropyl 2-(acetylamino)-2-deoxy-β-D-glucopyranoside (816 mg, 50 equiv).
ESI-MS: Calcd for C104H178N14O67: [M+2H]²⁺ 1348.5549 (most abundant mass), Found 1348.5491

### (Example 7-8) Synthesis of glycan donor (G-13)

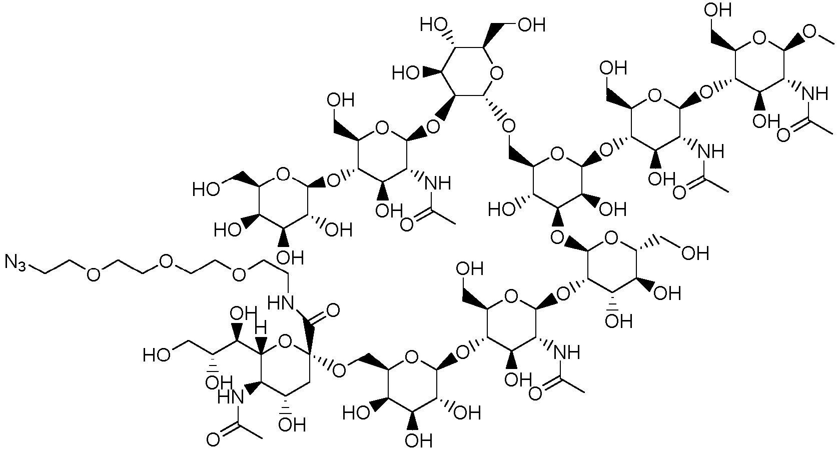

G-13 (50.0 mg) was obtained in the same manner as in Step (7-9-1) described in "Synthesis method 1 of G-14" by using glycan 3-1 (150 mg) synthesized in Step (7-1c) and Methyl 2-(acetylamino)-2-deoxy-β-D-glucopyranoside (569 mg, 50 equiv).
ESI-MS: Calcd for C82H139N9O56: [M+2H]²⁺ 1073.9226 (most abundant mass), Found 1073.9198

### (Example 7-10) Synthesis of [N₃-PEG(3)]₂-SGP-PEG(3)-N₃ (glycan donor G-15)

### (7-10a) Synthesis of SGP-tri-Fmoc form

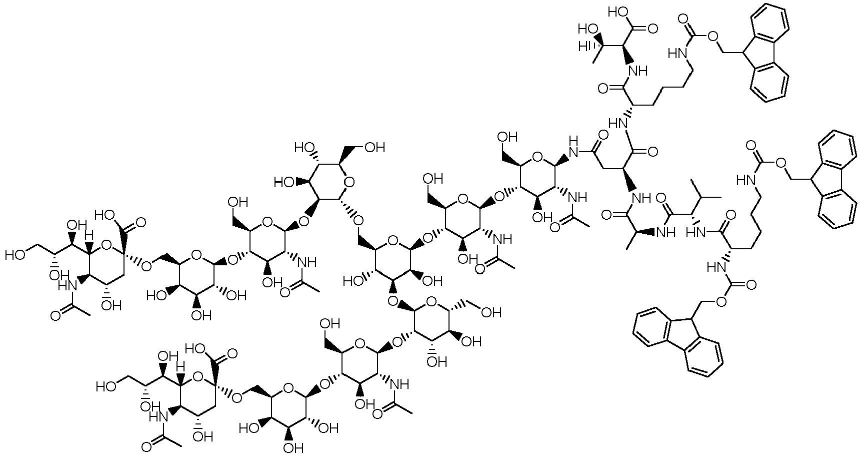

### [Fmoc protection of amino group]

N-[(9H-Fluoren-9-ylmethoxy)carbonyloxy]succinimide (188 mg, 0.56 ml) and N,N-diisopropylethylamine (0.12 ml, 0.70 mmol) were added to a mixed solution of a solution of SGP (200 mg) in N,N-dimethylformamide (2.1 ml)/distilled water (0.7 ml), and the resulting mixture was stirred at room temperature for 30 minutes.

### [Crude purification]

After completion of the reaction, the reaction solution was transferred to a centrifuge tube (50 ml) to which diethyl ether (30 ml) was added in advance. The reaction solution was separated into two layers by using a small centrifuge (Hitachi Koki, CF16RX II), and the supernatant was removed. This operation was repeated twice. Subsequently, methyl alcohol (2 ml) was added, ethyl acetate (20 ml) was added, a solid was precipitated by using a small centrifuge (Hitachi Koki, CF16RX II), and the supernatant was removed. This operation was repeated twice. Subsequently, the solid was washed with an appropriate amount of diethyl ether and then dried under reduced pressure to obtain a solid (412 mg) including the target compound. The solid was used for the next reaction without further purification. ESI-MS: Calcd for C157H219N15O76: [M+3H]³⁺ 1178.13 (most abundant mass), Found 1178.13.

### (7-10b) Synthesis of [N₃-PEG(3)]₂-SGP-PEG(3)-N₃ (glycan donor G-15)

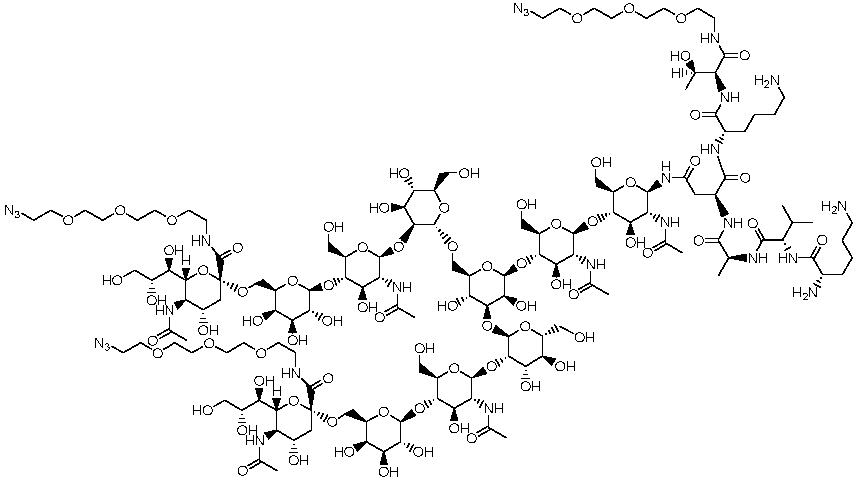

### [Condensation reaction]

A solution of 11-azido-3,6,9-trioxaundecan-1-amine (127 mg, 0.58 mmol) in N,N-dimethylformamide (1.6 ml), and N,N-diisopropylethylamine (0.18 ml, 1.40 mmol) were added to a mixed solution of the solid (412 mg) obtained in Step (7-10a) and 1H-benzotriazol-1-yloxytripyrrolidinophosphonium hexafluorophosphate (243 mg, 0.47 mmol) in N,N-dimethylformamide (6.7 ml), and the resulting mixture was stirred at 37°C for 1.5 hours and then stirred at room temperature for 19 hours.

### [Crude purification]

After completion of the reaction, the reaction solution (2.5 mL) was transferred to a centrifuge tube (50 ml) to which diethyl ether (35 ml) was added in advance. A solid was precipitated by using a small centrifuge (Hitachi Koki, CF16RX II), and the supernatant was removed. This operation was repeated until the reaction solution was exhausted. Subsequently, methyl alcohol (3 ml) was added, and the precipitate was broken up with a spatula. After that, ethyl acetate (30 ml) was added to separate out a solid, then the solid was precipitated by using a small centrifuge (Hitachi Koki, CF16RX II), and the supernatant was removed. This operation was repeated three times. Subsequently, the solid was washed with an appropriate amount of diethyl ether and then dried under reduced pressure to obtain a solid.

### [Deprotection of Fmoc group]

N,N-Dimethylformamide (5.0 ml) and piperidine (0.35 ml) were added to the above solid, and the resulting mixture was stirred at 37°C for 4 hours. After that, N,N-dimethylformamide (3.3 ml) was added, and the resulting mixture was stirred at room temperature for 16 hours.

### [Crude purification]

After completion of the reaction, the reaction solution (2.5 mL) was transferred to a centrifuge tube (50 ml) to which diethyl ether (35 ml) was added in advance. A solid was precipitated by using a small centrifuge (Hitachi Koki, CF16RX II), and the supernatant was removed. This operation was repeated until the reaction solution was exhausted. Subsequently, methyl alcohol (8 ml) was added, and the precipitate was broken up with a spatula. After that, ethyl acetate (16 ml) was added to separate out a solid, then the solid was precipitated by using a small centrifuge (Hitachi Koki, CF16RX II), and the supernatant was removed. This operation was repeated twice. Subsequently, the solid was washed with an appropriate amount of diethyl ether and then dried under reduced pressure to obtain a solid.

### [Purification]

The obtained solid was dissolved in a 0.1% trifluoroacetic acid aqueous solution. A 0.1% trifluoroacetic acid aqueous solution and a 0.1% trifluoroacetic acid acetonitrile solution were used as eluents, Purif-Rp2 (manufactured by Shoko Scientific) was used as an apparatus, and Inertsil ODS-3 (manufactured by GL Science) was used as a column. A fraction including the target product according to UV detection (220 nm) during the elution were freeze-dried. The freeze-dried fraction was dissolved again in distilled water, and before freeze-drying, an appropriate amount of aqueous ammonia was added to confirm that the solution was not acidic, and the solution was freeze-dried again to obtain the target compound G-15 (90 mg) as a freeze-dried powder.
ESI-MS: Calcd for C136H237N27O76: [M+2H]²⁺ 1733.78 (most abundant mass), Found 1733.78.

### (Example 7-11) Preparation of mixed solution of glycan donor raw materials (glycan raw material 8-1, glycan raw material 8-2, and glycan raw material 8-3)

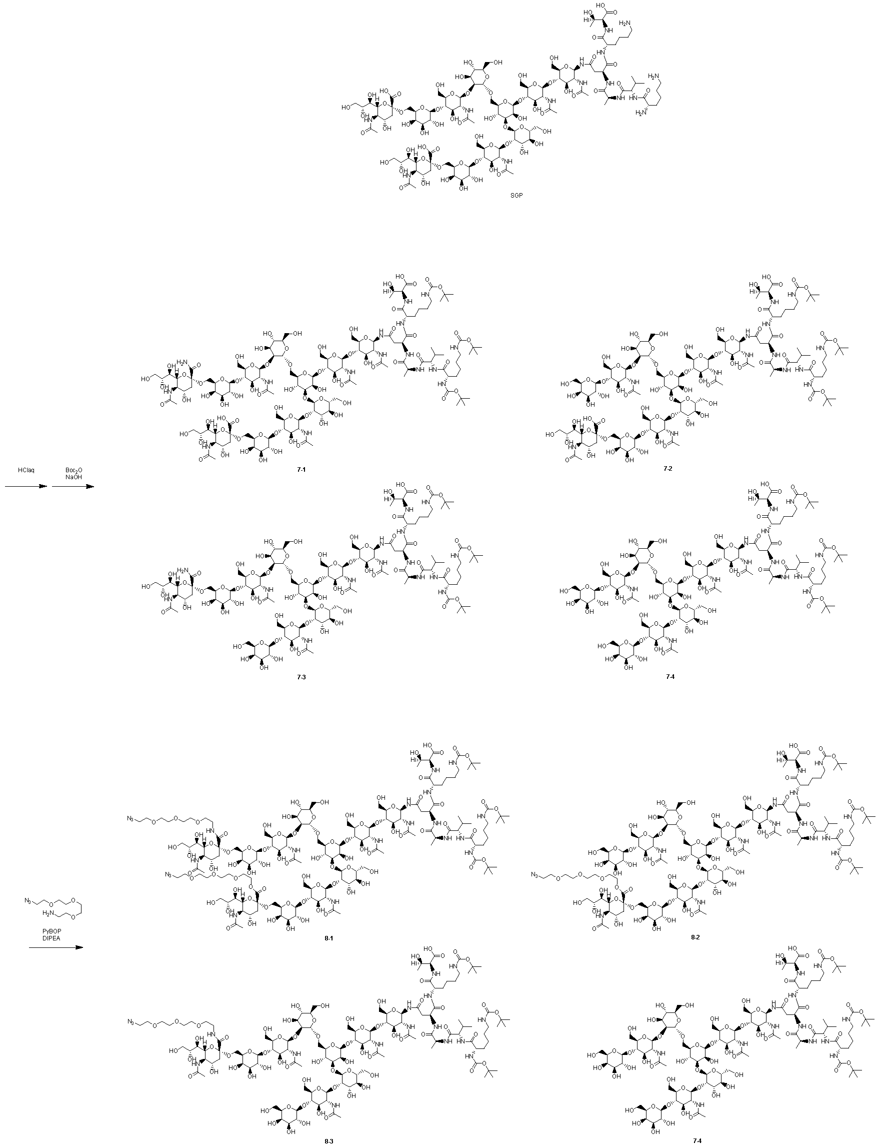

### (7-11a) [Hydrolysis reaction and N-Bocylation]

SGP (G-1) (10.0 g) was dissolved in purified water (100 mL), hydrochloric acid (1 N) was added while checking the pH with a pH meter to adjust the pH to 2.4, and then the resulting solution was stirred for 3 hours while keeping the temperature at 60°C. The solution was cooled to room temperature, and then a sodium hydroxide aqueous solution (1 N) was added while checking the pH with a pH meter to adjust the pH to 7.5. To one-fifth of the resulting solution, tert-butyl dicarbonate (708 mg) dissolved in tetrahydrofuran (6 mL) was added, then a sodium hydroxide aqueous solution (1 N) was added to adjust the pH to 12, and the resulting solution was stirred for 2 hours. Furthermore, tert-butyl dicarbonate (359 mg) was added, a sodium hydroxide aqueous solution (1 N, 2.3 mL) was added, and the resulting mixture was further stirred for 3 hours. Hydrochloric acid (1 N) was added to adjust the pH to 8 to obtain a solution including glycan raw material 7-1, glycan raw material 7-2, glycan raw material 7-3, and glycan raw material 7-4.

### (7-1 1b) [Condensation]

DMF (10 mL) was added to half the solution including glycan raw material 7-1, glycan raw material 7-2, glycan raw material 7-3, and glycan raw material 7-4 obtained in Step (7-11a), and then the operation of adding acetonitrile (10 mL) and distilling off acetonitrile under reduced pressure was repeated four times to carry out a dehydration operation. 11-Azido-3,6,9-trioxaundecan-1-amine (0.46 g) was added to the resulting DMF solution, and the resulting mixture was cooled to 0°C. Subsequently, 1H-benzotriazol-1-yloxytripyrrolidinophosphonium hexafluorophosphate (1.09 g) was added, and then the resulting mixture was stirred at room temperature for 1 hour to obtain a solution including glycan raw material 8-1, glycan raw material 8-2, glycan raw material 8-3, and glycan raw material 7-4.

### (Example 7-12) Synthesis of glycan donor (G-16)

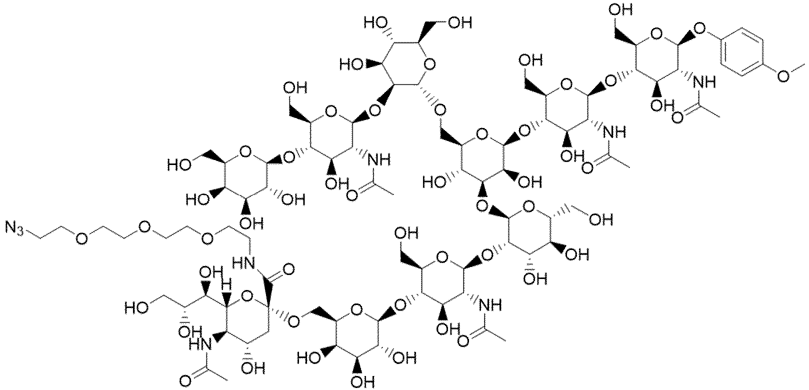

### (7-12a) [Glycan conversion reaction]

The solution of the mixture including glycan raw material 8-2 obtained in Step (7-11b) was subjected to buffer replacement with phosphate buffer (9.2 mL, pH 6.7, 0.2 mol/L) by using Amicon Ultra-15, 3 kDa (Merck). To this solution, 4-Methylphenyl 2-(acetylamino)-2-deoxy-β-D-glucopyranoside (280 mg, 7 equiv), toluene (1.2 mL), and Endo-Rp N172Q (2.6 mg) were added, the temperature was kept at 50°C, and the resulting mixture was stirred for 150 hours.

### (7-12b) [Purification]

One-third of the reaction solution obtained in Step (7-12a) was separated and purified by using acetonitrile and water as eluents and using X-Bridge C18 (manufactured by Waters) as a column. The peak of the target product detected by UV detection (210 nm) was collected and concentrated under reduced pressure. This concentrate was freeze-dried to obtain the target compound G-16 (16.3 mg) as a white powder.
ESI-MS: Calcd for C88H143N9O57: [M+2H]²⁺ 1119.94 (most abundant mass), Found 1119.93

### (Example 7-13) Synthesis of glycan donor (G-6)

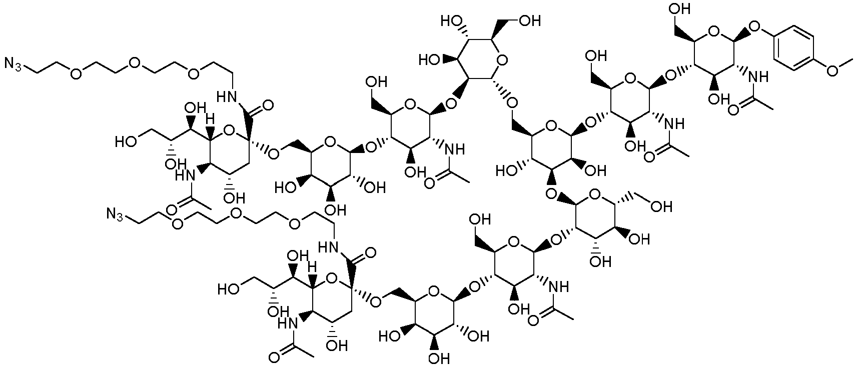

### (7-13) [Glycan exchange reaction]

G-6 (35 mg) was obtained in the same manner as in Step (7-5-1) described in "Synthesis method 1 of G-10" by using glycan raw material 3 (100 mg) obtained in Step (7-1c) and 4-Methylphenyl 2-(acetylamino)-2-deoxy-β-D-glucopyranoside (182 mg, 20 equiv).
ESI-MS: Calcd for C107H176N14O67: [M+2H]²⁺ 1365.55 (most abundant mass), Found 1365.54

When the direct transglycosylation reaction proceeds in the form shown in Figure 1 conceived this time, it is considered that in the case of the biantennary glycan structure recognized by Enzyme-B, it is not necessary to limit the structure on the non-reducing end side to an N-linked type, and that the target reaction proceeds with any structure. For example, the structure shown below or in Figure 2 can be used as a glycan donor.

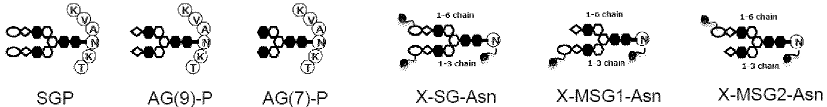

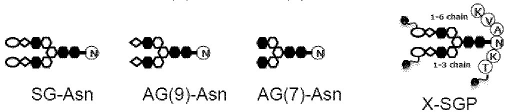

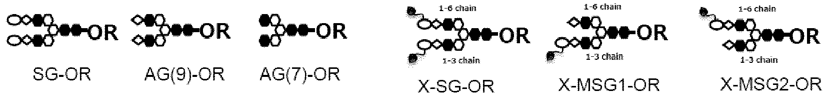

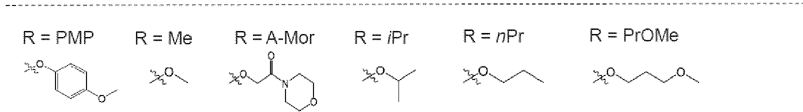

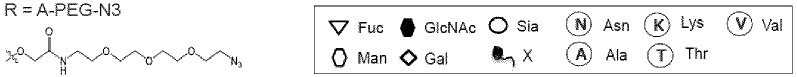

Therefore, in the following Examples, the following glycan derivatives were appropriately used.

**Table Z6. Glycan donors**

| **Glycan donor** | **Structure** | **Preparation method** |
|---|---|---|
| G-1 | SGP | Method disclosed in Example 1 of WO2017110984 or Example 1 of WO2014208742 |
| G-2 | AG(9)P | Step 1-17A of Example 1-17 of WO2014115797 |
| G-3 | X-SG-Asn | Step 1-1 2A of Example 1-12 of WO2018003983 |
| G-4 | X-MSG1-Asn | Step 3 of Example 154 of WO2019065964 |
| G-5 | X-MSG2-Asn | Step 3 of Example 154 of WO2019065964 |
| G-6 | X-SG-OPMP | Example 7-13 |
| G-7 | X-SG-OMe | Example 7-2 |
| G 8 | X-SG-OA-Mor | Example 7-3 |
| G-9 | X-SG-OA-PEG(3)-N₃ | Example 7-4 |
| G-10 | X-SG-OiPr | Example 7-5 |
| G-11 | X-SG-OnPr | Example 7-6 |
| G-12 | X-SG-OPrOMe | Example 7-7 |
| G-13 | Y-MSG1-OMe | Example 7-8 |
| G-14 | X-MSG1-OiPr | Example 7-9 |
| G-15 | X-SGP | Example 7-10 |
| G-16 | X-MSG1-OPMP | Example 7-12 |
| G-17 | AG(7)P | Step 1-17B of Example 1-17 of WO2014115797 |

| | | |
|---|---|---|
| (in the above table, "X" represents N₃-PEG(3)) | | |

### <Example 8> Reaction condition for "Step 1": Antibody concentration

It has been reported that when a generally widely used glycan donor or a chemically synthesized oxazoline form is used, glycation, which is a non-Endo-enzyme-mediated chemical reaction, of Lys in the antibody proceeds easily. Furthermore, in order to suppress this side reaction, it was recommended that the reaction be carried out at a weak acidity (pH 6.50) and an oxazoline form solution be repeatedly added to be reacted, that is, the reaction solution be diluted (Ref. Bioconjugate Chemistry 2019 30(5), 1343-1355). In this report, the antibody concentration of the reaction solution was set to 40 µM or less. In addition, a report on a detailed protocol of a transglycosylation reaction using a chemically synthesized oxazoline form also discloses that the concentration is "final concentration: Herceptin = 10 mg/ml" (Ref. Nature Protocols volume 12, pages 1702-1721 (2017)). As described above, the idea of reducing the total volume of the reaction solution, that is, carrying out a transglycosylation reaction with a high concentration antibody solution is not common in a transglycosylation reaction to an antibody.

On the other hand, when the direct transglycosylation reaction proceeds in the form shown in Figure 1 conceived this time, it is very effective to reduce the total volume of the reaction solution, that is, to carry out the reaction under a condition that increases the frequency of contact between molecules and at a high concentration. Even if a stable glycan donor representatively shown in Table Z6 and an antibody are mixed at a high concentration, glycation, which is a kind of chemical reaction, does not occur. This is because under the reaction condition for "Step 1" in Figure 1, the chemically synthesized oxazoline form solution, which causes the side reaction, is not added.

The acceptor molecule (for example, antibody) concentration in the reaction system applicable to the production method of the present invention is not limited, and in Example 8, in order to confirm that an antibody having a moderate (>80%) or higher transglycosylation rate can be obtained in the transglycosylation reaction regardless of the acceptor molecule (antibody) concentration in the reaction system, the antibody concentration in the system was set to 20 mg/mL to 80 mg/mL.

### (Example 8-1) When antibody concentration in reaction system is 20 mg/mL [Transglycosylation reaction standard protocol 1]

(Fucα1,6)GlcNAc-mAb1 (6 mg), a glycan donor (20 equivalents), Enzyme-A (1.6 to 1.7% w/w), Enzyme-B (0.2% w/w), and an appropriate amount of a 50 mM Tris-HCl buffer solution (pH 7.25) were added to make a total solution volume of 300 µL, and the resulting mixture was reacted at 30°C for 28 hours. Sampling was carried out 2 hours, 4 hours, 6 hours, 8 hours, 24 hours, and 28 hours after the start of the reaction. After completion of the reaction, the pH of the reaction solution was measured. At the time of sampling, a portion of the reaction solution was withdrawn, diluted with purified water such that the concentration of the antibody contained was 1 mg/mL, and stored frozen until the start of LabChip analysis.

According to transglycosylation reaction standard protocol 1, a transglycosylation reaction was carried out, and a sampling solution was provided. Here, acceptor molecule solution 1-1 provided in Reference Example 1 was used. Either the Enzyme-A solution (Si-001-2) provided in Example 2 or the Enzyme-A solution (S-001) provided in Example 5 was used. The Enzyme-B solution (Rp-001-2) provided in Example 3 was used. Any of glycan donor G-1, G-3, or G-4 provided according to Table Z6 was used. Depending on the structure of the glycan donor used in each experiment, the transglycosylation reaction shown in the schematic diagram below or Figure 3 proceeds.

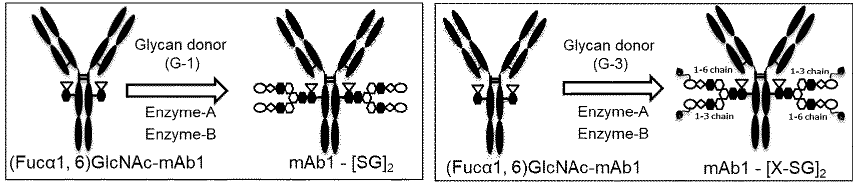

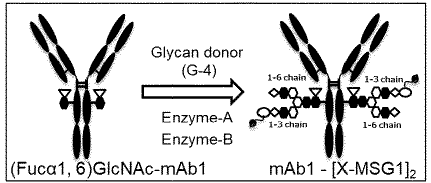

### [Transglycosylation rate measurement using LabChip analysis]

When the sampling solution is subjected to LabChip analysis according to the method described above, the unreacted product and the transglycosylated form appear as separated peaks in the resulting electropherogram. The transglycosylation rate was calculated from the peak area ratio of the unreacted product and the transglycosylated form by the following calculation expression. Transglycosylation rate (%) = [[peak area of H chain derived from transglycosylated antibody]/{[peak area of H chain derived from unreacted product] + [peak area of H chain derived from transglycosylated antibody]}] × 100

For each reaction condition used for (Fucα1,6)GlcNAc-mAb1, the transglycosylation rate at each reaction time was calculated (Table Z7.).

**Table Z7**

| **Example** | **Antibody (mg/m L)** | **Glycan donor** | **Enzyme-A** | **Enzyme-B** | **Transglycosylation rate (%)** | | | | | |
|---|---|---|---|---|---|---|---|---|---|---|
| | | | | | 2h | 4h | 6h | 8h | 24h | 28h |
| 8-1-1 | mAb1 (20) | G-1 (20eq.) | Endo-Si D241MQ311L | Endo-Rp N172H | 7.3 | 12.5 | 18.0 | 24.7 | 80.2 | 88.1 |
| 8-1-2 | mAb1 (20) | G-3 (20eq.) | Endo-Si D241MQ311L | Endo-Rp N172H | 7.6 | 19.5 | 31.6 | 44.9 | 88.3 | 89.3 |
| 8-1-3 | mAb1 (20) | G-4 (20eq.) | Endo-Si D241MQ311L | Endo-Rp V172H | 11.1 | 26.3 | 45.1 | 59.9 | 96.3 | 96.2 |
| 8-1-4 | mAb1 (20) | G-1 (20eq.) | Endo-S D233QE350Q | Endo-Rp N172H | 4.7 | 12.1 | 18.8 | 23.0 | 68.0 | 76.8 |
| 8-1-5 | mAb1 (20) | G-3 (20eq.) | Endo-S D233QE350Q | Endo-Rp N172H | 4.5 | 10.8 | 18. 9 | 26.0 | 70.2 | 73.1 |
| 8-1-6 | mAb1 (20) | G-4 (20eq.) | Endo-S D233QE350Q | Endo-Rp N172H | 7.4 | 14.4 | 21.5 | 29.1 | 64.1 | 66.1 |
| 8-1-7 | mAb1 (20) | G-1 (40eq.) | Endo-S D233QE350Q | Endo-Rp N172H | 15.4 | 32.8 | 49.4 | 65.1 | >99.9 | 96.5 |
| 8-1-8 | mAb1 (20) | G-3 (40eq.) | Endo-S D233QE350Q | Endo-Rp N172H | 16.8 | 39.2 | 59.4 | 73.7 | 88.6 | 87.7 |
| 8-1-9 | mAb1 (20) | G-4 (40eq.) | Endo-S D233QE350Q | Endo-Rp N172H | 24.0 | 49.4 | 68.3 | 80.3 | 89.0 | 87.9 |

Depending on the potential of Enzyme-A used, there are cases where the transglycosylation rate does not exceed 80% by 28 hours as in Example 8-1-4, Example 8-1-5, and Example 8-1-6. In such cases, increasing the amount of the glycan donor used is expected to improve the transfer efficiency. In Example 8-1-7, Example 8-1-8, and Example 8-1-9, Example 8-1-4, the amount of the glycan donor was changed from 20 equivalents to 40 equivalents in [Transglycosylation reaction standard protocol 1] used in Example 8-1-4, Example 8-1-5, and Example 8-1-6, respectively. As expected, the transglycosylation rate was improved. As described above, it can be seen that when it is desired to improve the transglycosylation rate without changing the combination of enzymes and without changing the final antibody concentration, it is sufficient to simply double the amount of the glycan donor.

### (Example 8-2) When antibody concentration in reaction system is 40 mg/mL

According to transglycosylation reaction standard protocol 2 instead of transglycosylation reaction standard protocol standard protocol 1, the transglycosylation reaction was carried out, and the transglycosylation rate was measured by using LabChip analysis in the same manner as in Example 8-1.

### [Transglycosylation reaction standard protocol 2]

(Fucα1,6)GlcNAc-mAb1 (6 mg), a glycan donor (20 equivalents), Enzyme-A (1.6 to 1.7% w/w), Enzyme-B (0.2% w/w), and an appropriate amount of a 50 mM Tris-HCl buffer solution (pH 7.25) were added to make a total solution volume of 150 µL, and the resulting mixture was reacted at 30°C for 28 hours. Sampling was carried out 2 hours, 4 hours, 6 hours, 8 hours, 24 hours, and 28 hours after the start of the reaction. After completion of the reaction, the pH of the reaction solution was measured. At the time of sampling, a portion of the reaction solution was withdrawn, diluted with purified water such that the concentration of the antibody contained was 1 mg/mL, and stored frozen until the start of LabChip analysis.

For each reaction condition used for (Fucα1,6)GlcNAc-mAb1, the transglycosylation rate at each reaction time was calculated in the same manner as in (Example 8-1) (Table Z8).

**Table Z8**

| **Example** | **Antibody (mg/mL)** | **Glycan donor** | **Enzyme-A** | **Enzyme-B** | **Transglycosylation rate (%)** | | | | | |
|---|---|---|---|---|---|---|---|---|---|---|
| | | | | | 2h | 4h | 6h | 8h | 24h | 28h |
| 8-2-1 | mAh1 (40) | G-1 (20eq.) | Endo-Si D241MQ311L | Endo-Rp N172H | 17.9 | 35.4 | 57.7 | 74.7 | 98.7 | 99.2 |
| 8-2-2 | mAb1 (40) | G-3 (20eq.) | Endo-Si D241MQ311L | Endo-Rp N172H | 18.2 | 44.3 | 66.2 | 79.8 | 94.1 | 93.8 |
| 8-2-3 | mAb1 (40) | G-4 (20eq.) | Endo-Si D241MQ311L | Endo-Rp N172H | 19.8 | 48.6 | 71.4 | 86.3 | 97.3 | 97.0 |
| 8-2-4 | mAb1 (40) | G-1 (20eq.) | Endo-S D233QE350Q | Endo-Rp N172H | 11.7 | 24.0 | 36.1 | 49.4 | 96.0 | 96.3 |
| 8-2-5 | mAb1 (40) | G-3 (20eq.) | Endo-S D233QE350Q | Endo-Rp N172H | 13.1 | 33.0 | 52.1 | 67.9 | 88.3 | 86.6 |
| 8-2-6 | mAb1 (40) | G-4 (20eq.) | Endo-S D233QE350Q | Endo-Rp N172H | 18.6 | 39.4 | 58.0 | 72.0 | 85.7 | 83.7 |

### (Example 8-3) When antibody concentration in reaction system is 60 mg/mL

According to transglycosylation reaction standard protocol 3 instead of transglycosylation reaction standard protocol standard protocol 1, the transglycosylation reaction was carried out, and the transglycosylation rate was measured by using LabChip analysis in the same manner as in Example 8-1.

### [Transglycosylation reaction standard protocol 3]

(Fucα1,6)GlcNAc-mAb1 (6 mg), a glycan donor (20 equivalents), Enzyme-A (1.6 to 1.7% w/w), Enzyme-B (0.2% w/w), and an appropriate amount of a 50 mM Tris-HCl buffer solution (pH 7.25) were added to make a total solution volume of 100 µL, and the resulting mixture was reacted at 30°C for 28 hours. Sampling was carried out 2 hours, 4 hours, 6 hours, 8 hours, 24 hours, and 28 hours after the start of the reaction. After completion of the reaction, the pH of the reaction solution was measured. At the time of sampling, a portion of the reaction solution was withdrawn, diluted with purified water such that the concentration of the antibody contained was 1 mg/mL, and stored frozen until the start of LabChip analysis.

For each reaction condition used for (Fucα1,6)GlcNAc-mAb1, the transglycosylation rate at each reaction time was calculated in the same manner as in Example 8-1 (Table Z9).

**Table Z9**

| **Example** | **Antibody (mg/mL)** | **Glycan donor** | **Enzyme-A** | **Enzyme-B** | **Transglycosylation rate (%)** | | | | | |
|---|---|---|---|---|---|---|---|---|---|---|
| | | | | | 2h | 4h | 6h | 8h | 24h | 28h |
| 8-3-1 | mAb1 (60) | G-1 (20eq.) | Endo-Si D241MQ311L | Endo-Rp N172H | 22.2 | 62.4 | 75.2 | 90.5 | 98.8 | 99.2 |
| 8-3-2 | mAb1 (60) | G-3 (20eq.) | Endo-Si D241MQ311L | Endo-Rp N172H | 29.8 | 64.5 | 83.7 | 91.4 | 95.1 | 94.4 |
| 8-3-3 | mAb1 (60) | G-4 (20eq.) | Endo-Si D241MQ311L | Endo-Rp N172H | 36.8 | 78.6 | 95.3 | 98.5 | 98.1 | 97.6 |
| 8-3-4 | mab1 (60) | G-1 (20eq.) | Endo-S D233QE350Q | Endo-Rp N172H | 17.7 | 39.6 | 60.6 | 77.5 | 97.7 | 97.9 |
| 8-3-5 | mAb1 (60) | G-3 (20eq.) | Endo-S D233QE350Q | Endo-Rp N172H | 25.9 | 60.3 | 81.1 | 89.8 | 90.3 | 88.6 |
| 8-3-6 | mAb1 (60) | G-4 (20eq.) | Endo-S D233QE350Q | Endo-Rp N172H | 33.3 | 67.7 | 85.7 | 91.3 | 89.4 | 86.4 |

### (Example 8-4) When antibody concentration in reaction system is 80 mg/mL

According to transglycosylation reaction standard protocol 4 instead of transglycosylation reaction standard protocol standard protocol 1, the transglycosylation reaction was carried out, and the transglycosylation rate was measured by using LabChip analysis in the same manner as in Example 8-1.

### [Transglycosylation reaction standard protocol 4]

(Fucα1,6)GlcNAc-mAb1 (6 mg), a glycan donor (20 equivalents), Enzyme-A (1.6 to 1.7% w/w), Enzyme-B (0.2% w/w), and an appropriate amount of a 50 mM Tris-HCl buffer solution (pH 7.25) were added to make a total solution volume of 75 µL, and the resulting mixture was reacted at 30°C for 28 hours. Sampling was carried out 2 hours, 4 hours, 6 hours, 8 hours, 24 hours, and 28 hours after the start of the reaction. After completion of the reaction, the pH of the reaction solution was measured. At the time of sampling, a portion of the reaction solution was withdrawn, diluted with purified water such that the concentration of the antibody contained was 1 mg/mL, and stored frozen until the start of LabChip analysis. However, here, acceptor molecule solution 1-2 provided in Reference Example 1 was used.

For each reaction condition used for Fucα1,6)GlcNAc-mAb1, the transglycosylation rate at each reaction time was calculated in the same manner as in Example 8-1 (Table Z10).

**Table Z10**

| **Example** | **Antibody (mg/mL)** | **Glycan donor** | **Enzyme-A** | **Enzyme-B** | **Transglycosylation rate (%)** | | | | | |
|---|---|---|---|---|---|---|---|---|---|---|
| | | | | | 2h | 4h | 6h | 8h | 24h | 28h |
| 8-4-1 | mAb1 (80) | G-1 (20eq.) | Endo-Si D241MQ311L | Endo-Rp N172H | 25.4 | 51.2 | 82.9 | 95.5 | 98.7 | 99.0 |
| 8-4-2 | mAb1 (80) | G-3 (20eq.) | Endo-Si D241MQ311L | Endo-Rp N172H | 49.9 | 84.7 | 94.1 | 96.3 | 95.7 | 95.1 |
| 8-4-3 | mAb1 (80) | G-4 (20eq.) | Endo-Si D241MQ311L | Endo-Rp N172H | 64.6 | 96.6 | 99.0 | 98.8 | 98.1 | >99. 9 |
| 8-1-4 | mAb1 (80) | G-1 (20eq.) | Endo-S D233QE350Q | Endo-Rp N172H | 28.1 | 62.0 | 85.5 | 96.0 | 98.2 | 97.3 |
| 8-4-5 | mAb1 (80) | G-3 (20eq.) | Endo-S D233QE350Q | Endo-Rp N172H | 12.9 | 81.7 | 92.7 | 94.2 | 90.7 | 88.6 |
| 8-4-6 | mAb1 (80) | G-4 (20eq.) | Endo-S D233QE350Q | Endo-Rp N172H | 48.0 | 84.9 | 93.3 | 91.2 | 89.8 | 87.3 |

In Example 8, a combination of a representative enzyme and a representative glycan donor was used, and the amount of the glycan donor was mainly fixed at 20 equivalents, and the transglycosylation rate was confirmed for up to 28 hours. It can be seen that in any combination of enzymes, the higher the final antibody concentration in the reaction system, the higher the transglycosylation rate. In addition, it can be confirmed that an antibody having a moderate (>80%) or higher transglycosylation rate can be obtained in the transglycosylation reaction regardless of the antibody concentration in the reaction system.

### <Example 9> Reaction condition for "Step 1": Glycan equivalent

The antibody concentration in the reaction system applicable to the production method of the present invention is not limited, and in Example 9, in order to confirm that an antibody having a moderate (>80%) or higher transglycosylation rate can be obtained in the transglycosylation reaction regardless of the amount of a glycan donor molecule added to the reaction system, the number of glycan equivalents added to the system was set to 10 equivalents to 40 equivalents.

### (Example 9-1) When using 10 equivalents of glycan for antibody

According to transglycosylation reaction standard protocol 5 instead of transglycosylation reaction standard protocol standard protocol 1, the transglycosylation reaction was carried out, and the transglycosylation rate was measured by using LabChip analysis in the same manner as in Example 8-1.

### [Transglycosylation reaction standard protocol 5]

(Fucα1,6)GlcNAc-mAb1 (6 mg), a glycan donor (10 equivalents), Enzyme-A (1.6 to 1.7% w/w), Enzyme-B (0.2% w/w), and an appropriate amount of a 50 mM Tris-HCl buffer solution (pH 7.25) were added to make a total solution volume of 100 µL, and the resulting mixture was reacted at 30°C for 28 hours. Sampling was carried out 2 hours, 4 hours, 6 hours, 8 hours, 24 hours, and 28 hours after the start of the reaction. After completion of the reaction, the pH of the reaction solution was measured. At the time of sampling, a portion of the reaction solution was withdrawn, diluted with purified water such that the concentration of the antibody contained was 1 mg/mL, and stored frozen until the start of LabChip analysis.

Here, acceptor molecule solution 1-1 provided in Reference Example 1 was used. Either the Enzyme-A solution (Si-001-2) provided in Example 2 or the Enzyme-A solution (S-001) provided in Example 5 was used. The Enzyme-B solution (Rp-001-2) provided in Example 3 was used. Any of glycan donor G-1, G-3, or G-4 provided according to Table Z6 was used. Depending on the structure of the glycan donor used in each experiment, the transglycosylation reaction shown in Figure 3 described above proceeds.

For each reaction condition used for (Fucα1,6)GlcNAc-mAb1, the transglycosylation rate at each reaction time was calculated in the same manner as in Example 8-1 (Table Z11).

**Table Z11**

| **Example** | **Antibody (mg/mL)** | **Glycan donor** | **Enzyme-A** | **Enzyme-B** | **Transglycosylation rate (%)** | | | | | |
|---|---|---|---|---|---|---|---|---|---|---|
| | | | | | 2h | 4h | 6h | 8h | 24h | 28h |
| 9-1-1 | mAb1 (60) | G-1 (10eq.) | Endo-Si D241MQ311L | Endo-Rp N172H | 7.6 | 16.4 | 24.4 | 31.5 | 84.3 | 90.5 |
| 9-1-2 | mAb1 (60) | G-3 (10eq.) | Endo-Si D241MQ311L | Endo-Rp N172H | 9.8 | 22.2 | 35.9 | 48.4 | 88.6 | 89.6 |
| 9-1-3 | mAb1 (60) | G-4 (10eq.) | Endo-Si D241MQ311L | Endo-Rp N172H | 13.4 | 29.0 | 48.7 | 61.7 | 96.2 | 96.1 |
| 9-1-4 | mAb1 (60) | G-1 (10eq.) | Endo-S D233QE350Q | Endo-Rp N172H | 5.0 | 13.1 | 19.3 | 26.2 | 72.6 | 79.7 |
| 9-1-5 | mAb1 (60) | G-3 (10eq.) | Endo-S D233QE350Q | Endo-Rp N172H | 7.8 | 18.9 | 31.4 | 43.5 | 81.4 | 82.0 |
| 9-1-6 | mAb1 (60) | G-4 (10eq.) | Endo-S D233QE350Q | Endo-Rp N172H | 9.7 | 23.2 | 35.8 | 48.7 | 82.1 | 81.9 |
| 9-1-7 | mAb1 (80) | G-1 (10eq.) | Endo-S D233QE350Q | Endo-Rp N172H | 14.0 | 26.3 | 38.4 | 50.6 | 93.8 | 94.3 |

When the transglycosylation rate does not exceed 80% by 28 hours as in Example 9-1-4, increasing the final antibody concentration is expected to improve the transfer rate. Therefore, in Example 9-1-7, the reaction was carried out under a condition in which the glycan donor (20 equivalents) was changed to the glycan donor (10 equivalents) in [Transglycosylation Reaction Standard Protocol 4] used in Example 8-4. As expected, the transglycosylation rate was improved. As described above, when it is desired to improve the transglycosylation rate without changing the number of glycan equivalents, the final antibody concentration in the reaction system may be increased.

### (Example 9-2) When using 30 equivalents of glycan for antibody

According to transglycosylation reaction standard protocol 6 instead of transglycosylation reaction standard protocol standard protocol 1, the transglycosylation reaction was carried out, and the transglycosylation rate was measured by using LabChip analysis in the same manner as in Example 8-1.

### [Transglycosylation reaction standard protocol 6]

(Fucα1,6)GlcNAc-mAb1 (6 mg), a glycan donor (30 equivalents), Enzyme-A (1.6 to 1.7% w/w), Enzyme-B (0.2% w/w), and an appropriate amount of a 50 mM Tris-HCl buffer solution (pH 7.25) were added to make a total solution volume of 100 µL, and the resulting mixture was reacted at 30°C for 28 hours. Sampling was carried out 2 hours, 4 hours, 6 hours, 8 hours, 24 hours, and 28 hours after the start of the reaction. After completion of the reaction, the pH of the reaction solution was measured. At the time of sampling, a portion of the reaction solution was withdrawn, diluted with purified water such that the concentration of the antibody contained was 1 mg/mL, and stored frozen until the start of LabChip analysis.

For each reaction condition used for (Fucα1,6)GlcNAc-mAb1, the transglycosylation rate at each reaction time was calculated in the same manner as in Example 8-1(Table Z12).

**Table Z12**

| **Example** | **Antibody (mg/mL)** | **Glycan donor** | **Enzyme-A** | **Enzyme-B** | **Transglycosylation rate (%)** | | | | | |
|---|---|---|---|---|---|---|---|---|---|---|
| | | | | | 2h | 4h | 6h | 8h | 24h | 28h |
| 9-2-1 | mAb1 (60) | G-1 (30eq.) | Endo-Si D241MQ311L | Endo-Rp N172H | 50.5 | 89.2 | 99.0 | 99.2 | 99.3 | 99.3 |
| 9-2-2 | mAb1 (60) | G-3 (30eq.) | Endo-Si D241MQ311L | Endo-Rp N172H | 68.1 | 92.8 | 96.8 | 97.3 | 96.1 | 95.7 |
| 9-2-3 | mAb1 (60) | G-4 (30eq.) | Endo-Si D241MQ311L | Endo-Rp N172H | 78.4 | 98.7 | 98.9 | 98.8 | 98.3 | 98.2 |
| 9-2-4 | mAb1 (60) | G-1 (30eq.) | Endo-S D233QE350Q | Endo-Rp N172H | 39.5 | 79.9 | 96.6 | 98.5 | 98.3 | 98.3 |
| 9-2-5 | mAb1 (60) | G-3 (30eq.) | Endo-S D233QE350Q | Endo-Rp N172H | 62.0 | 93.4 | 96.2 | 96.0 | 93.0 | 92.3 |
| 9-2-6 | mAb1 (60) | G-4 (30eq.) | Endo-S D233QE350Q | Endo-Rp N172H | 62.8 | 94.9 | 96.8 | 96.6 | 94.2 | 93.6 |

### (Example 9-3) When using 40 equivalents of glycan for antibody

According to transglycosylation reaction standard protocol 7 instead of transglycosylation reaction standard protocol standard protocol 1, the transglycosylation reaction was carried out, and the transglycosylation rate was measured by using LabChip analysis in the same manner as in Example 8-1.

### [Transglycosylation reaction standard protocol 7]

(Fucα1,6)GlcNAc-mAb1 (6 mg), a glycan donor (40 equivalents), Enzyme-A (1.6 to 1.7% w/w), Enzyme-B (0.2% w/w), and an appropriate amount of a 50 mM Tris-HCl buffer solution (pH 7.25) were added to make a total solution volume of 100 µL, and the resulting mixture was reacted at 30°C for 28 hours. Sampling was carried out 2 hours, 4 hours, 6 hours, 8 hours, 24 hours, and 28 hours after the start of the reaction. After completion of the reaction, the pH of the reaction solution was measured. At the time of sampling, a portion of the reaction solution was withdrawn, diluted with purified water such that the concentration of the antibody contained was 1 mg/mL, and stored frozen until the start of LabChip analysis.

For each reaction condition used for (Fucα1,6)GlcNAc-mAb1, the transglycosylation rate at each reaction time was calculated in the same manner as in Example 8-1 (Table Z13).

**Table Z13**

| **Example** | **Antibody (mg/mL)** | **Glycan donor** | **Enzyme-A** | **Enzyme-B** | **Transglycosylation rate (%)** | | | | | |
|---|---|---|---|---|---|---|---|---|---|---|
| | | | | | 2h | 4h | 6h | 8h | 24h | 28h |
| 9-3-1 | mAb1 (60) | G-1 (40eq.) | Endo-Si D241MQ311L | Endo-Rp N172H | 78.1 | 99.4 | 99.6 | 99.5 | 99.5 | >99.9 |
| 9-3-2 | mAb1 (60) | G-3 (40eq.) | Endo-Si D241NQ311L | Endo-Rp N172H | 88.4 | 97.4 | 97.9 | 97.8 | 96.5 | 96.1 |
| 9-3-3 | mAb1 (60) | G-4 (40eq.) | Endo-Si D241MQ311L | Endo-Rp N172H | 95.0 | 98.9 | 98.9 | 98.8 | 98.0 | 98.5 |
| 9-3-4 | mAb1 (60) | G-1 (40eq.) | Endo-S D233QE350Q | Endo-Rp N172H | 65.7 | 98.0 | 99.0 | 99.0 | 98.6 | 98.5 |
| 9-3-5 | mAb1 (60) | G-3 (40eq.) | Endo-S D233QE350Q | Endo-Rp N172H | 88.2 | 97.2 | 96.9 | 96.5 | 93.7 | 93.0 |
| 9-3-6 | mAb1 (60) | G-4 (40eq.) | Endo-S D233QE350Q | Endo-Rp N172H | 89.5 | 97.5 | 97.1 | 96.8 | 94.6 | 94.1 |

In Example 9, a combination of a representative enzyme and a representative glycan donor was used, the final antibody concentration in the reaction system was mainly fixed at 60 equivalents, the amount of the glycan donor was changed, and the transglycosylation rate was confirmed for up to 28 hours. It was confirmed that in any combination of enzymes, the higher the amount of the glycan donor used, the higher the transglycosylation rate. In addition, it can be confirmed that an antibody having a moderate (>80%) or higher transglycosylation rate can be obtained in the transglycosylation reaction regardless of the glycan equivalent added to the reaction system.

Based on Experimental Example 8 and Experimental Example 9, in "Step 1", a high transglycosylation rate can be achieved by appropriately regulating the amount of the glycan donor used and the final antibody concentration in the reaction system.

Subsequently, in Example 10 below, the amount (20 equivalents) of the glycan donor used and the final antibody concentration (60 mg/mL) in the reaction system were mainly fixed, and combinations of the structures of available glycan donors and enzymes were confirmed.

### <Example 10> Reaction condition for "Step 1": Combination of Enzyme-A and Enzyme-B

According to transglycosylation reaction standard protocol 3 used in (Example 8-3), the transglycosylation reaction was carried out, and the transglycosylation rate was measured by using LabChip analysis in the same manner as in (Example 8-1).

Here, the acceptor molecule solutions, the Enzyme-A solutions, the Enzyme-B solutions, and the glycan donors were properly used according to the combinations shown in Table Z14. Specifically, any one of acceptor molecule solutions 1-1, 2-1, 2-2, or 3-1 provided in Reference Example 1 to Reference Example 3 was used. Any one of the Enzyme-A solutions provided in Example 2 or Example 5 was used. Any one of the Enzyme-B solutions provided in Example 3 or Example 6 was used. Any one of glycan donors G-1 to G-17 provided according to Table Z6 was used. Depending on the structure of the glycan donor used in each experiment, the transglycosylation reaction shown in the schematic diagram below or Figure 4 proceeds.

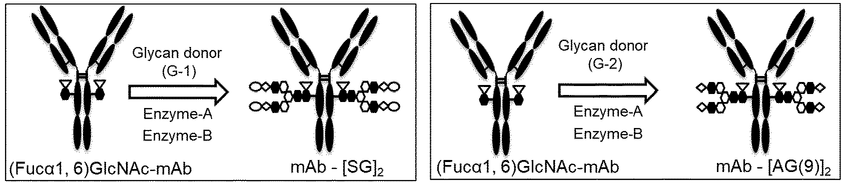

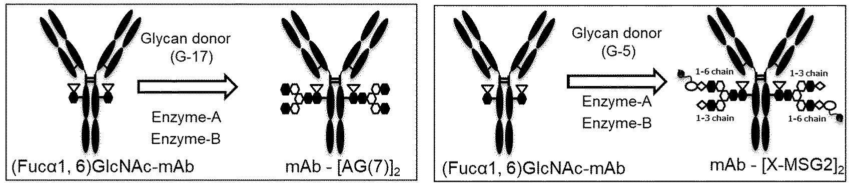

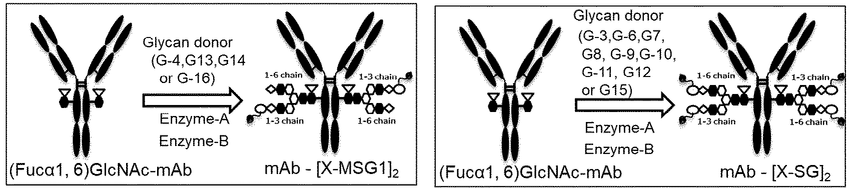

For each reaction condition used for (Fucα1,6)GlcNAc-mAb1, (Fucα1,6)GlcNAc-mAb2, or (Fucα1,6)GlcNAc-mAb3, the transglycosylation rate at each reaction time was calculated in the same manner as in Example 8-1 (Table Z14).

**Table Z14**

| **Example** | **Antibody (mg/m L)** | **Glycan donor** | **Enzyme-A** | **Enzyme-B** | **Transglycosylation rate (%)** | | | | | |
|---|---|---|---|---|---|---|---|---|---|---|
| | | | | | 2h | 4h | 6h | 8h | 24h | 28h |
| 10-1 | mAb1 (60) | G-2 (20eq.) | Endo-Si D241MQ311L | Endo-Rp N172H | 65.3 | 97.0 | 99.1 | 98.8 | 98.6 | 98.7 |
| 10-2 | mAb1 (60) | G-5 (20eq.) | Endo-Si D241MQ311L | Endo-Rp N172H | 60.6 | 91.2 | 96.8 | 97.5 | 96.4 | 96.2 |
| 10-3 | mAb1 (60) | G-14 (20eq.) | Endo-Si D241MQ311L | Endo-Rp N172H | 22.0 | 53.1 | 76.6 | 91.3 | 98.3 | 98.4 |
| 10-4 | mAb1 (60) | G-16 (20eq.) | Endo-Si D241MQ311L | Endo-Rp N172H | 44.3 | 85.4 | 97.7 | 98. 6 | 98.4 | 98.6 |
| 10-5 | mAb1 (60) | G-17 (20eq.) | Endo-Si D241MQ311L | Endo-Rp N172H | 92.1 | 97.1 | 96.9 | 96.2 | 92.3 | 91.8 |
| 10-6 | mAb2 (60) | G-1 (20eq.) | Endo-Si D241MQ311L | Endo-Rp N172H | 39. 2 | 36.6 | 62.2 | 81.6 | 99.3 | 99.3 |
| 10-7 | mAb2 (60) | G-1 (20eq.) | Endo-Si D241MQ311L | Endo-Rp N172V | 27.5 | 60.5 | 82.3 | 92.6 | 98.0 | 97.7 |
| 10-8 | mAb2 (60) | G-1 (20eq.) | Endo-Si D241MQ311L | Endo-Rp N172Q | 16.7 | 38.3 | 59.5 | 75.5 | 97.3 | 97.3 |
| 10-9 | mAb2 (60) | G-1 (20eq.) | Endo-Si D241MQ311L | Endo-M X175Q | 80.3 | 94.6 | 96.9 | 97.3 | 95.8 | 95.5 |
| 10-10 | mAb2 (60) | G-1 (20eq.) | Endo-Si D241MQ311L | Endo-M N175Q Y217F | 29.3 | 65.7 | 88.8 | 97.0 | 99.0 | 99.4 |
| 10-11 | mAb2 (60) | G-1 (20eq.) | Endo-Si D241Q | Endo-Rp N172H | 19.2 | 44.0 | 67.7 | 84.9 | 97.7 | 97.5 |
| 10-12 | mAb2 (60) | G-1 (20eq.) | Endo-Si D241M | Endo-Rp N172H | 20.6 | 47.3 | 70.7 | 85.0 | 94.4 | 93.7 |
| 10-13 | mAb2 (60) | G-1 (20eq.) | Endo-Si D241QE360Q | Endo-Rp N172H | 19.5 | 46.1 | 70.8 | 88.4 | 98.8 | 98.6 |
| 10-14 | mAb2 (60) | G-1 (20eq.) | Endo-Si D241ME360Q | Endo-Rp N172H | 20.8 | 48.2 | 73.2 | 89.4 | 97.7 | 97.4 |
| 10-15 | mAb2 (60) | G-1 (20eq.) | Endo-Si D241QQ311L | Endo-Rp N172H | 17.0 | 39. 1 | 60.1 | 79.8 | 99.1 | 99.4 |
| 10-16 | mAb2 (60) | G-1 (20eq.) | Endo-S D233QE350Q | Endo-Rp N172H | 11.9 | 29.5 | 48.1 | 65.1 | 97.2 | 97.0 |
| 10-17 | mAb2 (60) | G-1 (20eq.) | Endo-S D233QE350Q | Endo-Rp N172V | 24.5 | 56.8 | 80.2 | 91.7 | 96.5 | 96.3 |
| 10-18 | mAb2 (60) | G-1 (20eq.) | Endo-S D233QE350Q | Endo-Rp N172Q | 14.9 | 35.7 | 56. 7 | 72.9 | 95.7 | 95.3 |
| 10-19 | mAb2 (60) | G-1 (20eq.) | Endo-S D233QE350Q | Endo-Rp N172V Y214F | 24.3 | 56.5 | 81.3 | 93.5 | 97.8 | 97.7 |
| 10-21 | mAb2 (60) | G-2 (20eq.) | Endo-Si D241MQ311L | Endo-Rp N172II | 60.2 | 93.0 | 97.8 | 97.9 | 97.4 | 97.3 |
| 10-22 | mAb2 (60) | G-3 (20eq.) | Endo-Si D241MQ311L | Endo-Rp N172H | 32.0 | 68.5 | 85.7 | 91.9 | 94.0 | 93.2 |
| 10-23 | mAb2 (60) | G-3 (20eq.) | Endo-Si D241MQ311L | Endo-Rp N172V | 38. 7 | 66. 0 | 77.2 | 82.4 | 83. 8 | 81.7 |
| 10-24 | mAb2 (60) | G-3 (20eq.) | Endo-Si D241MQ311L | Endo-Rp N172Q | 34. 0 | 61.1 | 73. 1 | 78. 2 | 81.1 | 78. 8 |
| 10-25 | mAb2 (60) | G-3 (20eq.) | Endo-Si D241MQ311L | Endo-M N175Q | 65.9 | 79.1 | 82.9 | 84.0 | 75.8 | 73.1 |
| 10-26 | mAb2 (60) | G-3 (20eq.) | Endo-Si D241MQ311L | Endo-M N175Q Y217F | 29.9 | 64.2 | 81.8 | 89.2 | 92.7 | 92.0 |
| 10-27 | mAb2 (60) | G-3 (20eq.) | Endo-Si D241Q | Endo-Rp N172H | 35.0 | 72.4 | 88.7 | 92.0 | 88.9 | 85.7 |
| 10-28 | mAb2 (60) | G-3 (20eq.) | Endo-Si D241M | Endo-Rp N172H | 34.6 | 66.9 | 80.8 | 84.3 | 77.6 | 74.2 |
| 10-29 | mAb2 (60) | G-3 (20eq.) | Endo-Si D241QE360Q | Endo-Rp N172H | 37.7 | 77.7 | 91.9 | 94.2 | 91.1 | 89.6 |
| 10-30 | mAb2 (60) | G-3 (20eq.) | Endo-Si D241ME360Q | Endo-Rp N172H | 38.3 | 75.7 | 87.7 | 89.5 | 83.7 | 81.0 |
| 10-31 | mAb2 (60) | G-3 (20eq.) | Endo-Si D241QQ311L | Endo-Rp N172H | 27.2 | 56.5 | 74.7 | 84.7 | 94.3 | 93.6 |
| 10-32 | mAb2 (60) | G-3 (20eq.) | Endo-S D233QE350Q | Endo-Rp N172H | 22.0 | 52.5 | 72.7 | 84.0 | 89.7 | 88.5 |
| 10-33 | mAb2 (60) | G-4 (20eq.) | Endo-Si D241MQ311L | Endo-Rp N172H | 94.6 | 83.3 | 96.6 | 98.1 | 97.5 | 97.1 |
| 10-34 | mAb2 (60) | G 4 (20eq.) | Endo Si D241MQ311L | Endo Rp N172V | 56.0 | 86.5 | 93.9 | 95.2 | 92.5 | 91.4 |
| 10-35 | mAb2 (60) | G-4 (20eq.) | Endo-Si D241MQ311L | Endo-Rp N172Q | 56.9 | 85.6 | 92.8 | 94.2 | 91.0 | 89.8 |
| 10-36 | mAb2 (60) | G-4 (20eq.) | Endo-Si D241MQ311L | Endo-M N175Q | 83.9 | 91.6 | 92.6 | 92.3 | 83.8 | 81.2 |
| 10-37 | mAb2 (60) | G-4 (20eq.) | Endo-Si D241MQ311L | Endo-M N175Q Y217F | 57.8 | 92.5 | 98.0 | 98.3 | 97.4 | 96.9 |
| 10-38 | mAb2 (60) | G-4 (20eq.) | Endo-Si D241Q | Endo-Rp N172H | 35.4 | 70.0 | 84.2 | 87.7 | 78.0 | 76.1 |
| 10-39 | mAb2 (60) | G-4 (20eq.) | Endo-Si D241M | Endo-Rp N172H | 27.0 | 19.3 | 59.3 | 62.3 | 58.2 | 54.1 |
| 10-40 | mAb2 (60) | G-4 (20eq.) | Endo-Si D241QE360Q | Endo-Rp N172H | 46.2 | 83.6 | 91.9 | 92.6 | 88.1 | 85.7 |
| 10-41 | mAb2 (60) | G-4 (20eq.) | Endo-Si D241ME360Q | Endo-Rp N172H | 32.5 | 56.4 | 64.9 | 68.1 | 63.8 | 59.6 |
| 10-42 | mAb2 (60) | G-4 (20eq.) | Endo-Si D241QQ311L | Endo-Rp N172H | 45.0 | 88.5 | 98.0 | 98.7 | 97.9 | 97.7 |
| 10-43 | mAb2 (60) | G-4 (20eq.) | Endo-S D233QE350Q | Endo-Rp N172H | 28.1 | 61.4 | 81.6 | 90.2 | 89.1 | 88.1 |
| 10 44 | mAb2 (60) | G-5 (20eq.) | Endo-Si D241MQ311L | Endo-Rp N172H | 69.4 | 93.6 | 97.1 | 97.6 | 96.1 | 95.4 |
| 10-45 | mAb2 (60) | G-6 (20eq.) | Endo-Si D241MQ311L | Endo-Rp N172H | 17.7 | 53. 7 | 79. 9 | 91.7 | 98.2 | 98.0 |
| 10-46 | mAb2 (60) | G-6 (20eq.) | Endo-Si D241MQ311L | Endo-M N175Q Y217F | 9.6 | 23.5 | 39.1 | 54.1 | 94.5 | 95.1 |
| 10-17 | mAb2 (60) | G-6 (20eq.) | Endo-S D233QE350Q | Endo-Rp N172H | 16.2 | 41.7 | 63.6 | 79.0 | 93.2 | 92.4 |
| 10-48 | mAb2 (60) | G-7 (20eq.) | Endo-Si D241MQ311L | Endo-Rp N172H | 1.2 | 3.3 | 7.1 | 11.3 | 65.5 | 73.8 |
| 10-49 | mAb2 (60) | G-8 (20eq.) | Endo-Si D241MQ311L | Endo-Rp N172H | 9.8 | 35.8 | 60.9 | 79.5 | 97.9 | 97.6 |
| 10-50 | mAb2 (60) | G-8 (20eq.) | Endo-S D233QE350Q | Endo-Rp N172H | 9.4 | 23.1 | 38.0 | 52.0 | 89.7 | 89.9 |
| 10-51 | mAb2 (60) | G-9 (20eq.) | Endo-Si D2411dQ311L | Endo-Rp N172H | 28.4 | 71.8 | 91.1 | 96.8 | 98.1 | 98.0 |
| 10-52 | mAb2 (60) | G-9 (20eq.) | Endo-S D233QE350Q | Endo-Rp N172H | 21.1 | 51.8 | 73.9 | 85.9 | 92.5 | 91.8 |
| 10-53 | mAb2 (60) | G-10 (20eq.) | Endo-Si D241MQ311L | Endo-Rp N172H | 9.8 | 32.6 | 59.8 | 78.8 | 98.1 | 98.0 |
| 10-54 | mAb2 (60) | G-10 (20eq.) | Endo-S D233QE350Q | Endo-Rp N172H | 9.3 | 23.3 | 38.4 | 54.1 | 90.5 | 90.5 |
| 10-55 | mAb2 (60) | G-11 (20eq.) | Endo-Si D241MQ311L | Endo-Rp N172H | 4.6 | 12.9 | 27.1 | 43.7 | 95.9 | 96.8 |
| 10-56 | mAb2 (60) | G-11 (20eq.) | Endo-S D233QE350Q | Endo-Rp N172H | 6.2 | 15.2 | 25.1 | 35.9 | 85.4 | 87.3 |
| 10-57 | mAb2 (60) | G-12 (20eq.) | Endo-Si D241MQ311L | Endo-Rp N172H | 4.1 | 13.7 | 28.8 | 45.6 | 96.4 | 97.0 |
| 10-58 | mAb2 (60) | G-12 (20eq.) | Endo-S D233QE350Q | Endo-Rp N172H | 6.5 | 16.0 | 26.7 | 38.3 | 86.7 | 88.1 |
| 10-59 | mAb2 (60) | G-13 (20eq.) | Endo-Si D241MQ311L | Endo-Rp N172H | 9.9 | 10.6 | 16.3 | 23.8 | 70.4 | 77.7 |
| 10-60 | mAb2 (60) | G-14 (20eq.) | Endo-Si D241MQ311L | Endo-Rp N172H | 24.2 | 50.7 | 74.5 | 90.0 | 97.9 | 97.7 |
| 10-61 | mAb2 (60) | G-14 (20eq.) | Endo-S D233QE350Q | Endo-Rp N172H | 14.3 | 35.4 | 56.2 | 72.4 | 93.0 | 92.5 |
| 10-62 | mAb2 (60) | G-15 (20eq.) | Endo-Si D241MQ311L | Endo-Rp N172H | 20.1 | 46.4 | 66.9 | 80.4 | 93.9 | 93.9 |
| 10-63 | mAb2 (60) | G-16 (20eq.) | Endo-Si D241MQ311L | Endo-Rp N172H | 41.0 | 77.0 | 94.1 | 97.3 | 97.1 | 97.0 |
| 10-64 | mAb2 (60) | G-17 (20eq.) | Endo-Si D241MQ311L | Endo-Rp N172H | 88.8 | 94.3 | 94.1 | 93.3 | 87.5 | 86.4 |
| 10-65 | mAb3 (60) | G-1 (20eq.) | Endo-Si D241MQ311L | Endo-Rp N172H | 9.3 | 24.6 | 44.8 | 63.8 | 99.3 | 99.5 |
| 10-66 | mAb3 (60) | G-2 (20eq.) | Endo-Si D241MQ311L | Endo-Rp N172H | 44.8 | 80.0 | 96.0 | 98.0 | 97.8 | 97.6 |
| 10-67 | mAb3 (60) | G-3 (20eq.) | Endo-Si D241MQ311L | Endo-Rp N172H | 22.4 | 56.9 | 81.1 | 91.7 | 96.9 | 96.5 |
| 10-68 | mAb3 (60) | G-4 (20eq.) | Endo-Si D241MQ311L | Endo-Rp N172H | 34,6 | 76.6 | 95.9 | 99.0 | 98.9 | 98.7 |
| 10-69 | mAb3 (60) | G-5 (20eq.) | Endo-Si D241MQ311L | Endo-Rp N172H | 59.0 | 90.5 | 96.9 | 97.8 | 96.7 | 96.4 |
| 10 70 | mAb3 (60) | G-13 (20eq.) | Endo-Si D241MQ311L | Endo-Rp N172H | 4.0 | 8.2 | 13.3 | 18.8 | 67.0 | 76.3 |
| 10-71 | mAb3 (60) | G-14 (20eq.) | Endo-Si D241MQ311L | Endo-Rp N172H | 18.1 | 44.2 | 67.6 | 85.4 | 98.5 | 98.3 |
| 10-72 | mAb3 (60) | G-15 (20eq.) | Endo-Si D241MQ311L | Endo-Rp N172H | 14.7 | 35.4 | 55.1 | 70.5 | 94.5 | 94.5 |
| 10-73 | mAb3 (60) | G-16 (20eq.) | Endo-Si D241MR311L | Endo-Rp N172H | 33.3 | 69.9 | 91.2 | 97.4 | 97.6 | 97.4 |
| 10-74 | mAb3 (60) | G-17 (20eq.) | Endo-Si D241MQ311L | Endo-Rp N172H | 86.2 | 94.9 | 95.1 | 94.9 | 91.2 | 90.3 |

In Example 10, combinations of the structures of available glycan donors and enzymes were compared, and it can be confirmed that in any of the combinations, an antibody having a moderate (>80%) or higher transglycosylation rate can be obtained.

### (Residual hydrolysis activity of Enzyme-A)

When the direct transglycosylation reaction proceeds in the form shown in Figure 1 conceived this time, if Enzyme-A used in "Step 1 has residual hydrolysis activity," for example, when mAb-[X-MSG1]₂ is the target product, it is considered that the hydrolysis reaction shown in the schematic diagram below or in Figure 5 competes. That is, when the residual hydrolysis activity of Enzyme-A used is equal to or higher than that of the wild type enzyme, the apparent transglycosylation rate is low.

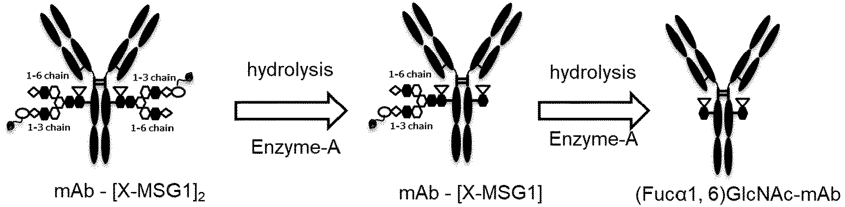

As an enzyme that can be used in "Step 1", an Endo-S mutant, an Endo-S2 mutant, and an Endo-Si mutant can be arbitrarily selected. In order to clarify an indicator of the degree of preferable residual hydrolysis activity as any mutant, the experiment of Example 11 was carried out.

### (Example 11) Hydrolysis of mAb2-[MSG1-N₃]₂

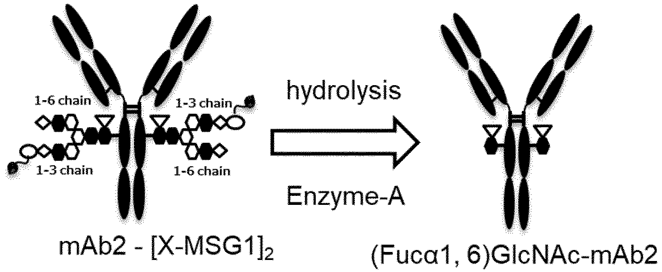

### [Hydrolysis reaction standard protocol 1]

mAb2-[MSG1-N₃]₂ (0.2 mg), Enzyme-A (2.0% w/w), and an appropriate amount of a 50 mM Tris-HCl buffer solution (pH 7.25) were added to make a total solution volume of 21 µL, and the resulting mixture was reacted at 30°C for 24 hours. Sampling was carried out 4 hours and 24 hours after the start of the reaction. At the time of sampling, a portion of the reaction solution was withdrawn, diluted with purified water such that the concentration of the antibody contained was 1 mg/mL, and stored frozen until the start of LabChip analysis.

Here, according to hydrolysis reaction standard protocol 1, hydrolysis was carried out, and a sampling solution was provided. Here, antibody solution 7-1 provided in Reference Example 7 was used. Any one of the Enzyme-A solutions provided in Example 2, Example 4, or Example 5 was used.

### [Transglycosylation rate measurement using LabChip analysis]

When the sampling solution is subjected to LabChip analysis according to the method described above, the unreacted product and the hydrolyzed form appear as separated peaks in the resulting electropherogram. The hydrolysis rate was calculated from the peak area ratio of the unreacted product and the hydrolyzed form by the following calculation expression. Hydrolysis rate (%) = [[peak area of H chain derived from hydrolyzed form]/{[peak area of H chain derived from unreacted product] + [peak area of H chain derived from hydrolyzed form]}] × 100

For each reaction condition used for mAb2-[MSG1-N₃]₂, the hydrolysis rate at each reaction time was calculated (Table Z15).

**Table Z15**

| **Example** | **Enzyme-A** | **Reaction solution pH, temperature** | **Hydrolysis rate (%)** | |
|---|---|---|---|---|
| | | | 4h | 24h |
| 11-1 | Endo-Si D241MQ311L | 7.25, 30°C | 10.1 | 21.2 |
| 11-2 | Endo-Si D241Q | 7.25, 30°C | 42.9 | 96.6 |
| 11-3 | Endo-Si D241Q E360Q | 7.25, 30°C | 31.4 | 71.2 |
| 11-4 | Endo-Si D241M | 7.25, 30°C | 87.0 | 99.3 |
| 11-5 | Endo-Si D241Q Q311L, | 7.25, 30°C | 8.1 | 22.6 |
| 11-6 | Endo-Si D241M E360Q | 7.25, 30°C | 73.1 | > 99.9 |
| 11-7 | Endo-S D233Q E350Q | 7.25, 30°C | 9.0 | 54.0 |
| 11-8 | Endo-S2 D184M | 7.25, 30°C | > 99.9 | > 99.9 |
| 11-9 | Endo-S2 T138Q | 7.25, 30°C | > 99.9 | > 99.9 |
| 11-10 | Endo-Si WT | 7.25, 30°C | 98.4 | 99.7 |
| 11-11 | Endo-S WT | 7.25, 30°C | > 99.9 | > 99.9 |
| 11-12 | Endo-S2 WT | 7.25, 30°C | > 99.9 | > 99.9 |

It was confirmed that enzymes that could be expected to have a high transglycosylation rate in "Step 1" had lower hydrolysis rates than the wild type enzymes thereof at the time point of 4 hours after the start of the reaction. In addition, at the time point of 24 hours after the start of the reaction, many of the enzymes had generally lower hydrolysis rates than the wild type enzymes thereof, but also included some mutants exhibiting equivalent hydrolysis rates.

On the other hand, it has been reported that Endo-S2 D184M and Endo-S2 T138Q exhibit a high transglycosylation rate when the oxazoline form is used as a glycan donor. However, when these were reacted with the transglycosylated antibody in the absence of the oxazoline form, theses exhibited almost the same hydrolysis activity as the wild type enzyme thereof at the time point of 4 hours after the start of the reaction and at the time point of 24 hours after the start of the reaction.

From the results of Example 10, Example 11, and Reference Example 8, it can be seen that an enzyme used in "Step 1" may be arbitrarily selected by using "having a hydrolysis rate of less than 90% at the time point of 4 hours after the start of the reaction" and "having a hydrolysis rate of less than 99.9% at the time point of 24 hours after the start of the reaction" as indicators.

### <Example 12> Confirmation of effect of combination of "Step 1" and "Step 2"

In Example 12, it was verified whether or not the transglycosylation rate could be improved in "Step 2" regardless of the conditions for "Step 1".

The identity of the acceptor molecular (the identity of the antibody), the glycan donor, and the enzyme to which the production method of the present invention can be applied is not limited, and in order to confirm the effect of the combination of "Step 1" and "Step 2" in several patterns, the combinations shown in Table Z16, that is, 5 glycan donors, 3 antibodies, 3 types of Enzyme-A, and 2 types of Enzyme-B, were selected as representative examples. In addition, the amount of the glycan donor used was 10 equivalents or 20 equivalents.

### [Loading solution preparation standard protocol 1 (Step 1)]

### <Step 1>

An appropriate amount of a 50 mM Tris-HCl buffer solution (pH 7.25) was added to a mixed solution of the acceptor molecule (Fucα1,6)GlcNAc-mAb, 10, 20, or 60 equivalents of a glycan donor molecule, 1.6% (w/w) Enzyme-A, and 0.2% (w/w) Enzyme-B, and the reaction solution volume was adjusted such that the final antibody concentration was 20 mg/mL or 60 mg/mL. Subsequently, the reaction solution was incubated at 30°C, and the reaction was terminated 28 hours after the start of the reaction.

### <Sampling>

After completion of the reaction, a trace amount of the reaction solution was sampled for the purpose of measuring the transglycosylation rate, diluted with purified water such that the antibody concentration was 1 mg/mL, and then stored frozen until the start of LabChip analysis.

### <Adjustment of pH and salt concentration of loading solution>

Subsequently, 100 mM acetate buffer (pH 3.9), a 1 M sodium chloride aqueous solution, 1 M hydrochloric acid, and purified water were added to the reaction solution to prepare a column loading solution. The loading solution was prepared in such a way as to have an antibody concentration of 5 mg/mL and to have the same pH and final sodium chloride concentration as those of the column equilibration solution for each experiment. After preparation of the loading solution, 0.5 mL was sampled for the purpose of confirming the transglycosylation rate of the loading solution.

Here, the acceptor molecule solutions, the Enzyme-A solutions, and the glycan donors were properly used according to the combinations shown in Table Z16. Specifically, any one of acceptor molecule solution 4-1 provided in Reference Example 4, acceptor molecule solution 5-1 provided in Reference Example 5, or acceptor molecule solution 6-1 provided in Reference Example 6 was used. Either the Enzyme-A solution (Si-001-2) provided in Example 2 or the Enzyme-A solution (S-001) provided in Example 5 was used. Either the Enzyme-B solution (Rp-001-3) provided in Example 3 or the Enzyme-B solution (M-002) provided in Example 6 was used. Any one of glycan donor G-1, G-3, G-4, G-10, or G-16 provided according to Table Z6 was used.

### [Cation exchange chromatography standard protocol 1 (Step 2)]

Apparatus: AKTA avant 25 (manufactured by Cytiva)
Column: POROS XS (1 ml) (manufactured by Thermo Fisher)
Flow rate: 0.3 ml/min

About 70 mL of the loading solution provided according to loading solution preparation standard protocol 1 was passed through the column (sample passing), and then 15 CV (column washing) of an equilibration solution (50 mM acetate buffer, 445 mM sodium chloride aqueous solution, pH 3.8) was applied. After that, 8 CV of an eluant (50 mM acetate buffer, 1 M sodium chloride aqueous solution, pH 4.0)) was applied for the purpose of column regeneration. Finally, 3 CV of a cleaning solution (50 mM acetate buffer, 0.5 M sodium hydroxide aqueous solution) was applied for the purpose of cleaning in place (CIP).

The target product was detected by absorbance at 280 nm, the flow-through solution was collected when the absorbance was 50 mAU/2 mm UV cell or more, and adjusted to pH 5.0 to 5.4 by adding a 1 M Tris solution, and this was used as a flow-through fraction. In addition, in the column washing step with the equilibration solution, 5 CV of a wash solution passed was collected in one container and used as a wash fraction. With respect to this collected solution, the antibody concentration was confirmed by using an ultraviolet-visible spectrophotometer system SoloVPE (manufactured by C Technologies), and the transglycosylation rate was confirmed by using a microchip type capillary fully automated electrophoresis system LabChip GX (manufactured by Perkin Elmer).

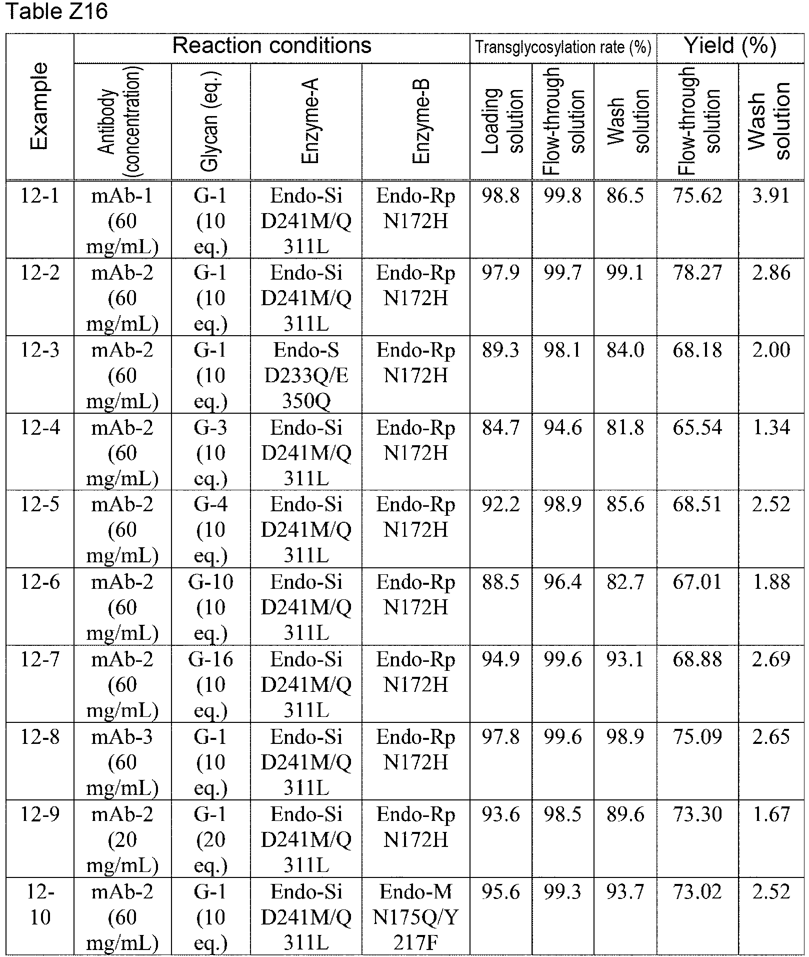

As shown here, "Step 1" was carried out by using 10 equivalents or 20 equivalents of a glycan donor molecule, and then "Step 2" was carried out, and as a result, it was possible to collect an antibody having an improved transglycosylation rate as compared with the loading solution in the flow-through solution in "Step 2", regardless of the types of the antibody, the enzyme, and the glycan used in "Step 1" This has shown that by the combination of "Step 1" and "Step 2", it is possible to obtain an Fc-containing molecule having a homogeneous glycan structure with high purity with the smallest possible amount of a glycan donor molecule used (40 equivalents or less, preferably 10 to 20 equivalents). In addition, it was confirmed that an antibody having an improved glycosylation rate as compared with the loading solution was also collected in the wash solution depending on the conditions as in Example 12-2 and Example 12-8. Because of this, it is considered that the collected solution and the wash solution can be mixed if necessary for the purpose of increasing the recovery.

### [Mass spectrometry of glycan-remodeled antibody and acceptor molecule]

Among the glycan-remodeled antibody molecules included in the collected solution obtained in Example 12, representative compounds and the acceptor molecule used in the preparation of each compound were confirmed by the following method. The glycan-remodeled antibody molecules were separated in an analytical column in the state of a complex of a heavy chain and a light chain, or the state of being fragmented into a heavy chain and a light chain, and introduced into a mass spectrometer. Orbitrap Exploris 240 (manufactured by Thermo Fisher Scientific Inc), Vanquish Flex UHPLC System (manufactured by Thermo Fisher Scientific Inc), and MAbPac RP (manufactured by Thermo Fisher Scientific Inc) (5 µm, 2.1 × 50 mm) were used as apparatuses, water/0.1% formic acid/0.02% trifluoroacetic acid solution was used as mobile phase A, acetonitrile/0.1% formic acid/0.02% trifluoroacetic acid solution was used as mobile phase B, and a gradient changing from 20% to 50% of mobile phase A over 10 minutes was used to carry out analysis at a flow rate of 0.4 mL/min and 80°C.

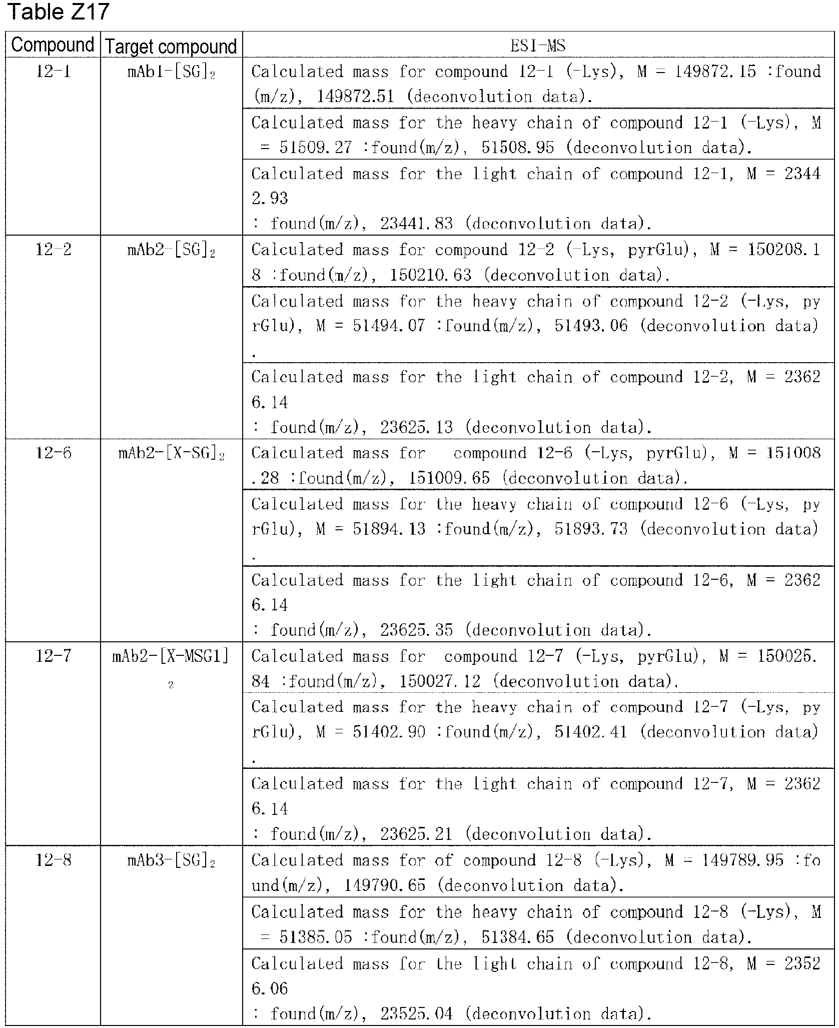

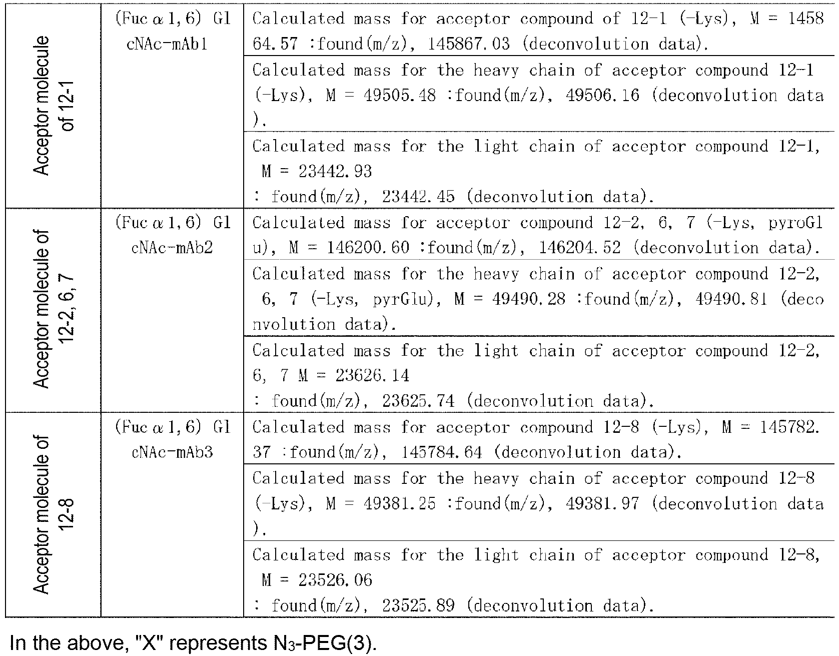

### <Example 13> Examination of the type of cation exchange chromatography medium

In Example 13, it was confirmed that the improving effect on the transglycosylation rate shown in Example 12 does not depend on a specific chromatography medium. Here, the loading solution provided according to loading solution preparation standard protocol 1 used in Example 12 was loaded onto a cation exchange chromatography medium commonly used in antibody production. In order to show that the method of the present invention can be applied to a wide range of cation exchange chromatography media such as a particulate one, a membranous one, or a monolithic one, representative products of various media were used for experiments, but the cation exchange chromatography medium in the present invention are not limited thereto.

### [Cation exchange chromatography standard protocol 2 (Step 2)]

In [Cation exchange chromatography standard protocol 1 (Step 2)] shown in Example 12, the type of the [cation exchange chromatography medium] was changed from POROS XS (1 ml) (manufactured by Thermo Fisher) to CEX particles (POROS HS (1 mL, manufactured by Thermo Fisher), SOURCE 15S (1.66 mL, manufactured by Cytiva), Eshmuno CP-FT (1 mL, manufactured by Merck)), a CEX membrane (Sartobind S (1 mL, manufactured by Sartorius), Mustang S (0.86 mL, manufactured by Pall)), or a CEX monolith (CIM monolith SO3 (1 mL, manufactured by BIA Separations)). Furthermore, each experiment shown in Table Z17 was carried out under operating conditions suitable for the above media. That is, the buffer or the loading solution was passed at a flow rate of a contact time of 3 minutes (0.3 mL/min or 0.55 mL/min) for the CEX particles and 5 membrane volume (MV)/min (5 mL/min or 4.3 mL/min) for the CEX membrane, and a contact time of 1 minute (1 mL/min) for the CEX monolith. 5 CV or 5 to 15 MV of the equilibration solution was fed to cause the equilibration, and then the above loading solution was loaded onto the CEX medium. About 29 mL of the loading solution was loaded onto POROS HS, Eshmuno CP-FT, Sartobind S, and CIM monolish SO3, 49 mL thereof was loaded onto SOURCE 15S, and 25 mL thereof was loaded onto Mustang S. The pH of the column loading solution and the equilibration solution was set to pH 3.7 or pH 3.8 depending on the carrier used, and the salt concentration was set in the range of 280 to 445 mM. The column was washed with 5 to 15 CV or 15 MV of the equilibration solution, and then the CEX medium was regenerated with 5 to 8 CV or 8 to 50 MV of an eluant (50 mM acetate buffer, 1 M sodium chloride solution, pH 4.0). The target product was detected by absorbance at 280 nm, the flow-through solution was fractionated and collected in 5 mL portions when the absorbance was 50 mAU/2 mm UV cell or more, and each fraction was mixed together into one, and then this was adjusted to pH 5.0 to 5.4 by adding a 1 M Tris solution, and used as a flow-through fraction. In addition, in the column washing step with the equilibration solution, 5 CV or 5 MV of a wash solution passed was collected in one container and used as a wash fraction.

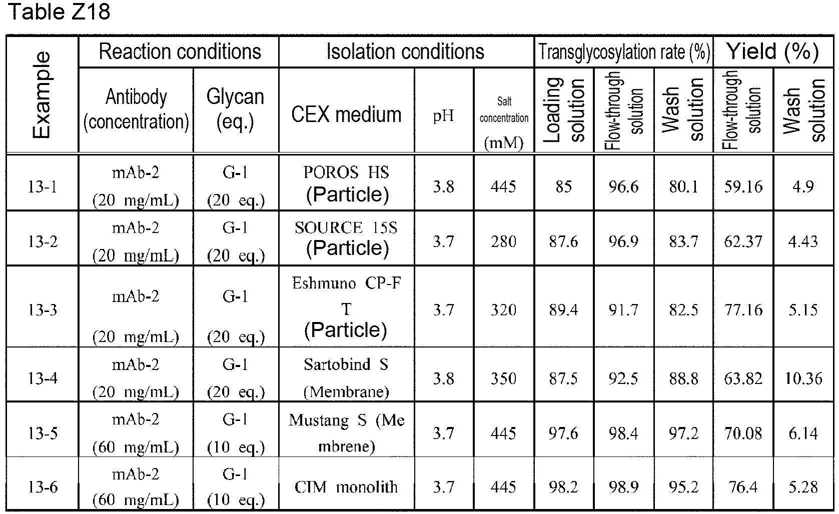

As shown here, it was confirmed that it was possible to improve the transglycosylation rate even when a medium other than the chromatography medium used in Example 12 was used.

### <Example 14> Examination of chromatography operating conditions

In order to clarify isolation conditions that could be expected to provide the improving effect on the transglycosylation rate in "Step 2", the reaction solution was treated under various isolation conditions by using two CEX media.

### [Cation exchange chromatography standard protocol 3 (Step 2)]

Apparatus: AKTA avant 25 (manufactured by Cytiva)
Columns: POROS HS (manufactured by Thermo Fisher) and SOURCE 15S (manufactured by Cytiva)

The isolation conditions followed [cation exchange chromatography standard protocol 2 (step 2)] used in Example 13. However, each experiment was carried out under the conditions of pH and salt concentration set in Table Z19. That is, the pH of the column loading solution and the equilibration solution was pH 3.5 to 5.0, and the salt concentration was in the range of 310 to 500 mM. In addition, in the column washing step with the equilibration solution, the first 1 CV of the 5 CV passed was collected in a container and used as a wash fraction.

**Table Z19**

| Example | Isolation conditions | | | Transglycosylation rate (%) | | | Yield (%) | |
|---|---|---|---|---|---|---|---|---|
| | CEX medium | pH | Salt concentration | Loading solution | Flow-through solution | Wash solution | Flow-through solution | Wash solution |
| 14-1 | POROS HS | 3.8 | 460 | 96.3 | 99.6 | 98.0 | 64.30 | 4.07 |
| 14-2 | POROS HS | 3.8 | 480 | 96.1 | 99.2 | 96.1 | 66.45 | 4.87 |
| 14-3 | POROS HS | 3.8 | 490 | 96.2 | 99.0 | 97.2 | 72.47 | 3.25 |
| 14-4 | POROS HS | 3.8 | 500 | 96.5 | 98.4 | 96.7 | 75.84 | 3.44 |
| 14-5 | POROS HS | 3.9 | 480 | 97.1 | 97.8 | 95.9 | 81.92 | 2.95 |
| 14-6 | POROS HS | 4.0 | 460 | 92.0 | 92.6 | 92.8 | 83.30 | 3.45 |
| 14-7 | POROS HS | 4.5 | 460 | 91.3 | 91.8 | 91.7 | 88.40 | 6.70 |
| 14-8 | SOURCE 15S | 3.7 | 310 | 96.4 | 98.4 | 96.6 | 76.80 | 4.20 |
| 14-9 | SOURCE 15S | 3.7 | 320 | 96.1 | 98.5 | 96.3 | 72.36 | 6.03 |
| 14-10 | SOURCE 15S | 3.7 | 330 | 96.4 | 97.7 | 96.1 | 75.94 | 5.05 |
| 14-11 | POROS HS | 5.0 | 460 | 91.1 | 90.2 | 89.9 | 86.90 | 2.51 |
| 14-12 | POROS HS | 3.5 | 460 | 92.6 | 99.4 | 99.3 | 43.36 | 0.97 |

As shown here, it was confirmed that even when any CEX medium was used, the pH of the loading solution and the equilibration solution affected the improving effect on the transglycosylation rate and the yield. The lower the pH of the loading solution and the equilibration solution, the higher the improving effect on the transglycosylation rate, and the improving effect on the transglycosylation rate decreased as the pH increased. In general, in the purification of an antibody by cation exchange chromatography, a range of pH 4.5 to 6.0 is selected as the pH of the loading solution and the equilibration solution (Groenberg A, Optimizing Cation-Exchange Chromatography with High-Throughput Process Development for mAb Purification. Biopharm Int. 2015; 28: 44-47, and Thermo Fisher Scientific. POROS XS and HS 50 chromatography resins. [Internet]. Thermo Fisher Scientific Inc; c2016 [cited 2022 Jan 13]. 3 p. Available from: https://assets.thermofisher.com/TFS-Assets/LSG/Application-Notes/POROS-viral-clearance-CEX-app-note.pdf POROS XS and HS 50 chromatography resins. Viral clearance capability of cation exchange and recommendations), but in the present experiment, the improving effect on the transglycosylation rate was not observed at all at pH 5.0, and in order to improve the transglycosylation rate, it was important to carry out the isolation in a pH range lower than pH 5.0 (for example, pH 3.5 to 4.5).

In addition, it was confirmed that the salt concentration, like the pH, affected the improving effect on the transglycosylation rate and the yield.

### <Example 15> Application examples related to "Step 1" (enzyme, glycan donor molecule, and glycan acceptor molecule)

### (Example 15a) Preparation of Enzyme-A used in Example 15

Example 11 and Reference Example 8 suggested that an enzyme having appropriate residual hydrolysis activity can be adopted as Enzyme-A used in "Step 1". Therefore, it was verified whether it was possible to use Endo-Se, Endo-Sd, and Endo-Sz, which are known to exhibit properties similar to those of Endo-S and Endo-S2, in Step 1. The enzymes were prepared in the same manner as in Example 2, Example 4, and Example 5 by introducing plasmids encoding genes corresponding to SEQ ID NOs: 76 to 78 into the methanol-assimilating yeast Ogataea minuta (Table Z20).

**Table Z20. Endo-S2 enzyme, Endo-Sd enzyme, Endo-Se enzyme, and Endo-Sz enzyme solutions**

| **Enzyme-A solution** | **Mutant** | **Concentration (mg/mL)** | **Volume (mL)** |
|---|---|---|---|
| S2-004 | Endo-S2 D184Q Q250L | 6.26 | 0.80 |
| S2-005 | Endo-S2 D184M Q250L | 7.52 | 1.10 |
| Sd-001 | Endo-Sd WT | 9.50 | 0.43 |
| Se-001 | Endo-Se D233M | 7.98 | 0.27 |
| Se-002 | Endo-Se WT | 8.82 | 0.30 |
| Sz-001 | Endo-Sz D234M | 8.11 | 0.34 |
| Sz-002 | Endo-Sz D234M Q304L | 7.98 | 0.31 |
| Sz-003 | Endo-Sz WT | 8.06 | 0.26 |

### (Example 15b) Hydrolysis of mAb2-[MSG1-N₃]₂

In order to clarify an indicator of the degree of preferable residual hydrolysis activity as an enzyme that can be used in "Step 1", the experiments shown in Table Z21 were carried out.

The hydrolysis rate at each reaction time was calculated in the same manner as in Example 11 by using Enzyme-A prepared in Example 15a (Table Z21). However, antibody solution 7-2 was used instead of antibody solution 7-1.

**Table Z21**

| Example | Enzyme-A | Reaction solution pH, temperature | Hydrolysis rate (%) | |
|---|---|---|---|---|
| | | | 4h | 24h |
| 15-1 | Endo-S2 D184Q Q250L | 7.25, 30°C | 15.7 | 52.1 |
| 15-2 | Endo-S2 D184M Q250L | 7.25, 30°C | 22.3 | 69.3 |
| 15-3 | Endo-Sd WT | 7.25, 30°C | >99.9 | >99.9 |
| 15-4 | Endo-Se D233M | 7.25, 30°C | 34.8 | >99.9 |
| 15-5 | Endo-Se WT | 7.25, 30°C | >99.9 | >99.9 |
| 15-6 | Endo-Sz D234M | 7.25, 30°C | 34.1 | 98.3 |
| 15-7 | Endo-Sz D234M Q304L | 7.25, 30°C | 8.4 | 28.1 |
| 15-8 | Endo-Sz WT | 7.25, 30°C | >99.9 | >99.9 |

### <Example 15c> Reaction condition for "Step 1": Combination of Enzyme-A and Enzyme-B

The transglycosylation reaction was carried out according to [Transglycosylation reaction standard protocol 2] described in (Example 8-2) to provide a sampling solution. After that, the transglycosylation rate at each reaction time was calculated in the same manner as in (Example 8-1) (Table Z22). However, the glycan donor, the glycan equivalent, and the enzymes used in each experiment were appropriately changed to those shown in Table Z22. In addition, in Example 15-19 to Example 15-21, GlcNAc-mAb4, (Fucα1,6)GlcNAc-Fc-A, and (Fucα1,6)GlcNAc-CLCH-A prepared in Reference Example 22 were used as acceptor molecules.

**Table Z22**

| **Example** | **Antibody (mg/mL)** | **Glycan donor** | **Enzyme-A** | **Enzyme-B** | **Transglycosylation rate (%)** | | | | | |
|---|---|---|---|---|---|---|---|---|---|---|
| | | | | | 2h | 4h | 6h | 8h | 24h | 28h |
| 15-9 | mAb1 (40) | G-1 (40eq.) | Endo-S2 D184M | Endo-M N 175Q | 88.0 | 86.7 | 84.1 | 83.0 | 64.5 | 59.4 |
| 15-10 | mAb1 (40) | G-1 (40eq.) | Endo-S2 T138Q | Endo-M N 175Q | 72.2 | 73.7 | 68.4 | 63.1 | 33.6 | 28.7 |
| 15-11 | mAb1 (40) | G-1 (40eq.) | Endo-S2 D184M/Q250L | Endo-M N 175Q | 98.4 | 99.7 | 99.8 | 99.6 | 99.2 | 98.8 |
| 15-12 | m4b1 (40) | G-1 (40eq.) | Endo-S2 D184Q/Q250L | Endo-M N 175Q | 98.4 | 99.8 | 98.4 | 99.8 | 99.5 | 98.9 |
| 15-13 | mAb1 (40) | G-1 (40eq.) | Endo-Se D233M | Endo-Rp N172V | 34.3 | 63.8 | 83.1 | 91.8 | 95.2 | 94.6 |
| 15-14 | mAb1 (40) | G-1 (40eq.) | Endo-Sz D234M | Endo-Rp N172V | 52.2 | 87.4 | 96.5 | 97.3 | 96.4 | 95.9 |
| 15-15 | mAb1 (40) | G-1 (40eq.) | Endo-Sz D234M/Q304L | Endo-Rp N172V | 40.8 | 75.0 | 93.1 | 98.0 | 98.2 | 98.1 |
| 15-16 | mAb1 (40) | G-1 (40eq.) | Endo-Si D241Q/Q311L | Endo-Rp N172V | 32.4 | 68.9 | 89.7 | 96.8 | 99.6 | 99.7 |
| 15-17 | mAb1 (40) | G-1 (40eq.) | Endo-Si D241 M | Endo-Rp N172V | 75.2 | 97.0 | 96.9 | 97.1 | 95.2 | 95.2 |
| 15-18 | mAb1 (40) | G-1 (40eq.) | Endo-S D233Q /E350Q | Endo-Rp N172V | 68.3 | 98.2 | 99.4 | 98.6 | 99.0 | 98.7 |
| 15-19 | m4b4 (40) | SG-1 (40eq.) | Endo-Si D241Q/Q311L | Endo-Rp N172V | 63.2 | 91.1 | 96.6 | 97.5 | 97.6 | 97.5 |
| 15-20 | Fc-A (40) | SG-1 (40eq.) | Endo-Si D241Q/Q311L | Endo-Rp N172V | N.A. | N.A. | N.A. | N.A. | 91.2 | N.A. |
| 15-21 | CLCH (40) | SG-1 (40eq.) | Endo-Si D241Q/Q311L | Endo-Rp N172V | N.A. | N.A. | N.A. | N.A. | 90.6 | N.A. |

| | | | | | | | | | | |
|---|---|---|---|---|---|---|---|---|---|---|
| However, in Example 15-20 and Example 15-21, the transglycosylation rate was evaluated only 24 hours after the start of the reaction, and thus the transglycosylation rates at other reaction times were denoted as N.A. | | | | | | | | | | |

As shown here, it was confirmed that it was possible to obtain an antibody having a moderate (>80%) or higher transglycosylation rate in a transglycosylation reaction with any combination of enzymes except for when Endo-S2 T138Q was used as Enzyme-A (Example 15-10). As shown in Reference Example 8, it is presumed that even when Endo-S2 T138Q is used, an antibody having a moderate or higher transglycosylation rate can be obtained by increasing the amount of the glycan donor used and introducing a mutation reducing the residual hydrolysis activity. In the present Example, Endo-S, Endo-Si, Endo-S2, Endo-Se, and Endo-Sz were used as Enzyme-A for the purpose of evaluating combinations of representative enzymes, but Enzyme-A in the present invention is not limited thereto, and can be arbitrarily selected depending on the purpose with the residual hydrolysis activity as an indicator. In addition, even when the fucose-less antibody, the Fc fragment, and "CLCH formed by combining CH consisting only of a constant region and CL consisting only of a constant region of a light chain obtained by deleting a variable region" were used as shown in Example 15-19 to Example 15-21, it was possible to obtain a moderate (>80%) or higher transglycosylation rate. This demonstrated that the type of an Fc-containing molecule is not limited to an antibody, and that "Step 1" of the present invention can be applied to a wide range of Fc-containing molecules.

### (Example 15d) Preparation of Enzyme-A/Enzyme-B fusion protein enzyme

A method involving binding two or more proteins or protein fragments by a chemical or genetic engineering technique to allow the same to function as one fusion protein is known. For example, Non Patent Literatures (F Grueninger-Leitch, et al., Protein Sci. 1996, 12, 2617-22, and Emily MK, et al., Protein Eng Des Sel. 2005, 10, 497-501) have reported an example in which a carbohydrate binding module or glutathione S-transferase as a purification tag was fused to an endo-β-N-acetylglucosaminidase. In addition, Patent Literature (WO2017137459) has reported a method for obtaining an antibody having a cleaved glycan in a single step from an antibody to which glycans having different basic structures, such as a high mannose type glycan and a complex type glycan, are added, by using an enzyme (such as a fusion protein of Endo-S and Endo-H, or Endo-F3 and Endo-H) in which endo-β-N-acetylglucosaminidases different in substrate selectivity are fused together. On the other hand, there have so far been no reports of an example of the use of a fusion protein for the purpose of improving the efficiency of a reaction transferring a glycan from a glycan donor molecule to an acceptor molecule. As described herein, in the present invention, Enzyme-A and Enzyme-B may be added to the reaction system in a directly or indirectly bound state as long as these play their respective desired roles. Here, as a representative, a fusion protein in which Enzyme-B and Enzyme-A were bound by a linker including consecutive amino acid residues was prepared, and the reactivity was confirmed. A strain producing the fusion protein was obtained by introducing expression plasmids encoding genes corresponding to SEQ ID NOs: 80 and 81 into the methanol-assimilating yeast Ogataea minuta. In the expression of the protein by methanol induction, in the case of shaking culture, a baffled flask was used, methanol induction was carried out on day 1 after inoculation, the culture was terminated on day 3 after inoculation, and cells were collected by centrifugation. The cells collected were disrupted by using YeastBuster(registered trademark) Protein Extraction Reagent (Merck, 71186), and centrifuged, and the resulting supernatant was purified by using Capto Chelating. Finally, the buffer was replaced with PBS(-) (FUJIFILM Wako Pure Chemical Corporation) by using Amicon Ultra-15, 30 kDa (Merck) to obtain an enzyme solution.

**Table Z23. Enzyme-B/Enzyme-A fusion protein enzyme solutions**

| **Enzyme solution** | **Enzyme-B/Enzyme-A fusion protein enzyme** | **Concentration (mg/mL)** | **Volume (mL)** |
|---|---|---|---|
| F-001 | [Endo-Rp N172H]- [Endo-Si D 241M/Q311L] | 8. 08 | 0. 38 |
| F-002 | [Endo-Rp N172V]- [Endo-Si D 241Q/Q311L] | 8.00 | 0.36 |

### (Example 15e) Hydrolysis of mAb2-[MSG1-N₃]₂

In order to clarify an indicator of the degree of preferable residual hydrolysis activity as an enzyme that can be used in "Step 1", the experiments shown in Table Z24 were carried out.

The hydrolysis rate at each reaction time was calculated in the same manner as in Example 11 by using the enzymes prepared in Example 15d (Table Z24).

**Table Z24**

| **Example** | **Enzyme-B/Enzyme-A fusion protein enzyme** | **Reaction solution pH, temperature** | **Hydrolysis rate (%)** | |
|---|---|---|---|---|
| | | | 4h | 24h |
| 15-22 | [Endo-Rp N172H]- [Endo-Si D241M/Q311L] | 7.25, 30°C | 8.7 | 33.2 |
| 15-23 | [Endo-Rp N172V]- [Endo-Si D241Q/Q311L] | 7.25, 30°C | 20.4 | 68.1 |

### (Example 15f) Reaction examples of Enzyme-A/Enzyme-B fusion protein enzyme

(Fucα1,6)GlcNAc-mAb1 (1 mg), a glycan donor (20 equivalents), an Enzyme-B/Enzyme-A fusion protein (3.2% w/w), and an appropriate amount of a 50 mM Tris-HCl buffer solution (pH 7.25) were added to make a total solution volume of 16.7 µL, and the resulting mixture was reacted at 30°C for 24 hours. Sampling was carried out 16 hours, 18 hours, 20 hours, 22 hours, and 24 hours after the start of the reaction. After completion of the reaction, the pH of the reaction solution was measured. At the time of sampling, a portion of the reaction solution was withdrawn, diluted with purified water such that the concentration of the antibody contained was 1 mg/mL, and stored frozen until the start of LabChip analysis.

After that, the transglycosylation rate at each reaction time was calculated in the same manner as in (Example 8-1) (Table Z25). However, the glycan donor, the glycan equivalent, and the enzymes used in each experiment were appropriately changed to those shown in Table Z25.

**Table Z25**

| **Example** | **Antibody** (mg/mL) | **Glycan donor** | **Enzyme-B/Enzyme-A fusion protein enzyme** | **Transglycosylation rate (%)** | | | | |
|---|---|---|---|---|---|---|---|---|
| | | | | 16h | 18h | 20h | 22h | 24h |
| 15-24 | mAb1 (60) | G-1 (20eq.) | [Endo-Rp N172H]- [Endo -Si D241M/Q311L] | 97.6 | 97.3 | 97.3 | 97.3 | 97.2 |
| 15-25 | mAb1 (60) | G-1 (20eq.) | [Endo-Rp N172V]- [Endo -Si D241Q/Q311L] | 72.4 | 73.9 | 74.1 | 74.4 | 76.3 |

As shown here, even when a fusion protein in which Enzyme-B and Enzyme-A were bound by amino acids was used, it was possible to obtain an antibody to which the desired glycan was added, as when the two enzymes were added as separate molecules in Step 1 (Example 10). In addition, the fusion protein of SEQ ID NO: 79 can also be prepared in the same manner as the fusion proteins of SEQ ID NOs: 80 and 81 according to the procedure described above. In the present Example, a fusion protein in which the full length of each enzyme was directly bound via amino acids was used as a representative fusion protein, but the fusion protein in the present invention is not limited thereto.

### <Example 16> Application examples of "Step 2"

### (Example 16-1) Purification using cation-hydrophobic multimodal resin

It was confirmed that it was also possible to obtain the improving effect on the transglycosylation rate shown in Example 12 when a cation-hydrophobic multimodal resin was used.

Here, the loading solution provided according to loading solution preparation standard protocol 2 below was loaded onto a cation-hydrophobic multimodal resin commonly used in antibody production. Representative commercial products were used in the experiment, but the cation-hydrophobic multimodal resin in the present invention is not limited thereto.

### [Loading solution preparation standard protocol 2 (Step 1)]

### <Step 1>

An appropriate amount of a 50 mM Tris-HCl buffer solution (pH 7.25) was added to a mixed solution of (Fucα1,6)GlcNAc-mAb (acceptor molecule solution, 2000 mg), 20 equivalents of a glycan donor molecule (G-1), 1.6% (w/w) Enzyme-A (S-001), and 0.2% (w/w) Enzyme-B (M-001), and the reaction solution volume was adjusted such that the final antibody concentration was 60 mg/mL. Subsequently, the reaction solution was incubated at 30°C, the reaction was terminated 24 hours after the start of the reaction, and the reaction solution was stored frozen until the start of a purification experiment.

### <Sampling>

After completion of the reaction, a trace amount of the reaction solution was sampled for the purpose of measuring the transglycosylation rate, diluted with purified water such that the antibody concentration was 1 mg/mL, and then stored frozen until the start of LabChip analysis.

### [Cation-hydrophobic multimode chromatography standard protocol 1 (Step 2)]

Apparatus: AKTA avant 25 (manufactured by Cytiva)
Columns (volume of 1 ml each): Capto MMC Impress (manufactured by Cytiva),
Eshmuno CMX (manufactured by Merck), and Nuvia cPrime (manufactured by BIO-RAD Laboratories)
Flow rate: 0.3 ml/min

100 mM acetate buffer (pH 3.9), a 1 M sodium chloride aqueous solution, 1 M hydrochloric acid, and purified water were added to the reaction solution provided according to loading solution preparation standard protocol 2 to prepare a column loading solution. The loading solution was prepared in such a way as to have an antibody concentration of 5 mg/mL and to have the same pH and final sodium chloride concentration as those of a column equilibration solution for each experiment. After preparation of the loading solution, 0.5 mL was sampled for the purpose of confirming the transglycosylation rate of the loading solution. About 30 mL of the loading solution prepared was passed through the column (sample passing), and then 5 CV of an equilibration solution (50 mM acetate buffer, 490 mM sodium chloride aqueous solution, pH 3.8) was applied (column washing). After that, 5 CV of an eluant (50 mM acetate buffer, 1 M sodium chloride aqueous solution, pH 4.0)) was applied for the purpose of column regeneration. Finally, 3 CV of a cleaning solution (0.5 M sodium hydroxide aqueous solution) was applied for the purpose of cleaning in place (CIP). The target product in the column flow-through solution during the sample passing was detected by absorbance at 280 nm, the flow-through solution was collected when the absorbance was 50 mAU/2 mm UV cell or more, and adjusted to pH 5.0 to 5.4 by adding a 1 M Tris solution, and this was used as a collected solution. With respect to this collected solution, the antibody concentration was confirmed by using an ultraviolet-visible spectrophotometer system SoloVPE (manufactured by C Technologies), and the transglycosylation rate was confirmed by using a microchip type capillary fully automated electrophoresis system LabChip GX (manufactured by Perkin Elmer) (Table Z26).

**Table Z26**

| **Example** | **Reaction conditions** | | **Medium** | **Transglycosylation rate (%)** | | **Yield (%)** |
|---|---|---|---|---|---|---|
| | **Antibody (concentration)** | **Glycan (eq.)** | | **Loading solution** | **Collected solution** | **Collected solution** |
| 16-1-1 | mAb-1 (60 mg/mL) | G-1 (20 eq.) | Capto MMC Impres s | 96.1 | 98.6 | 41.26 |
| 16-1-2 | | | Eshmuno CMX | 96.1 | 98.2 | 49.97 |
| 16-1-3 | | | Nuvia cPrime | 96.1 | 98.3 | 48.32 |

As shown here, it was confirmed that it was also possible to obtain the improving effect on the transglycosylation rate when the cation-hydrophobic multimodal resin was used.

### (Example 16-2) Comparison with method of purification in binding/elution mode

It was confirmed that even when the target product was adsorbed on a cation exchange column and then the eluant was passed to collect the same in an elution fraction, it was possible to obtain the improving effect on the transglycosylation rate. In order to make a direct comparison with the method involving collecting the target product in the cation exchange column flow-through fraction as described in Example 12, a reaction solution prepared by the same method (loading solution preparation standard protocol 2 (Step 1)) was used to carry out purification by cation exchange chromatography standard protocol 4 or cation exchange chromatography standard protocol 5.

### [Cation exchange chromatography standard protocol 4 (Step 2)]

Apparatus: AKTA avant 25 (manufactured by Cytiva)
Column: MonoS (1 ml) (manufactured by Cytiva)
Flow rate: 0.3 ml/min

To the reaction solution provided according to loading solution preparation standard protocol 2, a 11-fold volume of an equilibration solution (50 mM acetate buffer, pH 3.8, pH 4.0, or pH 4.2) was added to prepare a column loading solution. The loading solution was passed through the column such that the loading volume according to Table Z27 was obtained, to adsorb the target product on the column. 5 CV of an equilibration solution was applied to wash the column, then an eluant (50 mM acetate buffer, 1 M sodium chloride aqueous solution, pH adjusted to the equilibration solution) was used to elute the target product from the column by using a linear concentration gradient of sodium chloride (from 0 to 100% at 20 CV). The target product in the eluate was detected by absorbance at 280 nm, and the eluate was fractionated in 0.5 ml portions when the absorbance was 20 mAU/2 mm UV cell or more. With respect to this fractioned solution, the antibody concentration was confirmed by using an ultraviolet-visible spectrophotometer system SoloVPE (manufactured by C Technologies), and the transglycosylation rate was confirmed by using a microchip type capillary fully automated electrophoresis system LabChip GX (manufactured by Perkin Elmer), and only the fractions having an improved transglycosylation rate as compared with the loading solution were mixed to obtain a collected solution (Table Z27).

### [Cation exchange chromatography standard protocol 5 (Step 2)]

Apparatus: AKTA avant 25 (manufactured by Cytiva)
Column: MonoS (1 ml) (manufactured by Cytiva)
Flow rate: 0.3 ml/min

100 mM acetate buffer (pH 3.9), a 1 M sodium chloride aqueous solution, 1 M hydrochloric acid, and purified water were added to the reaction solution provided according to loading solution preparation standard protocol 2 to prepare a column loading solution. The loading solution was prepared in such a way as to have an antibody concentration of 5 mg/mL and to have the same pH and final sodium chloride concentration as those of a column equilibration solution for each experiment. About 30 mL of the loading solution was passed through the column (sample passing), and then 5 CV of an equilibration solution (50 mM acetate buffer, 490 mM sodium chloride aqueous solution, pH 3.8) was applied (column washing). After that, 5 CV of an eluant (50 mM acetate buffer, 1 M sodium chloride aqueous solution, pH 4.0)) was applied for the purpose of column regeneration. Finally, 3 CV of a cleaning solution (0.5 M sodium hydroxide aqueous solution) was applied for the purpose of cleaning in place (CIP). The target product in the column flow-through solution during the sample passing was detected by absorbance at 280 nm, the flow-through solution was collected when the absorbance was 50 mAU/2 mm UV cell or more, and adjusted to pH 5.0 to 5.4 by adding a 1 M Tris solution, and this was used as a collected solution. With respect to this collected solution, the antibody concentration was confirmed by using an ultraviolet-visible spectrophotometer system SoloVPE (manufactured by C Technologies), and the transglycosylation rate was confirmed by using a microchip type capillary fully automated electrophoresis system LabChip GX (manufactured by Perkin Elmer) (Table Z27).

**Table Z27**

| **Example** | **Purification conditions** | | | **Transglycosylation rate (%)** | | **Yield (%)** |
|---|---|---|---|---|---|---|
| | **Purification method** | **pH** | **Loading solution volume (mL)** | **Loading solution** | **Collected solution** | |
| 16-2-1 | Binding/elution (protocol 4) | 4.2 | 1.0 | 96.1 | 97.8 | 36.90 |
| 16-2-2 | Binding/elution (protocol 4) | 4.0 | 1.0 | 96.1 | 98.8 | 18.01 |
| 16-2-3 | Binding/elution (protocol 4) | 3.8 | 1.0 | 96.1 | 99.4 | 5.59 |
| 16-2-4 | Binding/elution (protocol 4) | 4.2 | 4.0 | 96.1 | 98.2 | 29.31 |
| 16-2-5 | Binding/elution (protocol 4) | 4.2 | 6.4 | 96.1 | 98.0 | 25.29 |
| 16-2-6 | Flow-through (protocol 5) | 3.8 | 29.8 | 96.1 | 98.4 | 83.38 |

As described here, it was confirmed that even when the target product was adsorbed on a cation exchange column and then the eluant was passed to collect the same in the elution fraction, it was possible to obtain the improving effect on the transglycosylation rate. In addition, it was confirmed that as when the target product was collected in the flow-through fraction (Example 14), the lower the pH, the higher the improving effect on the transglycosylation rate, and the improving effect on the transglycosylation rate decreased as the pH increased.

### (Example 16-3) When reaction solution was provided by using oxazoline method

It was confirmed that even when an oxazoline form was used as the glycan donor in Step 1, it was possible to obtain the improving effect on the transglycosylation rate shown in Example 12. First, an appropriate amount of the glycan donor used in Step 1 was confirmed.

### [Loading solution preparation standard protocol 3 (Step 1)]

### <Step 1>

(Fucα1,6)GlcNAc-mAb1 (acceptor molecule solution, 6 mg), the glycan donor N₃-PEG(3)]-MSG1(9)-Ox (hereinafter appropriately referred to as "N₃-MSG1-Ox," Patent Literature: WO2019065964) (2 equivalents, 4 equivalents, 6 equivalents, 8 equivalents, or 10 equivalents), Enzyme-A (0.5% w/w), and an appropriate amount of a 50 mM Tris-HCl buffer solution (pH 7.25) were added to make a total solution volume of 300 µL, and the resulting mixture was reacted at 30°C for 2 hours.

### <Sampling>

After completion of the reaction, a trace amount of the reaction solution was sampled for the purpose of measuring the transglycosylation rate, diluted with purified water such that the antibody concentration was 1 mg/mL, and then the transglycosylation rate was confirmed by using a microchip type capillary fully automated electrophoresis system LabChip GX (manufactured by Perkin Elmer) (Table Z28).

**Table Z28**

| **Example** | **Reaction conditions** | | **Transglycosylation rate (%)** |
|---|---|---|---|
| | **Glycan** | **Amount of glycan (eq.)** | |
| 16-3-1 | N₃-MSG1-Ox | 2 | 77.2 |
| 16-3-2 | | 4 | 99.4 |
| 16-3-3 | | 6 | >99.9 |
| 16-3-4 | | 8 | >99.9 |
| 16-3-5 | | 10 | >99.9 |

From the results shown in Table Z28, when 4 equivalents or more of the glycan donor N₃-MSG1-Ox was used in Step 1, the transglycosylation rate was very high, and thus this amount was considered to be unsuitable for evaluating the improving effect on the transglycosylation rate in Step 2. Because of this, 2 equivalents of the glycan donor N₃-MSG1-Ox was used in Step 1 to prepare a loading solution in Step 2.

### [Loading solution preparation standard protocol 4 (Step 1)]

In [Loading solution preparation standard protocol 3 (Step 1)], the amount of (Fucα1,6)GlcNAc-mAb1 (acceptor molecule solution) added was changed to 150 mg, and the amount of the glycan donor N₃-MSG1-Ox added was changed to 2 equivalents. Furthermore, the total reaction solution volume was 7.5 ml.

### <Adjustment of pH and salt concentration of loading solution>

Subsequently, 100 mM acetate buffer (pH 3.9), a 1 M aqueous sodium chloride solution, 1 M hydrochloric acid, and purified water were added to the reaction solution to prepare a column loading solution. The loading solution was prepared in such a way as to have an antibody concentration of 5 mg/mL and to have the same pH and final sodium chloride concentration as those of a column equilibration solution for each experiment. After preparation of the loading solution, 0.5 mL was sampled for the purpose of confirming the transglycosylation rate of the loading solution.

### [Cation exchange chromatography standard protocol 5 (Step 2)]

Apparatus: AKTA avant 25 (manufactured by Cytiva)
Column: POROS HS (1 ml) (manufactured by Thermo Fisher)
Flow rate: 0.3 ml/min

About 20 mL of the loading solution provided according to loading solution preparation standard protocol 4 was passed through the column (sample passing), and then 5 CV of an equilibration solution (50 mM acetate buffer, 490 mM sodium chloride aqueous solution, pH 3.8) was applied (column washing). After that, 5 CV of an eluant (50 mM acetate buffer, 1 M sodium chloride aqueous solution, pH 4.0)) was applied for the purpose of column regeneration. Finally, 3 CV of a cleaning solution (0.5 M sodium hydroxide aqueous solution) was applied for the purpose of cleaning in place (CIP). The target product included in the column flow-through solution during the sample passing and the wash solution during the column washing was detected by absorbance at 280 nm, these solutions were collected when the absorbance was 50 mAU/2 mm UV cell or more, and adjusted to pH 5.0 to 5.4 by adding a 1 M Tris solution, and these were used as a flow-through solution and a wash solution, respectively. With respect to these collected solutions, the antibody concentration was confirmed by using an ultraviolet-visible spectrophotometer system SoloVPE (manufactured by C Technologies), and the transglycosylation rate was confirmed by using a microchip type capillary fully automated electrophoresis system LabChip GX (manufactured by Perkin Elmer) (Table Z29).

**Table Z29**

| **Example** | **Reaction conditions** | | **Transglycosylation rate (%)** | | | **Yield (%)** | |
|---|---|---|---|---|---|---|---|
| | **Antibody** | **Glycan** | **Loading solution** | **Flow-through solution** | **Wash solution** | **Flow-through solution** | **Wash solution** |
| | **(Concentration)** | **(eq.)** | | | | | |
| 16-3-6 | mAb-1 (20 mg/mL) | N₃-MSG1-Ox (2 cq.) | 77.5 | 84.1 | 82.3 | 84.78 | 1.74 |

As shown here, it was confirmed that even when an oxazoline form was used as the glycan donor in Step 1, it was possible to obtain the improving effect on the transglycosylation rate in Step 2.

Enzyme A that can be used in the present invention is not limited to a known enzyme and a mutant thereof. Any enzyme can be selected as long as the enzyme satisfies the requirements described in Example 1. A new mutant may be searched for based on known enzyme sequence information. Therefore, a method for confirming an amino acid mutation of Enzyme-A that can be used in "Step 1" (hereinafter referred to as the "amino acid mutation confirmation method") will be shown in Example 17 to Example 19.

### <Example 17> Preparation of Endo-Si single mutants

Here, attention was focused on a catalytically active domain of Endo-Si in order to show an exhaustive search method. Endo-Si single mutants (D241X, T190X, Q311X, and E360X) were prepared in order to analyze the activity with a single mutation of an amino acid at a mutation position.

Single mutants of T190 and single mutants of D241, Q311, and E360 other than the mutants designed in Example 1 were prepared according to the method described in Example 2 (Table Z31). However, the reaction scale was different, and thus at the time of culture, a 96-well deep well plate was used instead of a 500 mL or 1000 mL baffled flask, and at the time of purification, a His-tag purification kit Capturem His-Tagged Purification (Clontech) was used instead of Capto Chelating to carry out simple purification.

**Table Z31**

| **Enzyme-A solution** | **Mutant** | **Concentration (mg/mL)** | **Volume (mL)** |
|---|---|---|---|
| Si-008 | T190A | 0.258 | 0.066 |
| Si-009 | T190R | 0.328 | 0.061 |
| Si-010 | T190V | 0.310 | 0.061 |
| Si-011 | T190C | 0.348 | 0.059 |
| Si-012 | T190D | 0.268 | 0.060 |
| Si-013 | T190E | 0.244 | 0.058 |
| Si-014 | T190G | 0.215 | 0.056 |
| Si-015 | T190II | 0.504 | 0.056 |
| Si-016 | T190I | 0.423 | 0.058 |
| Si-017 | T190K | 0.241 | 0.055 |
| Si-018 | T190L | 0.285 | 0.055 |
| Si-019 | T190M | 0.440 | 0.057 |
| Si-020 | T190F | 0.082 | 0.059 |
| Si-021 | T190P | 0.142 | 0.060 |
| Si-022 | T190S | 0.143 | 0.055 |
| Si-023 | T190W | 0.132 | 0.058 |
| Si-024 | T190Y | 0.357 | 0.062 |
| Si-025 | T190V | 0.138 | 0.062 |
| Si-026 | T190Q | 0.349 | 0.062 |
| Si-027 | D241A | 0.107 | 0.052 |
| Si-028 | D241R | 0.098 | 0.052 |
| Si-029 | D241N | 0.053 | 0.047 |
| Si-030 | D241C | 0.048 | 0.050 |
| Si-031 | D241E | 0.088 | 0.075 |
| Si-032 | D241G | 0.085 | 0.054 |
| Si-033 | D241H | 0.479 | 0.054 |
| Si-034 | D241I | 0.106 | 0.065 |
| Si-035 | D241L | 0.455 | 0.053 |
| Si-036 | D241K | 0.259 | 0.048 |
| Si-037 | D241F | 0.216 | 0.054 |
| Si-038 | D241P | 0.119 | 0.056 |
| Si-039 | D241S | 0.226 | 0.058 |
| Si-040 | D241T | 0.252 | 0.054 |
| Si-041 | D241W | 0.288 | 0.056 |
| Si-042 | D241Y | 0.113 | 0.054 |
| Si-043 | D241V | 0.255 | 0.062 |
| Si-044 | Q311A | 0.648 | 0.065 |
| Si-045 | Q311R | 0.161 | 0.076 |
| Si-046 | Q311N | 0.441 | 0.066 |
| Si-047 | Q311C | 0.207 | 0.063 |
| Si-048 | Q311D | 0.587 | 0.064 |
| Si-049 | Q311E | 0.539 | 0.069 |
| Si-050 | Q311G | 0.238 | 0.060 |
| Si-051 | Q311H | 0.365 | 0.060 |
| Si-052 | Q311I | 0.397 | 0.055 |
| Si-053 | Q311K | 0.147 | 0.058 |
| Si-054 | Q311M | 0.615 | 0.063 |
| Si-055 | Q311F | 0.123 | 0.058 |
| Si-056 | Q311P | 0.583 | 0.067 |
| Si-057 | Q311S | 0.115 | 0.058 |
| Si-058 | Q311T | 1.037 | 0.057 |
| Si-059 | Q311W | 0.151 | 0.060 |
| Si-060 | Q311Y | 0.239 | 0.052 |
| Si-061 | Q311V | 0.305 | 0.065 |
| Si-062 | Q311L | 0.491 | 0.067 |
| Si-063 | E360A | 0.158 | 0.062 |
| Si-064 | E360R | 0.156 | 0.063 |
| Si-065 | E360K | 0.430 | 0.058 |
| Si-066 | E360C | 0.514 | 0.062 |
| Si-067 | E360D | 0.474 | 0.062 |
| Si-068 | E360H | 0.203 | 0.060 |
| Si-069 | E360I | 0.164 | 0.065 |
| Si-070 | E360K | 0.154 | 0.078 |
| Si-071 | E360L | 0.349 | 0.063 |
| Si072 | E360M | 0.223 | 0.074 |
| Si-073 | E360P | 0.238 | 0.070 |
| Si-074 | E360S | 0.098 | 0.070 |
| Si-075 | E360T | 0.120 | 0.071 |
| Si-076 | E360W | 0.086 | 0.068 |
| Si-077 | E360Y | 0.102 | 0.066 |
| Si-078 | E360V | 0.215 | 0.070 |

### <Example 18> Hydrolysis of mAb2-[MSG1-N₃]₂

Next, a method for narrowing down an enzyme having residual hydrolysis activity equal to or lower than that of Endo-Si WT will be described. Any antibody may be used as the antibody used here as long as the antibody is an antibody to which a glycan is added.

A total of 23.2 µL of a reaction solution including mAb2-[MSG1-N₃]₂ (50 µg), an Endo-Si single mutant (1 µg) provided in Example 17, and 50 mM Tris-HCl buffer (pH 7.25) was prepared and reacted at 30°C. The reaction solution was sampled 1 hour, 4 hours, and 24 hours after the start of the reaction. At the time of sampling, a portion of the reaction solution was withdrawn, diluted with purified water such that the concentration of the antibody contained was 1 mg/mL, and stored frozen until the start of LabChip analysis. The hydrolysis rate at each reaction time was calculated in the same manner as in Example 11. This test was carried out a total of two times (Table Z32).

**Table Z32**

| **Example** | **Enzyme-A solution** | **Enzyme-A solution used** | **Hydrolysis rate (%) [1st/2nd]** | | |
|---|---|---|---|---|---|
| | | | 1h | 4h | 24h |
| 18-1 | Endo-Si T190A | Si-008 | > 99.9 / >99.9 | > 99.9 / >99.9 | > 99.9 / >99.9 |
| 18-2 | Endo-Si T190C | Si-011 | > 99.9 / >99.9 | > 99.9 / >99.9 | > 99.9 / >99.9 |
| 18-3 | Endo-Si T190D | Si-012 | > 99.9 / >99.9 | > 99.9 / >99.9 | > 99.9 / >99.9 |
| 18 4 | Endo-Si T190E | Si 013 | > 99.9 / >99.9 | > 99.9 / >99.9 | > 99.9 / >99.9 |
| 18-5 | Endo-Si T190F | Si-020 | 6.5 / 16.3 | 33.9 / 47.8 | 92.8 / >99.9 |
| 18-6 | Endo-Si T190G | Si-014 | > 99.9 / >99.9 | > 99.9 / >99.9 | > 99.9 / >99.9 |
| 18-7 | Endo-Si T190H | Si-015 | 86.2 / 82.3 | > 99.9 / >99.9 | > 99.9 / >99.9 |
| 18-8 | Endo-Si T190I | Si-016 | > 99.9 / >99.9 | > 99.9 / >99.9 | > 99.9 / >99.9 |
| 18-9 | Endo-Si T190K | Si-017 | 2.0 / 4.0 | 11. 2 / 4.4 | 28.2 / 30.9 |
| 18-10 | Endo-Si T190L | Si-018 | > 99.9 / >99.9 | > 99.9 / >99.9 | > 99.9 / >99.9 |
| 18-11 | Endo-Si T190M | Si-019 | 91.4 / 91.3 | > 99.9 / >99.9 | > 99.9 / >99.9 |
| 18-12 | Endo-Si T190N | Si-029 | > 99.9 / >99.9 | > 99.9 / >99.9 | > 99.9 / >99.9 |
| 18-13 | Endo-Si T190P | Si-021 | > 99.9 / >99.9 | > 99.9 / >99.9 | > 99.9 / >99.9 |
| 18 14 | Endo Si T190Q | Si 026 | 60.2 / 70.9 | > 99.9 / >99.9 | > 99.9 / >99.9 |
| 18-15 | Endo-Si T190R | Si-009 | 2.1 / 4.1 | 14.3 / 6.1 | 36.7 / 34.5 |
| 18-16 | Endo-Si T190S | Si-022 | > 99.9 / >99.9 | > 99. 9 / >99.9 | > 99.9 / >99.9 |
| 18 17 | Endo Si T190V | Si 025 | > 99.9 / >99.9 | > 99.9 / >99.9 | > 99.9 / >99.9 |
| 18-18 | Endo-Si T190W | Si-023 | 7.8 / 14.0 | 34.5 / 37.0 | 85.3 / 82.0 |
| 18-19 | Endo-Si T190Y | Si-024 | 12.5 / 22.3 | 54.6 / 56.6 | 94.1 / 95.2 |
| 18-20 | Endo-Si D241A | Si-027 | 64.4 / 62.8 | > 99.9 / >99.9 | > 99.9 / >99.9 |
| 18-21 | Endo-Si D241C | Si-030 | > 99.9 / >99.9 | > 99.9 / >99.9 | > 99.9 / >99.9 |
| 18-22 | Endo-Si D241E | Si-031 | 37.7 / 48.2 | 97.0 / 97.0 | > 99.9 / >99.9 |
| 18-23 | Endo-Si D241F | Si-037 | 0.9 / <0.1 | 2.2 / 4.1 | 15.8 / 8.9 |
| 18-24 | Endo-Si D241G | Si-032 | > 99.9 / >99.9 | > 99.9 / >99.9 | > 99.9 / > 99.9 |
| 18-25 | Endo-Si D241H | Si-033 | 2.3 / <0.1 | 4.1 / 9.0 | 25.2 / 16.9 |
| 18-26 | Endo-Si D2-11I | Si-034 | 8.1 / 11.0 | 32.6 / 15.2 | > 99.9 / >99.9 |
| 1827 | Endo-Si D241K | Si 036 | 0.7 / <0.1 | 0.8 / 1.7 | <0.1 / 0.6 |
| 18-28 | Endo-Si D241L | Si-035 | 4.5 / 4.4 | 15.1 / 24.8 | 74.9 / 77.2 |
| 18-29 | Endo-Si D241N | Si-029 | > 99.9 / >99.9 | > 99.9 / >99.9 | > 99.9 / >99.9 |
| 18-30 | Endo-Si D241P | Si-038 | 2.4 / <0.1 | 8.1 / 15.6 | 60.6 / 62.7 |
| 18-31 | Endo-Si D241R | Si-028 | 0.6 / <0.1 | 0.7 / 2.0 | 2.2 / 0.8 |
| 18-32 | Endo-Si D241S | Si-039 | 87.2 / 93.9 | > 99.9 / >99. 9 | > 99. 9 / >99. 9 |
| 18-33 | Endo-Si D241T | Si-040 | 35.0 / 37.3 | > 99.9 / 94.2 | > 99.9 / >99.9 |
| 18-34 | Endo-Si D241V | Si-043 | 30.5 / 36.8 | 96.0 / 91.9 | 96.0 / >99.9 |
| 18-35 | Endo-Si D241W | Si-041 | 1.0 / <0.1 | 2.4 / 4.8 | 34.7 / 15.0 |
| 18-36 | Endo-Si D241Y | Si-042 | 0.8 / <0.1 | 1.5 / 3.0 | 10.4 / 9.0 |
| 18-37 | Endo-Si Q311A | Si-044 | > 99.9 / >99.9 | > 99.9 / >99.9 | > 99.9 / >99.9 |
| 18-38 | Endo-Si Q311C | Si-047 | > 99.9 / >99.9 | > 99.9 / >99.9 | > 99.9 / >99.9 |
| 18-39 | Endo-Si Q311D | Si-048 | > 99.9 / >99.9 | > 99.9 / >99.9 | > 99.9 / >99.9 |
| 18-40 | Endo-Si Q311E | Si-049 | > 99.9 / >99.9 | > 99.9 / >99.9 | > 99.9 / >99.9 |
| 18-41 | Endo-Si Q311F | Si-064 | 5.0 / 3.6 | 24.4 / 24.4 | > 99.9 / 94.0 |
| 18-12 | Endo-Si Q311G | Si-050 | > 99.9 / >99.9 | > 99.9 / >99.9 | > 99.9 / >99.9 |
| 18-43 | Endo-Si Q311H | Si-051 | > 99.9 / >99.9 | > 99.9 / >99.9 | > 99.9 / >99.9 |
| 18-44 | Endo-Si Q311I | Si-052 | > 99.9 / >99.9 | > 99.9 / >99.9 | > 99.9 / >99.9 |
| 18-15 | Endo-Si Q311K | Si-053 | 4.7 / 3.9 | 32.0 / 28. 7 | > 99.9 / 93.5 |
| 18-46 | Endo-Si Q311L | Si-062 | > 99.9 / >99.9 | > 99.9 / >99.9 | > 99.9 / >99.9 |
| 18-47 | Endo-Si Q311M | Si-054 | > 99.9 / >99.9 | > 99.9 / >99.9 | > 99.9 / >99.9 |
| 18-48 | Endo-Si Q311N | Si-046 | 46.8 / 52.9 | > 99.9 / >99.9 | > 99.9 / >99.9 |
| 18-49 | Endo-Si Q311P | Si-056 | > 99.9 / >99.9 | > 99.9 / >99.9 | > 99.9 / >99.9 |
| 18-50 | Endo-Si Q311R | Si-045 | 0.7 / <0.1 | 0.8 / 2.8 | 2.0 / 3.8 |
| 18-51 | Endo-Si Q311S | Si-057 | > 99.9 / >99.9 | > 99.9 / >99.9 | > 99.9 / >99.9 |
| 18-52 | Endo-Si Q311T | Si-058 | > 99.9 / >99.9 | > 99.9 / >99.9 | > 99.9 / >99.9 |
| 18-53 | Endo-Si Q311V | Si-061 | > 99.9 / >99.9 | > 99.9 / >99.9 | > 99.9 / >99.9 |
| 18-54 | Endo-Si Q311W | Si-059 | 0.7 / <0.1 | 3.7 / 1.5 | 9.2 / 4.5 |
| 18-55 | Endo-Si Q311Y | Si-060 | 1.3 / <0.1 | 5.0 / 4.8 | 26.0 / 26.6 |
| 18-66 | Endo-Si E360A | Si-063 | > 99.9 / >99.9 | > 99.9 / >99.9 | > 99.9 / >99.9 |
| 18-57 | Endo-Si E360C | Si-066 | > 99.9 / >99.9 | > 99.9 / >99.9 | > 99.9 / >99.9 |
| 18-58 | Endo-Si E360D | Si-067 | > 99.9 / >99.9 | > 99.9 / >99.9 | > 99.9 / >99.9 |
| 18-59 | Endo-Si E360H | Si-068 | > 99.9 / >99.9 | > 99.9 / >99.9 | > 99.9 / >99.9 |
| 18-60 | Endo-Si E360I | Si-069 | > 99.9 / >99.9 | > 99.9 / >99.9 | > 99.9 / >99.9 |
| 18-61 | Endo-Si E360K | Si-070 | 71.3 / 94.5 | > 99.9 / >99. 9 | > 99.9 / >99.9 |
| 18-62 | Endo-Si E360L | Si-071 | > 99.9 / >99.9 | > 99.9 / >99.9 | > 99.9 / >99.9 |
| 18-63 | Endo-Si E360M | Si-072 | > 99.9 / >99.9 | > 99.9 / >99.9 | > 99.9 / >99.9 |
| 18-64 | Endo-Si E360N | Si-065 | > 99.9 / >99.9 | > 99.9 / >99.9 | > 99.9 / >99.9 |
| 18-65 | Endo-Si E360P | Si-073 | > 99.9 / >99.9 | > 99.9 / >99.9 | > 99.9 / >99.9 |
| 18-66 | Endo-Si E360R | Si-064 | > 99.9 / >99.9 | > 99.9 / >99.9 | > 99.9 / >99.9 |
| 18-67 | Endo-Si E360S | Si-074 | > 99.9 / >99.9 | > 99.9 / >99.9 | > 99.9 / >99.9 |
| 18-68 | Endo-Si E360T | Si-075 | > 99.9 / >99.9 | > 99.9 / >99.9 | > 99.9 / >99.9 |
| 18-69 | Endo-Si E360V | Si-078 | > 99.9 / >99.9 | > 99.9 / >99.9 | > 99.9 / >99.9 |
| 18-70 | Endo-Si E360W | Si-076 | 55.8 / 71.6 | 88.8 / 97.2 | > 99.9 / >99.9 |
| 18-71 | Endo-Si E360Y | Si-077 | > 99.9 / >99.9 | > 99.9 / >99.9 | > 99.9 / >99.9 |

In this evaluation, it is possible to narrow down enzymes to an enzyme having residual hydrolysis activity equal to or lower than that of Endo-Si WT. It can be determined that an "enzyme exhibiting a hydrolysis rate of >99.9% at the time point of 1 hour" exhibits residual hydrolysis activity equal to or higher than the hydrolysis activity of Endo-Si WT measured in Example 11. Therefore, the evaluation of Example 19 was carried out for "enzymes that did not exhibit a hydrolysis rate of >99.9% at the time point of 1 hour."

### <Example 19> Confirmation of transglycosylation reaction using glycan donor G-1 (SGP)

Next, a method for identifying an amino acid mutation in an enzyme that can be adopted in "Step 1" will be described.

A total of 28 µL of a reaction solution including (Fucα1,6)GlcNAc-mAb2 (50 µg), the glycan donor G-1 (122.72 µg, 50 equivalents), an Endo-Si single mutant provided in Example 17 (2 µg), Endo-Rp N172H (5.7 µg) prepared in Example 3-2, and 50 mM Tris-HCl buffer (pH 7.25) was prepared and reacted at 30°C.

The reaction solution was sampled 4 hours, 8 hours, and 24 hours after the start of the reaction. At the time of sampling, a portion of the reaction solution was withdrawn, diluted with purified water such that the concentration of the antibody contained was 1 mg/mL, and stored frozen until the start of LabChip analysis. The transglycosylation rate at each reaction time was calculated in the same manner as in Example 8-1 (Table Z33).

**Table Z33**

| **Example** | **Enzyme-A** | **Enzyme-A solution used** | **Transglycosylation rate (%)** | | |
|---|---|---|---|---|---|
| | | | 4h | 8h | 24h |
| 19-1 | Endo-Si T190F | Si-020 | 21.2 | 47.4 | 75.4 |
| 19-2 | Endo-Si T190H | Si-015 | 37.0 | 61.3 | 65.5 |
| 19-3 | Endo-Si T190K | Si-017 | 11.2 | 30.4 | 61.5 |
| 19-4 | Endo-Si T190M | Si-019 | 40.3 | 64.4 | 69.6 |
| 19-5 | Endo-Si T190Q | Si-026 | 32.7 | 61.9 | 74.2 |
| 19-6 | Endo-Si T190R | Si-009 | 9.0 | 27.6 | 56.4 |
| 19-7 | Endo-Si T190W | Si-023 | 27.6 | 58.3 | 87.9 |
| 19-8 | Endo-Si T190Y | Si-024 | 42.1 | 73.9 | 88.2 |
| 19-9 | Endo-Si D241A | Si-027 | 27.9 | 51.1 | 70.7 |
| 19-10 | Endo-Si D241E | Si-031 | 26.7 | 43.4 | 48.5 |
| 19-11 | Endo-Si D241F | Si-037 | <0.1 | 4.2 | 19.4 |
| 19 12 | Endo-Si D241H | Si-033 | 39.4 | 63.2 | 87.0 |
| 19-13 | Endo-Si D241I | Si-034 | 48.1 | 74.2 | 50.0 |
| 19-14 | Endo-Si D241K | Si-036 | <0.1 | <0.1 | <0.1 |
| 19-15 | Endo-Si D241L | Si-035 | 57.7 | 81.9 | 91.6 |
| 19-16 | Endo-Si D241P | Si-038 | 34.0 | 61.8 | 84.9 |
| 19-17 | Endo-Si D241R | Si-028 | <0.1 | 2.5 | 4.0 |
| 19-18 | Endo-Si D241S | Si-039 | 34.3 | 54.4 | 59.4 |
| 19-19 | Endo-Si D241T | Si-040 | 50.2 | 74.8 | 77.9 |
| 19-20 | Endo-Si D241V | Si-043 | 59.2 | 86.4 | 89.6 |
| 19-21 | Endo-Si D241W | Si-041 | 10.3 | 23.6 | 49.3 |
| 19-22 | Endo-Si D241Y | Si-042 | 12.8 | 28.6 | 52.6 |
| 19-23 | Endo-Si Q311F | Si-064 | 5.7 | 15.1 | 29.2 |
| 19-24 | Endo-Si Q311K | Si-053 | <0.1 | <0.1 | 4.8 |
| 19-25 | Endo-Si Q311N | Si-046 | 34.4 | 62.5 | 81.1 |
| 19-26 | Endo-Si Q311R | Si-045 | <0.1 | <0.1 | <0.1 |
| 19-27 | Endo-Si Q311W | Si-059 | <0.1 | <0.1 | <0.1 |
| 19 28 | Endo-Si Q311Y | Si-060 | <0.1 | 5.7 | 10.8 |
| 19-29 | Endo-Si E360K | Si-070 | 2.6 | 7.5 | 15.0 |
| 19-30 | Endo-Si E360W | Si-076 | <0.1 | <0.1 | <0.1 |

The results shown in Table Z33 confirmed the following.
"When inserting a single mutation in D241"

The Endo-Si single mutant that can be used as Enzyme-A is not necessarily limited to D241X₁ wherein X₁ = Q or X₁ = M, and as one aspect, it can be considered that X₁ is any amino acid except that X₁ = K or X₁ = R.
"When not inserting a single mutation in D241"

Here, as representative examples, three locations T190X₂, Q311X₃, and E360X₄ were subjected to exhaustive analysis. As one aspect, T190X₂ can be considered to have X₂ = F, H, K, M, Q, R, W, or Y, Q311X₃ can be considered to have X₃ = F, N, or Y, and E360X₄ can be considered to have X₄ = K.

The experiment of Example 19 was carried out on an extremely small scale, and thus the antibody concentration in the reaction was remarkably lower than the antibody concentrations in the reactions carried out in Example 8 to Example 10, and was 4.46 mg/mL. Considering that as shown in Example 8, when the antibody concentration in the reaction is increased, the transglycosylation rate improves, if the optimal conditions for each enzyme are used, as one aspect, most of the enzymes except enzymes having a transglycosylation rate of <0.1% at the time point of 24 hours are considered to be adoptable in "Step 1" (Table Z34).

**Table Z34 Amino acid mutations considered to be highly effective according to amino acid mutation confirmation method**

| | **Enzyme-A** | | **Enzyme-A** |
|---|---|---|---|
| A-13 | Endo-Si T190F | A-26 | Endo-Si D241L |
| A-14 | Endo-Si T190H | A-27 | Endo-Si D241P |
| A-15 | Endo-Si T190K | A-28 | Endo-Si D241R |
| A-16 | Endo-Si T190M | A-29 | Endo-Si D241S |
| A-17 | Endo-Si T190Q | A-30 | Endo-Si D241T |
| A-18 | Endo-Si T190R | A-31 | Endo-Si D241V |
| A-19 | Endo-Si T190W | A-32 | Endo-Si D241W |
| A-20 | Endo-Si T190Y | A-33 | Endo-Si D241Y |
| A-21 | Endo-Si D241A | A-34 | Endo-Si Q311F |
| A-22 | Endo-Si D241E | A-35 | Endo-Si Q311K |
| A-23 | Endo-Si D241F | A-36 | Endo-Si Q311N |
| A-24 | Endo-Si D241H | A-37 | Endo-Si Q311Y |
| A-25 | Endo-Si D241I | A-38 | Endo-Si E360K |

Similarly, exhaustive analysis of single mutations of all amino acids near the catalytically active center of Endo-Si is possible. Therefore, any amino acid mutation may be included in the catalytically active domain of Enzyme-A that can be used in "Step 1".

Furthermore, this method is not limited to Endo-Si, Endo-S, and Endo-S2, and can be used for all endo-β-N-acetylglucosaminidases, which prefer, as a substrate, an N297-linked glycan of an Fc-containing molecule. Therefore, Enzyme-A that can be used in Step 1 is not limited to the specific Endo-Si mutants, Endo-S mutants, Endo-S2 mutants, Endo-Se mutants, Endo-Sd mutants, and Endo-Sz mutants described in the Examples of the present invention. Those skilled in the art can refer to the present specification to identify the structure of Enzyme-A that satisfies the requirements described in Example 1, which is not described as an example in the present Example and herein. Therefore, any enzyme can be selected in "Step 1" as long as the enzyme satisfies the requirements described in Example 1.

### <Example 20> Direct exchange reaction of host cell-derived N297 glycan

As shown in the above Examples, in the present invention, an enzyme having residual hydrolysis activity can be selected as Enzyme-A. Therefore, in one aspect of the present invention, an acceptor molecule can be prepared in situ by the action of Enzyme-A on an Fc-containing molecule having a host cell-derived heterogeneous glycan. In addition, by transferring a glycan from a glycan donor molecule to an acceptor molecule prepared in situ, as shown in Example 10 or the like, an antibody to which the desired glycan is homogeneously added can be obtained.

Specifically, by adding Enzyme-A, Enzyme-B, and a glycan donor molecule to an antibody having a host cell-derived N297-linked glycan, an antibody to which the desired glycan derived from the glycan donor molecule is homogeneously added can be obtained. In the present idea, Enzyme-A exhibits both hydrolysis reaction on a host cell-derived N297-linked glycan and transglycosylation reaction on a glycan donor molecule in the same reaction tank. That is, first, Enzyme-A acts on an antibody having a host cell-derived N297-linked glycan and hydrolyzes the host cell-derived N297-linked glycan to generate an acceptor molecule in situ. Subsequently, Enzyme-A also acts on the glycan donor molecule activated by the action of Enzyme-B in the same reaction tank, making it possible to transfer the glycan to the acceptor molecule. The series of reactions described in the present section is hereinafter referred to as the "direct exchange reaction of the host cell-derived N297 glycan."

In the direct exchange reaction of the host cell-derived N297 glycan, it is considered that the host cell-derived N297-linked glycan can be substituted with the desired glycan structure, as shown in Figure xx2. For example, it is considered that when G-1 is selected as the glycan donor molecule, mAb-[SG]₂ is obtained. In addition, it is considered that when G-4, G-13, G-14, or G-16 is selected as the glycan donor molecule, mAb-[X-MSG1]₂ is obtained, and furthermore, it is considered that when G-3, G-6, G-7, G-8, G-9, G-10, G-11, G-12, or G-15 is selected as the glycan donor molecule, mAb-[X-SG]₂ is obtained.

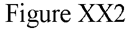

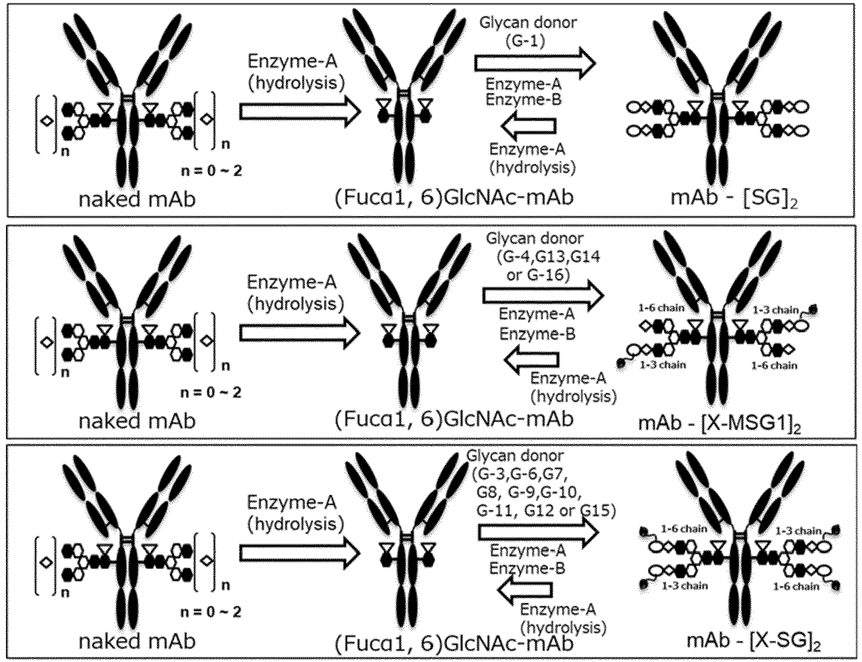
Enzyme-A
(hydrolysis)
Glycan donor
(G-1)
Enzyme-A
Enzyme-B
Enzyme-A
(hydrolysis)
n
n
n = 0 ~ 2
naked mAb
(Fucα1, 6)GlcNAc-mAb
mAb - [SG]2

Enzyme-A
(hydrolysis)
Glycan donor
(G-4,G13,G14
or G-16)
Enzyme-A
Enzyme-B
Enzyme-A
(hydrolysis)
1-6 chain
1-3 chain
1-3 chain
1-6 chain
n
n
n = 0 ~ 2
naked mAb
(Fucα1, 6)GlcNAc-mAb
mAb - [X-MSG1]2

Enzyme-A
(hydrolysis)
Glycan donor
(G-3,G-6,G7,
G8, G-9,G-10,
G-11, G12 or G15)
Enzyme-A
Enzyme-B
Enzyme-A
(hydrolysis)
1-6 chain
1-3 chain
1-3 chain
1-6 chain
n
n
n = 0 ~ 2
naked mAb
(Fucα1, 6)GlcNAc-mAb
mAb - [X-SG]2

### [N297 glycan direct exchange reaction standard protocol 1]

An antibody to which a host cell-derived N297-linked glycan was heterogeneously added (naked mAb1, 12 mg), the glycan donor G-1, G-10, or G-16 (40 equivalents), Enzyme-A (1.6% w/w), Enzyme-B (0.2% w/w), and an appropriate amount of a 50 mM Tris-HCl buffer solution (pH 7.25) were added to make a total solution volume of 200 µL, and the resulting mixture was reacted at 30°C for 72 hours. Sampling was carried out 24 hours, 48 hours, and 72 hours after the start of the reaction. At the time of sampling, a portion of the reaction solution was withdrawn, diluted with a 50 mM Tris-HCl buffer solution (pH 7.25) solution such that the concentration of the antibody contained was 1.5 mg/mL, and stored frozen until the start of analysis. Mass spectrometry and/or LabChip analysis was used to confirm that the host cell-derived N297-linked glycan was exchanged with the desired glycan.

### [Mass spectrometry]

A glycan-remodeled antibody molecule was separated by an analytical column in the state of being fragmented into a heavy chain and a light chain, and introduced into a mass spectrometer. TCEP (tris(2-carboxyethyl)phosphine) was used as a reducing agent for fragmentation. Thereby, the Staudinger reaction also proceeded on the glycan site N₃ group derived from the glycan donor introduced into the antibody by the transglycosylation reaction, and the mass when the N₃ group was reduced to a NH₂ group appeared as the main peak.

Q Exactive (manufactured by Thermo Fisher Scientific Inc), an Ultimate 3000 UHPLC system (manufactured by Thermo Fisher Scientific Inc), and PLRP-S 1000A (manufactured by Agilent Technologies) (8 µm, 2.1 × 50 mm) were used as apparatuses, water/0.1% formic acid/0.02% trifluoroacetic acid solution was used as mobile phase A, acetonitrile was used as mobile phase B, and a gradient changing from 20% to 50% of mobile phase A over 4 minutes was used to carry out analysis at a flow rate of 0.6 mL/min and 60°C to obtain deconvoluted spectra (Table Z36).

**Table Z35 MS peaks derived from host cell-derived H chain (measured values) and MS peaks derived from H chain during reaction progress (theoretical values)**

| **Target compound** | ESI-MS for the heavy chain (-Lys) |
|---|---|
| naked mAb1 | found, M = 50556.59, 50600.70 (most abundant), 50 630.62, 50762.43 (deconvolution data) |
| **Hydrolyzed form** (Fuc α 1,6) GlcNAc-mAb1 | Calculated mass, M = 49505.48 |
| **Transglycosylated form** mAb1-[SG]₂ | Calculated mass, M = 51509.27 |
| **Transglycosylated form** mAb1-[X-MSG1]₂ | Calculated mass, M = 51418.25 |
| **Transglycosylated form** mAb1-[X-SG]₂ | Calculated mass, M = 51909.75 |

In the above, "X" represents N₃-PEG(3). However, when the N₃ group is detected as a peak at which the N₃ group is reduced to an NH₂ group, a numerical value (about 26 Da) that accounts for changes for two nitrogen atoms and two hydrogen atoms per N₃ group appear as a difference from the theoretical value.

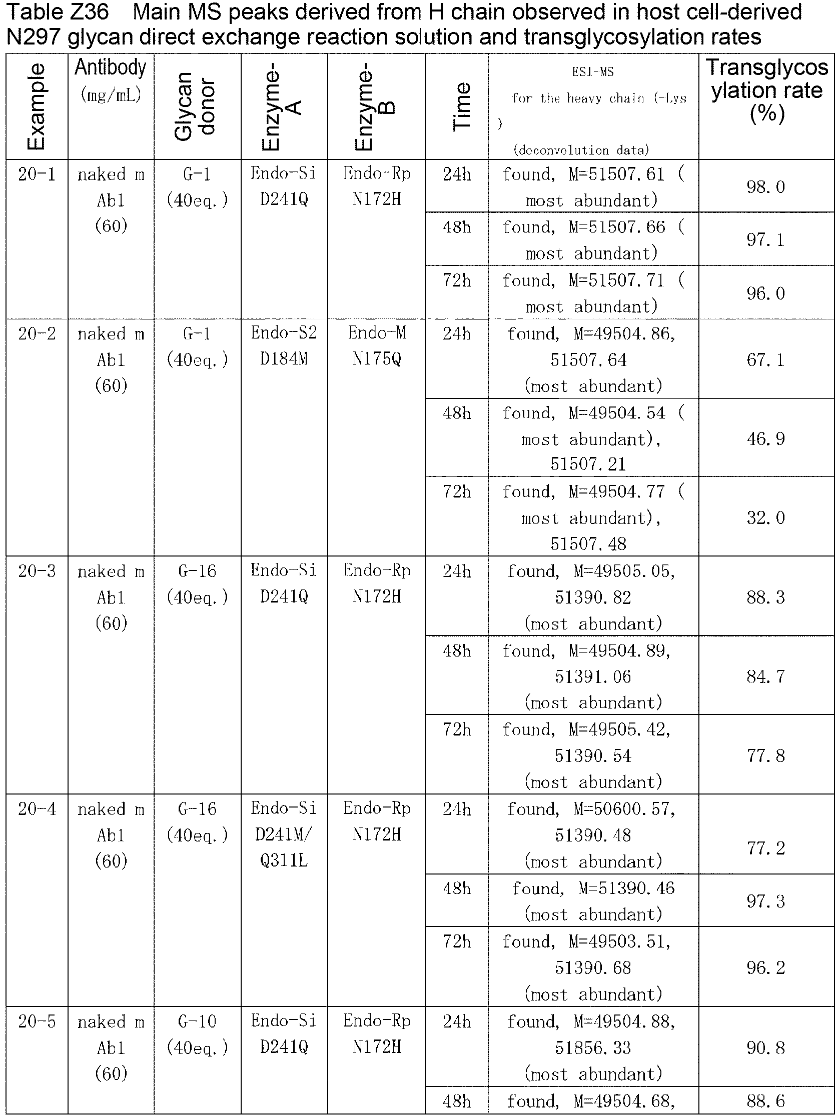

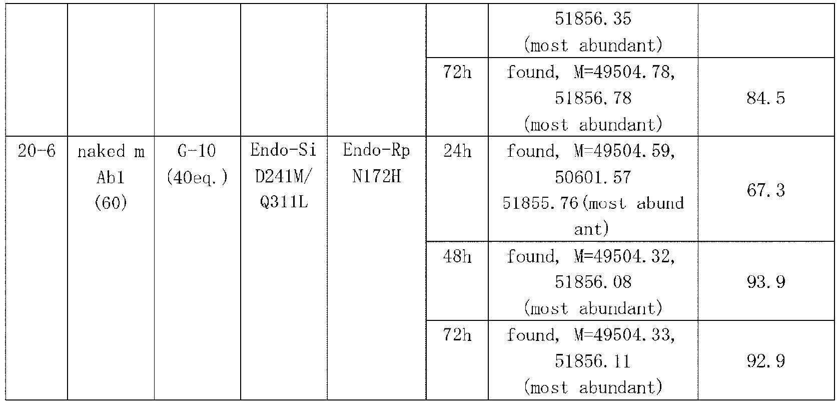

Naked mAb1 is an antibody to which a host cell-derived N297-linked glycan is heterogeneously added, and thus in MS analysis, the structure in which a host cell-derived N297 glycan is added to an H chain was confirmed as a plurality of MS peaks (Table Z36). Focusing on Example 20-1, the host cell-derived N297-linked glycan was almost hydrolyzed and replaced with the G-1-derived glycan structure at the time point of 24 hours after the start of the reaction. This indicates that the main structure of the antibody in the reaction solution is mAb1-[SG]₂. On the other hand, focusing on Example 20-2, the host cell-derived N297-linked glycan was almost hydrolyzed and replaced with the G-1-derived glycan structure and the structure of the resulting hydrolyzed form at the time point of 24 hours after the start of the reaction. This indicates that the main structure of the antibody in the reaction solution is mAb1-[SG]₂ and (Fucα1,6)GlcNAc-mAb1.

Subsequently, focusing on Example 20-3 and Example 20-4 using G-16 as a glycan donor, or Example 20-5 and Example 20-6 using G-10 as a glycan donor, MS peaks as the glycan structure derived from G-16 and the glycan structure derived from G-10, respectively, were detected. In addition, even when Endo-Si D241M/Q303L, which has much weaker residual hydrolysis activity than Endo-Si D241Q, was used, the host cell-derived N297 glycan was already almost hydrolyzed at the time point of 48 hours after the start of the direct exchange reaction. This indicates that the main structure of the antibody in the reaction solution are mAb1-[X-SG]₂ and (Fucα1,6)GlcNAc-mAb1, or mAb1-[X-MSG1]₂ and (Fucα1,6)GlcNAc-mAb1. As shown here, even when Enzyme-A was caused to act on an antibody having a host cell-derived heterogeneous glycan, it was possible to obtain an antibody to which the glycan derived from the glycan donor molecule was homogeneously added. In addition, it was confirmed that even when different combinations of enzymes and glycan donor molecules were used, the same results were obtained. In consideration of the results obtained up to Example 19, it was demonstrated that when the combinations of enzymes and glycan donor molecules described in the present Example were used, the direct exchange reaction of the host cell-derived N297 glycan is also included in "Step 1".

Furthermore, those skilled in the art can refer to the present specification to identify the structures of Enzyme-A, Enzyme-B, and a glycan donor molecule that satisfy the requirements described in Example 20, which are not described as an example in the present Example and herein. Therefore, when the direct exchange reaction of the host cell-derived N297 glycan is adopted in "Step 1", any enzyme and glycan donor molecule can be selected.

### <Reference Examples>

Hereinafter, Reference Examples will be presented. Herein, the Reference Examples do not disclose aspects outside the scope of the present invention, but disclose merely for reference, Production Examples of various acceptor molecules, antibodies, glycan donor molecules, and the like, and Test Examples using these, applied embodiments of the present invention, or the like.

A (Fucα1,6)GlcNAc-Fc-containing molecule solution (acceptor molecule solution) used as an acceptor molecule in a direct transglycosylation reaction, an antibody-[MSG1-N₃]₂ solution (antibody solution) used in a hydrolysis reaction, and an antibody-[SG-N₃]₂ solution (antibody solution) were prepared by the following methods.

### (Reference Example 1) Preparation (1) of (Fucα1,6)GlcNAc-mAb1 solution

A solution containing (Fucα1,6)GlcNAc-mAb1, which can be prepared by the method disclosed in Example 3 of WO2017010559, was buffer exchanged by concentration dialysis (UFDF) using an ultrafiltration membrane Amicon Ultra-15, 30 kDa (Merck) and 50 mM Tris-HCl (pH 7.5) to provide (Fucα1,6)GlcNAc-mAb solutions (acceptor molecule solution 1-1 and acceptor molecule solution 1-2) shown in Table Y-2.

### (Reference Example 2) Preparation (1) of (Fucα1,6)GlcNAc-mAb2 solution

A solution containing (Fucα1,6)GlcNAc-mAb2, which can be prepared by the method disclosed in Step 1 in Example 61 of WO2019065964, was buffer exchanged by concentration dialysis (UFDF) using an ultrafiltration membrane Amicon Ultra-15, 30 kDa (Merck) and 50 mM Tris-HCl (pH 7.5) to provide (Fucα1,6)GlcNAc-mAb solutions (acceptor molecule solution 2-1 and acceptor molecule solution 2-2) shown in Table Y-2.

### (Reference Example 3) Preparation (1) of (Fucα1,6)GlcNAc-mAb3 solution

A solution containing (Fucα1,6)GlcNAc-mAb3, which can be prepared by the method disclosed in Step 1 in Example 85 of WO2020050406, was buffer exchanged by concentration dialysis (UFDF) using an ultrafiltration membrane Amicon Ultra-15, 30 kDa (Merck) and 50 mM Tris-HCl (pH 7.5) to provide a (Fucα1,6)GlcNAc-mAb solution (acceptor molecule solution 3-1) shown in Table Y-2.

### (Reference Example 4) Preparation (2) of (Fucα1,6)GlcNAc-mAb1 solution

50 mM Tris-HCl (pH 7.25) was used instead of 50 mM Tris-HCl (pH 7.5) used in (Reference Example 1), and a (Fucα1,6)GlcNAc-mAb solution (acceptor molecule solution 4-1) shown in Table Y-2 was provided in the same manner as in (Reference Example 1).

### (Reference Example 5) Preparation (2) of (Fucα1,6)GlcNAc-mAb2 solution

50 mM Tris-HCl (pH 7.25) was used instead of 50 mM Tris-HCl (pH 7.5) used in (Reference Example 2), and a (Fucα1,6)GlcNAc-mAb solution (acceptor molecule solution 5-1) shown in Table Y-2 was provided in the same manner as in (Reference Example 2).

### (Reference Example 6) Preparation (2) of (Fucα1,6)GlcNAc-mAb3 solution

50 mM Tris-HCl (pH 7.25) was used instead of 50 mM Tris-HCl (pH 7.5) used in (Reference Example 3), and a (Fucα1,6)GlcNAc-mAb solution (acceptor molecule solution 6-1) shown in Table Y-2 was provided in the same manner as in (Reference Example 3).

### (Reference Example 7) Preparation of mAb2-[MSG1-N₃]₂ solution

A solution containing mAb2-[MSG1-N₃]₂, which can be prepared by the method disclosed in Step 2 in Example 61 of WO2019065964, was buffer exchanged by concentration dialysis (UFDF) using an ultrafiltration membrane Amicon Ultra-15, 30 kDa (Merck) and 50 mM Tris-HCl (pH 7.25) to provide (mAb2-[MSG1-N₃]₂ solutions (acceptor molecule solution 7-1 and acceptor molecule solution 7-2) shown in Table Y-2.

**Table Y-2 Preparation of test solutions**

| **Solution name** | **Fc-containing) molecule** | **Concentrati on (mg/mL)** | **Volume (mL)** |
|---|---|---|---|
| **Acceptor molecule solution 1-1** | (Fuc α 1,6)GlcNAc-mAb1 | 106.40 | 26.05 |
| **Acceptor molecule solution 1-2** | (Fuc α 1,6) GlcVAc-mAb1 | 156.38 | 2.50 |
| **Acceptor molecule solution 2-1** | (Fuc α 1,6) GlcNAc-mAb2 | 91.83 | 7.99 |
| **Acceptor molecule solution 2-2** | (Fuc a 1,6) GlcNAc-mAb2 | 103.5 | 3.51 |
| **Acceptor molecule solution 3-1** | (Fuc α 1,6)GlcNAc-mAb3 | 92.86 | 1.85 |
| **Acceptor molecule solution 4-1** | (Fuc α 1,6)GlcNAc-mAb1 | 92.46 | 7.76 |
| **Acceptor molecule solution 4-2** | (Fuc α 1,6)GlcNAc-mAb1 | 98.42 | 34.30 |
| **Acceptor molecule solution 5-1** | (Fuc α 1,6)GlcNAc-mAb2 | 91.27 | 155.07 |
| **Acceptor molecule solution 6-1** | (Fuc α 1,6)GlcNAc-mAb3 | 90.11 | 3.79 |
| **Antibody solution 7-1** | mAb2-[MSG1-N₃]₂ | 10.00 | 0.2 |
| **Antibody solution 7-2** | mAb2-[MSG1-N₃]₂ | 35.10 | 0.125 |

### (Reference Example 8) Confirmation of reactivity of Endo-S2 mutant

According to any of the standard protocols used in the Examples, conditions were appropriately set as shown in Table Z38 below. Various glycan donors (10 equivalents to 40 equivalents, or 100 equivalents), Enzyme-A (0.8% w/w), Enzyme-B (0.2% w/w), and an appropriate amount of a 50 mM Tris-HCl buffer solution (pH 7.25) were added to (Fucα1,6)GlcNAc-mAb1 (6 mg) to make a total solution volume of 100 to 300 µL, and the resulting mixture was reacted at 30°C for 28 hours. Sampling was carried out 2 hours, 4 hours, 6 hours, 8 hours, 24 hours, and 28 hours after the start of the reaction. After completion of the reaction, the pH of the reaction solution was measured. At the time of sampling, a portion of the reaction solution was withdrawn, diluted with purified water such that the concentration of the antibody contained was 1 mg/mL, and stored frozen until the start of LabChip analysis. The transglycosylation rate was measured in the same manner as in the Examples.

**Table Z38**

| **Reference Example** | **Antibody (mg/mL)** | **Glycan donor** | **Enzyme-A** | **Enzyme-B** | **Transglycosylation rate (%)** | | | | | |
|---|---|---|---|---|---|---|---|---|---|---|
| | | | | | 2h | 4h | 6h | 8h | 24h | 28h |
| 8-1 | mAb1 (20) | G-1 (40eq. ) | Endo-S2 D184M | Endo-M N175Q | 62.2 | 77.1 | 75.2 | 71.6 | 52.3 | 47.1 |
| 8-2 | mAb1 (40) | G-1 (40eq. ) | Endo-S2 D184M | Endo-M N175Q | 80.1 | 83.7 | 81.3 | 78.1 | 58.2 | 54.1 |
| 8-3 | mAb1 (60) | G-1 (40eq. ) | Endo-S2 D184M | Endo-M N175Q | 64.0 | 54.9 | 89.5 | 88.0 | 83.8 | 82.8 |
| 8-4 | mAb1 (80) | G-1 (40eq. ) | Endo-S2 D184M | Endo-M N175Q | 88.2 | 87.8 | 85.4 | 83.2 | 65.6 | 61.5 |
| 8-5 | mAb1 (60) | G-1 (10eq. ) | Endo-S2 D184M | Endo-M N175Q | 24.2 | 42.0 | 47.1 | 47.7 | 24.4 | 19.3 |
| 8-6 | mAb1 (60) | G-3 (10eq. ) | Endo-S2 D184M | Endo-M N175Q | 5.6 | 6.0 | 5.3 | 4.1 | 0.8 | 0.5 |
| 8-7 | mAb1 (60) | G-4 (10eq. ) | Endo-S2 D184M | Endo-M N175Q | 7.6 | 6.7 | 5.5 | 4.7 | 6.3 | 0.8 |
| 8-8 | mAb1 (60) | G-1 (20eq. ) | Endo-S2 D184M | Endo-M N175Q | 54.6 | 69.6 | 68.8 | 66.3 | 36.7 | 30.5 |
| 8-9 | mAb1 (60) | G 3 (20eq. ) | Endo S2 D184M | Endo M N175Q | 18.5 | 17.0 | 14.1 | 10.6 | 2.0 | 0.9 |
| 8-10 | mAb1 (60) | G-4 (20eq. ) | Endo-S2 D184M | Endo-M N175Q | 20.9 | 16.9 | 13.0 | 12.1 | 3.7 | 0.4 |
| 8-11 | mAb1 (60) | G-1 (30eq. ) | Endo-S2 D184M | Endo-M N175Q | 78.9 | 81.3 | 78.9 | 76.5 | 53.2 | 44.9 |
| 8-12 | mAb1 (60) | G-3 (30eq. ) | Endo-S2 D184M | Endo-M N175Q | 31.2 | 23.3 | 19.3 | 26.9 | 3.2 | 1.3 |
| 8-13 | mAb1 (60) | G-4 (30eq. ) | Endo-S2 D184M | Endo-M N175Q | 26.6 | 19.1 | 15.8 | 12.1 | 2.0 | 1.2 |
| 8-14 | mAb1 (60) | G-3 (40eq. ) | Endo-S2 D184M | Endo-M N175Q | 4.6 | 1.6 | 37.8 | 26.6 | 21.5 | 18.3 |
| 8-15 | mAb1 (60) | G-4 (40eq. ) | Endo-S2 D184M | Endo-M N175Q | 32.7 | 23.3 | 22.3 | 15.5 | 1.8 | 2.2 |
| 8-16 | mAb1 (60) | G-3 (100eq. ) | Endo-S2 D184M | Endo-M N175Q | 63.7 | 50.8 | 44.3 | 35.9 | 18.0 | 14.2 |
| 8-17 | mAb1 (60) | G-4 (100eq. ) | Endo-S2 D184M | Endo-M N175Q | 71.3 | 61.7 | 54.7 | 46.0 | 28.2 | 24.7 |

As shown in Example 11, Endo-S2 D184M, which has almost the same residual hydrolysis activity as the wild type, can achieve a transglycosylation rate of more than 80% if appropriate conditions are set in [Step 1] when G-1 without glycan modification is used as the glycan donor.

On the other hand, when a chemically-modified glycan donor was used, the antibody in which the glycan was once transferred was easily hydrolyzed with Endo-S2 D184M, which has almost the same residual hydrolysis activity as the wild type, and thus the transglycosylation rate did not exceed 80% with an amount of the glycan donor used of 40 equivalents or less. However, from the results of Reference Example 8-16 and Reference Example 8-17, it was confirmed that the transglycosylation rate tended to be improved by increasing the amount of the glycan donor used. In such a case, it can be expected that by introducing a mutation that reduces the residual hydrolysis activity, the resulting enzyme can be adopted in "Step 1".

### Reference Example 9: Preparation of anti-CD70 antibody 1

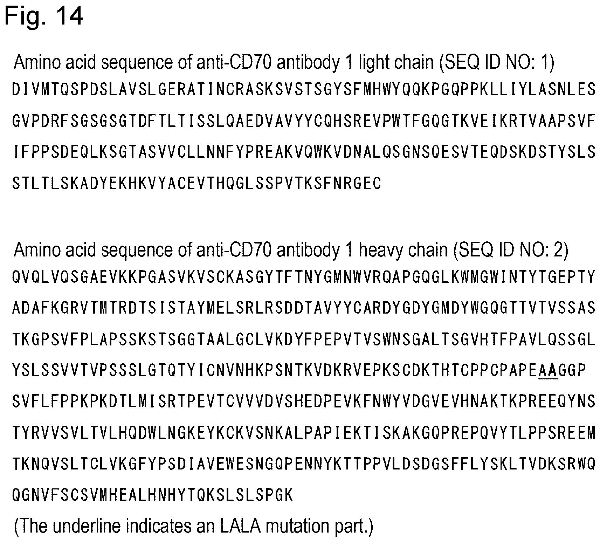

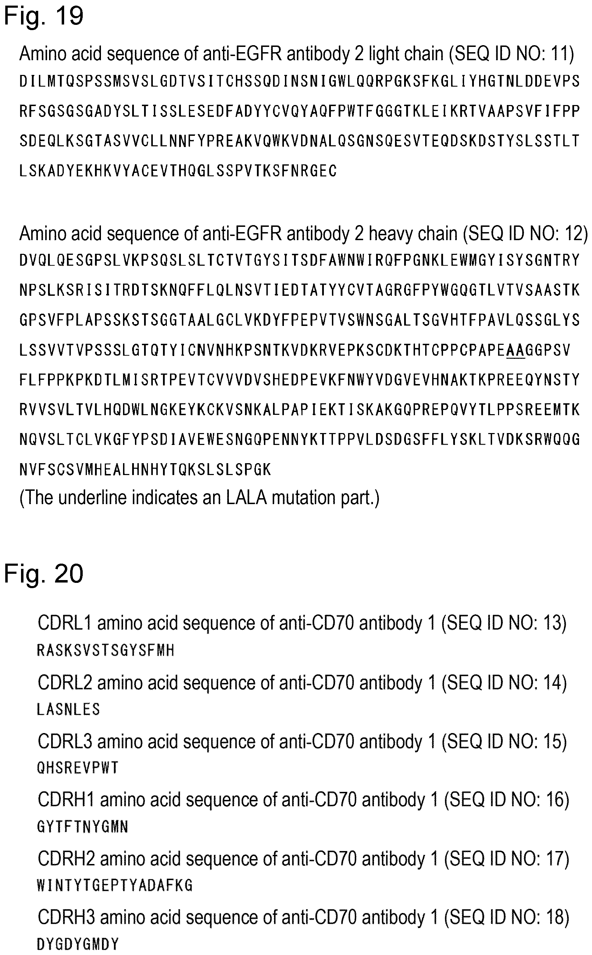

Anti-CD70 antibody 1 was prepared with reference to WO2004/073656. The anti-CD70 antibody 1 has an isotype of IgG1 and an LALA mutation (IgG1-L234A and L235A). The light chain amino acid sequence (SEQ ID NO: 1) and the heavy chain amino acid sequence (SEQ ID NO: 2) of the anti-CD70 antibody 1 are shown in Figure 14. In the light chain sequence shown in SEQ ID NO: 1 (Figure 14), the amino acid sequence consisting of amino acid residues 1 to 112 is a light chain variable region, and in the heavy chain sequence shown in SEQ ID NO: 2 (Figure 14), the amino acid sequence consisting of amino acid residues 1 to 118 is a heavy chain variable region. The CDRL1 amino acid sequence (SEQ ID NO: 13), the CDRL2 amino acid sequence (SEQ ID NO: 14), the CDRL3 amino acid sequence (SEQ ID NO: 15), the CDRH1 amino acid sequence (SEQ ID NO: 16), the CDRH2 amino acid sequence (SEQ ID NO: 17), and the CDRH3 amino acid sequence (SEQ ID NO: 18) of the antibody are shown in Figure 20.

### Reference Example 10: Preparation of anti-CD70 antibody 2

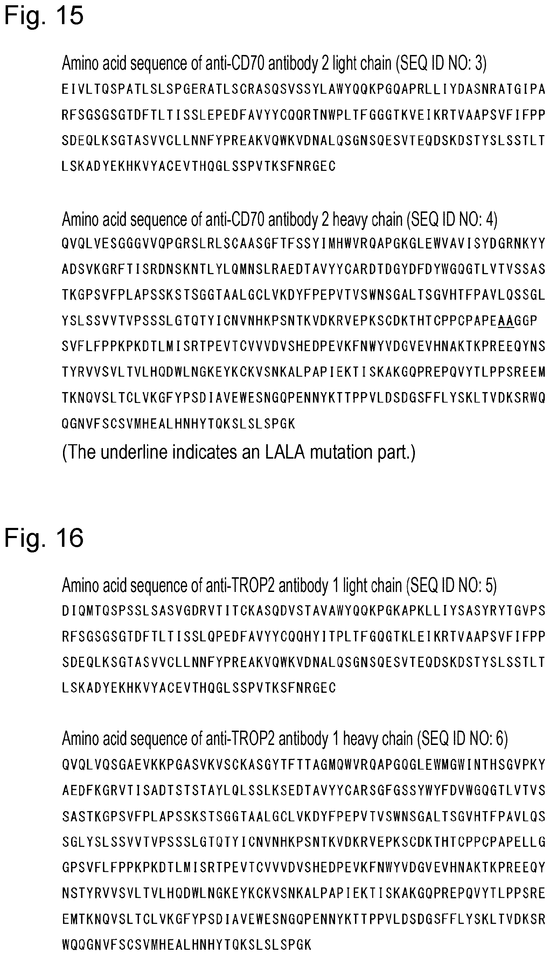

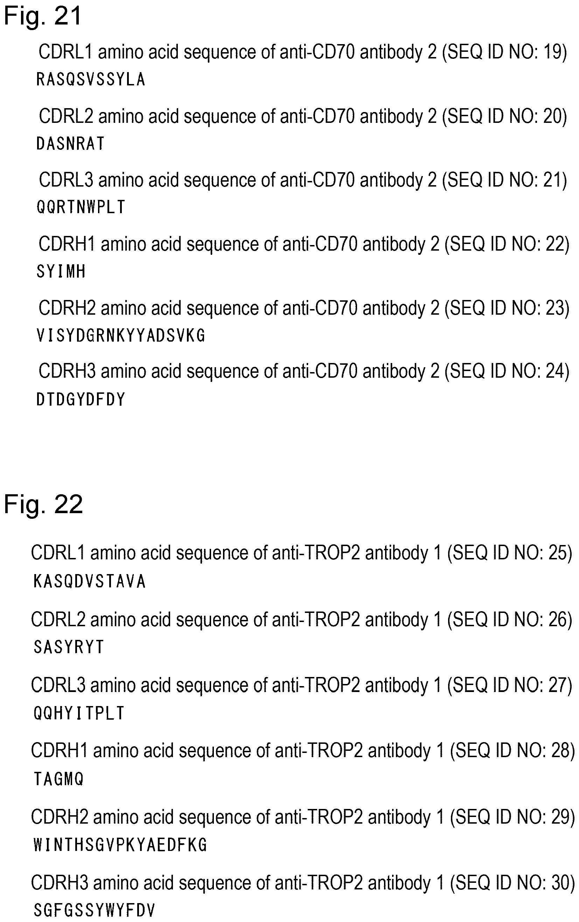

Anti-CD70 antibody 2 was prepared with reference to WO2007/038637. The anti-CD70 antibody 2 has an isotype of IgG1 and has an LALA mutation. The light chain amino acid sequence (SEQ ID NO: 3) and the heavy chain amino acid sequence (SEQ ID NO: 4) of the anti-CD70 antibody 2 are shown in Figure 15. In the light chain sequence shown in SEQ ID NO: 3 (Figure 15), the amino acid sequence consisting of amino acid residues 1 to 108 is a light chain variable region, and in the heavy chain sequence shown in SEQ ID NO: 4 (Figure 15), the amino acid sequence consisting of amino acid residues 1 to 118 is a heavy chain variable region. The CDRL1 amino acid sequence (SEQ ID NO: 19), the CDRL2 amino acid sequence (SEQ ID NO: 20), the CDRL3 amino acid sequence (SEQ ID NO: 21), the CDRH1 amino acid sequence (SEQ ID NO: 22), the CDRH2 amino acid sequence (SEQ ID NO: 23), and the CDRH3 amino acid sequence (SEQ ID NO: 24) of the antibody are shown in Figure 21.

### Reference Example 11: Preparation of anti-TROP2 antibody 1

Anti-TROP2 antibody 1 was prepared with reference to WO2015/098099. The anti-TROP2 antibody 1 has an isotype of IgG1. The light chain amino acid sequence (SEQ ID NO: 5) and the heavy chain amino acid sequence (SEQ ID NO: 6) of the anti-TROP2 antibody 1 are shown in Figure 16. In the light chain sequence shown in SEQ ID NO: 5 (Figure 16), the amino acid sequence consisting of amino acid residues 1 to 108 is a light chain variable region, and in the heavy chain sequence shown in SEQ ID NO: 6 (Figure 16), the amino acid sequence consisting of amino acid residues 1 to 121 is a heavy chain variable region. The CDRL1 amino acid sequence (SEQ ID NO: 25), the CDRL2 amino acid sequence (SEQ ID NO: 26), the CDRL3 amino acid sequence (SEQ ID NO: 27), the CDRH1 amino acid sequence (SEQ ID NO: 28), the CDRH2 amino acid sequence (SEQ ID NO: 29), and the CDRH3 amino acid sequence (SEQ ID NO: 30) of the antibody are shown in Figure 22.

### Reference Example 12: Preparation of anti-TROP2 antibody 2

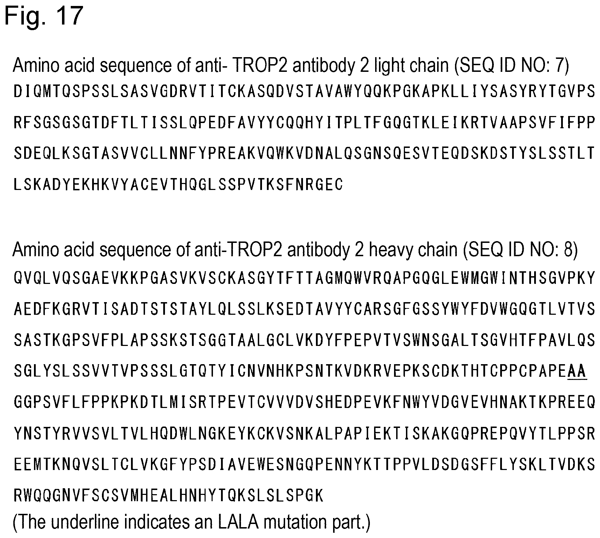

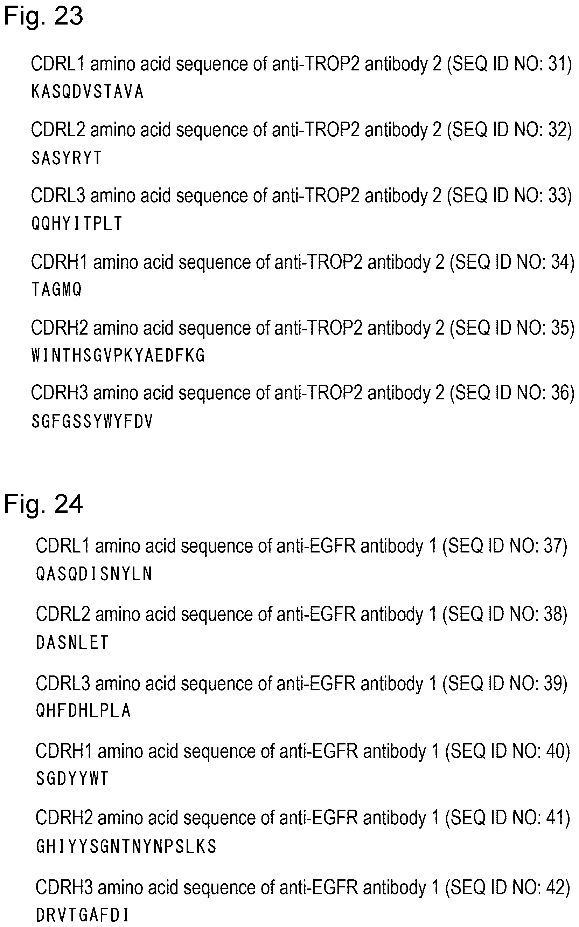

Anti-TROP2 antibody 1 was prepared with reference to WO2015/098099. The anti-TROP2 antibody 1 has an isotype of IgG1. Anti-TROP2 antibody 2 is obtained by introducing an LALA mutation into anti-TROP2 antibody 1. The light chain amino acid sequence (SEQ ID NO: 7) and the heavy chain amino acid sequence (SEQ ID NO: 8) of the anti-TROP2 antibody 2 are shown in Figure 17. In the light chain sequence shown in SEQ ID NO: 7 (Figure 17), the amino acid sequence consisting of amino acid residues 1 to 108 is a light chain variable region, and in the heavy chain sequence shown in SEQ ID NO: 8 (Figure 17), the amino acid sequence consisting of amino acid residues 1 to 121 is a heavy chain variable region. The CDRL1 amino acid sequence (SEQ ID NO: 31), the CDRL2 amino acid sequence (SEQ ID NO: 32), the CDRL3 amino acid sequence (SEQ ID NO: 33), the CDRH1 amino acid sequence (SEQ ID NO: 34), the CDRH2 amino acid sequence (SEQ ID NO: 35), and the CDRH3 amino acid sequence (SEQ ID NO: 36) of the antibody are shown in Figure 23.

### Reference Example 13: Preparation of anti-EGFR antibody 1

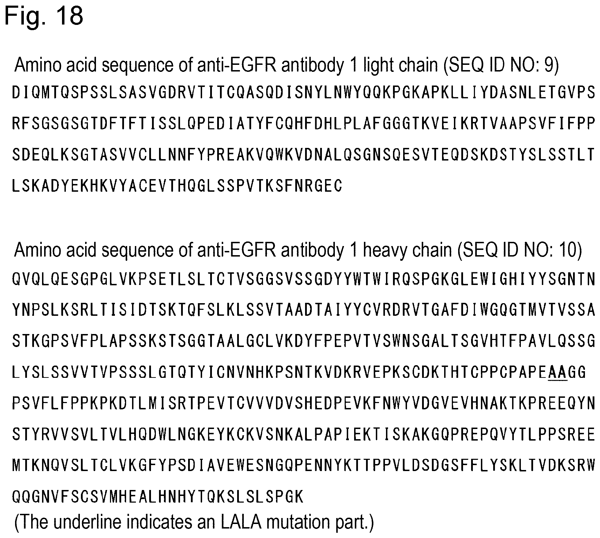

Anti-EGFR antibody 1 was prepared with reference to the Vectibix Intravenous Infusion 100 mg Examination Result Report (March 5, 2010, Evaluation and Licensing Division, Pharmaceutical and Food Safety Bureau). The anti-EGFR antibody 1 has an isotype of IgG1 and has an LALA mutation. The light chain amino acid sequence (SEQ ID NO: 9) and the heavy chain amino acid sequence (SEQ ID NO: 10) of the anti-EGFR antibody 1 are shown in Figure 18. In the light chain sequence shown in SEQ ID NO: 9 (Figure 18), the amino acid sequence consisting of amino acid residues 1 to 108 is a light chain variable region, and in the heavy chain sequence shown in SEQ ID NO: 10 (Figure 18), the amino acid sequence consisting of amino acid residues 1 to 119 is a heavy chain variable region. The CDRL1 amino acid sequence (SEQ ID NO: 37), the CDRL2 amino acid sequence (SEQ ID NO: 38), the CDRL3 amino acid sequence (SEQ ID NO: 39), the CDRH1 amino acid sequence (SEQ ID NO: 40), the CDRH2 amino acid sequence (SEQ ID NO: 41), and the CDRH3 amino acid sequence (SEQ ID NO: 42) of the antibody are shown in Figure 24. The CDR sequences were referenced from WO1998/050433.

### Reference Example 14: Preparation of anti-EGFR antibody 2

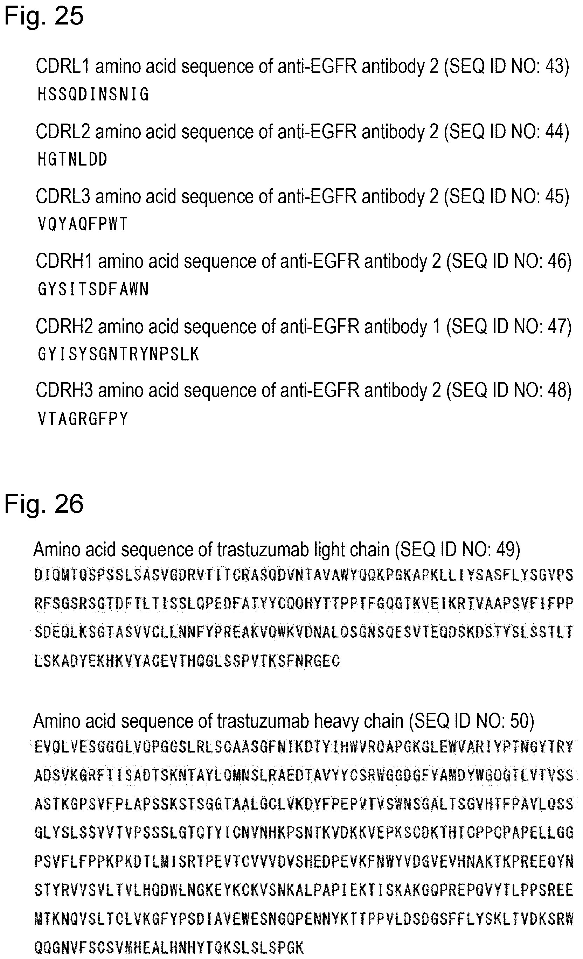

Anti-EGFR antibody 2 was prepared with reference to WO2002/092771. The anti-EGFR antibody 2 has an isotype of IgG1 and has an LALA mutation. The light chain amino acid sequence (SEQ ID NO: 11) and the heavy chain amino acid sequence (SEQ ID NO: 12) of the anti-EGFR antibody 2 are shown in Figure 19. In the light chain sequence shown in SEQ ID NO: 11 (Figure 19), the amino acid sequence consisting of amino acid residues 1 to 108 is a light chain variable region, and in the heavy chain sequence shown in SEQ ID NO: 12 (Figure 19), the amino acid sequence consisting of amino acid residues 1 to 116 is a heavy chain variable region. The CDRL1 amino acid sequence (SEQ ID NO: 43), the CDRL2 amino acid sequence (SEQ ID NO: 44), the CDRL3 amino acid sequence (SEQ ID NO: 45), the CDRH1 amino acid sequence (SEQ ID NO: 46), the CDRH2 amino acid sequence (SEQ ID NO: 47), and the CDRH3 amino acid sequence (SEQ ID NO: 48) of the antibody are shown in Figure 25.

### Reference Example 15: Preparation of anti-HER2 antibody

Herein, "trastuzumab" is sometimes also referred to as HERCEPTIN(registered trademark), huMAb4D5-8, or rhuMAb4D5-8, and is a humanized IgG1 antibody having a light chain consisting of the amino acid sequence set forth in SEQ ID NO: 49 and a heavy chain consisting of the amino acid sequence set forth in SEQ ID NO: 50. The amino acid sequences were referenced from US5821337. The light chain amino acid sequence (SEQ ID NO: 49) and the heavy chain amino acid sequence (SEQ ID NO: 50) of trastuzumab are shown in Figure 26.

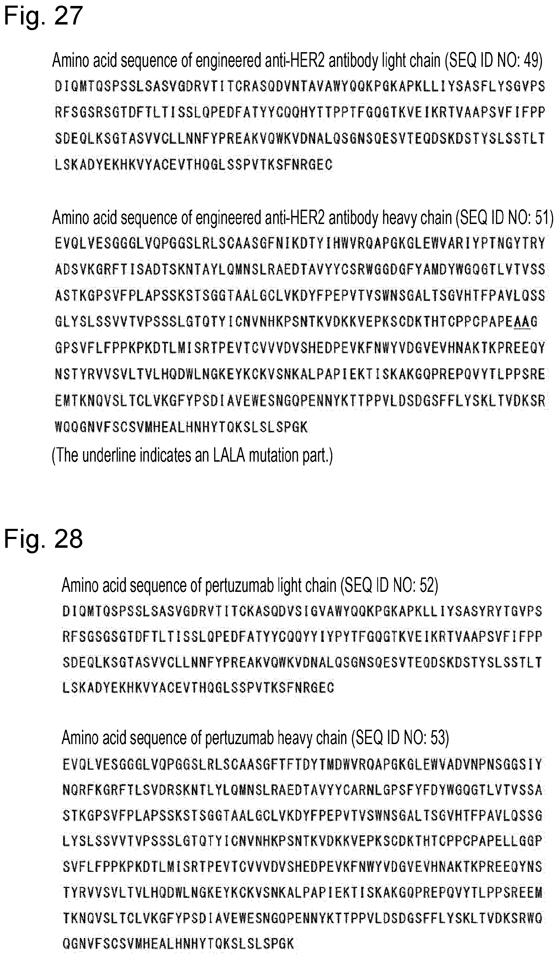

The anti-HER2 antibody can be designed and prepared as a trastuzumab constant region-engineered IgG1 antibody in which leucine (L) at position 234 and leucine (L) at position 235 as numbered according to EU index numbering of the heavy chain amino acid sequence of trastuzumab are each mutated to alanine (A) (also referred to herein as an LALA mutation) (also referred to herein as an engineered anti-HER2 antibody). The light chain amino acid sequence (SEQ ID NO: 49) and heavy chain amino acid sequence (SEQ ID NO: 51) of the engineered anti-HER2 antibody are shown in Figure 27.

### Reference Example 16: Preparation of anti-HER2 antibody 2

"Pertuzumab" is sometimes also referred to as PERJETA(registered trademark), and is a humanized IgG1 antibody having a light chain consisting of the amino acid sequence set forth in SEQ ID NO: 52 and a heavy chain consisting of the amino acid sequence set forth in SEQ ID NO: 53. The amino acid sequences were referenced from WO2004/008099. Pertuzumab is also referred to herein as anti-HER2 antibody 2. The light chain amino acid sequence (SEQ ID NO: 52) and the heavy chain amino acid sequence (SEQ ID NO: 53) of pertuzumab are shown in Figure 28. In the light chain sequence shown in SEQ ID NO: 52 (Figure 28), the amino acid sequence consisting of amino acid residues 1 to 108 is a light chain variable region, and in the heavy chain sequence shown in SEQ ID NO: 53 (Figure 28), the amino acid sequence consisting of amino acid residues 1 to 119 is a heavy chain variable region, and the positions of the variable regions were determined according to the IMGT definition.

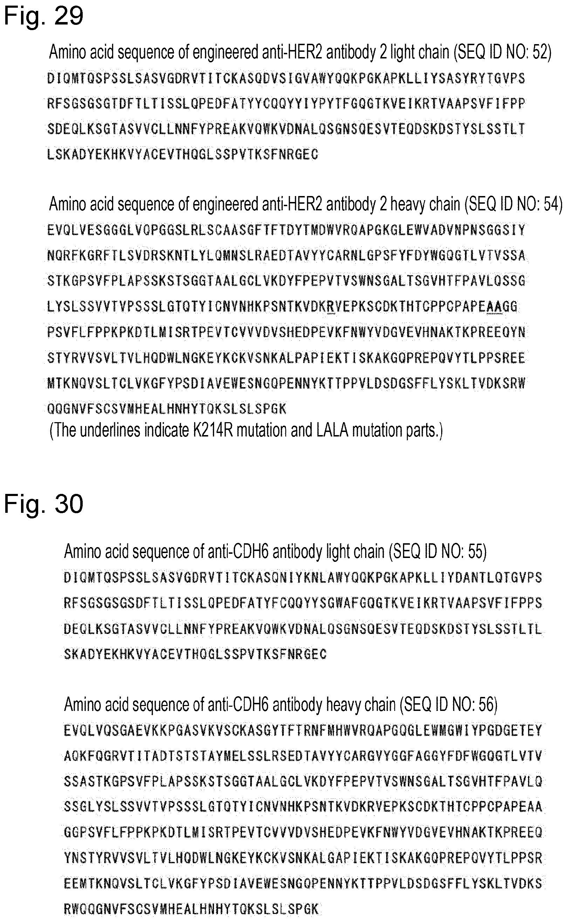

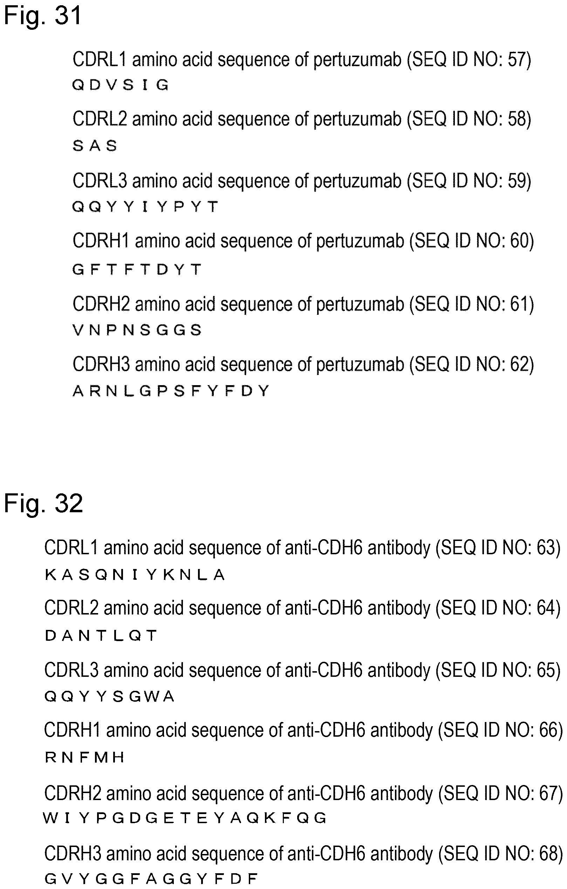

Anti-HER2 antibody 2 can be designed and prepared as anti-HER2 antibody 2 having a G1m3 allotype constant region in which lysine (K) at position 214 as numbered according to EU index numbering in the heavy chain amino acid sequence is mutated to arginine (R) in addition to the LALA mutation (also referred to herein as engineered anti-HER2 antibody 2). The light chain amino acid sequence (SEQ ID NO: 52) and the heavy chain amino acid sequence (SEQ ID NO: 54) of the engineered anti-HER2 antibody 2 are shown in Figure 29. The CDRL1 amino acid sequence (SEQ ID NO: 57), the CDRL2 amino acid sequence (SEQ ID NO: 58), the CDRL3 amino acid sequence (SEQ ID NO: 59), the CDRH1 amino acid sequence (SEQ ID NO: 60), the CDRH2 amino acid sequence (SEQ ID NO: 61), and the CDRH3 amino acid sequence (SEQ ID NO: 62) of the antibody are shown in Figure 31. In the light chain sequence shown in SEQ ID NO: 52 (Figure 29), the amino acid sequence consisting of amino acid residues 1 to 108 is a light chain variable region, and in the heavy chain sequence shown in SEQ ID NO: 54 (Figure 29), the amino acid sequence consisting of amino acid residues 1 to 119 is a heavy chain variable region, and the positions of the variable regions were determined according to the IMGT definition.

### Reference Example 17: Preparation of anti-CDH6 antibody

The anti-CDH6 antibody can be prepared with reference to WO2018/212136. The anti-CDH6 antibody has an isotype of IgG1, and in addition to the LALA mutation, has a mutation of proline (P) at position 329 as numbered according to EU index numbering in the heavy chain amino acid sequence to glycine (G). The light chain amino acid sequence (SEQ ID NO: 55) and the heavy chain amino acid sequence (SEQ ID NO: 56) of the anti-CDH6 antibody are shown in Figure 30. In the light chain sequence shown in SEQ ID NO: 55 (Figure 30), the amino acid sequence consisting of amino acid residues 1 to 107 is a light chain variable region, and in the heavy chain sequence shown in SEQ ID NO: 56 (Figure 30), the amino acid sequence consisting of amino acid residues 1 to 122 is a heavy chain variable region. The CDRL1 amino acid sequence (SEQ ID NO: 63), the CDRL2 amino acid sequence (SEQ ID NO: 64), the CDRL3 amino acid sequence (SEQ ID NO: 65), the CDRH1 amino acid sequence (SEQ ID NO: 66), the CDRH2 amino acid sequence (SEQ ID NO: 67), and the CDRH3 amino acid sequence (SEQ ID NO: 68) of the antibody are shown in Figure 32.

### Reference Example 18: Application Method Example 1 of "Step 1"

In recent years, examples of transferring a glycan structure that is not easily degraded by Endo-S2 WT to an antibody that is an Fc-containing molecule have been reported [Non Patent Literature: ACS Chem. Biol. 2021, 2502-2514., Non Patent Literature: Acta Pharm. Sin. B 2022, 2417-2428]. Therefore, by appropriately selecting the structure of a glycan donor molecule, it is possible to obtain a desired product in which a glycan derived from the glycan donor molecule is transferred to an acceptor molecule even when a wild type enzyme is used. For example, with reference to the structure of the glycan oxazoline form disclosed in these reports, a non-oxazolinated O-linked glycan donor molecule or N-linked glycan donor molecule having one GlcNAc extended on the reducing end side is provided, and furthermore, with reference to conditions herein, a wild type Enzyme-A is caused to act thereon in the presence of an appropriate Enzyme-B that can act on O-linked glycans or N-linked glycans of these glycan donors, thereby making it possible to obtain a reaction solution including the desired product. Therefore, such an application example is included in "Step 1" of the present invention. In addition, those skilled in the art can refer to the present specification to identify the structures of Enzyme-A and Enzyme-B that work concertedly, which are not described as an example in the present Example and herein, and examples thereof include a wild type Enzyme-A selected from Endo-S2, Endo-S, Endo-Si, and the like, and an Enzyme-B selected from Endo-M, Endo-Rp, Endo-CC (SEQ ID NO: 6), Endo-Om (SEQ ID NO: 7), Endo-A (GenBank Accession number: AAD10851), Endo-CE (GenBank Accession number: BAB84821), Endo-Tsp1006 (GenBank Accession number: CCY37287), Endo-Tsp1263 (GenBank Accession number: CDD88945), Endo-Tsp1457 (GenBank Accession number: CDD89351), Endo-BB (GenBank Accession number: AAN25135), Endo-BH (GenBank Accession number: BAB04504.1), Endo-BN (GenBank Accession number: WP_007484749.1), Endo-CC1 (GenBank Accession number: XP_001839402.1), Endo-CC2 (GenBank Accession number: XP_002911817.1), Endo-D (GenBank Accession number: AAK74656.1), Endo-Pm (GenBank Accession number: ADK97032.1), and the like.

### Reference Example 19: Application Method Example 2 of "Step 1"

In the Experimental Examples herein, only examples in in vitro reactions are shown, but even when Enzyme-A and Enzyme-B are co-expressed in an antibody-producing cell, it is expected that the same situation as in Figure 1 or Figure XX2 will be reproduced in the producing cell. Those skilled in the art can refer to the present specification and appropriately set a co-expression condition to obtain a reaction solution including the desired product (when the target reaction proceeds even in a culture solution, the culture solution corresponds to the reaction solution). Therefore, such an application example is included in "Step 1" of the present invention.

### Reference Example 20: Application Method Example 3 of "Step 1"

Enzyme-A of the present invention is an enzyme that can act on a glycan of an Fc-containing molecule. For example, when a structure having affinity for an Fc fragment of an antibody is introduced into an enzyme partially including an amino acid sequence such as Endo-BI1 (GenBank Accession number: ACJ53522.1), Endo-BI2 (GenBank Accession number: BAJ71450.1), Endo-BT-3987 (GenBank Accession number: AAO79092.1), Endo-E (GenBank Accession number: AAR20477.1), Endo-F (GenBank Accession number: AAA24922.1), Endo-F2 (GenBank Accession number: AAA24923.1), Endo-F3 (GenBank Accession number: AAA24924.1), Endo-CoM (GenBank Accession number: XP006673222), or Endo-SB (GenBank Accession number: BBB35949) (hereinafter referred to as an Enzyme-A fragment), it is expected that the Enzyme-A fragment works as Enzyme-A of the present invention.

It was known that when an oxazoline form is used as a glycan donor and an Endo-F3 mutant is used, a larger amount of a glycan or an enzyme is required than when Endo-S or the like is used, that is, the efficiency is poor from the viewpoint of mass production (Non Patent Literature: J. Biol. Chem. 2016, 9356-9370). On the other hand, it has been reported that by giving an Fc-binding peptide to an Endo-F3 mutant, it becomes possible to efficiently transfer a glycan to the antibody Fc portion (Non Patent Literature: Org. Biomol. Chem. 2022, 3086-3095). From the above, it was shown that the Endo-F3-Fc-binding peptide complex works like Endo-S, that is, by combining an Enzyme-A fragment and an Fc-binding structure, it is possible to artificially prepare an enzyme that can act on a glycan of an Fc-containing molecule. Even when artificially prepared Enzyme-A is used, those skilled in the art can refer to the present specification to identify the structure of Enzyme-A and the structures of Enzyme-A and Enzyme-B that work concertedly, which are not described as an example in the present Example and herein, and obtain a reaction solution including the desired product. Therefore, such an application example is included in "Step 1" of the present invention (however, the Fc binding structure is any peptide, nucleic acid, small molecule, or the like that exhibits affinity for an Fc fragment).

### Reference Example 21: Application Method Example 4 of "Step 1"

The glycan donor molecule of the present invention is an N-linked glycan or an O-linked glycan that includes GlcNAc having an unactivated reducing end and has an optionally chemically-modified non-reducing end. The glycan donor molecule is not limited to the glycans used in the Examples of the present specification, and it is reasonably expected that Step 1 can also be applied to a glycan donor having a triantennary type glycan structure, a tetraantennary type glycan structure, a glycan structure loaded with a payload, or the like. That is, by appropriately selecting Enzyme-B that can act on the above glycan donor molecule and Enzyme-A, the target transglycosylation reaction proceeds.

For example, it has been reported that when Endo-S or a mutant thereof, Endo-S2 or a mutant thereof, or Endo-F3 or a mutant thereof is appropriately used depending on the purpose, the desired compound can be obtained if triantennary type glycan oxazoline or tetraantennary type glycan oxazoline, or a glycan oxazoline form loaded with a payload is used as a glycan donor (Non Patent Literatures: Bioorg. Med. Chem, 2018, 1347-1355., J Biol. Chem. 2016, 16508-16518, Acta Pharm. Sin. B 2022, 2417-2428, Patent Literatures: WO2017124084, US2019002542, JP2021-145548). From the above, it can be expected that by appropriately using a condition with reference to the present specification, Endo-S2 acts on an Fc-containing molecule including a GlcNAc to work as Enzyme-A for introducing a triantennary type glycan, a tetraantennary type glycan, or a glycan loaded with a payload. That is, even when a glycan donor molecule having a triantennary type glycan structure, a tetraantennary type glycan structure, a glycan structure loaded with a payload, or the like is used, those skilled in the art can refer to the present specification to identify the structure of Enzyme-A and the structures of Enzyme-A and Enzyme-B that work concertedly, which are not described as an example in the present Example and herein, and obtain a reaction solution including the desired product. Therefore, such an application example is included in "Step 1".

### Reference Example 22 Preparation of antibody solutions used in Example 15 and Example 20

Available Trastuzumab or Mogamulizumab (Poteligeo 20 mg, Kyowa Kirin Co., Ltd.) was used as the antibody, and buffer exchange by concentration dialysis (UFDF) using an ultrafiltration membrane Amicon Ultra-15, 30 kDa (Merck) and 50 mM Tris-HCl (pH 7.25) was carried out to provide antibody solutions shown in Table Y-3.

In addition, according to the method disclosed in Example 3 of WO2017010559, Mogamulizumab (Poteligeo 20 mg, Kyowa Kirin Co., Ltd.) was used as the antibody, the glycan was cleaved with wild type Endo-S, and after purification, buffer exchange by concentration dialysis (UFDF) using an ultrafiltration membrane Amicon Ultra-15, 30 kDa (Merck) and 50 mM Tris-HCl (pH 7.25) was carried out to provide acceptor molecule solution 9-1.

Furthermore, a (Fucα1,6)GlcNAc-Fc-A solution prepared by the method disclosed in Example 3-9A of WO2018003983 was used, and buffer exchange by concentration dialysis (UFDF) using an ultrafiltration membrane Amicon Ultra-500, 10 kDa (Merck) and 50 mM Tris-HCl (pH 7.5) was carried out to provide acceptor molecule solution 10-1 shown in Table Y-3.

A (Fucα1,6)GlcNAc-CLCH-A solution prepared by the method disclosed in Example 3-3A of WO2018003983 was used, and buffer exchange by concentration dialysis (UFDF) using an ultrafiltration membrane Amicon Ultra-500, 10 kDa (Merck) and 50 mM Tris-HCl (pH 7.25) was carried out to provide acceptor molecule solution 11-1 shown in Table Y-3.

**Table Y-3**

| **Solution name** | **Fc-containing molecule** | **Concentration (mg/mL)** | **Volume (mL)** |
|---|---|---|---|
| **Antibody solution 8-1 (Trastuzumab)** | naked -mAb1 | 95.32 | 3.5 |
| **Acceptor molecule solution 9-1** | GlcNAc-mAb4 | 90.75 | 0.080 |
| **Acceptor molecule solution 10-1** | (Fuc α 1,6)GlcNAc- Fc-A | 118.16 | 0.10 |
| **Acceptor molecule solution 11-1** | (Fuc α 1,6)GlcNAc- CLC H-A | 108.89 | 0.10 |

Reference Example 23 Preparation of glycan donor G-18 (mixture of M9-Asn, M8-Asn, M7-Asn, and M6-Asn) and confirmation of [Step 1] Reference Example 23-1 Preparation of glycan donor G-18

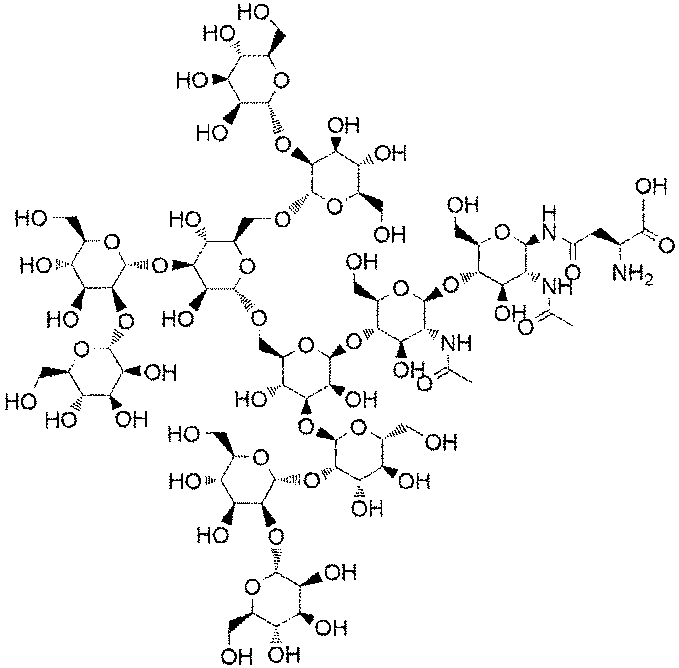

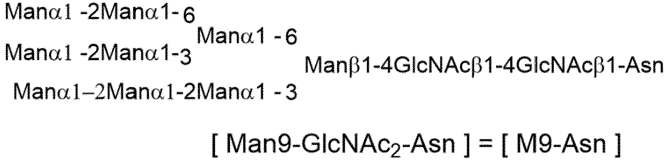

Soybean powder is known to include the structure of [M9-Asn] (Non Patent literature: Chem. Biol. 2004, 127-134). M8-Asn, M7-Asn, or M6-Asn has a structure lacking one, two, or three mannose residues, respectively, from M9-Asn.

### [Insoluble component removal (1)]

Commercially available soybean powder (Soybean Flour Type I, 1 Kg, manufactured by Sigma-Aldrich) was suspended in a 0.15 N sodium hydroxide aqueous solution (5 L), and the resulting suspension was stirred for 120 minutes and then centrifuged (8000 rpm, 30 minutes) to collect supernatant 1 (3.8 L).

### [Removal of water-soluble component]

Subsequently, ammonium sulfate (1.14 Kg) was added to supernatant 1. After that, the resulting mixture was centrifuged (8000 rpm, 30 minutes), and supernatant 2 was removed to obtain precipitate 1.

### [Insoluble component removal (2)]

Precipitate 1 was dissolved in a 0.15 N sodium hydroxide aqueous solution (4 L), and then an appropriate amount of 5 N hydrochloric acid was added to adjust the pH to 5.0. After that, the resulting mixture was centrifuged (8000 rpm, 30 minutes), and supernatant 3 was removed to obtain precipitate 2.

### [Enzyme reaction]

Precipitate 2 was dissolved in a 0.15 N sodium hydroxide aqueous solution (3 L), and then an appropriate amount of 5 N hydrochloric acid was added to adjust the pH to 5.0. After that, Actinase AS (300 g, manufactured by Kaken Pharmaceutical Co., Ltd.) was added, and then the resulting mixture was stirred at 37°C for 48 hours. After completion of the reaction, an appropriate amount of 5 N hydrochloric acid was added to adjust the pH to 5.0, and the resulting mixture was filtered through Celite under reduced pressure (precoat: Celite 505, body feed: Celite 545) to obtain filtrate A (2.7 L).

### [Insoluble component removal (3)]

A 3-fold volume of acetone was added to filtrate A (2.7 L), and for the purpose of collecting the resulting precipitate 3, the resulting mixture was centrifuged (8000 rpm, 30 minutes) to separate supernatant 4. Subsequently, the same volume of acetone as the first time was added to supernatant 4 separated, and for the purpose of collecting the resulting precipitate 4, the resulting mixture was centrifuged (8000 rpm, 30 minutes) to separate supernatant 5. Precipitate 3 and precipitate 4 obtained in the first operation and the second operation were combined into one, and this was dissolved in distilled water (2 L). Subsequently, the resulting solution was filtered under reduced pressure (filter paper: ADVANTEC No. 2) to obtain filtrate B.

### [Insoluble component removal (4)]

Acetone was removed from filtrate B under reduced pressure, then an appropriate amount of 5 N hydrochloric acid was added to adjust the pH to 3.0, and then the resulting mixture was filtered through Celite (precoat: Celite 505, body feed: Celite 545) under reduced pressure to obtain filtrate C (1.5 L).

### [Crude purification (1): Column purification]

Filtrate C (1.5 L) was passed through a column packed with SEPABEADS SP207 (200 mL, manufactured by Mitsubishi Chemical Corporation) to collect a flow-through solution. After that, distilled water was added to make up to 5.0 L to obtain a column treated solution (5.0 L).

### [Crude purification (2): Membrane purification]

The column treated solution (5.0 L) was ultrafiltered by using an ultrafiltration membrane (Hydrosart 2 kDa, 0.1 m², manufactured by Sartorius) while adding 10 L of distilled water, to obtain an ultrafiltration treated solution (3 L).

### [Obtaining extract]

A 2-fold volume of acetone was added to the ultrafiltration treated solution (3 L), and for the purpose of collecting the resulting precipitate 5, the resulting mixture was centrifuged (8000 rpm, 30 minutes) to separate supernatant 6. Subsequently, the same volume of acetone as the first time was added to supernatant 6 separated, and for the purpose of collecting the resulting precipitate 6, the resulting mixture was centrifuged (8000 rpm, 30 minutes) to separate supernatant 7. Precipitate 5 and precipitate 6 obtained in the first operation and the second operation were combined into one, and this was freeze-dried to obtain an extract (78.6 g).

### [Column purification]

Distilled water including 0.1% formic acid and acetonitrile were used as eluents, Agilent 1100 series LC (manufactured by Agilent technologies) was used as an apparatus, Hypercarb (20Φ × 150 mm, manufactured by Thermo Fisher Scientific Inc) was used as a column, a portion (about 20 g) of the extract obtained above was separated and purified. The main peak detected by MS detection was collected and freeze-dried. The resulting freeze-dried powder was dissolved in distilled water, and an appropriate amount of aqueous ammonia was added to adjust the pH to 7.0. After that, the resulting mixture was freeze-dried again to obtain glycan raw material G-18 (46.7 mg) as a freeze-dried powder.

### [Mass spectrometry]

Agilent 1200 (manufactured by Agilent Technologies), Orbitrap Elite (manufactured by Thermo Fisher Scientific Inc), and Hypercarb (manufactured by Thermo Fisher Scientific Inc) (5 µm, 4.6 × 150 mm) were used apparatuses, and water was used as mobile phase A, acetonitrile was used as mobile phase B, and a gradient changing from 5% to 15% of mobile phase B over 15 minutes was used to carry out analysis at a flow rate of 1.0 mL/min and 40°C. Mainly 4 peaks were observed.

ESI-MS: Calcd for M9-Asn C74H122N4O58: [M-2H]²⁻ 997.34 (most abundant mass), Found 997.34.

Man8GlcNAc2-Asn [M8-Asn] has a structure lacking one mannose residue on the non-reducing end side of M9-Asn.
ESI-MS: Calcd for M8-Asn C68H112N4O53: [M-2H]²⁻ 916.31 (most abundant mass), Found 916.31.

Man7GlcNAc2-Asn [M7-Asn] has a structure lacking two mannose residues on the non-reducing end side of M9-Asn.
ESI-MS: Calcd for M7-Asn C62H102N4O48: [M-2H]²⁻ 835.28 (most abundant mass), Found 835.28.

Man6GlcNAc2-Asn [M6-Asn] has a structure lacking three mannose residues on the non-reducing end side of M9-Asn.
ESI-MS: Calcd for M6-Asn C62H102N4O48: [M-2H]²⁻ 754.26 (most abundant mass), Found 754.26.

### [HPLC analysis]

Agilent 1260 (manufactured by Agilent Technologies) and Hypercarb (manufactured by Thermo Fisher Scientific Inc) (5 µm, 4.6 × 150 mm) were used apparatuses, water was used as mobile phase A, acetonitrile was used as mobile phase B, and a gradient changing from 5% to 15% of mobile phase B over 20 minutes was used to carry out analysis at a flow rate of 1.0 mL/min and 40°C. Mainly 4 peaks were observed.

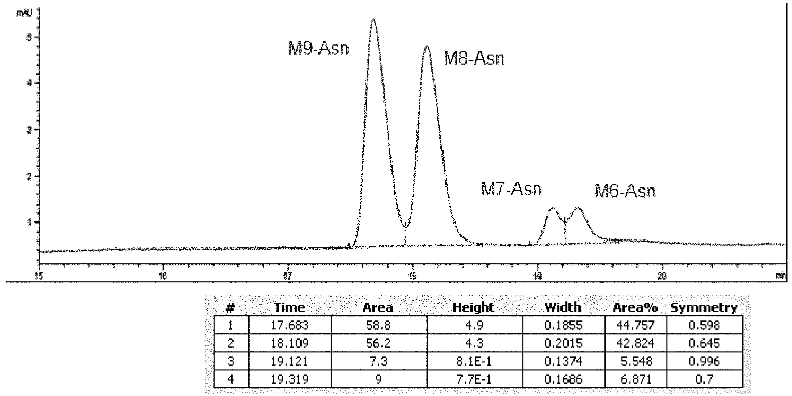
mAU
5
M9-Asn
M8-Asn
4
3
2
M7-Asn
M6-Asn
1
15 16 17 18 19 20 mi

# | Time | Area | Height | Width | Area% | Symmetry
1 | 17.683 | 58.8 | 4.9 | 0.1855 | 44.757 | 0.598
2 | 18.109 | 56.2 | 4.3 | 0.2015 | 42.824 | 0.645
3 | 19.121 | 7.3 | 8.1E-1 | 0.1374 | 5.548 | 0.996
4 | 19.319 | 9 | 7.7E-1 | 0.1686 | 6.871 | 0.7

### Reference Example 23-2 Transglycosylation reaction using glycan donor G-18

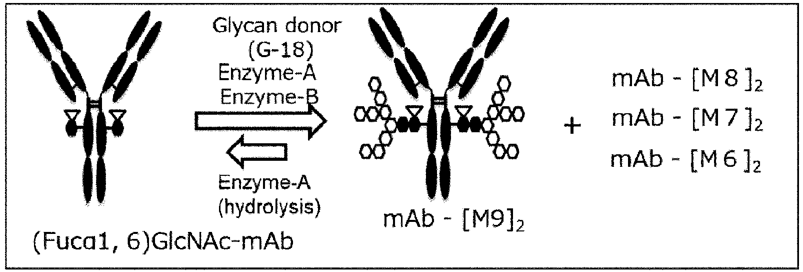

(In the figure, M8, M7, or M6 has a structure lacking one, two, or three mannose residues, respectively, from M9.)

(Fucα1,6)GlcNAc-mAb1 (0.6 mg), a glycan donor (40 equivalents), Enzyme-A (0.8 to 1.6% w/w), Enzyme-B (0.02% to 0.2% w/w), and an appropriate amount of a 50 mM Tris-HCl buffer solution (pH 7.25) were added to make a total solution volume of 15 µL, and the resulting mixture was reacted at 30°C for 24 hours. Sampling was carried out 2 hours, 4 hours, 6 hours, 8 hours, 22 hours, and 24 hours after the start of the reaction. After completion of the reaction, the pH of the reaction solution was measured. At the time of sampling, a portion of the reaction solution was withdrawn, diluted with purified water such that the concentration of the antibody contained was 1 mg/mL, and stored frozen until the start of LabChip analysis.

For each reaction condition used for (Fucα1,6)GlcNAc-mAb1, the transglycosylation rate at each reaction time was calculated in the same manner as in (Example 8-1) (Table ZZ17).

**Table ZZ17**

| **Reference Example** | **Antibody (mg/mL)** | **Glycan donor** | **Enzyme-A** | **Enzyme-B** | **Transglycosylation rate (%)** | | | | | |
|---|---|---|---|---|---|---|---|---|---|---|
| | | | | | 2h | 4h | 6h | 8h | 22h | 24h |
| 23-1 | mAb1 (40) | G-18 | Endo-S2 D184Q/Q250L | Endo-Rp N172H (0.2%) | 30.3 | 33.8 | 32.4 | 28.2 | 12. 3 | 11.2 |
| 23-2 | mAb1 (40) | G-18 | Endo-S2 D184Q/Q250L | Endo-Rp N172H (0. 1%) | 18.5 | 29.9 | 36.6 | 39.5 | 36.3 | 34.9 |
| 23-3 | mAb1 (40) | G-18 | Endo-S2 D184Q/Q250L | Endo-Rp N172H (0. 02%) | 7.1 | 9.9 | 12.2 | 14.8 | 28.1 | 28.8 |
| 23-4 | mAb1 (40) | G-18 | Endo-S2 D184Q/Q250L | Endo-M N 175Q (0.2%) | 13.8 | 15.1 | 15.0 | 13.3 | 7.2 | 6.9 |
| 23-5 | mAb1 (40) | G-18 | Endo-S2 D184Q/Q250L | Endo-M N 175Q (0.1%) | 17.5 | 20.5 | 20.4 | 19.0 | 11.2 | 10.4 |
| 23-6 | mAb1 (40) | G-18 | Endo-S2 D184Q/Q250L | Endo-M N 175Q (0.02%) | 10.2 | 14.2 | 17.5 | 19.8 | 26.1 | 26.4 |

From the results of Example 23-1 to Example 23-6, it was confirmed that "changing the addition ratio of Enzyme-B," that is, "changing the speed of activation of the glycan donor molecule by Enzyme-B" affects the maximum transglycosylation rate.

The essence of Enzyme-B that can be used in "Step 1" is the ability to activate a fucose-free glycan donor molecule to a state in which Enzyme-A can be used for a transglycosylation reaction to an Fc-containing molecule (activated intermediate). On the other hand, as shown in Example 11, the transglycosylation reaction by Enzyme-A always competes with the hydrolysis reaction of a transglycosylated form, which is a product thereof. Under such circumstances, if the speed of activation of the glycan donor molecule by Enzyme-B is insufficient, the expected transglycosylation rate is expected to be low.

In order to solve this problem, it is necessary to properly use Enzyme-B according to the glycan structure of the glycan donor molecule. It is reasonably expected that a higher transglycosylation rate can be obtained by, for example, screening Endo-A (GenBank Accession number: AAD10851), Endo-CE (GenBank Accession number: BAB84821), Endo-Tsp1006 (GenBank Accession number: CCY37287), Endo-Tsp1263 (GenBank Accession number: CDD88945), Endo-Tsp1457 (GenBank Accession number: CDD89351), Endo-BB (GenBank Accession number: AAN25135), Endo-BH (GenBank Accession number: BAB04504.1), Endo-BN (GenBank Accession number: WP_007484749.1), Endo-CC1 (GenBank Accession number: XP_001839402.1), Endo-CC2 (GenBank Accession number: XP_002911817.1), Endo-D (GenBank Accession number: AAK74656.1), or Endo-Pm (GenBank Accession number: ADK97032.1) to identify a structure of Enzyme-B suitable for G-18, in addition to changing the mutant of Endo-M or Endo-Rp.

## Claims

1. A method for producing an Fc-containing molecule having an N297-linked glycan comprising a glycan derived from a glycan donor molecule, comprising the following Steps 1 and 2:
[Step 1] reacting an acceptor molecule, which is an Fc-containing molecule having, as an N297-linked glycan, a core GlcNAc to which fucose is optionally added, with a glycan donor molecule comprising a glycan in the presence of an endo-β-N-acetylglucosaminidase (Enzyme-A), which prefers, as a substrate, an N297-linked glycan of an Fc-containing molecule, so as to obtain a reaction mixture; and
[Step 2] contacting the reaction mixture with a cation exchange chromatography medium or a multimode chromatography medium under an acidic condition and collecting the Fc-containing molecule having an N297-linked glycan comprising the glycan derived from the glycan donor molecule.

2. The method according to claim 1, wherein the glycan donor molecule comprises a GlcNAc having an unactivated reducing end.

3. The method according to claim 2, wherein in Step 1, the acceptor molecule is reacted with the glycan donor molecule in the presence of Enzyme-A and an endo-β-N-acetylglucosaminidase (Enzyme-B), which prefers, as a substrate, a glycan of a glycan donor molecule, so as to obtain a reaction mixture.

4. The method according to claim 3, wherein the endo-β-N-acetylglucosaminidase (Enzyme-B), which prefers, as a substrate, a glycan of a glycan donor molecule, is an enzyme having a property of acting on the glycan donor molecule in the presence of Enzyme-A to promote a transglycosylation reaction of the glycan derived from the glycan donor molecule to the acceptor molecule by Enzyme-A.

5. The method according to claim 3 or 4, wherein Enzyme-B is an enzyme having an activity of transglycosylation from SGP to a GlcNAc derivative.

6. The method according to any one of claim 3 to 5, wherein Enzyme-B is a mutant enzyme of Endo-M, Endo-Om, Endo-CC, or Endo-Rp, and the mutant enzyme has lower hydrolysis activity than a corresponding wild type enzyme thereof.

7. The method according to any one of claims 3 to 6, wherein Enzyme-B is selected from the group consisting of Endo-Rp N172Q, Endo-Rp N172H, Endo-Rp N172A, Endo-Rp N172C, Endo-Rp N172D, Endo-Rp N172E, Endo-Rp N172G, Endo-Rp N172I, Endo-Rp N172L, Endo-Rp N172M, Endo-Rp N172P, Endo-Rp N172S, Endo-Rp N172T, Endo-Rp N172V, Endo-Rp W278F/S216V, Endo-Rp W278F/N246D, Endo-Rp W278F/D276N, Endo-Rp W278F/A310D, Endo-Rp W278F/N172D/F307Y, Endo-Rp W278F/N172D/F307H, Endo-Rp W278F/N172D/A310D, Endo-Rp W214F/F307Y/L306I, Endo-M N175Q, Endo-M N175Q/Y217F, Endo-CC N180H, and Endo-Om N194Q.

8. The method according to any one of claims 3 to 7, wherein Enzyme-A and Enzyme-B are fused.

9. The method according to any one of claims 1 to 8, wherein in Step 1, 2 to 40 equivalents of the glycan donor molecule are used per 1 equivalent of the acceptor molecule.

10. The method according to any one of claims 1 to 9, wherein in Step 1, a transglycosylation rate exceeds 80%.

11. The method according to any one of claims 1 to 10, wherein the glycan included in the glycan donor molecule is an N-linked glycan or an O-linked glycan having an optionally chemically-modified non-reducing end.

12. The method according to claim 11, wherein the glycan donor molecule is SGP, AG(9)-P, AG(7)-P, SG-Asn, AG(9)-Asn, AG(7)-Asn, SG-OR, AG(9)-OR, AG(7)-OR, (MSG1)-Asn, (MSG2)-Asn, a mixture of (MSG1)-Asn and (MSG2)-Asn, MSG1-OR, MSG2-OR, or a mixture of MSG1-OR and MSG2-OR, having an optionally chemically-modified non-reducing end, wherein R is any substituent linked to the oxygen atom via at least one carbon atom.

13. The method according to claim 12, wherein R represents a C1-C6 alkyl group or an optionally substituted aryl group.

14. The method according to claim 13, wherein R is selected from the group consisting of a methyl group, an ethyl group, a n-propyl group, an i-propyl group, a n-butyl group, an i-butyl group, a s-butyl group, a t-butyl, a n-pentyl group, and a n-hexyl group, or is selected from the group consisting of a phenyl group, a benzyl group, an indenyl group, a naphthyl group, a fluorenyl group, an anthranyl group, and a phenanthrenyl group, optionally substituted with a C1-C6 alkoxy group, a C1-C6 alkyl group, or a halogen group.

15. The method according to any one of claims 12 to 14, wherein R is selected from the group consisting of PMP, Me, A-Mor, iPr, nPr, PrOMe, and A-PEG-N₃ wherein A represents an acetyl group in a glycolic acid unit, that is, a moiety present between an anomeric hydroxyl group and an amino group in Mor or PEG.

16. The method according to claim 11 or 12, wherein the glycan donor molecule is ([N₃-PEG(3)]₂-SG)-P-PEG(3)-N₃, ([N₃-PEG(3)]₂-SG)-Asn-PEG(3)-N₃, ([N₃-PEG(3)]-MSG1)-Asn-PEG(3)-N₃, ([N₃-PEG(3)]-MSG2)-Asn-PEG(3)-N₃, or a mixture of ([N₃-PEG(3)]-MSG1)-Asn-PEG(3)-N₃ and ([N₃-PEG(3)]-MSG2)-Asn-PEG(3)-N₃.

17. The method according to any one of claims 12 to 15, wherein the glycan donor molecule is ([N₃-PEG(3)]₂-SG)-OR, ([N₃-PEG(3)]-MSG1)-OR, ([N₃-PEG(3)]-MSG2)-OR, or a mixture of ([N₃-PEG(3)]-MSG1)-OR and ([N₃-PEG(3)]-MSG2)-OR.

18. The method according to any one of claims 11 to 17, wherein the glycan donor molecule is a compound represented by a formula selected from the group consisting of the following formulas. 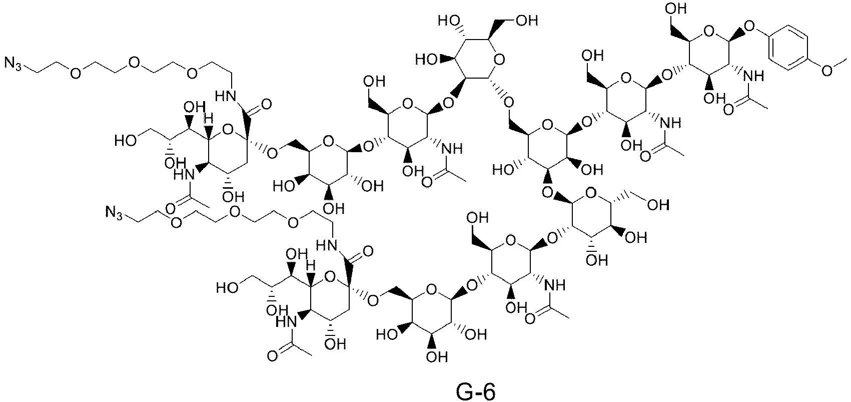 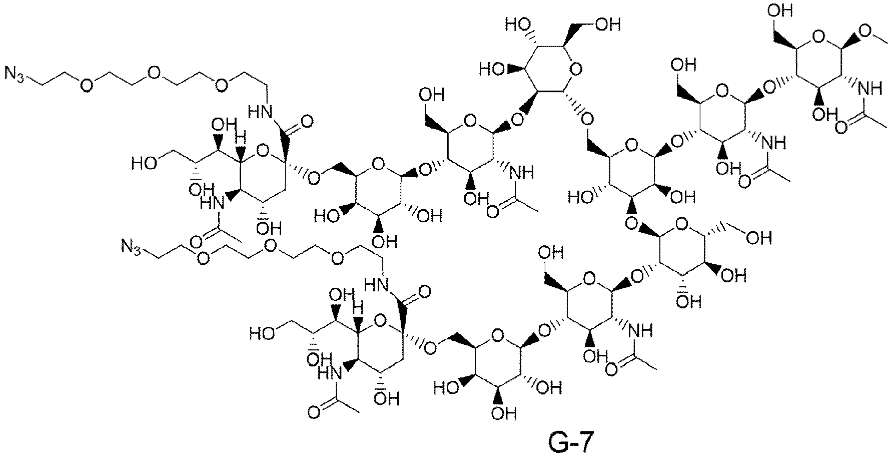 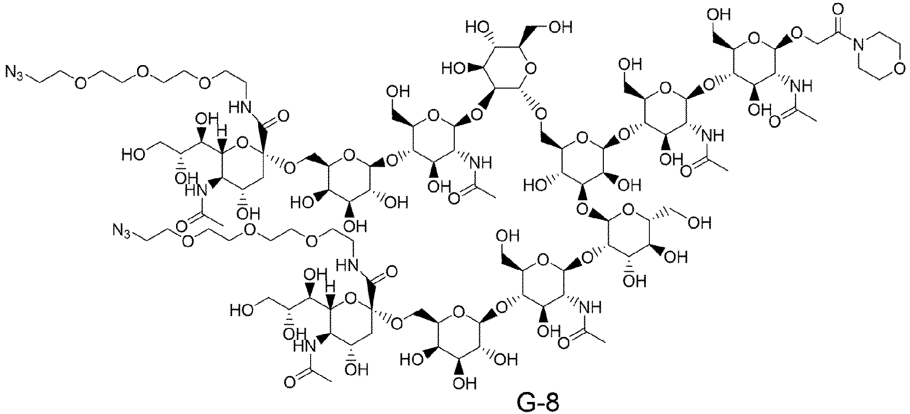 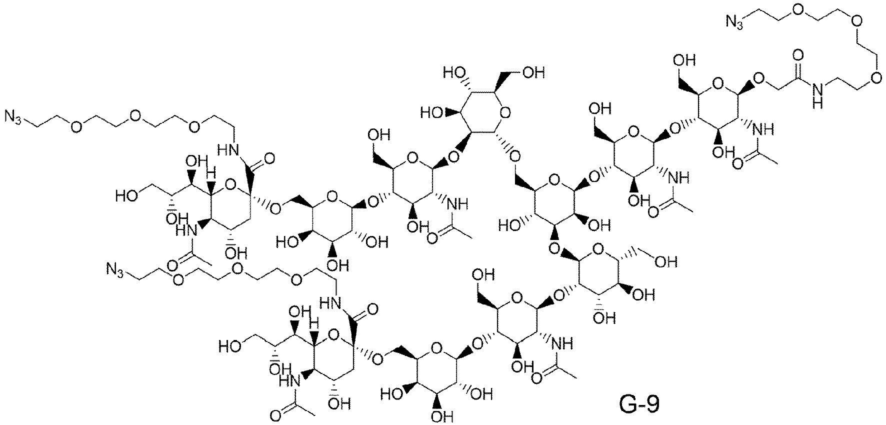 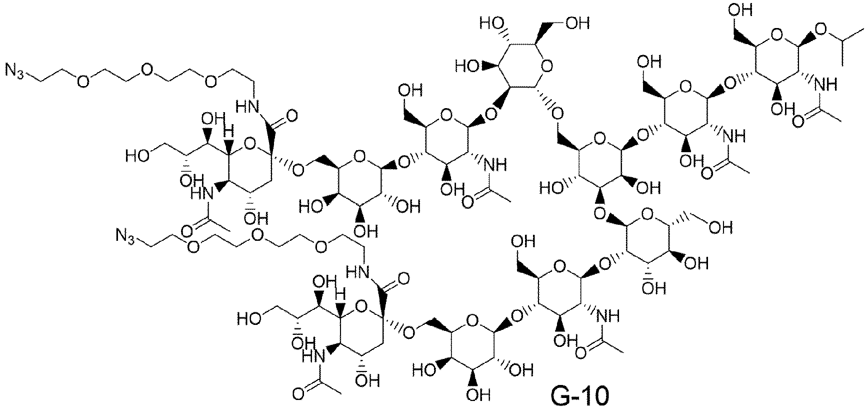 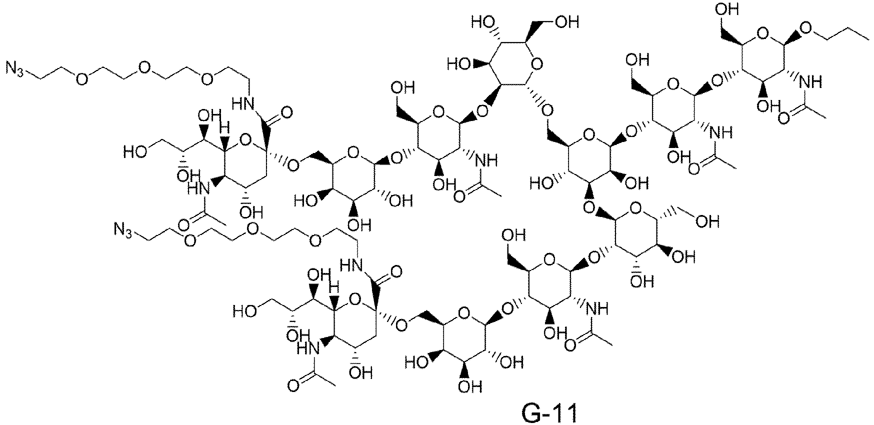 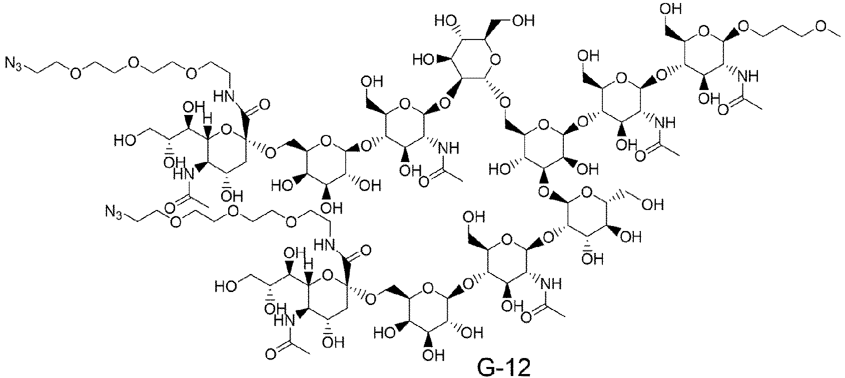 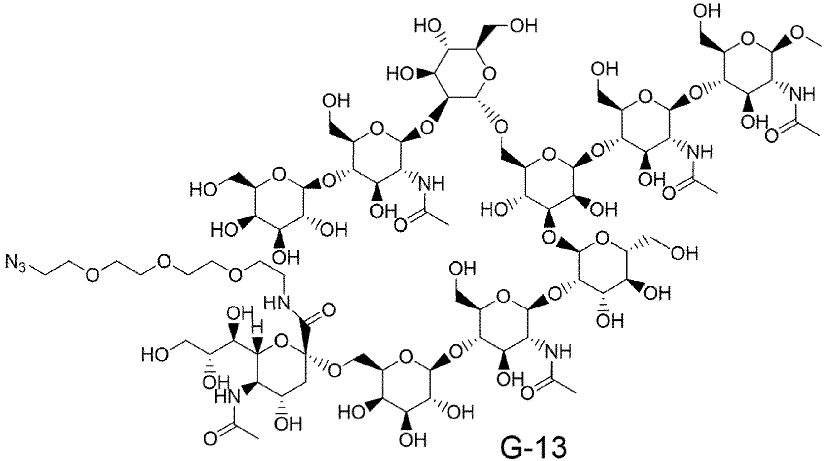 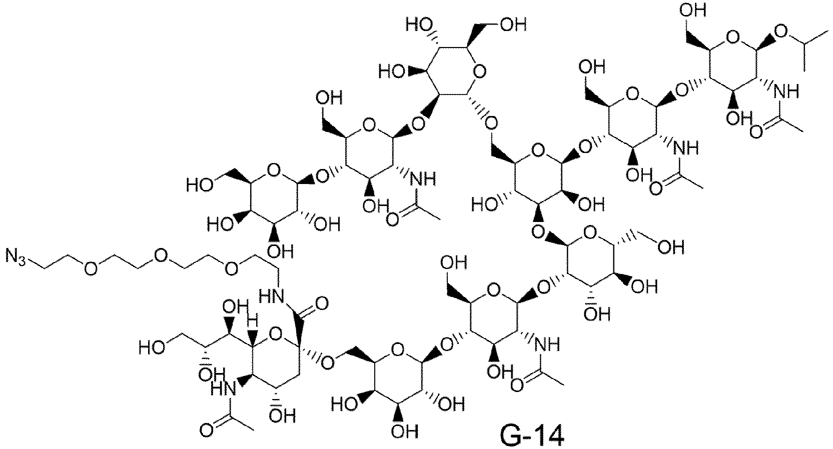 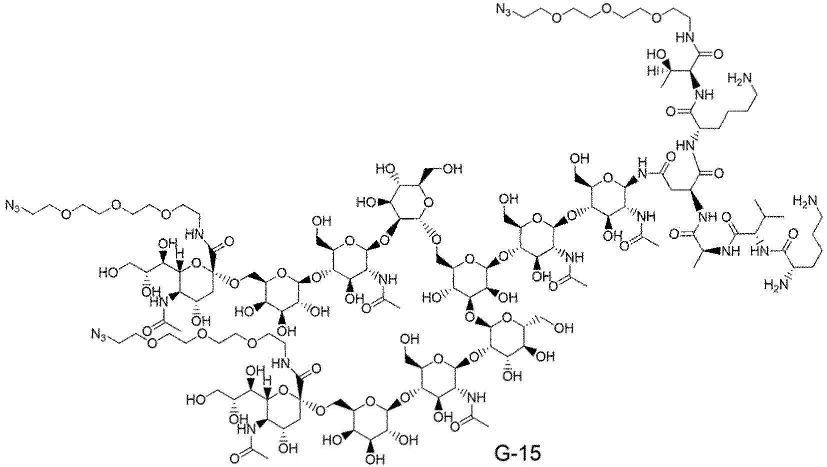 and

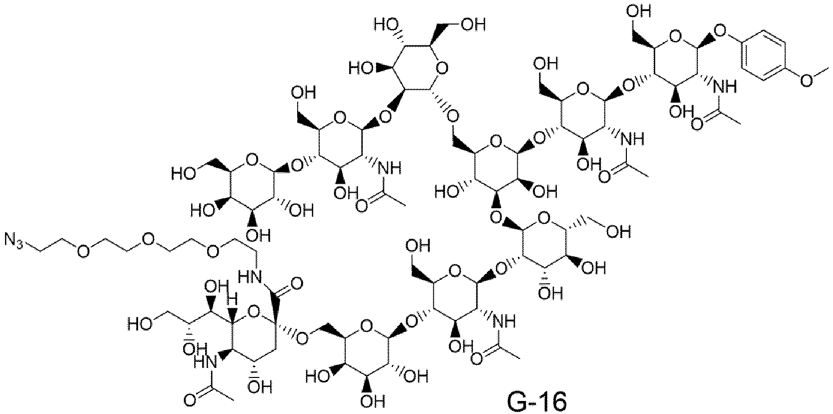

19. The method according to any one of claims 1 to 18, wherein the Fc-containing molecule is an immunoglobulin, CLCH, or an Fc-containing fragment.

20. The method according to any one of claims 1 to 19, wherein the endo-β-N acetylglucosaminidase (Enzyme-A), which prefers, as a substrate, an N297-linked glycan, is an enzyme that can act on the glycan of the Fc-containing molecule and optionally has residual hydrolysis activity.

21. The method according to any one of claims 1 to 20, wherein Enzyme-A is a mutant enzyme of Endo-S, Endo-S2, Endo-Si, Endo-Se, Endo-Sd, or Endo-Sz, and the mutant enzyme has lower hydrolysis activity than a corresponding wild type enzyme thereof.

22. The method according to claim 21, wherein Enzyme-A is Endo-S D233Q, Endo-S D233Q/Q303L, Endo-S D233Q/E350A, Endo-S D233Q/E350Q, Endo-S D233Q/E350D, Endo-S D233Q/E350N, Endo-S D233Q/D405A, Endo-Si D241Q, Endo-Si D241Q/Q311L, Endo-Si D241Q/E360Q, Endo-Si D241M, Endo-Si D241M/Q311L, Endo-Si D241M/E360Q, Endo-Si T190Q, Endo-Si T190/D241Q, Endo-Si T190Q/D241M, Endo-S2 D184M, Endo-S2 T138Q, Endo-S2 D184Q/Q250L, Endo-S2 D184M/Q250L, Endo-Se D233M, Endo-Sz D234M, Endo-Sz D234M/Q304L, Endo-Si D241X₁ wherein X₁ represents any amino acid residue other than K, R, and D, Endo-Si T190X₂ wherein X₂ represents an amino acid residue of F, H, K, M, Q, R, W, or Y, Endo-Si Q311X₃ wherein X₃ represents an amino acid residue of F, N, or Y, or Endo-Si E360K.

23. The method according to any one of claims 1 to 22, wherein pH in a reaction system in Step 1 is in the range of 6.5 to 8.0.

24. The method according to any one of claims 1 to 23, wherein a final acceptor molecule concentration in a reaction system in Step 1 is 20 mg/mL or more and 100 mg/mL or less.

25. The method according to any one of claims 1 to 24, wherein the Fc-containing molecule having an N297-linked glycan comprising the glycan derived from the glycan donor molecule is collected in a flow-through fraction from the cation exchange chromatography medium or the multimode chromatography medium.

26. The method according to any one of claims 1 to 25, wherein the acidic condition in Step 2 means a pH range of 3.5 to 4.5.

27. The method according to any one of claims 1 to 26, wherein the chromatography medium is particulate, membranous, or monolithic.

28. The method according to any one of claims 1 to 27, wherein the chromatography medium is a cation exchange chromatography medium.

29. The method according to any one of claims 1 to 28, wherein the glycan has a non-reducing end chemically-modified with a substituent having an azido group (N₃-), and the method further comprises reacting a molecule having an alkyne structure with the azido group (N₃-) of the glycan.

30. The method according to claim 29, wherein the molecule having an alkyne structure is selected from a chemotherapeutic agent, a molecular target drug, an immunostimulant, a toxin, a photosensitizer, an antimicrobial agent, an antiviral agent, a diagnostic agent, a protein, a peptide, an amino acid, a nucleic acid molecule, a nucleic acid, an antigen, a vitamin, and a hormone.

31. The method according to claim 30, wherein the chemotherapeutic agent is selected from camptothecin, pyrrolobenzodiazepine, doxorubicin, auristatin, taxane, and derivatives thereof.

32. The method according to claim 30, wherein the immunostimulant is selected from a STING agonist, a TLR agonist, and an A2AR antagonist.

33. The method according to any one of claims 29 to 32, wherein the molecule having an alkyne structure is selected from the group consisting of (A) to (E):
(A) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide,
(B) N-[4-(11,12-didehydrodibenzo[b,f]azocin-5(6H)-yl)-4-oxobutanoyl]glycylglycyl-L-valyl-N-[4-({[(11'S,11'aS)-11'-hydroxy-7'-methoxy-8'-(3-{[(11aS)-7-methoxy-2-(4-methoxyphenyl)-5-oxo-5,10,11,11a-tetrahydro-1H-pyrrolo[2,1-c][1,4]benzodiazepin-8-yl]oxy}propoxy)-5'-oxo-11',11'a-dihydro-1'H,3'H-spiro[cyclopropane-1,2'-pyrrolo[2,1-c][1,4]benzodiazepine]-10'(5'H)-carbonyl]oxy}methyl)phenyl]-L-alaninamide,
(C) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',11a'-dihydro-1'H-spiro[cyclopropane-1,2'-pyrrolo[2,1-c][1,4]benzodiazepin]-8'-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide,
(D) N-[4-(11,12-didehydrodibenzo[b,f]azocin-5(6H)-yl)-4-oxobutanoyl]glycylglycyl-L-valyl-N-{4-[({[(11'S,11a'S)-11'-hydroxy-7'-methoxy-8'-[(5-{[(11a'S)-7'-methoxy-5'-oxo-5',10',11',11a'-tetrahydro-1'H-spiro[cyclopropane-1,2'-pyrrolo[2,1-c][1,4]benzodiazepin]-8'-yl]oxy}pentyl)oxy]-5'-oxo-11',11a'-dihydro-1'H-spiro[cyclopropane-1,2'-pyrrolo[2,1-c][1,4]benzodiazepin]-10'(5'H)-yl]carbonyl}oxy)methyl]phenyl}-L-alaninamide, and
(E) (bis(N,N-diethylethanaminium)N-[4-(11,12-didehydrodibenzo[b,f]azocin-5(6H)-yl)-4-oxobutanoyl]glycylglycyl-L-phenylalanyl-N-[(2-{9-[(5R,7R,8R,12aR,14R,15R,15aR,16R)-15-fluoro-16-hydroxy-2,10-dioxo-2,10-disulfide-14-(6,7,8,9-tetrahydro-2H-2,3,5,6-tetraazabenzo[cd]azulen-2-yl)octahydro-2H,10H,12H-5,8-methano-2λ⁵,10λ⁵-furo[3,2-1][1,3,6,9,11,2,10]pentaoxadiphosphacyclotetradecyn-7-yl]-6-oxo-6,9-dihydro-1H-purin-1-yl} ethoxy)methyl]glycinamide.

34. An Fc-containing molecule produced by the method according to any one of claims 1 to 33.

35. A method for isolating an Fc-containing molecule having an N297-linked glycan, comprising contacting a solution comprising the Fc-containing molecule and one or more impurities with a cation exchange chromatography medium or a multimode chromatography medium under an acidic condition to selectively trap the impurities in the cation exchange chromatography medium or the multimode chromatography medium, or to selectively trap the Fc-containing molecule in the cation exchange chromatography medium or the multimode chromatography medium.

36. The method according to claim 35, wherein the N297-linked glycan comprises a complex type glycan.

37. The method according to claim 35 or 36, wherein the N297-linked glycan is not a core GlcNAc to which fucose is optionally added, and the one or more impurities comprise a molecule that is the same as the Fc-containing molecule except for having, as an N297-linked glycan, a core GlcNAc to which fucose is optionally added.

38. The method according to any one of claims 35 to 37, wherein the chromatography medium is a cation exchange chromatography medium.

39. A method for isolating an Fc-containing molecule having an N297-linked glycan comprising a glycan derived from a glycan donor molecule, comprising the following Steps 1 and 2:
[Step 1] reacting an acceptor molecule, which is an Fc-containing molecule having, as an N297-linked glycan, a core GlcNAc to which fucose is optionally added, with a glycan donor molecule comprising a glycan in the presence of an endo-β-N-acetylglucosaminidase (Enzyme-A), which prefers, as a substrate, an N297-linked glycan of an Fc-containing molecule, so as to obtain a reaction mixture; and
[Step 2] contacting the reaction mixture with a cation exchange chromatography medium or a multimode chromatography medium under an acidic condition and isolating the Fc-containing molecule having an N297-linked glycan comprising the glycan derived from the glycan donor molecule.

40. The method according to claim 39, wherein the chromatography medium is a cation exchange chromatography medium.

41. The method or the Fc-containing molecule according to any one of claims 1 to 40, wherein the Fc-containing molecule having an N297-linked glycan is derived from an antibody selected from the group consisting of an anti-HER2 antibody, an anti-HER3 antibody, an anti-DLL3 antibody, an anti-FAP antibody, an anti-CDH11 antibody, an anti-CDH6 antibody, an anti-A33 antibody, an anti-CanAg antibody, an anti-CD19 antibody, an anti-CD20 antibody, an anti-CD22 antibody, an anti-CD30 antibody, an anti-CD33 antibody, an anti-CD56 antibody, an anti-CD70 antibody, an anti-CD98 antibody, an anti-TROP2 antibody, an anti-CEA antibody, an anti-Cripto antibody, an anti-EphA2 antibody, an anti-G250 antibody, an anti-MUC1 antibody, an anti-GPNMB antibody, an anti-Integrin antibody, an anti-PSMA antibody, an anti-Tenascin-C antibody, an anti-SLC44A4 antibody, an anti-Mesothelin antibody, an anti-ENPP3 antibody, an anti-CD47 antibody, an anti-EGFR antibody, an anti-GPR20 antibody, and an anti-DR5 antibody.

42. A compound represented by a formula selected from the group consisting of the following formulas. 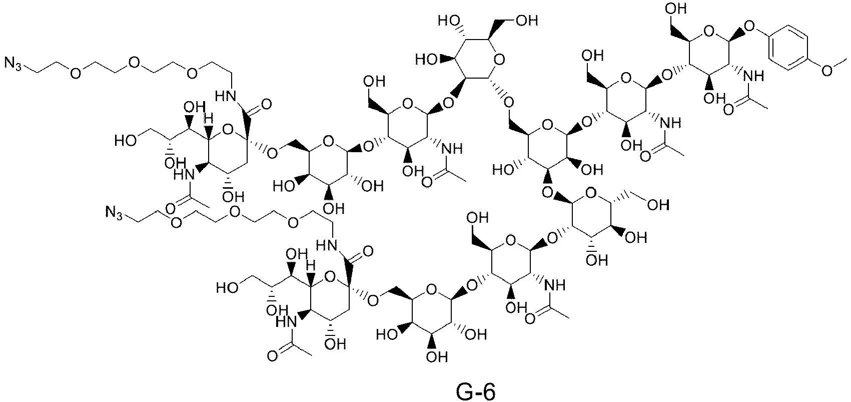 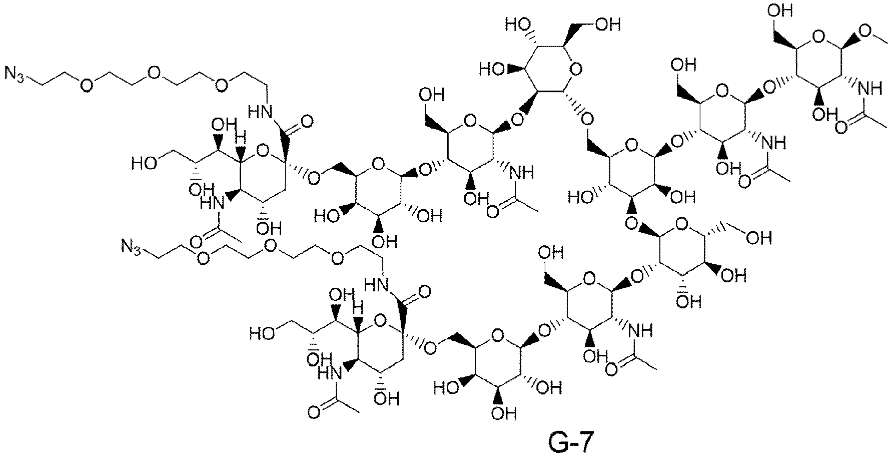 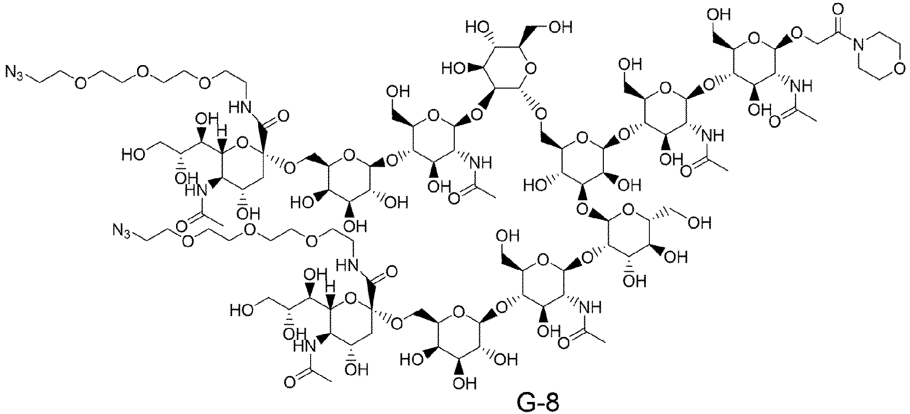 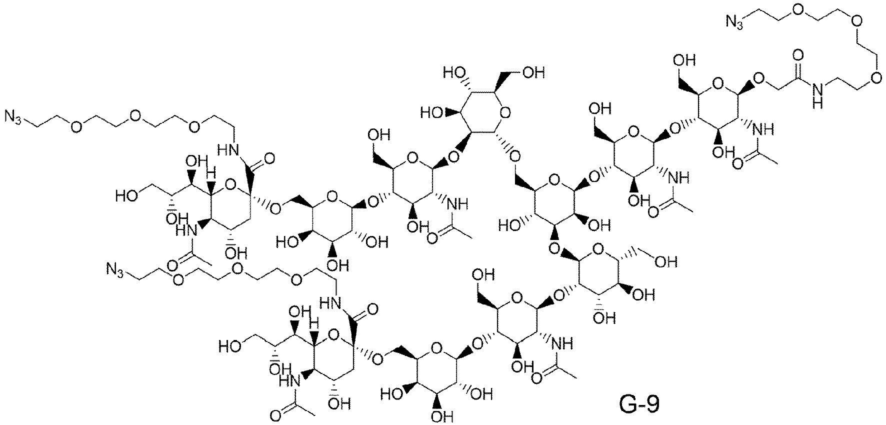 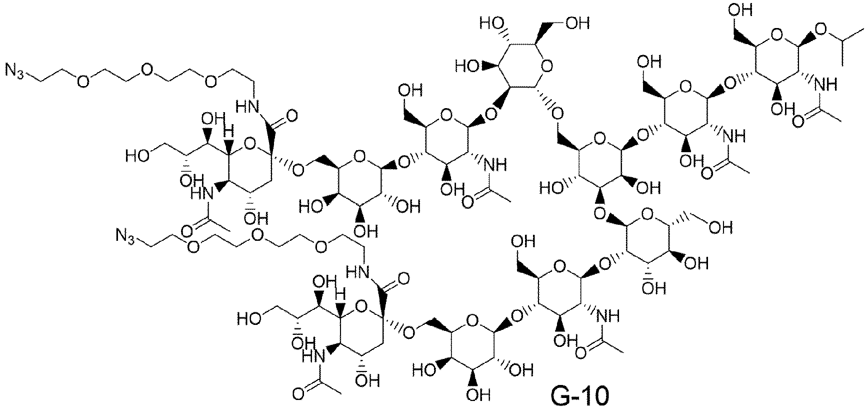 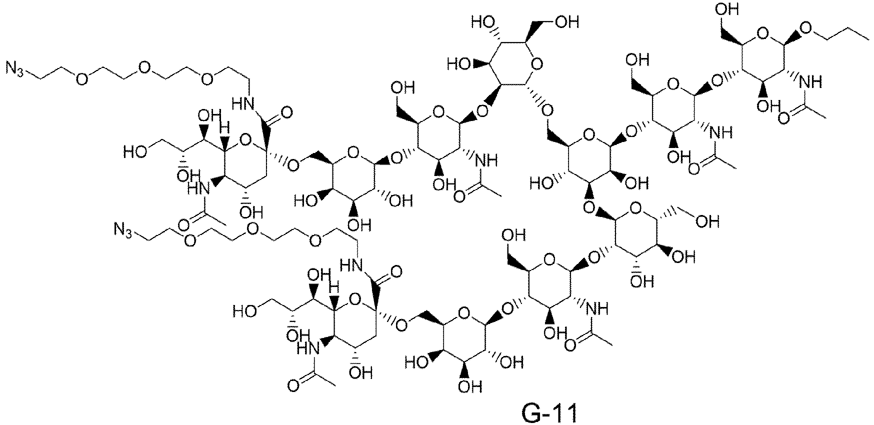 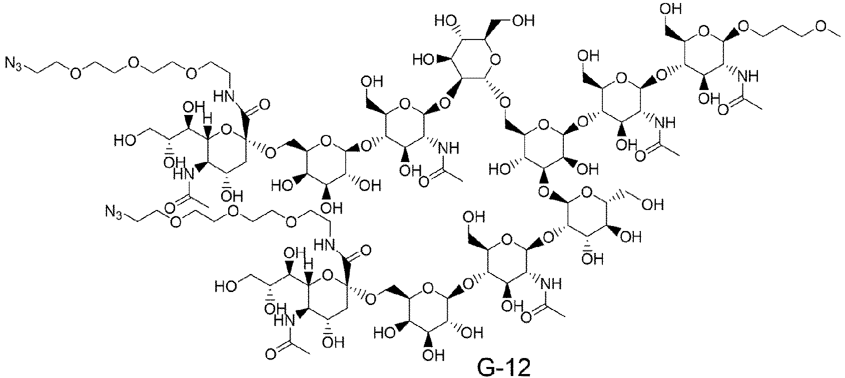 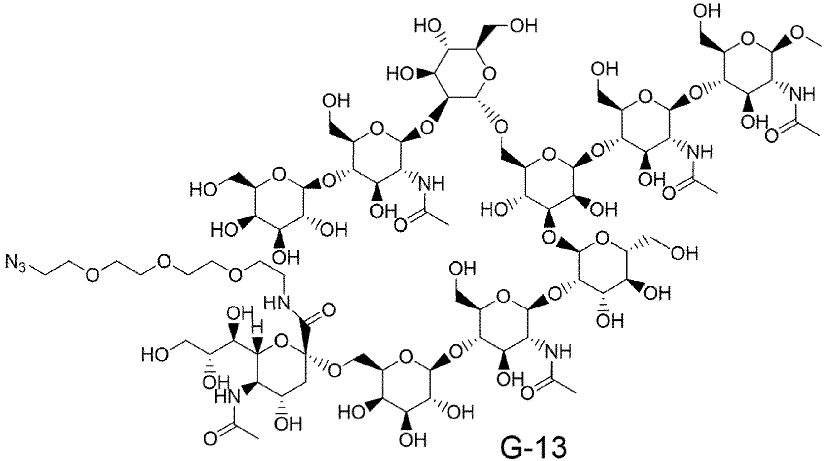 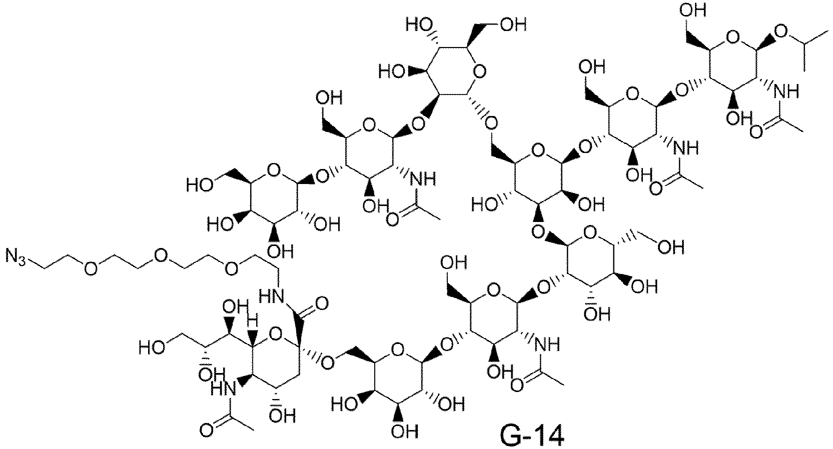 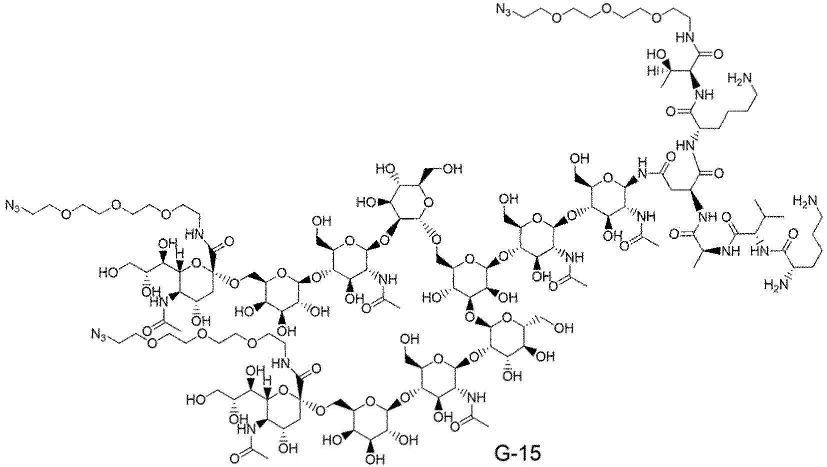 and

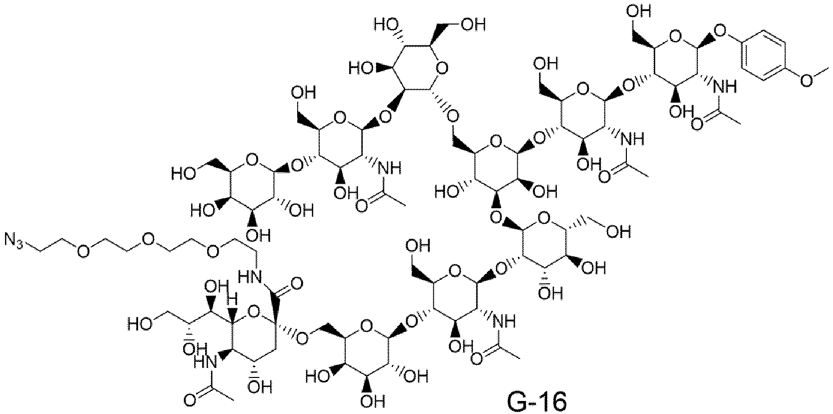

43. A fusion protein of Enzyme-A and Enzyme-B, wherein
Enzyme-A is an endo-β-N-acetylglucosaminidase, which prefers, as a substrate, an N297-linked glycan of an Fc-containing molecule, and
Enzyme-B is an endo-β-N-acetylglucosaminidase, which prefers, as a substrate, a glycan of a glycan-containing molecule having a GlcNAc having an unactivated glycan reducing end, but does not prefer, as a substrate, the N297-linked glycan, or has low reactivity with the N297-linked glycan.
